# EUROPEAN PATENT APPLICATION

(11) **EP 1 270 741 A1**
(43) Date of publication of application: **02.01.2003**
(21) Application number: 01202420.4
(22) Date of filing: 22.06.2001
(51) Int. Cl.: C12Q 1/68

(54) **Nucleotide sequences involved in plant disease resistance**

(71) Applicant: Keygene N.V., 6700 AE Wageningen (NL)
(72) Inventor: Turk, Stefan Cornelis Hendrikus Jozef, 2266 MA Leidschendam (NL); Takken, Franciscus Lambertus Wilhelmus, 1098 KW Amsterdam (NL); de Jong, Camiel Frido, 6701 DB Wageningen (NL); Joosten, Matthieu Henri Antoon Jozef, 6707 AN Wageningen (NL); de Wit, Peter Jozef Gerard Marie, 3911 XM Rhenen (NL)
(74) Representative: Jorritsma, Ruurd

(57) **Abstract**

Method for the identification and isolation of expressed nucleic acid sequences that are associated with disease resistance of a plant against a plant pathogen, isolated nucleic acid molecules and variants and/or homologues thereof, constructs, plants and polypeptides encoded by said nucleotide sequences and the use of these sequences in the development of resistance in plants against pathogens.

## Description

### Field of the invention

The present invention relates to the isolation and characterisation of nucleotide sequences that can be used to confer desired properties to a plant or plant cell. In particular the invention relates to nucleotide sequences involved in resistance of a plant against a pathogen.

### Background of the invention

Plants are under continuous attack of a wide variety of taxonomically very different pathogens such as bacteria, viruses, fungi, nematodes, aphids, insects and other pests. Most of these encounters between a pathogen and a plant stop at passive defence lines such as wax layers, cell walls and chemical barriers. However, when this primary barrier is overcome, the interaction between the plant and the pathogen can result in an invasion by the pathogen. This invasion may result in disease symptoms that can often lead to damage to the plant or even to death of the infected plant. The pathogen invasion may also lead to the infection of only a limited area of the plant or even only a few cells. To limit pathogen infections plants have developed a second line of defence. To this second line of defence a wide variety of highly complex and not yet elucidated mechanisms lay afoot. The basic principle resides in the mounting of the defence line by proteins derived from the transcription of specific resistance genes (R-genes). Takken *et al.* in the European Journal of Plant Pathology 106:699-713 (2000) recognises four different fundamental mechanisms: i). the product encoded by the R-gene inactivates a toxin which is produced by the pathogen and which toxin normally induces necrosis or inhibits the induction of other active defence responses; ii). the R-gene products encodes a pathogenicity target such that in absence of this target, plant resistance occurs; iii). the R-gene product primes the plant defence responses; and iv). the R-gene product mediates specific recognition of a specific pathogen that expresses a matching avirulence (Avr) gene (i.e. gene-for-gene resistance). Specific recognition of a pathogen derived Avr gene product, a so-called elicitor, by the host activates a signal transduction cascade that involves protein phosphorylation, ion fluxes, generation of reactive oxygen species and other signals. These signals subsequently trigger transcription of plant defence genes encoding proteins such as glutathione S-transferases, peroxidases, cell wall proteins, proteinase inhibitors, hydrolytic enzymes, pathogenesis related (PR) proteins and enzymes involved in secondary metabolism. In addition thereto, plant cells that are in direct contact with the invading pathogen die. This phenomenon is referred to as the hypersensitive response (HR). The HR is regarded as the hallmark of the gene-for-gene resistance principle. HR is an active process that requires protein syntheses and is often correlated with the induction of resistance in non-inoculated parts of the plant. This systemic acquired resistance (SAR) results in a significant reduction of disease symptoms caused by many pathogens.

The effect of a pathogen on a plant and on the economics of crop production can be very serious. Especially in the case of agronomically important crops such as tomato, potato, rice and corn this effect can be enormous if not disastrous. Accordingly there is a need for plants that are less vulnerable to pathogens and/or to plants that are more capable of defending themselves against invading pathogens.

In order to create such plants, whether transgenic or not, a further understanding of the underlying biochemistry and genetics of pathogen resistance is needed, and a further identification of components of the defence system of the plant is needed. To this end the pathway leading to a defence response in a plant has been studied by using a model system. In the course of these studies a large number of components that are involved in the induction of a defence response to a pathogen have been identified.

It is one of the goals of the present invention to provide for durable and/or broad resistance in crops and/or plants. It is a further goal of the present invention to identify nucleotide sequences and/or polypeptides that are related to, or capable of controlling, inducing, contributing or otherwise exercise a negative or positive influence or stimulus to, the signal transduction pathway. It is also a goal of the present invention to provide for methods, compounds or compositions that find use and/or application in the activation or regulation (up or down) of the defense mechanism of plants and in particular of the defense mechanism of plants against pathogens, more in particular the HR. It is further a goal of the present invention to identify and/or provide for downstream signaling components that are related to or are involved in the regulation of resistance, preferably race-specific resistance. It is a further goal of the present invention to identify and/or provide for promoters that drive pathogen inducible expression. Other goals of the present invention will become apparent from the description of the invention and its embodiments.

### Description of the invention

The present invention relates to the isolation and characterisation of nucleotide sequences that are involved in the defence mechanism of a plant against a plant pathogen. More in particular, the invention relates to the isolation and characterisation of nucleotide sequences that can be used to confer upon a plant one or more desired and/or favourable properties relating to the defence mechanism of a plant against a plant pathogen.

### A. Methods for the identification and isolation of differentially expressed nucleic acid fragments

In a first major aspect the invention relates to a method for the identification and isolation of expressed nucleic acid fragments that are associated with pathogen resistance. For this purpose, expression profiles of at least two plants that differ in at least one property relating to disease resistance. The plants that differ are preferably of the same genus. More preferably the plants that differ are (genotypically) identical but placed in different conditions or the plants are isogenic except for a genetic determinant involved in, or associated with disease resistance. In particular, the method may be used to identify expressed nucleic acid fragments that are part of transcripts of genes that are associated with disease resistance in plants against pathogens. The corresponding genes may subsequently be isolated and used to confer advantageous properties relating to disease resistance to a plant. The expressed fragments may e.g. be provided in the form of a library consisting of nucleic acid sequences or fragments, and may in particular be in the form of one or more (amplified) restriction fragments, e.g. obtained through cDNA AFLP® as described in more detail hereinbelow.

Preferably, the method for identification and isolation of expressed nucleic acid fragments that are associated with disease resistance in a plant against a pathogen comprises at least the steps of:
(a) providing a first plant material;
(b) providing at least a second plant material, which differs from the first plant material in a property relating to disease resistance;
(c) optionally providing an additional plant material, which also differs from the first plant material in a property relating to disease resistance;
(d) comparing a transcript profile of the first plant material with a transcript profile of the second plant material, and optionally with a transcript profile of the additional plant material;
(e) identifying in the transcript profiles a nucleic acid fragment that corresponds to transcript that is differentially expressed between the first plant material and the second plant material, and optionally between the first plant material and the additional plant material.

In the method of the invention, the property relating to disease resistance preferably is a property associated with the HR in a plant, such as e.g. the presence or absence of the HR, or time after induction of the HR.

The plant materials to be used in the method of the invention, i.e. the first, second and additional plant materials, are preferably obtained from the same genus, same species, same variety, same cultivar or from the same plant. Yet the first plant material differs from the second and additional plant materials in at least one property relating to disease resistance. Such a difference may be the result of a genotypic difference: e.g. the second and additional plant materials may be obtained from plants that are mutants of the plant from which the first material is obtained. In such instances, the plants are preferably isogenic except for the genotypic difference. Alternatively, the plant materials are obtained from isogenic plants whereby the difference is the result of different tissues, different developmental stages or different conditions to which the plants have been subjected.

Preferably, the first plant material and the second plant material will be selected such that the desired property or properties of disease resistance is improved (e.g. increased) in the second plant material compared to the first plant material (although the invention in its broadest sense is not limited thereto). For instance, the first plant material may be a plant (material) with a normal or wild-type level of a property relating to disease resistance- i.e. used as a reference - in which case the second plant material may be a plant (material) with abnormal level of the property, i.e. improved, increased, or decreased. Optionally, the transcription profile of the first plant material - and optionally also of the second plant material - may be further compared with the transcription profile(s) of one or more additional plant materials, e.g. a third, a fourth and further plant materials. Preferably, any such additional plant materials are selected such that they differ from the first plant material in a property relating to disease resistance. They are preferably further selected such that they also differ from the second plant material in a property relating to disease resistance (e.g. the same as above), and/or differ from each other in said property. For example, a series or collection of plant materials may be ranked according to the level or degree of a property relating to disease resistance, upon which the plant material with the lowest level or degree of the property relating to disease resistance is used as the first plant material, and the remaining plant materials are used as the second plant material and additional plant materials (e.g. ranked according to the increase in the property or properties). Alternatively, from a collection of plant materials, the plant material with the highest level or degree of a property relating to disease resistance may be compared with the plant material with the lowest level or degree of the property or properties, in which the plant material with the highest level of the property or properties will generally be used as the second plant material and the plant material with the lowest level of the property or properties will generally be used as the first plant material. Thereafter, the first plant material and/or second plant material may optionally be further compared with one or more of the additional plant materials from the collection. With respect the above, it will be clear to the skilled person that, instead of the first plant material, also a known reference plant or plant material and/or data for such a known reference plant or plant material may be used.

Preferably, in the method of the invention, the first plant material is obtained from a plant that does not produce the HR and the second and optional additional plant material are obtained from one or more plants in which the HR has been induced. Preferably, the induction of the HR is suppressible, such that the second and optional additional plants may be grown under conditions suppressing the HR to a desired stage after which the suppressing conditions are removed to allow for induction of the HR. The suppression of the HR is preferably conveniently controlled by temperature. In a preferred embodiment of the invention, the second and additional plant materials have been obtained at different time intervals after induction of the HR. E.g., the second and additional plant materials may have been obtained at 0, 30, 60 and 90 minutes, from the onset of the HR. In a particular embodiment, the first plant material may be the plant material obtained at the onset of the HR, i.e. t = 0, to be compared with the transcript profiles obtained from the materials obtained at the later timepoints.

In the method of the invention, the first plant material is preferably obtained from a plant that does not comprise a plant-pathogen derived avirulence (trans)gene. Avirulence genes are part of the gene-for-gene resistance mechanism through which plants, harbouring a specific resistance (*R*) gene, recognise pathogens carrying matching avirulence genes.

In the method of the invention, the second and optional additional plant material are preferably obtained from transgenic plants comprising a matching pair of (a) a plant pathogen derived avirulence gene, and (b) a plant resistance gene, whereby the matching pair of the resistance and avirulence genes is capable of inducing the HR. A range of matching pairs of resistance and avirulence genes are known in the art for a variety of plant species and their pathogens as reviewed by, and listed in Table 1, of Takken et al. (*supra*), which is incorporated by reference herein. Preferably, the plant pathogen providing the avirulence gene is a fungus, more preferably the fungus is *Cladosporium fulvum.* In a most preferred embodiment, the matching pair of the plant pathogen derived avirulence gene and the matching plant resistance gene is selected from the group consisting of the pairs *Avr2* and *Cf-2*; *Avr4* and *Cf-4*; *Hcr9-4E* and *Cf-4E*; *Avr5* and *Cf-5*; and, *Avr9* and *Cf-9.*

Generally, the plant materials used for generating the transcription profile(s) may be selected from one or more entire plants and/or from one or more parts, tissues and/or organs from one or more plants, including but not limited to the roots, stems, stalks, leaves, petals, fruits, seeds, tubers, meristems, sepals, and flowers as long as the plants or plant materials selected at least differ in a property relating disease resistance. Also, the plant materials selected may be representative for different stages of development of a plant, for different stages of a disease that may affect a plant, for plants that are exposed to different levels of stress (e.g. due to lack of water, light, nutrients, damage, etc.); again as long as the plant materials selected at least differ in a property relating to disease resistance. Usually, when the expression profiles of two or more such different plants are compared, the expression profiles of essentially same part(s), tissue(s) or organ(s) of such different plants will be compared.

When the expression profiles of two or more such different plants are compared, these plants will usually belong at least to the same genus, and preferably to the same species. E.g. plants from different varieties or lines showing differences in a property relating to disease resistance may be compared such as e.g. varieties or mutants with different properties relating to resistance, or any of these mutations in heterozygotes, homozygotes or hybrids.

Also, the plant materials may be two or more different parts, tissues and/or organs of a plant, which parts, tissues or organs differ in a property relating to disease resistance. Preferably, when the expression profiles of two or more such different parts, tissues or organs are compared, these parts, tissues or organs will be derived from plants belonging to the same species, more preferably from plants belonging to the same line or variety, and most preferably from the same plant or isogenic plants.

In the method of the invention preferably "comparable" expression profiles will be generated from each of the respective plant materials, by which is generally meant that these expression profiles should be such that they allow for the detection and identification of one or more differentially expressed fragments as outlined above. Generally, this means that the expression profiles will be obtained using the same technique and usually also using the same conditions. For instance, when cDNA AFLP® is used to generate such "comparable" expression profiles, such cDNA AFLP® will usually be carried out using the same restriction enzymes, adapters, primers, detection technique(s) and further conditions for essentially all plant materials used. However, the skilled person will understand that also several different comparable expression profiles may be generated from of the respective plant materials - e.g. using different restriction enzymes, different primer combinations (e.g. with different selective nucleotides), etc.- to provide several sets of expressed fragments.

For obtaining the transcript profiles that are compared in step (d), any technique known per se for transcript profiling may be used, and in particular any technique known per se that allows for the detection of one or more differences in expression between the respective plant materials. More in particular, a technique will be used that allows for the detection of differences in the transcripts or in the transcript levels as they are present in the respective plant materials, i.e. differences in mRNAs, mRNA levels or cDNAs corresponding to such mRNAs. Accordingly, the technique used for generating the transcript profiles will usually be such that the one or more expressed fragments are detected and subsequently identified that can be considered representative for one or more transcripts that are differentially expressed in the respective plant materials; preferably the levels of the expressed fragments as detected in the technique is such that they are representative for the levels the corresponding transcripts in the respective plant materials. Usually, these expressed fragments will be detected as, or will be identified by means of differences in the detection signals of the individual expressed fragments, e.g. bands, between the expression profiles generated from the respective plant materials. Of particular interest will be those markers the signal of which changes when the property or properties of disease resistance that are of interest (e.g. those mentioned above) changes (e.g. increases), for instance from the first plant material to the second plant material and/or from the first plant material to the additional plant materials, as further described below. Thus, for example, the expressed fragment's detectable signal - e.g. a band on a gel or autoradiograph, or a peak in a chromatogram - as present in the transcript profile(s) generated for the second plant material - and/or for any of the additional plant materials - may be absent or present at reduced levels in transcript profile(s) generated for the first plant material. The signals of the expressed fragments may also be increased - e.g. more intense - in the expression profile(s) generated for the second plant material - and/or for any of the additional plant materials - compared to the expression profile(s) generated for the first plant material. However, generally speaking the invention generally comprises the selection of any expressed fragment that is representative for any difference(s) in expression between the first plant material and the second plant material (and/or any of the additional plant materials). Thus, e.g. the invention also comprises (the selection of) fragments the signal of which changes (e.g. increases) when the property or properties of disease resistance that are of interest (e.g. those mentioned above) changes (e.g. increases), e.g. from the first plant material to the second plant material and/or from the first plant material to the additional plant materials. The invention also for instance comprises markers that are only associated with disease resistance during a certain - e.g. specific and/or limited - period of time, e.g. during development of the plant, during the development of the pathogen after invasion of the plant by the pathogen (e.g. during the development or activation of the defence response system of the plant or after the plant has been harvested.

The technique used for obtaining the transcription profiles is preferably such that the expressed fragments that are representative for the differentially expressed transcripts are provided in the form of nucleic acid fragment, such as amplified restriction fragments, that may be subjected to sequence analysis so as to provide the corresponding nucleic acid sequence. Such transcript-derived nucleotide sequences will be referred to herein as "expressed nucleic acid fragments".

When the one or more fragments are generated in the form of such expressed fragments, the method of the invention may also comprise the optional further step of: (f) isolating, purifying and/or sequencing the expressed fragment identified in step (e).

In particular, such expressed fragments may be (provided as) nucleotide sequences that are part of the differentially expressed transcripts, including e.g. restriction and/or PCR fragments derived from such transcripts.

Preferably, the expressed fragments are obtained by means of cDNA AFLP®, as further described below. In such a case, the expressed fragments will be provided as amplified restriction fragments - e.g. cDNA AFLP® fragments - that are identified as in bands with different intensities in the DNA fingerprints generated from the respective plant materials using cDNA AFLP®. Even more preferred, the transcript profiles may be generated by means of cDNA AFLP® carried out essentially as described hereinbelow, e.g. using the restriction enzymes, adapters, primers and further conditions as described below. The cDNA AFLP® -technique to be used preferably for the generation of the transcript profiles from the respective plant materials is based on the AFLP® method described by Vos *et al.* (1995, Nucl. Acids Res., 23: 4407-4414), whereby the starting DNA is a mixture of cDNA's obtained in the conventional manner by converting the mRNA pool isolated from the plant material of interest into a mixture of cDNA's using a reverse transcriptase. Total RNA extracted from the plant material may be used as template for the reverse transcriptase reaction but preferably the RNA template consists of poly-A⁺ enriched RNA obtained e.g. by oligo-dT chromatography.

The principle steps of the AFLP® method are as follows: a starting DNA (which is a mixture of cDNA's in the case of cDNA AFLP®) is digested with restriction enzymes. Preferably a combination of (1) a rare-cutting enzyme, e.g. a "six-cutter" such as *Eco*RI, and (2) a frequent-cutting enzyme, e.g. a "four-cutter" such as *Mse*I, is used. Alternatively, a combination of two (different) four-cutters can be used. Oligonucleotide adapters are ligated onto the ends of the restriction fragments thus obtained. A pre-amplification PCR is then performed such that only the fragments with a rare-cutter-site (*Eco*RI) on one end and a frequent-cutter-site (*Mse*I) on the other end are amplified. In the case of two four-cutters, the fragments that have two different end are preferentially amplified. The product of the pre-amplification is then used as template in a second and selective amplification under stringent conditions. The primers used in the selective amplification are complementary to the oligonucleotide adapters and in addition comprise at their 3'-ends arbitrarily chosen selective nucleotides which allow to amplify only a subset of the fragments amplified in the pre-amplification. The selective primers will usually contain between 1 and 5 selective nucleotides at their 3'-ends. Under these conditions only those fragments the ends of which are perfectly complementary to the selective primers will be amplified. E.g. two selective primers with each 1 selective nucleotide at their 3'-ends will amplify 1 out of 16 fragments, whereas two selective primers with each 2 selective nucleotide at their 3'-ends will amplify 1 out of 256 fragments. The amplified fragments may subsequently be separated by e.g. gel-electrophoresis, preferably on polyacrylamide gels and visualised by methods known per se. Each fragment may be defined by its size, i.e. length and by the combination of selective primers that have allowed it to be amplified. As mentioned, in the case of cDNA AFLP®, the starting DNA is cDNA obtained by reverse transcription of a mRNA population to be analysed. After pre-amplification and selective amplification the obtained fragments are derived from expressed transcripts and therefore herein referred to as "expressed fragments". The intensity of the "expressed fragment" on the gel allows to evaluate the expression level of the corresponding transcript in the RNA population. Thus, cDNA AFLP®-technique can be used to provide amplified restriction fragments that have been derived from - or that, more generally, that correspond to at least part of - the transcripts that are present in the respective plant materials. Advantageously, in the cDNA AFLP®-technique, the intensity of a particular amplified restriction fragments as obtained is generally a measure for the level of the corresponding transcript in the plant material. Thus, for instance, when the transcript profiles of two or more plant materials are to be compared, expressed fragments useful in the invention may be conveniently selected on the basis of any differences in the intensities between corresponding band(s) in the respective cDNA AFLP® fingerprints generated for each of the respective plant materials. Also, the actual DNA fragments corresponding to the expressed fragments may conveniently by obtained e.g. by cutting the bands from the gel and subsequently isolating and purifying excised fragments. Thereupon, the fragments may optionally be cloned, re-amplified and/or sequenced.

The method described above may be used to provide a fragment, and usually a collection of at least two fragments, that are associated with/representative for differences in expression between the respective plant materials. In a preferred embodiment, the method of the invention may be used to provide an expressed fragment, or usually rather a collection of such expressed fragments, that is associated with a difference in a property relating disease resistance between the respective plant materials. Generally, such a collection of expressed fragments will contain at least 5 such fragments, e.g. between 6 and 1000 such fragments, preferably between 50 and 350 such fragments. In particular, the method of the invention may be used to provide a "bank" or "library" of such expressed fragments, e.g. a collection of two or more such fragments that comprises essentially all such fragments obtained from the plant materials used (or at least from the first plant material and the second plant material), e.g. covering essentially all the differences in the transcript profiles generated using the method of the invention for the respective plant materials. Generally, such a library or bank of expressed fragments will contain at least 5 such fragments, e.g. between 6 and 1000 such fragments, preferably between 50 and 350 such fragments. The expressed fragments obtained using the method of the invention, as well as any collection, bank or library of such fragments, form a further aspect of the invention.

The expressed fragments are preferably in the form of nucleotide sequences or nucleic acid fragments, in preferably in the form of amplified restriction fragments, and more preferably in the form of cDNA AFLP® fragments. These sequences and/or fragments, as well as collections, banks or libraries thereof (as defined above), form further aspects of the invention. Optionally, the nucleotide sequences/fragments may also be provided as one or more (isolated) nucleic acids, and these isolated nucleic acids, as well as collections, banks or libraries thereof (as defined above), form further aspects of the invention.

These expressed fragments, the manner in which they were obtained, and the manner in which the transcription profiles were generated using cDNA AFLP®, are further described in the Examples hereinbelow. The transcript profiles thus obtained were compared, which provided a collection of expressed fragments. Essentially, this collection constitutes a bank or library of all the expressed fragments that are representative for the differences in expression between the various plant materials as mentioned above, e.g. associated with the differences in a property relating to disease resistance between these plant materials as detected using cDNA AFLP® carried out as outlined above. In particular, expressed fragments in the collection correspond to genes the expression of which is regulated, directly or indirectly by the *R*- genes. This collection of expressed fragments, as well as the individual expressed fragments thereof, form further aspects of the invention.

### B. Uses of the differentially expressed fragments.

In a further aspect the invention relates to the various uses of the differentially expressed fragments of the invention.

A particularly preferred use of the expressed fragments of the invention concerns their use in the isolation of plant nucleotide sequences corresponding to the differentially expressed fragments. Useful plant nucleotide sequences to be isolated by means of the expressed fragments of the invention include e.g. full-length or partial genes corresponding to the expressed fragments, full-length cDNAs corresponding to those genes, regulatory sequences associated with those genes, flanking sequences upstream or downstream from those genes or even neighbouring genes. Most preferred nucleotide sequences are, however, genomic or cDNA sequences comprising a full-length coding sequence corresponding to the expressed fragment. The nucleotide sequences to be isolated by means of the expressed fragments of the invention are used to alter disease resistance in a plant or to confer upon a plant one or more favourable properties relating to disease resistance as described herein below.

There are a number of means available and known per se to the skilled person for isolation of the nucleotide sequences corresponding to the expressed fragments of the invention. The expressed fragments themselves may e.g. be used as hybridisation probes in screening genomic- or cDNA-libraries to isolate the corresponding clones. Such use does not even require knowledge of the sequence of the expressed fragment. Alternatively, the sequence of the expressed fragment may be determined and subsequently used to design oligonucleotides to be used as primers in variety of possible amplification reactions aimed at specifically amplifying genomic or cDNA fragments which may then be assembled into their full-length counterparts.

In another preferred aspect the invention relates to the use of the expressed fragments to down regulate expression of the corresponding genes by means of expression of (part of) the sequence of the expressed fragment as an antisense RNA molecule, being capable of interfering with translation of the corresponding mRNA to which the antisense expressed fragment is complementary. For this purpose the (part of) the sequence of the expressed fragment is cloned in antisense orientation in an expression vector. The antisense expression vector is constructed and subsequently introduced and expressed in a desired plant host cell or organism as described herein below.

In a further embodiment the expressed fragments of the invention are used in determining whether a plant or plant material has the (capacity to express) a desired property relating to disease resistance. E.g. the expressed fragments may be used in analysing the genome of a plant for the presence of genes corresponding to such a desired property relating to disease resistance; and/or for analysing a plant or plant material for the expression of transcripts associated with such desired property. The expressed fragments of the invention may also be used in the identification of loci - including e.g. QTL's - involved in a desired property relating to disease resistance, and/or to identify alleles of involved in such a desired property. When the expressed fragments of the invention are used in genome or expression analysis, a preferred aspect of the invention concerns a solid support containing one or more immobilised expressed fragments of the invention.

Such solid supports or carriers containing one or more immobilised nucleic acid fragments are known as (micro)arrays or DNA-chips for analysing nucleic acid sequences or mixtures thereof (see e.g. WO 97/27317, WO 97/22720, WO 97/43450, EP 0 799 897, EP 0 785 280, WO 97/31256, WO 97/27317 and WO 98/08083). Such an array will generally comprise at least 10, 20, 50, 100, 200, 420 or 1000 different expressed fragments of the invention. For a "high-density array" or "micro-array", the total number of different expressed fragments can be in the range of 1000- 100.000 per cm² of surface area. The fragments are preferably bound to the carrier in such a way that each fragment is attached to a specific and distinct part of the carrier, so as to form an independently detectable area on the carrier, such as a spot or band. This makes it possible to "read" the array by scanning (i.e. visually or otherwise) the areas to which each fragment is attached. Preferably, the independently detectable area's containing the individual fragments are organised on the carrier in a predetermined, and preferably regularly distributed pattern, i.e. in one or more lines, columns, rows, squares, rectangles, etc, preferably in an "addressable" form, this also includes beads as the carrier. This further facilitates analysis of the array. The density of the different fragments will generally be in the range of 1-100,000 different fragments/cm², usually 5-50,000 fragments/cm², generally between 10-10,000 fragments/cm². The solid support (i.e. carrier) for the array may be any solid material to which nucleic acid sequences can be attached such as e.g. plastics, resins, polysaccharides, silica or silica-based materials, functionalised glass, modified silicon, carbon, metals, inorganic glasses, membranes, nylon, natural fibres such as silk, wool and cotton, and polymer materials such as polystyrene, polyethylene glycol terephthalate, polyvinyl acetate, polyvinyl chloride, polyvinyl pyrrolidone, polyacrylonitrile, polymethyl methacrylate, polytetrafluoroethylene, butyl rubber, styrenebutadiene rubber, natural rubber, polyethylene, polypropylene, (poly)tetrafluoroethylene, (poly)vinylidenefluoride, polycarbonate and polymethylpentene. Further suitable support materials are mentioned for instance mentioned in US-A-5,427,779, WO 97/22720, WO 97/43450, WO 97/31256, WO 97/27317 and EP 0 799 897. Preferably, the carrier will have an essentially flat, rectangular shape, with the fragments bound to one surface thereof. The size of the array, as well as of the individual areas corresponding to each of the different fragment may vary, depending upon the total amount of fragments, as well as the intended method for analysing the array. The fragments may be bound to the carrier in any manner known per se, and the specific technique used will mainly depend upon the carrier used. Binding will be at the 5'-end, or somewhere else on the fragments, as appropriate, but preferably not at the 3'-end, so as to allow for primer extension reactions. Preferably, the fragments are covalently linked to the array, i.e. by a suitable chemical technique. Suitable methods for attaching the fragments to the carrier will be clear to the skilled person. In general, any method for attaching a nucleic acid to a solid support can be used, including the methods described in US-A-5,427,779; US-A-4,973,493; US-A-4,979,959; US-A-5,002,582; US-A-5,217,492; US-A-5,525,041; US-A-5,263,992; WO 97/46313 and WO 97/22720, as well as the references cited therein.

Further applications of the expressed fragments according to the invention will be clear to the skilled person, e.g. based upon the known uses of known/comparable expressed fragments.

### C. Nucleic acid sequences, polypeptides, DNA constructs and transgenic plants.

One aspect of the invention relates to a nucleic acid molecule, preferably in isolated form that comprises a nucleotide sequence as defined in any of SEQ ID NO 1 to SEQ ID NO 420. In another aspect, the invention relates to a nucleic acid molecule, preferably in isolated form, that essentially consists of a nucleotide sequences as defined in any of SEQ ID NO 1 to SEQ ID NO 420. The invention further relates to mutants, variants, homologues, analogues, alleles, parts and/or fragments of the nucleotide sequences as defined in any of SEQ ID NO 1 to SEQ ID NO 420; and to nucleic acid molecules, preferably in isolated form, that comprise such mutants, variants, homologues, analogues, alleles, parts and/or fragments. Such mutants, variants, homologues, analogues, alleles, parts and/or fragments may differ from or may be derived from one of the nucleotide sequences of SEQ ID NO 1 to SEQ ID NO 420 by addition, substitution, insertion and/or deletion (herein collectively referred to as "nucleotide alterations") of one or more bases/nucleotides at one or more positions. The mutant, variant, homologue analogue, allele, part and/or fragment of the nucleic acid molecules shall collectively be referred to as variants, variant nucleotide sequences or variant nucleic acid molecules. Preferably, such variants will have a degree of sequence identity of at least 40%, 50%, 60%, 70%, 80%, 90%, or more than 95%, 97%, 98% or 99% with one of the nucleotide sequences of SEQ ID NO 1 to SEQ ID NO 420.

For this purpose, the percentage of "sequence identity" between a given nucleotide sequence and one of the nucleotide sequences of SEQ ID NO 1 to SEQ ID NO 420 is calculated using a known computer algorithm for sequence alignment such as BLAST, PC-gene or Pedant-Pro, which may be used at suitable and/or at standard settings. For instance, the degree of homology may be calculated using the Pedant-Pro program at (one or more of) the settings given in Table 1:

**Table 1:**

| BLAST settings. | | | | |
|---|---|---|---|---|
| Method | E-value | Max. number of alignments shown | Max. number of description lines shown | Number of iterations used |
| BLASTPGP | 0.001 | 10 | 500 | 1 |
| BLASTX | 0.01 | 5 | 500 | 5 |
| BLASTN | 10 | 5 | 500 | 1 |

| Other settings: | | | | |
|---|---|---|---|---|
| Minimal ORF length in ESTs (nt) | | | | 9 |
| Maximal number of BLOCKS alignments shown | | | | 10 |
| Blimps (BLOCKS) score threshold | | | | 1100 |
| Threshold for reporting coiled coil | | | | 0.95 |
| Minimal percentage of low complexity sequence to be reported, % | | | | 20 |
| Threshold for reporting non-globular regions by SEG | | | | 0 |
| Threshold for reporting PFAM hits | | | | 0.001 |

More specifically, in the Examples the settings given in Table 2 were used.

**Table 2:**

| BLAST settings used in the Experimental Part: | | | | |
|---|---|---|---|---|
| Method | E-value | Max. number of alignments shown | Max. number of description lines shown | Number of iterations used |
| BLASTPGP against the protein databank | 0.001 | 10 | 500 | 1 |
| All-against-one alignment via BLASTPGP | 0.0001 | 100 | 500 | 1 |
| BLASTPGP against functional categories | 0.001 | 10 | 500 | 5 |
| BLASTPGP against COGs | 0.01 | 50 | 500 | 1 |
| BLASTPGP threshold for extracting PIR keywords, superfamily information, and EC numbers | 1e-05 | Not applicable | Not applicable | Not applicable |
| BLASTX against the protein databank | 0.01 | 5 | 500 | 5 |
| BLASTN against EMBL | 10 | 5 | 500 | 1 |
| BLASTN against EMBLEST | 0.5 | 5 | 500 | 1 |
| BLASTPGP against known 3D structures | 0.001 | 50 | 500 | 1 |
| BLASTPGP against SCOP domains | 0.001 | 500 | 500 | 5 |

| Other settings: | | | | |
|---|---|---|---|---|
| Minimal ORF length in ESTs (nt) | | | | 9 |
| Maximal number of BLOCKS alignments shown | | | | 10 |
| Blimps (BLOCKS) score threshold | | | | 1100 |
| Threshold for reporting coiled coil | | | | 0.95 |
| Minimal percentage of low complexity sequence to be reported, % | | | | 20 |
| Threshold for reporting non-globular regions by SEG | | | | 0 |
| Threshold for reporting PFAM hits | | | | 0.001 |

Any part or fragment of a nucleotide sequence of SEQ ID NO 1 to SEQ ID NO 420 will preferably contain at least 30 %, 40%, 50%, 60%, 70%, 80%, 90%, or more than 95% of the total number of nucleotides of the full nucleotide sequence of the pertinent SEQ ID. Two or more such parts or fragments of a nucleotide sequence of SEQ ID NO 1 to SEQ ID NO 420 may be joined or otherwise combined to provide a nucleotide sequence useful in the invention, in which case the total number of bases/nucleotides present in the combined parts of fragments should again preferably be within the percentages indicated above. Also, any such parts or fragments may contain further nucleotide alterations as defined above.

A variant nucleotide sequence of SEQ ID NO 1 to SEQ ID NO 420 may also be defined by its capability to hybridise with any of the nucleotide sequences of SEQ ID NO 1 to SEQ ID NO 420 under moderate, or preferably under stringent hybridisation conditions. Stringent hybridisation conditions are herein defined as conditions that allow a nucleic acid sequences of at least about 25, preferably about 50 nucleotides, 75 or 100 and most preferably of about 200 or more nucleotides, to hybridise at a temperature of about 65°C in a solution comprising about 1 M salt, preferably 6 x SSC or any other solution having a comparable ionic strength, and washing at 65°C in a solution comprising about 0,1 M salt, or less, preferably 0,2 x SSC or any other solution having a comparable ionic strength. Preferably, the hybridisation is performed overnight, i.e. at least for 10 hours and preferably washing is performed for at least one hour with at least two changes of the washing solution. These conditions will usually allow the specific hybridisation of sequences having about 90% or more sequence identity. Moderate conditions are herein defined as conditions that allow a nucleic acid sequences of at least 50 nucleotides, preferably of about 200 or more nucleotides, to hybridise at a temperature of about 45°C in a solution comprising about 1 M salt, preferably 6 x SSC or any other solution having a comparable ionic strength, and washing at room temperature in a solution comprising about 1 M salt, preferably 6 x SSC or any other solution having a comparable ionic strength. Preferably, the hybridisation is performed overnight, i.e. at least for 10 hours, and preferably washing is performed for at least one hour with at least two changes of the washing solution. These conditions will usually allow the specific hybridisation of sequences having up to 50% sequence identity. The person skilled in the art will be able to modify these hybridisation conditions in order to specifically identify sequences varying in identity between 50% and 90%.

A particularly preferred nucleic acid molecule according to the invention comprises a coding sequence and a nucleotide sequence of SEQ ID NO 1 to SEQ ID NO 420 or a variant thereof. SEQ ID NO 1 to SEQ ID NO 420 comprise differentially expressed fragments and are thus derived from actively expressed transcripts that contain sequences coding for polypeptides. The nucleic acid molecule comprising a coding sequence and a nucleotide sequences of SEQ ID NO 1 to SEQ ID NO 420, preferably comprises a functional coding sequence, more preferably a full-length coding sequence. The nucleotide sequences of SEQ ID NO 1 to SEQ ID NO 420 will usually be present within the coding sequence, although the skilled person will appreciate that in some instances part or all of the nucleotide sequences of SEQ ID NO 1 to SEQ ID NO 420 may correspond to non-translated sequences in the transcript from which the nucleotide sequences of SEQ ID NO 1 to SEQ ID NO 420 is derived. In such instances the nucleotide sequences of SEQ ID NO 1 to SEQ ID NO 420 may be adjacent to or partly overlap with the coding sequence in the nucleic acid molecule. Thus, the invention relates to a nucleic acid molecule comprising a functional coding sequence for a plant polypeptide, wherein the coding sequence is part of a plant transcript that comprises a nucleotide sequence as defined in one of SEQ ID NO 1 to SEQ ID NO 420 is. The coding sequences in the nucleic acid molecules of the invention and variants thereof encode polypeptides that are further defined hereinbelow.

A nucleotide sequence comprising a sequence of SEQ ID NO 1 to SEQ ID NO 420 and the variants thereof is preferably such that the expression thereof in a suitable cell or organism, i.e. a host cell or host organism, leads to a "significant biological change" in the host cell or host organism. A "significant biological change" is understood to mean a detectable or measurable change in a biological property or activity of the host cell or host organism, as may be determined using one or more suitable detection methods known per se, such as one or more biological assays for the one or more biological properties or activities. In particular, a significant biological change may mean that the host cell or host organism, when maintained under conditions conducive to the expression of the nucleotide sequence or variant thereof, will show at least one of the biological properties and/or activities that are natively associated with the nucleotide sequences comprising a sequence of SEQ ID NO 1 to SEQ ID NO 420, and in particular one or more of the desired properties relating to disease resistance.

In particular, a variant of a nucleotide sequence comprising a sequence of SEQ ID NO 1 to SEQ ID NO 420 is such that, upon expression the nucleotide sequence and or the amino acid sequence encoded thereby, a biological property or activity is provided to the host cell or host organism in an amount of at least 30%, preferably at least 40%, more preferably at least 50%, even more preferably at least 75% and up to 100% or more, of the amount that is provided by a nucleotide sequences comprising a sequence of SEQ ID NO 1 to SEQ ID NO 420 in (essentially) the same host organism and under (essentially) the same conditions, as measured by a suitable assay for the a biological property or activity (in which it is to be understood that by "providing the biological property" also a reduction of one or more undesired properties may be meant, in which case the percentages mentioned above refer to the amount in which the undesired property is reduced compared to the reduction provided by one of the nucleotide sequences comprising a sequence of SEQ ID NO 1 to SEQ ID NO 420).

The nucleotide sequences comprising a sequence of SEQ ID NO 1 to SEQ ID NO 420 or variants thereof preferably encode an amino acid sequence of a polypeptide, such as a protein or an enzyme. It may also encode an RNA sequence that can influence one or more desired properties relating to disease resistance in or of the host. As will be clear to the skilled person, because of the degeneracy of the genetic code, a variant of a nucleotide sequence comprising a sequence of SEQ ID NO 1 to SEQ ID NO 420 may encode the same amino acid sequence as encoded by the nucleotide sequence comprising a sequence of SEQ ID NO 1 to SEQ ID NO 420. Such isocoding variant nucleotide sequences are included in the invention.

However, variants of the nucleotide sequences comprising a sequence of SEQ ID NO 1 to SEQ ID NO 420, may also encode a mutant, variant, homologue, analogue, allele, part and/or fragment of an amino acid sequence that is encoded by one of the nucleotide sequences comprising a sequence of SEQ ID NO 1 to SEQ ID NO 420. Again, such mutants, variants, homologues, analogues, alleles, parts and/or fragments of the amino acid sequences or polypeptides shall collectively be referred to as variants, variant amino acid sequences or variant polypeptides, proteins or enzymes. Such variant amino acid sequence may differ from the native amino acid sequence by addition, substitution, insertion and/or deletion of one or more amino acid residues at one or more positions, herein collectively referred to as "amino acid alterations". Preferably, the variant amino acid sequences encoded by the nucleotide sequences of the invention will have a degree of amino acid sequence identity of at least 40%, 50%, 60%, 70%, 80%, 90%, or more than 95%, 97%, 98% or 99% with one of the an amino acid sequence encoded by a nucleotide sequence comprising a sequences of SEQ ID NO 1 to SEQ ID NO 420.

The degree of amino acid identity may be calculated using a known computer algorithm for sequence alignment such as BLAST, PC-gene or Pedant-Pro. E.g., the degree of amino acid homology may be calculated using the Pedant-Pro program at the settings given in Table 3.

**Table 3:**

| BLAST settings. | | | | |
|---|---|---|---|---|
| Method | E-value | Max. number of alignments shown | Max. number of description lines shown | Number of iterations used |
| BLASTPGP | 0.001 | 10 | 500 | 1 |
| BLASTX | 0.01 | 5 | 500 | 5 |
| BLASTN | 10 | 5 | 500 | 1 |

| Other settings | | | | |
|---|---|---|---|---|
| Minimal ORF length in ESTs (nt) | | | | 9 |
| Maximal number of BLOCKS alignments shown | | | | 10 |
| Blimps (BLOCKS) score threshold | | | | 1100 |
| Threshold for reporting coiled coil | | | | 0.95 |
| Minimal percentage of low complexity sequence to be reported, % | | | | 20 |
| Threshold for reporting non-globular regions by SEG | | | | 0 |
| Threshold for reporting PFAM hits | | | | 0.001 |

In particular, in the Examples the settings given in Table 4 were used.

**Table 4:**

| BLAST settings. | | | | |
|---|---|---|---|---|
| Method | E-value | Max. number of alignments shown | Max. number of description lines shown | Number of iterations used |
| BLASTPGP against the protein databank | 0.001 | 10 | 500 | 1 |
| All-against-one alignment via BLASTPGP | 0.0001 | 100 | 500 | 1 |
| BLASTPGP against functional categories | 0.001 | 10 | 500 | 5 |
| BLASTPGP against COGs | 0.01 | 50 | 500 | 1 |
| BLASTPGP threshold for extracting PIR keywords, superfamily information, and EC numbers | 1e-05 | Not applicable | Not applicable | Not applicable |
| BLASTX against the protein databank | 0.01 | 5 | 500 | 5 |
| BLASTN against EMBL | 10 | 5 | 500 | 1 |
| BLASTN against EMBL-EST | 0.5 | 5 | 500 | 1 |
| BLASTPGP against known 3D structures | 0.001 | 50 | 500 | 1 |
| BLASTPGP against SCOP domains | 0.001 | 500 | 500 | 5 |

| Other settings | | | | |
|---|---|---|---|---|
| Minimal ORF length in ESTs (nt) | | | | 9 |
| Maximal number of BLOCKS alignments shown | | | | 10 |
| Blimps (BLOCKS) score threshold | | | | 1100 |
| Threshold for reporting coiled coil | | | | 0.95 |
| Minimal percentage of low complexity sequence to be reported, % | | | | 20 |
| Threshold for reporting non-globular regions by SEG | | | | 0 |
| Threshold for reporting PFAM hits | | | | 0.001 |

Optionally, in determining the degree of amino acid identity or homology, the skilled person may also take into account so-called "conservative" amino acid substitutions, as will be clear to the skilled person. Conservative amino acid substitutions refer to the interchangeability of residues having similar side chains. For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulphur-containing side chains is cysteine and methionine. Preferred conservative amino acids substitution groups are: valine-leucine-isoleucine, phenylalanine-tyrosine, lysine-arginine, alanine-valine, and asparagine-glutamine. Substitutional variants of the amino acid sequence disclosed herein are those in which at least one residue in the disclosed sequences has been removed and a different residue inserted in its place. Preferably, the amino acid change is conservative. Preferred conservative substitutions for each of the naturally occurring amino acids are as follows: Ala to ser; Arg to lys; Asn to gln or his; Asp to glu; Cys to ser or ala; Gln to asn; Glu to asp; Gly to pro; His to asn or gln; Ile to leu or val; Leu to ile or val; Lys to arg; gln or glu; Met to leu or ile; Phe to met, leu or tyr; Ser to thr; Thr to ser; Trp to tyr; Tyr to trp or phe; and, Val to ile or leu.

In the present invention, the nucleotide sequences comprising a sequence of SEQ ID NO 1 to SEQ ID NO 420 are preferably derived from varieties or lines of tomato, i.e. *Lycopersicon esculentum*, including *Lycopersicon esculentum* varieties like MoneyMaker or other *Lycopersicon esculentum* lines. However, it is envisaged that on the basis of the disclosure herein, the skilled person will be able to obtain nucleotide sequences homologous and preferably corresponding to nucleotide sequences comprising a sequence of SEQ ID NO 1 to SEQ ID NO 420 from other biological sources. Preferably such homologous sequences are obtained from plants, more preferably from dicotylous plants. Such homologous nucleotide or amino acid sequences will be referred to herein as "natural homologues" and are included within the scope of the present invention, as variants of such natural homologues. Preferably, any such natural homologue will have a degree of sequence identity of at least 30 %, 40%, 50%, 60%, 70%, 80%, 90%, or more than 95%, 97%, 98% or 99% with of the nucleotide sequences comprising a sequence of SEQ ID NO 1 to SEQ ID NO 420 or an amino acid sequence encoded thereby. The percentage of sequence identity is calculated as set out above. Natural homologues of the nucleotide sequences comprising a sequence of SEQ ID NO 1 to SEQ ID NO 420 or variants thereof may also be defined by their capability to hybridise with a nucleotide sequences of SEQ ID NO 1 to SEQ ID NO 420 under moderate or preferably stringent hybridisation conditions as defined above. It will be understood that a natural homologue of one of the nucleotide sequences comprising a sequence of SEQ ID NO 1 to SEQ ID NO 420 or a variant thereof is preferably such that, in the biological source from which it was obtained, it has a biological property or is capable of a biological activity that is essentially the same or at least comparable to the biological properties or activities natively provided by the nucleotide sequences comprising a sequence of SEQ ID NO 1 to SEQ ID NO 420. Moreover, a natural homologue according to the invention is preferably such that the expression thereof in a host cell or host organism leads to a significant biological change as defined above in the host cell or host organism, in particular a significant biological change with respect to one or more desired properties relating to disease resistance as outlined above.

Hereinbelow, all of the nucleotide sequences and nucleic acid molecules described above, i.e. any and all nucleotide sequences comprising a sequence of SEQ ID NO 1 to SEQ ID NO 420, any natural homologues thereof, as well as any variants thereof will collectively be referred to as "nucleotide sequences of the invention". The term "nucleotide sequences of the invention" also includes any and all nucleic acid molecules comprising such nucleotides sequences in the form of a nucleic acid construct as defined hereinbelow. These nucleotide sequences of the invention may be in the form of a double stranded nucleic acid or in form of a single stranded nucleic acid, in which the latter also includes any nucleic acid complementary to a nucleotide sequence of the invention. As such, the nucleotide sequences/nucleic acids of the invention may be a DNA sequence and/or an RNA sequence, which is again preferably in isolated form and/or in the form of a nucleic acid construct as defined hereinbelow. They may e.g. be genomic DNA sequences that - besides the one or more ORFs and/or exon sequences - may contain one or more intron sequences, 5'-UTRs or 3'-UTRs. They may also be cDNA sequences and/or RNA sequences, including but not limited to mRNA sequences.

Also included in the nucleotides sequences of the invention is any nucleotide sequence that is functionally homologous to any of the nucleotide sequences of SEQ ID NO 1 to 420 By the term "functionally homologous" encompasses the following. A sequence (for instance a gene) is considered functionally homologous if that sequence (gene) is homologous to another sequence, hence at least one nucleotide is deleted, inserted, replaced such as inversed (in case of more than one nucleotide) or transversion (purine-pyrimidine or pyrimidine-purine substitution) or transition (purine-purine or pyrimidine-pyrimidine substitution) while the function of said sequence (gene) is substantially maintained. This may also apply to chemically modified sequences. When a sequence is functionally homologous, there may very well be a low percentage of homology, but the functionality of that sequence is substantially maintained.

Also hereinbelow, any and all amino acid sequences encoded by the nucleotide sequences of the invention - and in particular those which meet the definitions for such amino acid sequences set out above - will be referred to hereinbelow as "polypeptides of the invention". These polypeptides thus include oligopeptides, enzymes and non-catalytic proteins. The term functionally homologous as hereinbefore defined also applies to the amino acid sequences of the present invention. According to the invention, upon expression in a host cell or host organism, any polypeptide of the invention may also be subjected to one or more post-translational modifications, e.g. as may occur in the host cell or host organism, and any and all derivatives obtained as a result of such post-translational modification(s) are also included within the term "polypeptide(s) of the invention " as used herein. It will be clear to the skilled person that one or more of these post-translational modifications may increase, or may even be required for, one or more of the intended or desired biological properties or activities of the polypeptide. Instead of a polypeptide, a nucleotide sequence of the invention may also encode an RNA sequence, such as an RNA sequence having (up- or down-) regulatory activity with respect to one or more biological processes or pathways within the host cells, and in particular with respect to one or more of the properties relating to disease resistance. In such instances, the term "polypeptide of the invention" is to be read as "expression product of the invention".

In yet another aspect, the invention also provides genes, e.g. full-length genes preferably including appropriate expression signals, containing one or more of the nucleotide sequences of the invention. More in particular, according to this aspect, the invention provides a nucleic acid molecule, preferably in isolated form, whereby the nucleic acid molecule comprises the following sequence elements:
- a first nucleotide sequence encoding an expression product, preferably a polypeptide;
- a second nucleotide sequence chosen from the group consisting of SEQ ID NO 1 to SEQ ID NO 420 or a variant thereof, whereby preferably at least part of the second nucleotide sequence is present within the first nucleotide sequence;
- preferably, a translational start codon at the 5' end of the first nucleotide sequence;
- preferably, a translational stop codon at the 3' end of the first nucleotide sequence;
- preferably, one or more elements operably linked to the (5'-end of) first nucleotide sequence, which elements are capable of initiation and/or control of transcription of at least the first nucleotide sequence, such as a promoter, an enhancer, or an upstream activating sequence; and
- preferably, a element operably linked to the (3'-end of) first nucleotide sequence, which element is capable of termination of transcription downstream of at least the first nucleotide sequence.

The nucleic acid molecule may be in any suitable form, and may for instance be in the form of single stranded or double stranded DNA or RNA (with *ds*DNA being preferred); and/or may be part of or in the form of a nucleic acid construct, e.g. as further described hereinbelow. Such nucleic acid may have any suitable size, for instance between 50 and 10000 nucleotides, in particular between 100 and 5000. The nucleic acid may optionally also be present in a host or host cell e.g. as defined below, including but not limited to bacterial cells (e.g. of *Agrobacterium*) or a plant or animal cell, and as such may be integrated in the genome of the host cell or may be maintained episomally in the host cell. It will be clear to the skilled person that nucleotide sequences comprising a full-length gene and containing a nucleotide sequence of the invention - optionally in the form of a nucleic acid molecule as described above - may be used in the same way, and for the same purposes, as the nucleotide sequences of the invention; and that for many applications, the use of such nucleic acid molecules may even be preferred. Thus, in the invention in its broadest sense, such nucleic acid molecules essentially comprising a full-length gene and comprising a nucleotide sequence of the invention are also comprised within the term "nucleotide sequence of the invention". Also, the invention in its broadest sense comprises polypeptides as defined above encoded by the full-length genes of the invention. Furthermore, the invention in it broadest sense comprises variants and natural and functional homologues of such full-length genes, as well as variants and natural and functional homologues of such polypeptides.

The pathogen in terms of the present invention encompasses all pathogens relevant to the plants or crops as mentioned hereinbelow. Such pathogens can be a soil or a leaf pathogen and can be an obligate, biotroph or necrotroph pathogen, more in particular, the pathogen is selected from the group of bacteria, viruses, nematodes, aphids, fungi and/or pests. Preferably the pathogen is selected from *Cladosporium fulvum,* TMV, PVX, *Pseudomonas syringae*, CMV, CGMMV, TRV, *Alternaria alternata*, *Odium lycopersici*, *Phytophtorea infestans*, *Botrytis cinerea*, *Fusarium oxysporum*, and *Fusarium* race 2 and 3.

The nucleotide sequences of the invention are used to impart valuable properties on a host cell or host organism (as defined below), and in particular on a plant or plant cell. In particular, the nucleotide sequences of the invention may be used with advantage to provide a host cell or host organism, and in particular to confer upon a plant, one or more desired properties relating to the development of disease resistance of a plant against a plant pathogen. More in particular the nucleotide sequences of the invention may be used to provide a plant which is susceptible to a pathogen with an improved resistance against that pathogen. To achieve this the nucleotide sequences of the invention can be transformed into the genetic material of the plant by any method known in the art. Upon expression of the nucleotide sequence of the invention, the defence mechanism of the plant can be induced and the plant can express a systemic HR leading to resistance against the pathogen.

This proposed utility of the nucleotide sequences of the invention is based on observations, which are further described in the Examples. These observations are based on a model system. This model system is based on an extensive differential cDNA AFLP® analysis that was performed on Cf4 tomato, 10-90 minutes after the onset of a systemic HR due to specific recognition of the Avr4 elicitor of the biotrophic fungus *Cladosporium fulvum*, and non-responding control plants. The tomato (*Lycopersicon esculentum*) that formed part of the model system is a transgenic tomato that expresses both the Avr4 elicitor of *C. fulvum* and the matching Cf4 resistance gene. Seeds derived from a cross between a tomato carrying Cf4 and a plant expressing Avr4 germinate normally. However, generally within a few days the seedlings become necrotic and die.

By a specific temperature treatment, however, the HR induction and subsequent death of the plants can be suppressed, with the result that the plant develops normally. When the suppressive temperature is released (to a permissive temperature) the plant develop necrotic spots and the expression of many defence related genes is induced. Subsequently, these tomato plants develop a systemic HR within a very short time span. One of the many advantages of this system is that no wounding of the plant occurs. Wounding is known to induce the expression of many genes including defence genes. Furthermore as no pathogen is directly involved there is also a diminished risk of isolation of contaminating genes. This model system advantageously allows for the isolation of an virtually unlimited amount of plant material in which gene expression is synchronised. cDNA AFLP® is a technique that can advantageously be used to identify differentially expressed genes. By comparing the gene expression patterns between transgenic Cf::Avr plants which are either repressed or are not repressed in HR, the genes involved in the induction of plant defence are identified. Analysis of the (RNA) sequences isolated at different time points after the de-repression of HR results in the identification of a specific classes of genes related to the signal transduction pathway. The first genes that are induced are those that encode components of the signal transduction cascades and/or transcription factors. The products of these genes will subsequently activate the expression of the genes involved in the defence system. When a specific time frame is selected, for instance in a very early stage of the HR, it is possible to search for specific genes that play a role in that particular time frame, for instance for genes that exert their function in the initiation of plant defence responses.

Differentially expressed genes are sequenced and the sequences are compared to sequences present in the databases. To this end a variety of algorithms and computer programs is available to the skilled man such as Blast searches, Bestfit, FASTA, TFASTA, the algorithms as disclosed by Smith and Waterman Adv. Appl. Math. 2:482 (1981), Needleman and Wunsch J. Mol. Biol. 48:443 (1970), Pearson and Lipman Proc. Natl. Acad. Sci. (U.S.A.) 85:2444 (1988), and Pedant Pro (Biomax).

The expression patterns are examined individually, by northern blots or in the alternative simultaneously by spotting the AFLP® fragments on microarrays. These fragments can subsequently hybridised with cDNA isolated at different time points after the release of HR repression. Techniques such as silencing, for instance Virus Induced Gene Silencing (VIGS) provides for identification of relevant genes. Expression of homologous plant gene sequences in for instance potato Virus X (PVX) result in post-transcriptional gene silencing. The silenced plants are tested for their ability to induce HR when provoked with a suitable (race-specific) elicitor. The candidate genes are used in the production of transgenic plants, for instance tomato or Arabidopsis to study (over)expression and/or inducible expression.

Possible further applications or uses of the nucleotide sequences of the invention include e.g.:
- utilisation as probe to detect homologous sequences;
- utilisation in classification of species and sub -species and individuals;
- use in heterologous production of polypeptides in for example micro-organisms;
- use in or for overexpression in original source;
- use in or for overexpression in a heterologous source or host;
- use in or for silencing in original source;
- use in or for silencing in a heterologous source or host;
- use as marker(s) for indirect selection;
- use on DNA arrays or chips.

The nucleotide sequences of the invention share as a common technical feature that they may be used to impart on a host cell or host organism one or more of the properties and/or activities mentioned above.

The nucleotide sequences of the invention may be isolated from natural source(s) - i.e. as indicated above - or from any other suitable source. Alternatively, the nucleotide sequences of the invention may be provided by recombinant techniques known per se, including but not limited to automated DNA synthesis; site-directed mutagenesis; combining two or more parts of one or more of the nucleotide sequences of the invention (e.g. using one or more restriction enzymes and/or ligases); introduction of mutations that lead to the expression of a truncated polypeptide; introduction of mutations that lead to an altered expression level and/or profile, and other modifications known to the skilled person per se. E.g. a specific technique involves the introduction of mutations by means of a PCR reaction using one or more "mismatched" primers and using one or more of the nucleotide sequences of the invention as a template. The amplified products/fragments thus obtained may then be ligated to form new variants of the nucleotide sequences of the invention. These and other suitable techniques are for instance described in Sambrook et al, "Molecular Cloning: A Laboratory Manual" (2nd.ed.), Vols. 1-3, Cold Spring Harbor Laboratory (1989) of F. Ausubel et al, eds., "Current protocols in molecular biology", Green Publishing and Wiley Interscience, New York (1987) and Saiki *et al*., Science 239 (1988), 487-491 or PCR Protocols, 1990, Academic Press, San Diego, California, USA.

In a further aspect, the invention relates to a nucleic acid construct, the construct at least comprising a nucleotide sequence of the invention; and optionally further elements of nucleic acid constructs known per se.

Such a nucleic acid construct may be in the form of a DNA sequence or RNA sequence, and is preferably in the form of a double stranded DNA sequence. It may also be in the form of plasmid or vector. The nucleic acid construct is preferably in a form suitable for transforming the intended host cell or host organism. In particular, the nucleic acid construct may be in a form suitable for transforming a plant or plant cell. The nucleic acid construct may also be such that, upon transformation, it is stably maintained, replicated and/or inherited in the host cell or host organism. Alternatively, the nucleic acid construct may be such that - upon transformation - it allows for (at least) the nucleotide sequence of the invention to be integrated into the DNA - and preferably into the genomic DNA - of the host cell or host organism. The nucleic acid construct may also be such that it allows for the expression of the nucleotide sequence of the invention in the host cell or host organism. It is preferably also such that it allows for the nucleotide sequence of the invention to be inherited from one generation of the host organism to the next. For either or both of these purposes, upon transformation, the nucleic acid construct may first be integrated into the DNA of the host cell or host organism as described above.

The "optional further elements" that may be present in the nucleic acid constructs of the invention include, but are not limited to, one or more expression regulating sequences such as a promoter, leader sequences, terminators, enhancers, integration factors, selection markers and/or reporter genes, intron sequences and/or matrix attachment (MAR) sequences. Preferably, any such optional further elements are "operably linked" to the nucleotide sequence of the invention and/or to each other. by which is generally meant that they are in a functional relationship with each other. For instance, a promoter is operably linked to a coding sequence if the promoter is able to initiate or otherwise control/regulate the transcription and/or expression of a coding sequence, in which case the coding sequence should be understood as being "under the control of" the promoter. Generally, when two nucleotide sequences are operably linked, they will be in the same orientation and usually also in the same reading frame. They will usually also be essentially contiguous, although this may not be required.

Preferably, the optional further elements of the nucleic acid construct are such that they are capable of providing their intended biological function in the host cell or host organism. For instance, a promoter, enhancer and/or terminator should be "operable" in the host organism, by which is meant that, in the pertinent host cell or host organism, the promoter should be capable of initiating or otherwise controlling/regulating the transcription and/or the expression of a nucleotide sequence - e.g. a coding sequence - to which it is operably linked as defined above.

A promoter for use in the nucleic acid constructs of the invention may be a constitutive promoter or an inducible promoter as further mentioned below; and suitable non-limiting examples of such promoters for use in for instance plants, animals, as well as bacteria, yeast(cells) algae/fungi and/or viruses are for instance described in WO 95/07463, WO 96/23810, WO 95/07463, WO 95/21191, WO 97/11094, WO 97/42320, WO 98/06737 and WO 98/21355.

A selection marker for use in the nucleic acid constructs of the invention should be capable of distinguishing - e.g. allow the detection and/or selection of - host organisms or host cells that contain the nucleic acid construct. For instance, such a selection marker may be any gene that can be used to select - under suitable conditions such as the use of a suitable selection medium - plants, plant material and plant cells that contain - e.g. as the result of a successful transformation - the nucleic acid construct containing the marker. A particularly preferred selection marker is the *npt*-gene, which can be selected using kanamycin.

A leader sequence for use in the nucleic acid constructs of the invention should be such that - in the pertinent host cell or host organism - it may allow for the desired post-translational modifications in the host cell or host organism; may direct the transcribed mRNA to a desired part or organelle of the host cell; and/or it may allow for secretion of the polypeptide from the host cell. As such, it may be a pro-, pre-, or pre/pro-sequence operable is the host cell or host organism.

A reporter gene for use in the nucleic acid constructs of the invention should be such that, in the pertinent host cell or host organism, it allows for the expression of a nucleotide sequence present on the nucleic acid construct to be detected.

Some non-limiting examples of such further elements such as terminators, transcriptional and/or translational enhancers and/or integration factors for use in for instance plants, animals, as well as bacteria, yeast(cells), algae/fungi and/or viruses are for instance described in WO 95/07463, WO 96/23810, WO 95/07463, WO 95/21191, WO 97/11094, WO 97/42320, WO 98/06737 and WO 98/21355.

Also, in the invention, an nucleotide sequence of the invention may be operably linked to a other sequence that encodes a further amino acid sequence such as a protein or polypeptide, so as to provide - upon expression - a fusion of an polypeptide of the invention and the further amino acid sequence.

The construct of the invention can be provided in a manner known per se, which generally involves techniques such as restricting and linking nucleic acids/nucleic acid sequences, for which reference is made to the standard handbooks, such as Sambrook et al, "Molecular Cloning: A Laboratory Manual" (2nd.ed.), Vols. 1-3, Cold Spring Harbor Laboratory (1989) of F. Ausubel et al, eds., "Current protocols in molecular biology", Green Publishing and Wiley Interscience, New York (1987).

When the nucleic acid construct is to be used for the transformation of plants or plant cells and/or for the expression of a nucleotide sequence of the invention in a plant or plant cell, the nucleic acid construct may e.g. contain, in addition to a nucleotide sequence of the invention, one or more of the following genetic elements:
(a) a promoter operable in a plant or plant cell, including but not limited to constitutive promoters such as the nos-promoter, de CaMV 35S promoter, de actin promoter en de *mas* (mannopinesynthase) promoter; inducible promoters such as the WIN ("wound inducible") promoter and the HMGR (HydroxymethylglutarylCoA reductase) promoter; inducible promoters such as described in WO 97/41228, an ethanol inducible promoter (WO 99/29881) and the steroid inducible promoter (PNAS 88 23, 10421-25; 1991), copper ion inducible promoter (Transgenic Res. 5, 2, 105-113; 1996), Tet repressor regulated promoter (Mol Gen Genet. 227-2, 229-237; 1991); tissue- or organ-specific promoters such as the patatin promoter, the GBSS promoter and the plastocyanine promoter and those mentioned in WO 97/41228, the fruit specific promoter 2A11 (WO 8809334), E4 and E8 (WO 9201790) and the promoters described in WO 9727308, WO 9717452, WO 9831812; and/or promoters that are specific for a particular phase in the life cycle and/or development of a plant such as the SAG12 promoter (derived from the *Arabidopsis* "senescence associated gene");
(b) a terminator operable in a plant or plant cell such as nos-3' terminator, CaMV 3' terminator or tml terminator;
(c) an enhancer operable in a plant or plant cell such as the CaMV 35S enhancer sequence;
(d) a reporter gene suitable for use in plant or plant cell such as the *gfp*-gene, *gus* gene or *luc* gene;
(e) a selection marker such as the *npt* gene, *hpt* gene or *bar* gene.

A nucleic acid construct for the transformation of plants may be in the form of a plasmid, cosmid or vector, including but not limited to a binary vectors or co-integration vectors, e.g. suitable for use in the transformation of a plant or plant cell using *Agrobacterium*; and *Agrobacterium* strains comprising a nucleic acid construct as described herein form a further aspect of the invention. Examples of suitable vectors systems for use with *Agrobacterium* are for instance binary vectors such as pBI121 and derivatives thereof; co-integration vectors such as pGV1500 and derivatives of pBR322.

In a further aspect, the invention relates to a host organism or host cell that contains and/or that is transformed with a one or more of the nucleotide sequences of the invention. The host organism or host cell may be (a cell of) an unicellular organism or (a cell of) a multicellular organism. Examples of unicellular organisms that can used as host organism/host cells in the invention include, but are not limited to micro-organisms such as viruses, bacteria, algae, fungi, yeast and/or protozoa's. Examples of multicellular organisms that can used as host organisms - and/or the cells of which can be used as host cells - in the invention include, but are not limited to higher organisms such as plants and animals, including but not limited to non-humans mammals.

Preferably, the host organism/host cell is a plant/plant cell. In particular, the host organism/host cell may be a (a cell of) a mono- or dicotylous plant, preferably (a cell of) an agronomically important plant or crop, such as e.g. watermelon, avocado, raspberry, pineapple, grape, apple, pear, orange, wheat, barley, rye, maize, tomato, pepper, lettuce, cucumber, rice, pulse, clover, citrus fruits, banana, grapes, cassava, pea, strawberry, cotton, chilli, brinjal, bhindi, sugarbeet, soybean, oil seed rape, sunflower, cauliflower, beans, peas, of which tomato, pepper and aubergine (egg plant) are particularly preferred. Other agronomically important species, varieties and/or lines of plants/crops will be clear to the skilled person. And may for instance be selected from species from the genera of *Avena, Agrostis, Antirrhinum, Arabidopsis, Asparagus, Atropa, Brassica, Beta, Bromus, Browaalia, Capsicum, Ciahorum, Citrullus, Citrus, Composita, Cucumis, Cucurbita, Datura, Daucus, Digitalis, Festuca, Fragaria, Geranium, Glycine, Gramina, Helianthus, Heterocallis, Hordeum, Hyoscyamus, Juglans, Lactuca, Linum, Lolium, Lotus, Lycopersicon*, *Majorana, Manihot, Medicago, Nemesis, Nicotiana, Onobrychis, Oryza, Panieum, Pelargonium, Pennisetum, Petunia, Phaseolus, Pisum, Ranunculus, Raphanus, Rosa, Salpiglossis, Secale, Senecio, Sinapis, Solanum, Sorghum, Trifolium, Trigonella, Triticum, Vigna, Vita* and *Zea.*

The plant or plant cell is preferably *solanacea,* such as exemplified by tomato, eggplant, pepper, tobacco, potato and the like, more preferably tomato.

The host cell may be present in a host organism *in vivo.* For instance, when the host cell is a cell of a multicellular (host) organism, the host cell may be present in the multicellular host organism and/or in any part, tissue or organ of the multicellular host organism; and any multicellular organism containing such a transformed host cell should be considered included within the term "*multicellular host organism*"as used herein. Similarly, when reference is made herein to a "*multicellular host organism*", this also includes any part, tissue, organ or cell of such a multicellular host organism; and or any material derived from and/or for such a multicellular host organism.

For instance, when the multicellular host is a plant, the term *"plant"* also includes any part, tissue, organ or cell of such a plant, including but not limited to roots, stems, stalks, leaves, petals, fruits, seeds, tubers, meristems, sepals, and flowers. It also includes material of or for such a plant, such as (again) fruits or other materials of the plant that are intended to be harvested; material that can be regenerated into a (mature) plant, including but not limited protoplasts and/or callus tissue; or material that can be cultivated into a mature plant, such as seed, seedlings and/or (other) propagation material and/or cultivation material, e.g. as mentioned below.

The host cell may also be present *in vitro,* e.g. in a culture of such host cells. This may be a culture of the unicellular host cell(s), such as a culture of one of the micro-organisms mentioned above. It may also be a culture of host cells derived from a multicellular organism, such as an *in vitro* culture of plant or animal cells, including but not limited to a culture of cells or of a cell line derived from native plant cells or animal cells.

According to one embodiment, the host cell or host organism that contains and/or that has been transformed with the a nucleotide sequence of the invention is capable of expressing the nucleotide sequence. In particular, according to this embodiment, the nucleotide sequence of the invention is expressed in the host cell, in the host organism or in a part, tissue, organ or cell thereof. More preferably, the expression in such that is leads to a significant biological change (as defined above) in the host cell or host organism.

The host cell may also be a cell suitable for transforming another (second) host cell. In such a case, the nucleotide sequence of the invention is preferably in a form of a nucleic acid construct suitable for transforming the other (second) host cell, in which the nucleic acid construct is preferably also such that it can be (independently) maintained and/or replicated in the (first) host cell. E.g., the first host cell may be a virus or micro-organism suitable for transforming (cells of) a higher organism, such as an *Agrobacterium* strain suitable for transforming plants or plant cells.

The nucleotide sequence of the invention, as well as the one or more further optional elements of the nucleic acid construct may be homologous or heterologous to the host cell or host organism. Suitable sources and/or methods for obtaining the nucleotide sequences of the invention have been described hereinabove. The nucleotide sequences encoding the further elements of the constructs may have been isolated and/or derived from a naturally occurring source - for instance as cDNA - and/or from known available sources (such as available plasmids, etc.), and/or may have been provided synthetically using known DNA synthesis techniques.

The nucleic acid construct can be transformed into the host cell or host organism by any suitable transformation technique known per se. Suitable transformation systems and techniques for the transformation of plants, animals, as well as bacteria, yeast(cells), algae/fungi and/or viruses are for instance described in WO 95/07463, WO 96/23810, WO 95/07463, WO 95/21191, WO 97/11094, WO 97/42320, WO 98/06737 and WO 98/21355, as well as in Sambrook et al, "Molecular Cloning: A Laboratory Manual" (2nd.ed.), Vols. 1-3, Cold Spring Harbor Laboratory (1989) of F. Ausubel et al, eds., "Current protocols in molecular biology", Green Publishing and Wiley Interscience, New York (1987). Some examples of techniques and vector systems for the transformation of plants or plant cells include transformation using *Agrobacterium;* transformation using "denuded" DNA, for instance using particle bombardment or transformation of protoplasts using electroporation or treatment with PEG; as well as the transformation techniques mentioned in WO 97/41228. Suitable vector systems for transformation using denuded DNA include but are not limited to *E. coli*-vectors with a high copy number such as pUC-vectors and pBluescript II (SK+) vectors. Suitable vector systems for use with *Agrobacterium* have been mentioned hereinabove.

As mentioned above, upon such transformation, the nucleic acid construct may be incorporated into the genomic DNA of the host (cell), or it may be maintained and inherited independent from the host genome, as an episomal genetic element in the host cell.

Upon transformation, the host organism or host cell may be kept under or exposed to conditions such that expression of the nucleotide sequence of the invention is obtained, e.g. in a cell, tissue, part or organ thereof. Such conditions may depend upon the host cell or host organism used and/or on the promoter that is used to regulate the expression of the nucleotide sequence of the invention. Such conditions may for instance include the presence of a suitable inducing conditions, factors and/or compounds. In case of plants, suitable conditions for expression may for instance also include growing and/or cultivating the transformed plant, e.g. until it is mature and/or carries fruits or seed. Suitable conditions may also include growing or keeping the plant under conditions where the expression of the nucleotide sequence of the invention may be induced by factors such as stress - e.g. lack of water, light or nutrients - and/or where the plant may be subject to attack or damage by disease and/or pathogens such as viruses, fungi, bacteria, insects, nematodes or other pests; and/or damage by herbivores.

In a further aspect the invention pertains to a method for inducing, or preferably regulating the induction of the HR response in a plant by means temperature control. The method preferably comprises the steps of (a) growing a plant expressing a matching pair of a plant pathogen derived avirulence gene and a plant resistance gene, at a temperature that suppresses induction of the hypersensitive response; and (b) reducing the temperature of the plant to a temperature that is permissive for the induction of the hypersensitive response.

The transgenic plant that may be applied in the method of the invention forms another aspect of the invention. The plant preferably comprising a matching pair of a plant pathogen derived avirulence gene and a plant resistance gene. The plant resistance gene may be an endogenous gene, but may also be an exogenously provided transgene. The pathogen derived avirulence gene usually is a transgene. The plant resistance gene and the avirulence gene are preferably present and/or expressed in each (somatic) cell of the plant and are preferably integrated in the genome of the plant's cells. The plant resistance gene and the avirulence gene are preferably driven by plant promoters, or at least promoters that are active in plant cells, e.g. promoters derived from plant viruses. The promoters are preferably active during (most of) the vegetative phase of the plant and preferably in all tissues of the plant. Alternatively, the promoter driving the expression of at least one of the plant resistance gene and the avirulence gene, is a regulated and/or tissue specific promoter such that the induction of the hypersensitive response may be regulated and/or confined to one or more specific tissues. The transgenic plant is preferably grown to at least 4, 5, 6, 7, 8, 9, 10 or 11 days post-emergence of the hypocotyls, alive and viable and preferably also free of necrosis, necrotic spots and/or a hypersensitive response. More preferably the plant is fullgrown, alive and viable and preferably also free of necrosis, necrotic spots and/or a hypersensitive response.

In the method of the invention, the transgenic plants are grown at a temperature that suppresses induction of the HR. A suppressive temperature will usually be higher than room temperature (i.e. 20°C), or at least higher than the temperature at which the plants are conventionally grown. Preferably, a suppressive temperature is at least 5, 10, 15 or 20 °C above room temperature, or more preferably above the temperature at which the plants are conventionally grown. On the other hand the temperature at which the plants are grown only needs to be so high that the induction of the HR is suppressed and is preferably not chosen so high that the growth of the plants is significantly affected. A suitable suppressive temperature will depend on the plant species, variety or line and on the given matching pair of avirulence gene and resistance gene and may even depend on other conditions. A suitable suppressive temperature, however, can easily be determined by the skilled person. E.g. by allowing seeds for the transgenic plants to germinate at a range of temperatures to determine the lowest temperature at which the seeds germinate and grow out without developing necrotic spots. Usually the permissive temperature will be equal or close to room temperature (i.e. 20°C), or equal or close to the temperature at which the plants are conventionally grown. By growing the transgenic plants under an regime comprising such an elevated temperature, the onset of the plant defence response reaction by the interaction of the (products of) the avirulence gene and the resistance gene is suppressed. When the temperature is subsequently lowered to the permissive temperature, the defence response is mounted. It is possible that the permissive temperature is below room temperature or below the temperature at which the plants are conventionally grown. Again, a suitable permissive temperature can easily be established by the skilled person without undue experimentation, simply by lowering the temperature, e.g. stepwise or with a gradient, and at regular intervals determining the onset of the defence response by methods known in the art and especially by the method described in this application. As an example, for transgenic tomato plants of the MoneyMaker variety that are transgenic for the *Cf-4* resistance gene and for the *C. fulvum Avr4* avirulence gene, 33°C is a suitable suppressive temperature and 20°C is a suitable permissive temperature.

When the method according to the invention is applied to a group of transgenic plants it is possible to simultaneously mount the defence response in a group of plants. In one embodiment, the invention according pertains to a method for the simultaneous induction of a HR in a group of at least two transgenic plants containing an avirulence gene and a resistance gene corresponding to said avirulence gene comprising a step wherein the plants are subjected to a temperature controlled step. The present invention also relates to the use of this method in the regulation of the, preferably simultaneous and/or systemic, induction of the HR.

### Examples

### Example 1: Identification of sequences involved in the hypersensitive response

Disease resistance in plants to pathogens is often based on the presence of specific resistance (*R*) genes in the plant and avirulence (*Avr*) genes in the pathogen. When the *R* and *Avr* gene match, the plant induces a large array of defence responses. One of these is the HR in which cells around the infection site undergo programmed cell death. The HR, in combination with other defence responses, prevents further ingress of the pathogen.

Transgenic tomato lines have been constructed from a construct comprising the plant resistance gene *Cf-4* and a plant expressing the *Cladosporium fulvum* avirulence gene *Avr4* to result in transgenic plants that express both the *Cladosporium fulvum* avirulence gene *(Avr4)* and the matching plant resistance gene *(Cf-4).* The construction of these *Cf-4*/*Avr4* transgenic tomato lines (as well as the construction of *Cf-9*/*Avr9* transgenic tomato lines) is described by Cai et al. (2001) Mol. Plant Pathol. **2**: 77-86. When grown under repressive conditions (high temperature: about 33°C) these plants grow normal, but under permissive conditions (room temperature: about 20°C) the plant defence responses are activated. This results in a systemic and synchronised induction of the HR and whole plant death in approximately 48 hours. Using cDNA AFLP®, with RNA isolated at different time-points after temperature shift, the (key)genes involved in the induction and regulation of these defence responses are identified.

RNA was isolated, at ten different time-points after the temperature shift, from *Cf4-Avr4* plants and *Cf0* control plants. This material was used for cDNA AFLP® analysis resulting in fingerprints with a consistent- and highly reproducible banding pattern. Test gels were run and analysed to determine the most informative time-points for performing the expression analysis with the material isolated and to determine whether the screen should be carried out with +2/+2 or with +2/+3 primers. Based on these results it was decided to use the time-points 0, 30, 60 and 90 minutes after the temperature shift for the complete analysis using the Taq/Mse enzyme combination and all 1024 +2/+3 primer combinations.

### Adapters:

Taq-rare: 5'-CTCGTAGACTGCGTAC –3'
3'- TCTGACGCATGGC –5'

Mse :	5'- GACGATGAGTCCTGAG-3'
`	3'-TACTCAGGACTCAT –5'

Primers: Taq rare 5'- GTAGACTGCGTACCGA –3'
Mse: 5'- GATGAGTCCTGAGTAA –3'

At the 3' end of each primer the +2/+3 selective nucleotides are located.

Running and analysing the gels with all 1024 primer combinations resulted in the expression pattern of approximately 50 000 "C*f*0 bands" that were compared to the "*Cf4*::*Avr*4" homologues. Bands that are differentially expressed were selected and the exact levels of expression were determined using the software Quantar-Pro (obtainable from Keygene NV, Wageningen, the Netherlands). Subsequently the difference in expression level per time-point between the *Cf*4::*Avr*4 and the *Cf*0 control plants was determined. Based on these data 442 fragments were selected that showed a difference in expression of a factor ≥3 at two or more time-points or of a factor between *2.5* and 3 at three or four time-points. Of these 442 differentials 328 are up-regulated in the *Cf*4::*Avr*4 plants and 114 are down regulated. For approximately 75 % of these fragments the expression in the *Cf*4::*Avr*4 plants was already up- or down-regulated at timepoint t=0. Some of these differentials result from differences in genetic background (e.g. *GusA,* N*pt*II, *Avr*4, C*f*4) but it could also be an indication that the repression of plant defence signaling at higher temperature is not absolute and some defence-related signalling already takes place. All 442 differentials were isolated from gel, re-amplified and subcloned in the pCR2.1 vector using the TA cloning strategy. Reamplification primers were:

M00L : 5'- GACGATGAGTCCTGAGTAA –3'

TR00L: 5'- CTCGTAGACTGCGTACCGA –3'

After transformation four independent clones were picked and the insert sizes were validated on a sequence gel. In cases were less than four colonies were obtained after transformation, or when all four clones contained an insert of the wrong size, the fragments were re-isolated from gel. Finally 420 clones were obtained that contained an insert of the correct size. 16 fragments could not be cloned and are omitted from further analysis.

All 420 clones were sequenced and the obtained sequences trimmed by APES. This software tool, developed by the applicant, recognises and removes the AFLP® adapter sequences and determines the quality of the sequence. In order to determine the potential function of the differentials, most of the sequences have been characterised using PEDANT. This program performs blast searches against all known public databases and determines the sequence homology on protein and DNA level. Furthermore it determines the homology on the predicted 3D protein structure and recognisable protein domains. Based on these analyses the 420 sequence fragments can be divided into three groups.

The first group (Table 5) contains the fragments that can be matched to the "internal controls". These "internal controls" result from known differences in the genetic background between *Cf*0 and *Cf*4::*Avr*4 such as the *Cf-4* introgression fragment and the T-DNA (that contains *Avr4* together with *GusA* and *Npt*II (Kanamycin) as selectable markers). The difference in expression levels of these genes varies between the 4.5 and 150. This difference is at least partially due to the fact the genes on the T-DNA are under the control of the constitutive CaMV 35S promoter while the *Cf*4 gene is under the control of its own promoter. All expected "internal controls" have been retrieved. One of these genes is the *Cf4A (Hcr9-4E)* resistance gene of which the expression is very low. This gene is also a member of a very homologous multi-gene family and therefore, almost impossible to identify on a traditional northern blot. The fact that this gene fragment is retrieved shows that cDNA AFLP® is a very powerful technique to identify differentially expressed genes of which the expression is very low.

The second group (Table 6) contains all those sequence fragments that share homology or similarity with known sequences in publicly available databases. To determine the function of the genes belonging to the second group blast searches were performed. Homology searches on protein level show that approximately 40% of these sequences show significant homology to known plant sequences. A subset of the genes that have been identified so far are present in tomato cDNA libraries that were made from plants treated with biotic elicitors (underscored in the column marked 'description') or from developing tissues (ripening fruits, flowerbuds, roots and callus). This observation suggests that plant development and plant defence systems use, at least partially, the same signalling pathways. Based on the results obtained the genes are subdivided in 21 super-families (Table 7). These super-families are shown together with one or two references of the gene(s) with the highest homology. Most genes found have high homology to plant genes involved in plant defence. Besides many (receptor like) kinases, which are known to be involved in defence signaling also phospholipases are found that are involved in lipid-based signal transduction over the membranes. One of the first responses to elicitors is the oxidative burst and many genes are found that encode for peroxidases and cytochromes involved in the generation of these active oxygen species. This burst triggers expression of many late defence genes and accordingly a substantial number of genes coding for transcription factors is found. For the onset of the HR, which is a form of programmed cell death, it is known that many proteins are specifically degraded. The expression of many proteases is indeed up-regulated. Furthermore, in the first 90 minutes after the HR-induction some genes are already up-regulated that encode for proteins that play a role in later stages of defence. These proteins are for instance involved in phytoalexin production (like catechol oxidase) or known as PR proteins (chitinaseA).

### Example 2: Virus induced gene silencing.

To functionally characterise the obtained nucleotide sequences are cloned in a virus which upon infection of the plant will result in virus induced gene silencing (VIGS). Not only the viral genes will be silenced but also the genes that are homologous to the nucleotides of the invention. Gene functionality is subsequently determined by analysing the silenced plants for the ability to mount a defence response upon elicitor treatment. Potato Virus X (PVX) is used as an expression vector and as a vector to induce gene silencing in Nicotiana Benthamania expressing Cf4 or Cf9. The differentials found are sub-cloned into the PVX vector. These clones are used for silencing on tobacco (N. Benthamania). As controls viruses containing part of the Cf-4 resistance gene (loss of HR) and the phytoene desaturase (PDS) gene (induces photobleaching) in silenced tissue, resulting in white leaves are included. After reaching satisfactory levels of silencing, the silenced plants are challenged with the AVR4 elicitor and scored for induction of HR. Those genes that appear essential for the HR are selected. Also the tobacco rattle virus (TRV)-based gene silencing system is used for the induction of silencing in both tobacco and tomato. Genes that are essential for HR on Cf-4/t Cf-9 tobacco plants are analysed in the TRV system, together with other genes that were not silenced in tobacco in this system.

### Example 3: Gene expression analysis in other systems using cDNA AFLP®.

RNA was isolated from control plants together with tomato plants that were inoculated with at least four different pathogens. Using cDNA AFLP® the expression of these genes is studied. Many of the fragments found in the screen are derived from the same gene or are derived from genes that are involved Cladosporium-tomato interactions. These cDNA AFLP® fragments are omitted. The expression analysis is only carried out on the other cDNA AFLP® fragments. The expression data provide criteria to select the novel genes that are early expressed and induced in various systems. These genes are used for the generation of broad and durable resistance and/or for the isolation of a pathogen inducible promoter.

Gene expression analysis experiments for the determination of the expression of the cDNA AFLP® identified genes in other systems.

| Pathogen | Type of pathogen | Resistant/susceptible lines |
|---|---|---|
| Cladosporium fulvum | Biotroph | R+ S lines |
| *Alternaria alternata* | Necrotroph, AAL toxin | R+ S lines |
| *Oidium lycopersici* | Obligate biotroph | S line |
| *Phytophthora infestans* | Semi biotroph | S line |
| Pseudomonas syringae | Bacteria | |
| Tobacco mosaic virus | Virus | |
| Tobacco rattle virus | Virus | |

### Example 4: Functional analysis of candidate disease resistance genes and characterisation of full length transcripts.

Cloning of cDNA AFLP® fragments is carried out by cloning them into suitable vectors that facilitate handling and construction of other vectors and bacterial strains. Subsequently characterisation of full length transcripts is performed in order to obtain sequence information of the full length transcripts. In the absence of information on sequence homology in the database, the cDNA sequence is obtained by RACE. Southern hybridisation experiments confirms the single copy nature of the clones

After cloning the full-length cDNAs they are inserted under the control of suitable promoters in transformations vectors. After transfer of the vector to Agrobacterium the strains are used for the generation of transgenic tomato pants. From each construct a number of independent diploid transformants are generated and after ploidy analysis, copy number counts and verifying the intact integration, the fully grown plants are allowed to set seed ad the next generation is analysed to reveal the exact function of the gene -product in plant defence. The plants are further analysed for morphological differences and the timing of the induction of a defence response for instance by using northern blot hybridisation with known PR genes, microarray gene expression analysis of cDNA AFLP® in either the absence or the presence of various pathogens.

**Table 5:**

| cDNA-AFLP fragments that can be assigned to known differences in genetic background. | | | | | |
|---|---|---|---|---|---|
| SEQ ID NO | Fragment | Accesion | Protein | Homology | Up-regulation |
| 28 | Km3f_tr11-76r | TREMBL:A G146 1 | Neomycin phosphotransferase resistance protein; Kanamycin resistance | 8e-22 | 10x |
| 60 | Km3f_tr11-53r | PIR:T07015 | Cf-4A protein-tomato | 1e-52 | 4.5x |
| 166 | km3f_t23-G05r | | HygromycinB phosphotransferase | 2e-38 | 14x |
| 362 | Km3f_tr15-32r | PIR:S41047 | AVR4 protein - fungus (Cladosporium fulvum) | 2e-10 | 28x |

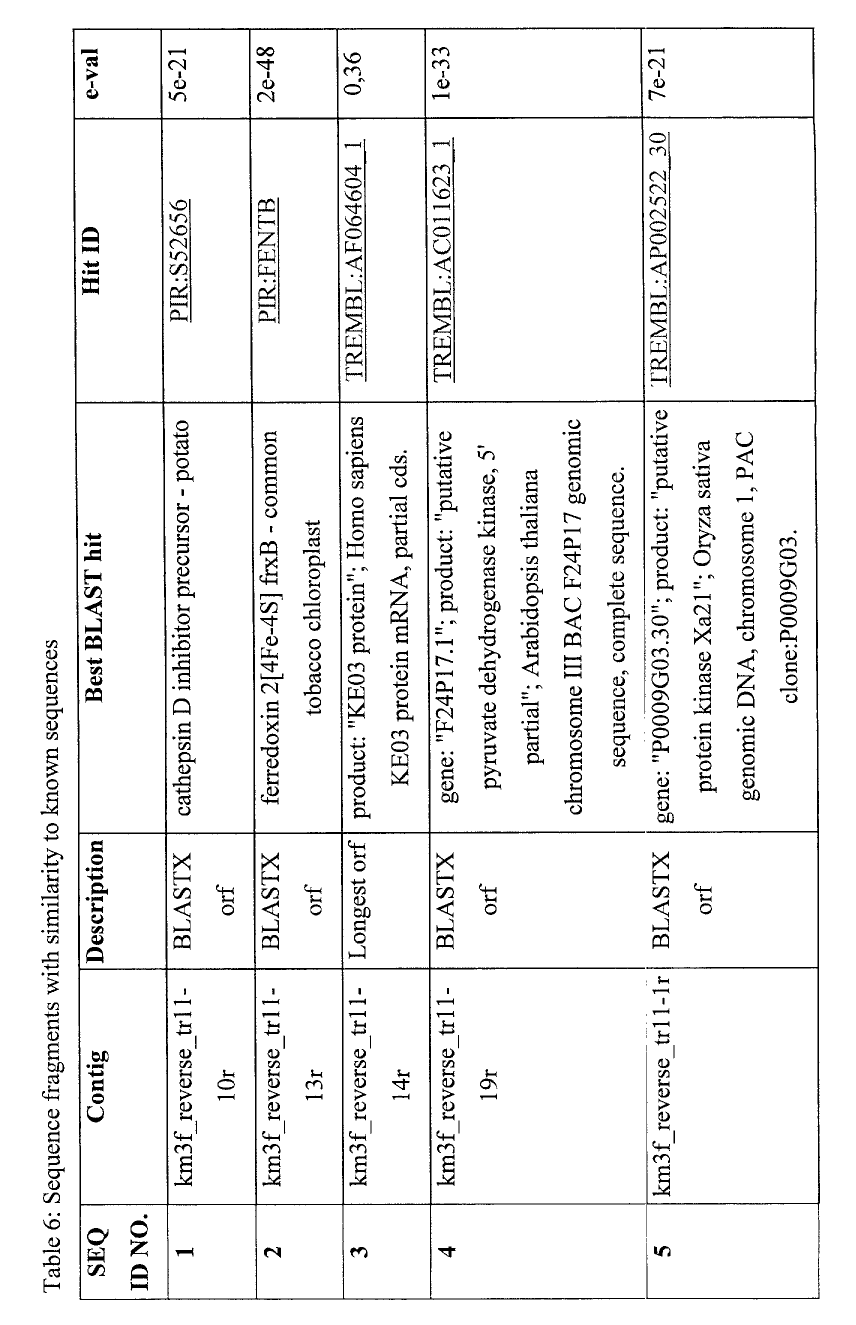

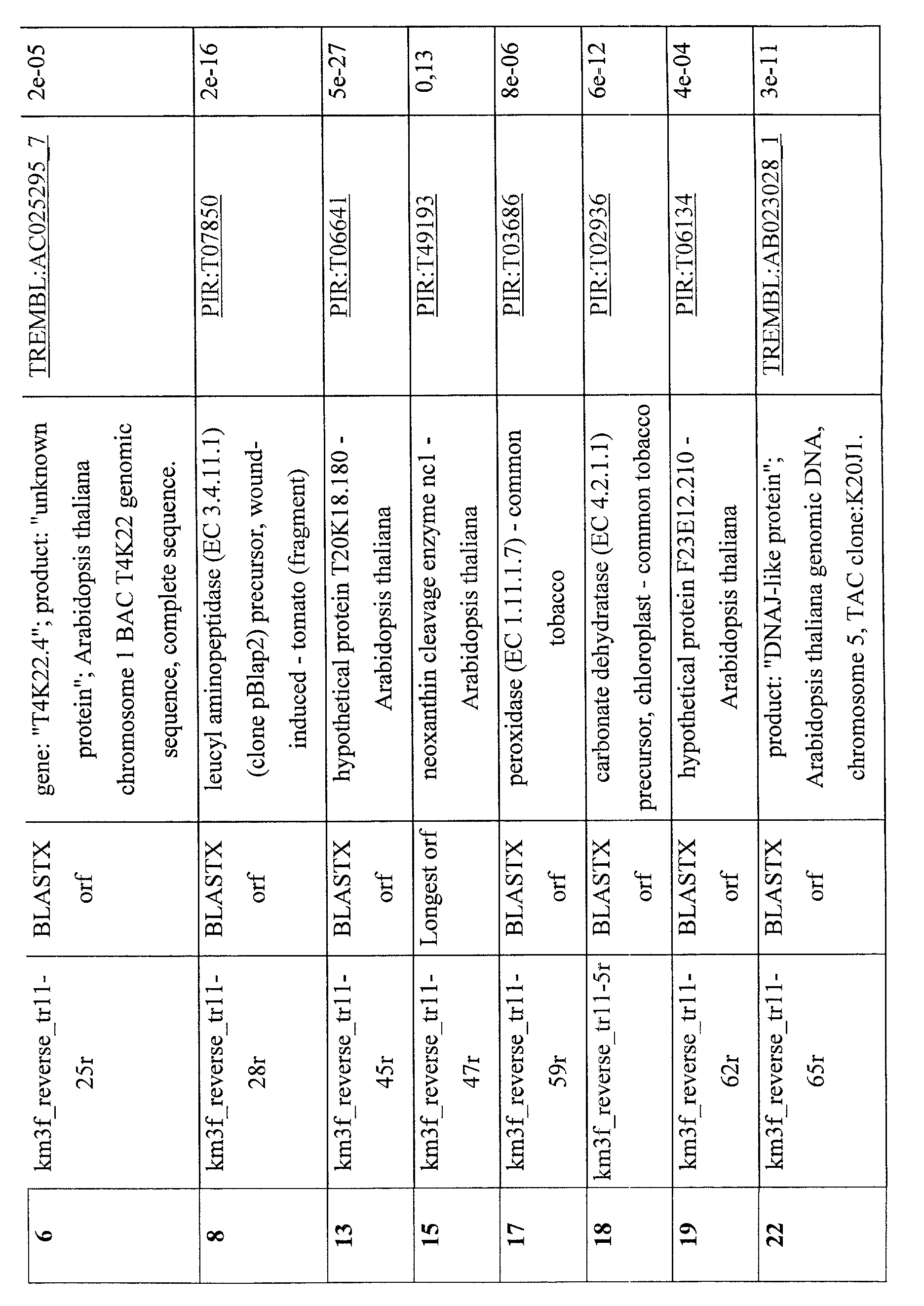

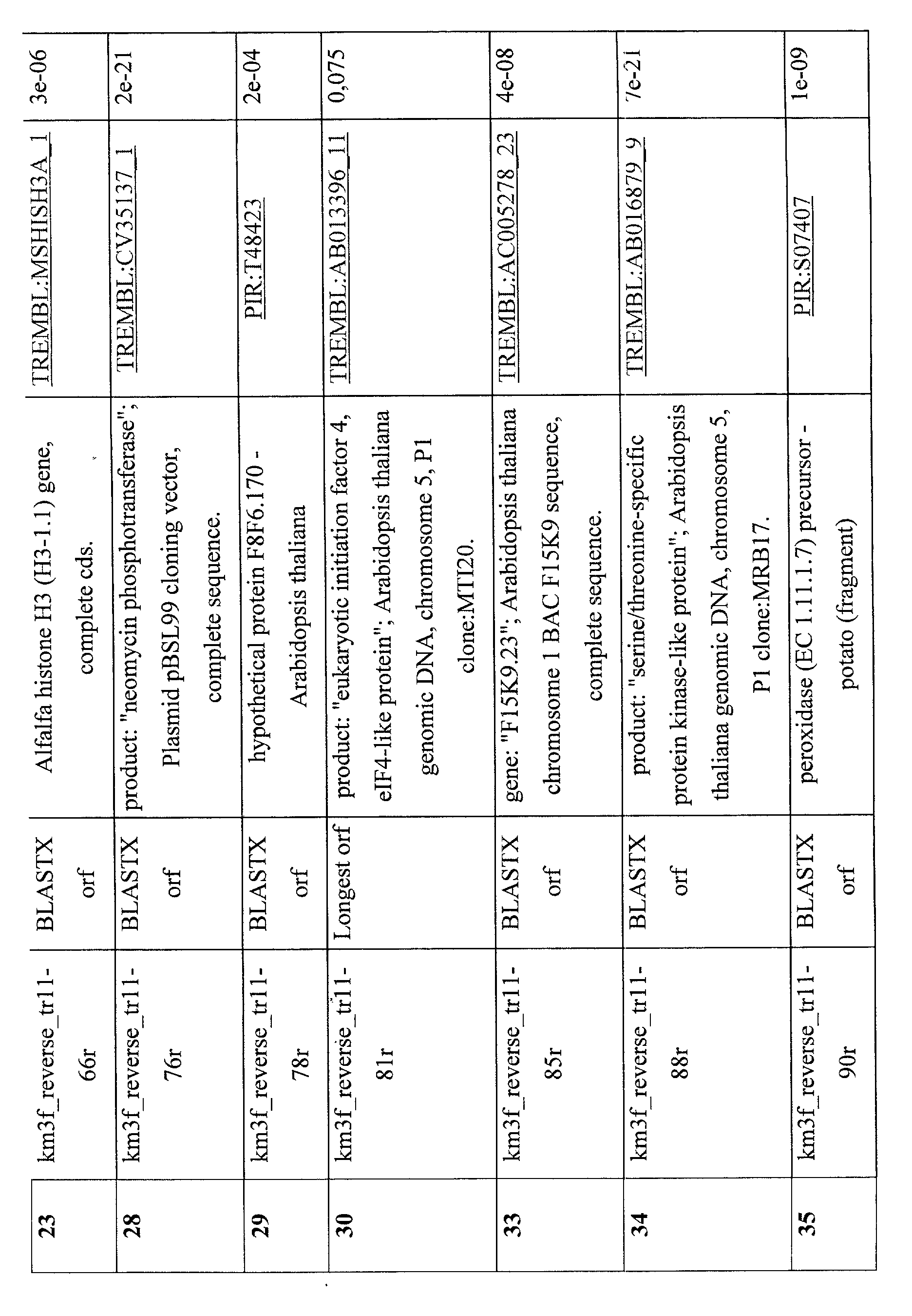

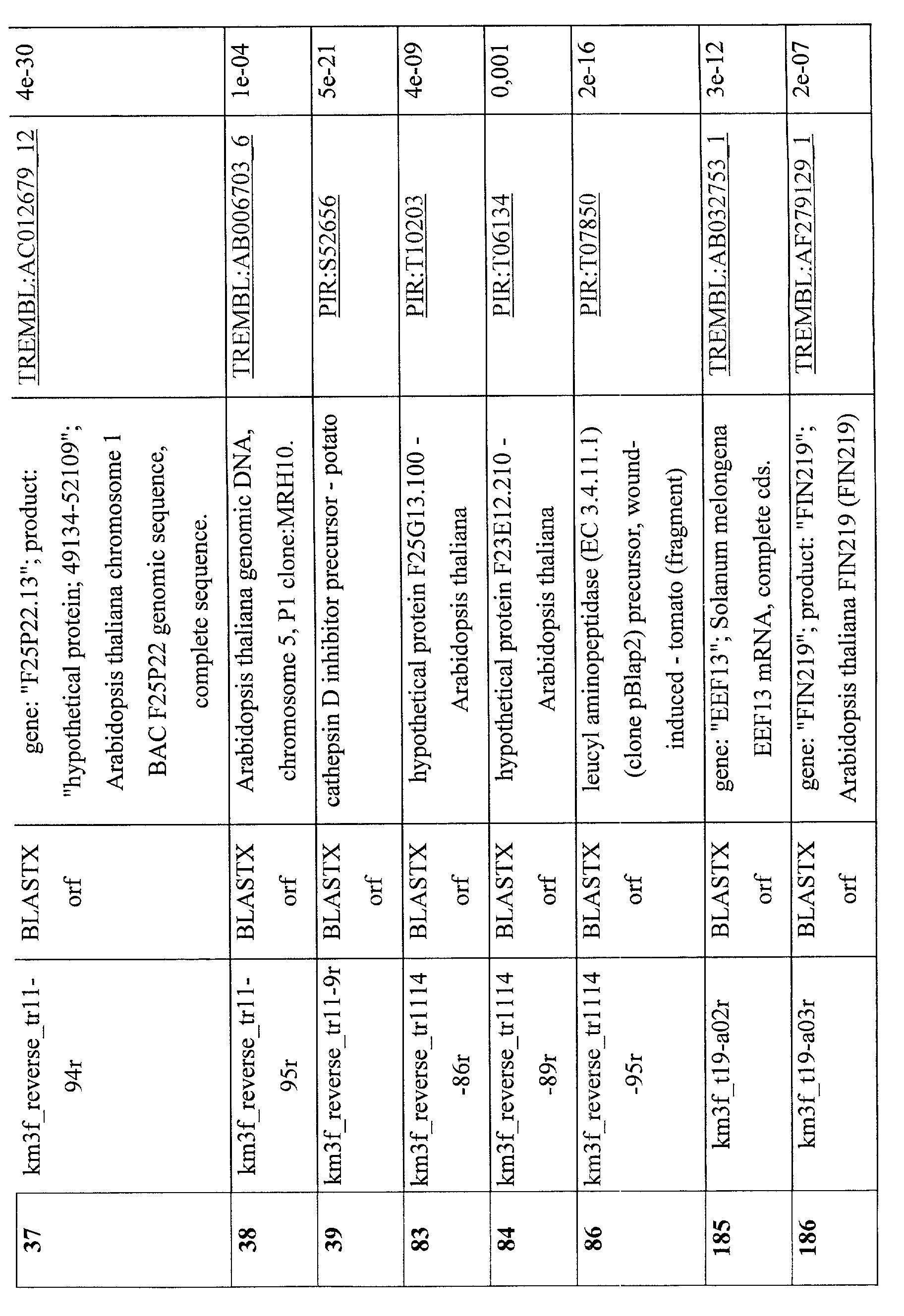

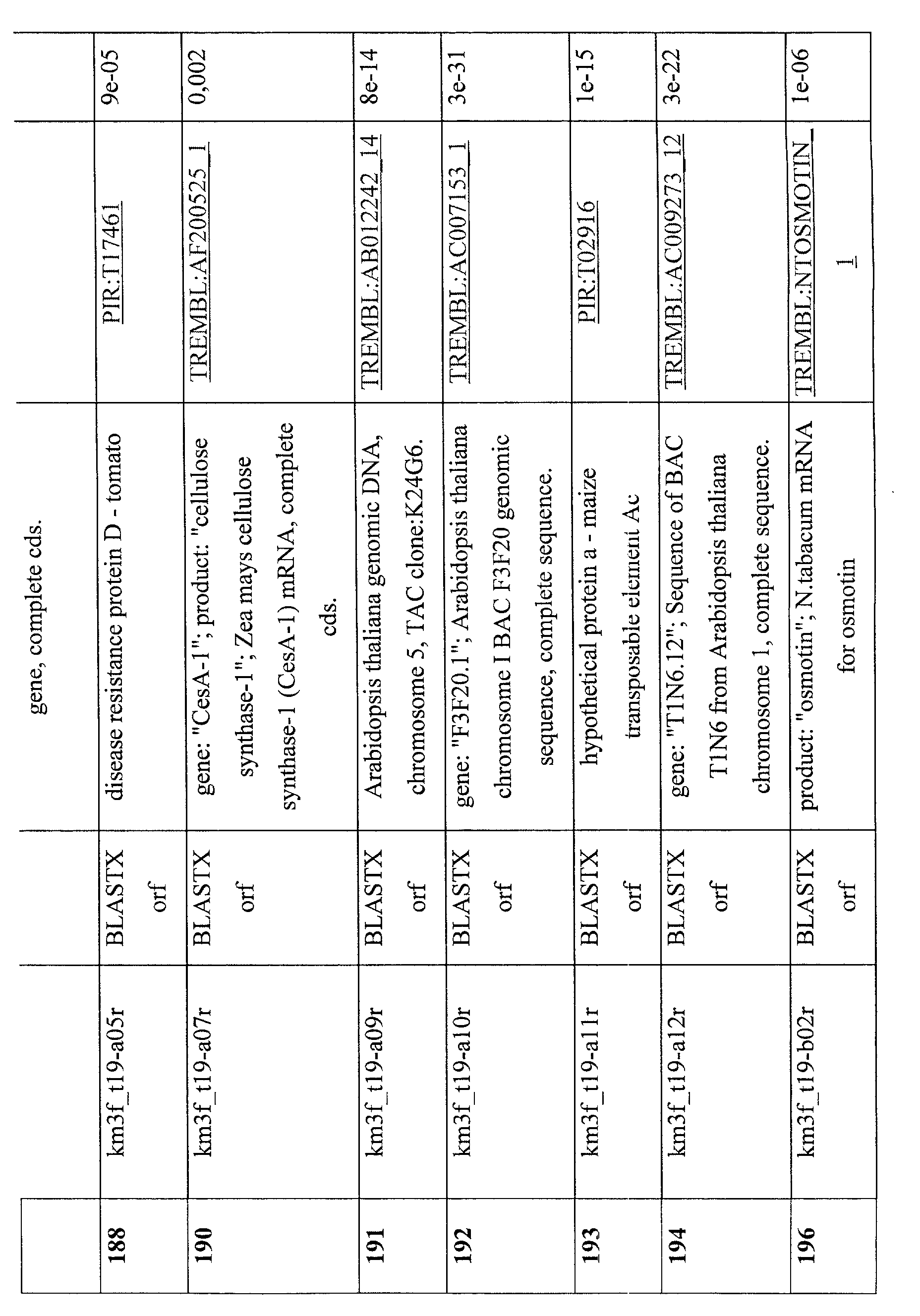

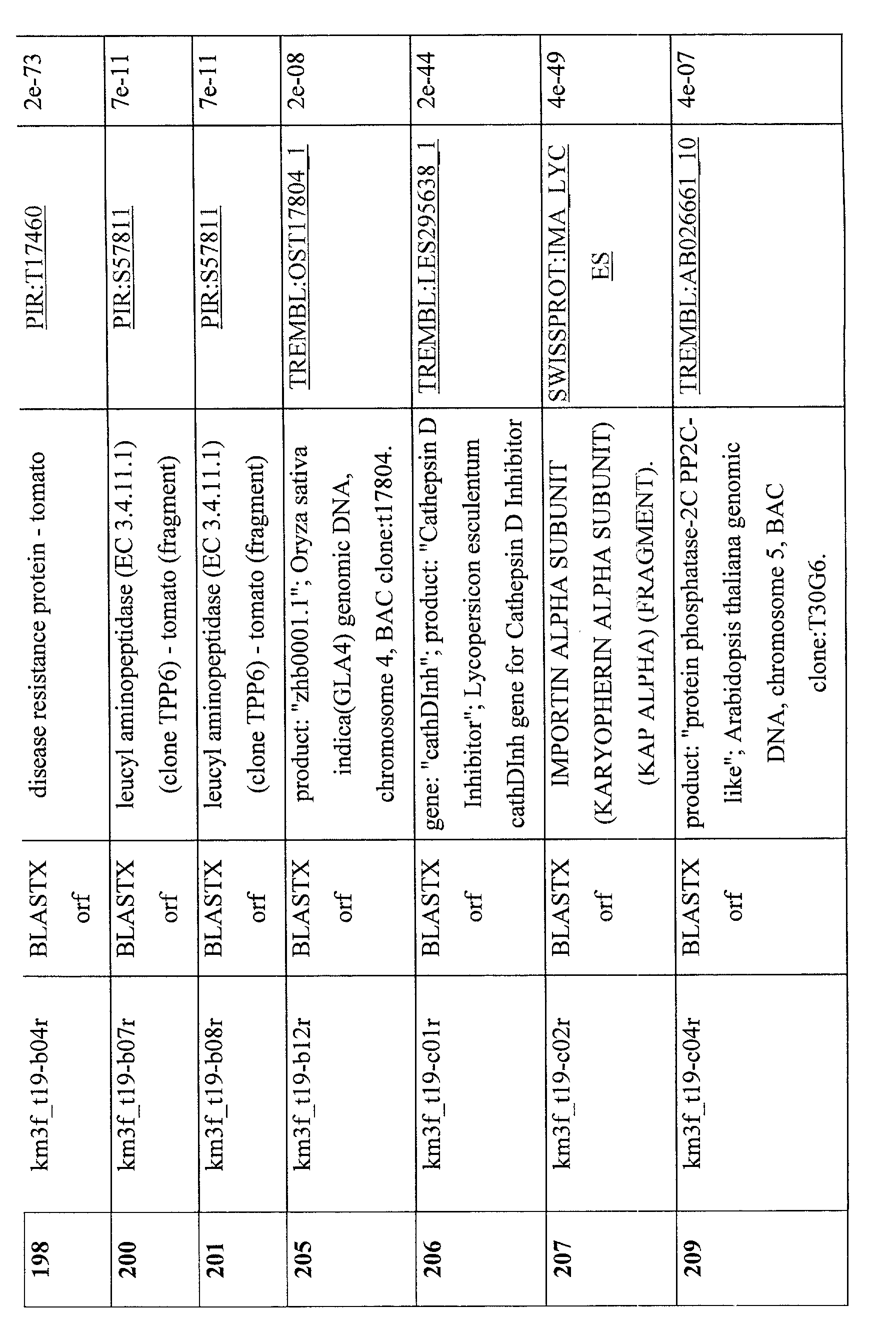

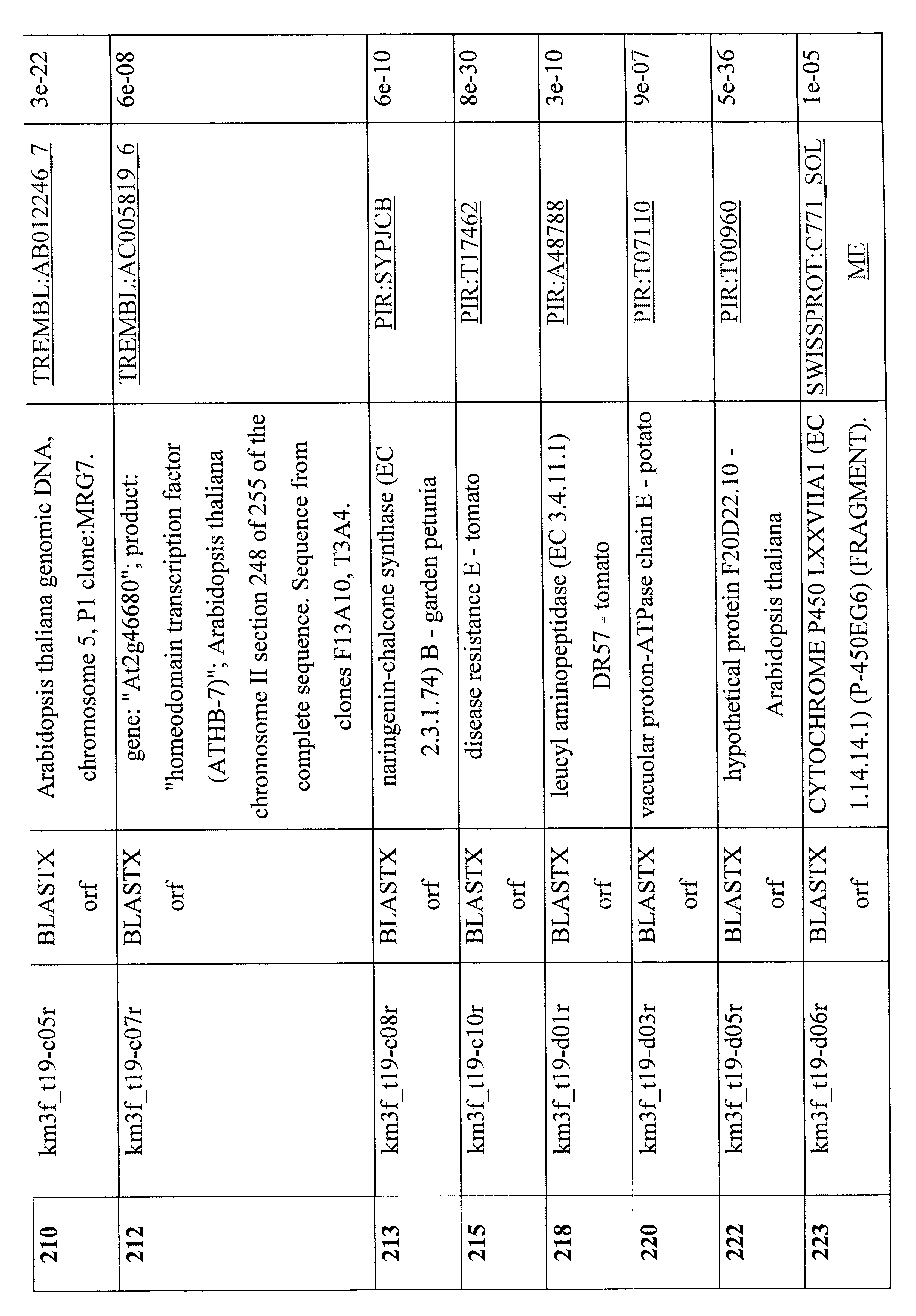

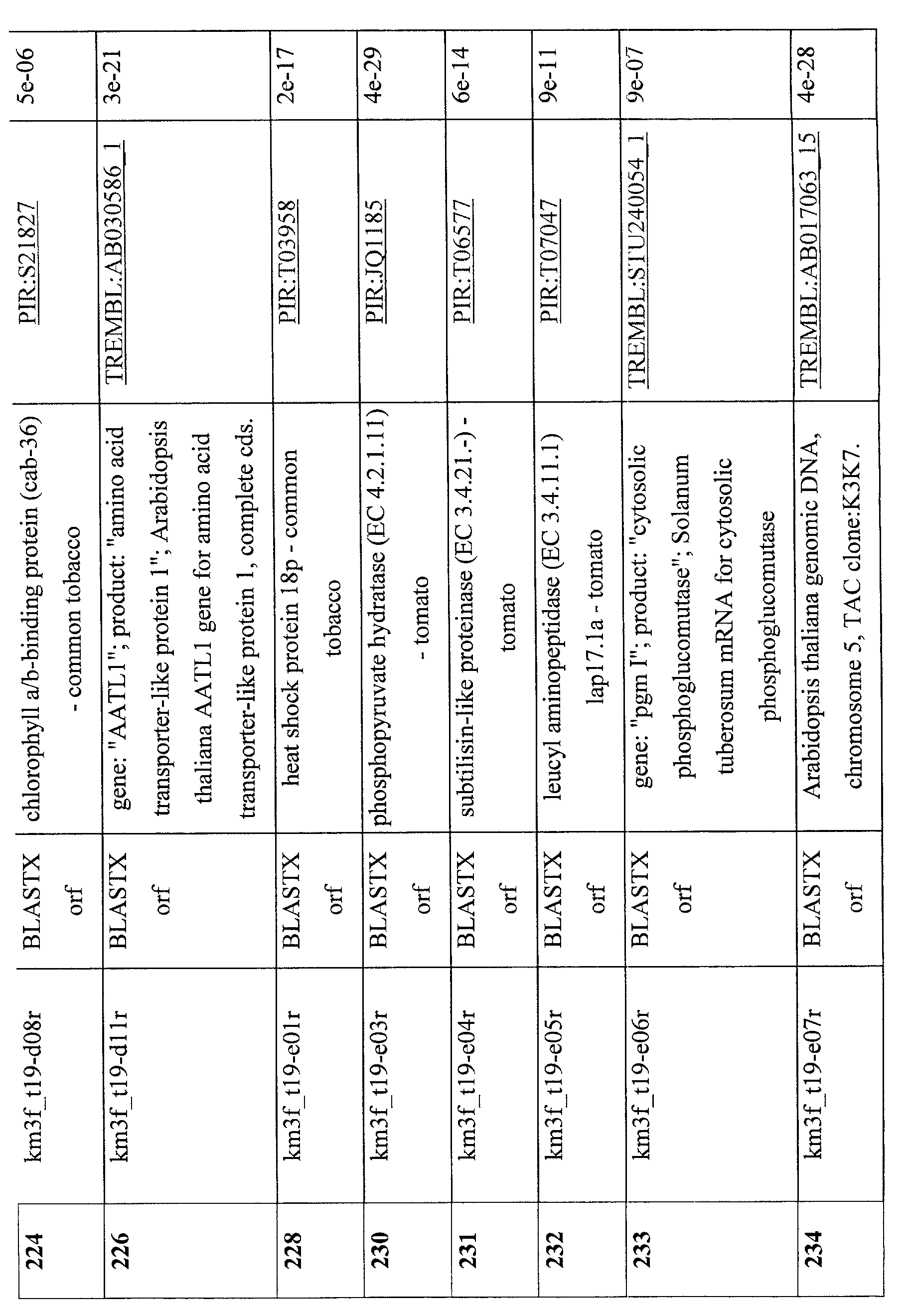

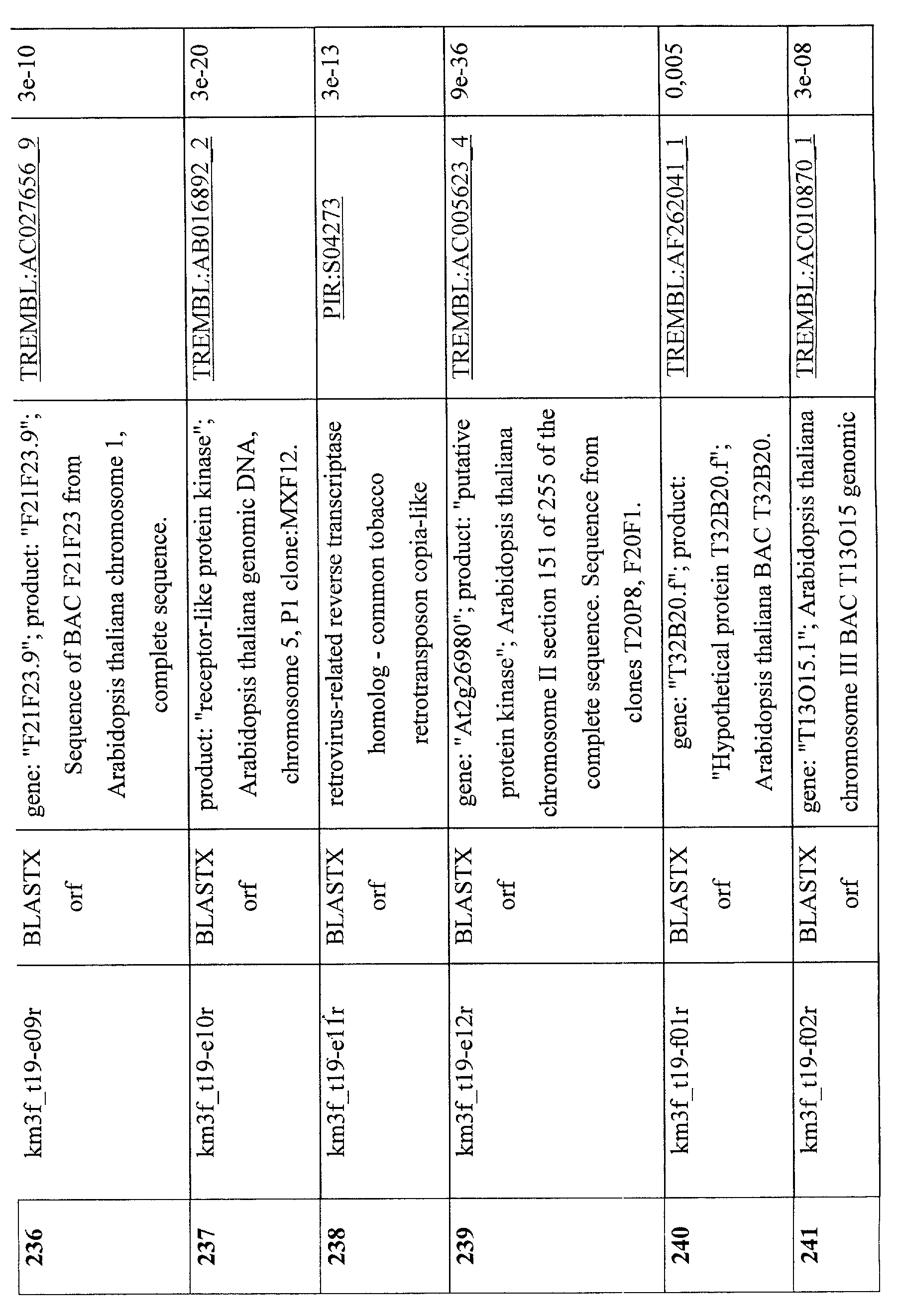

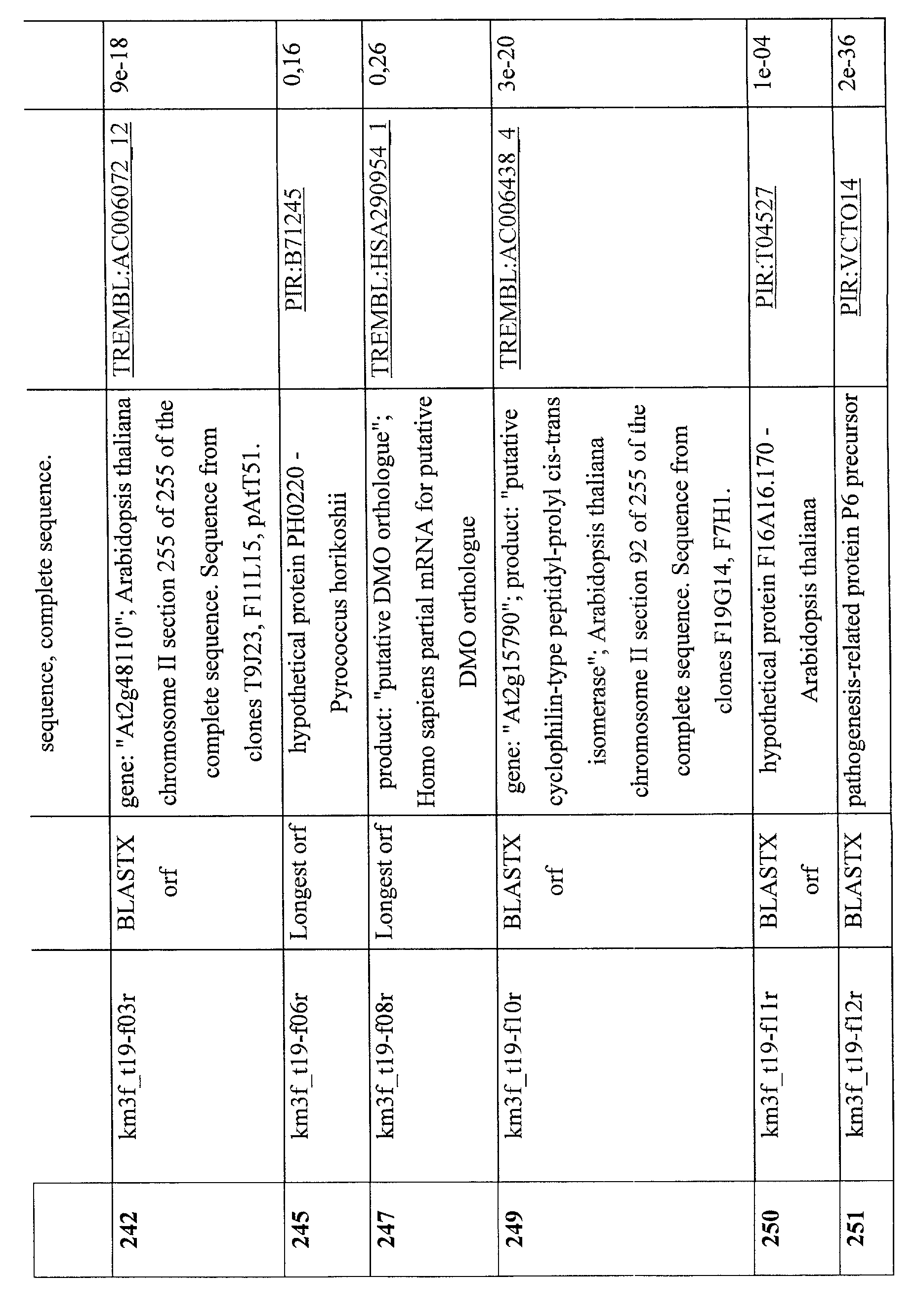

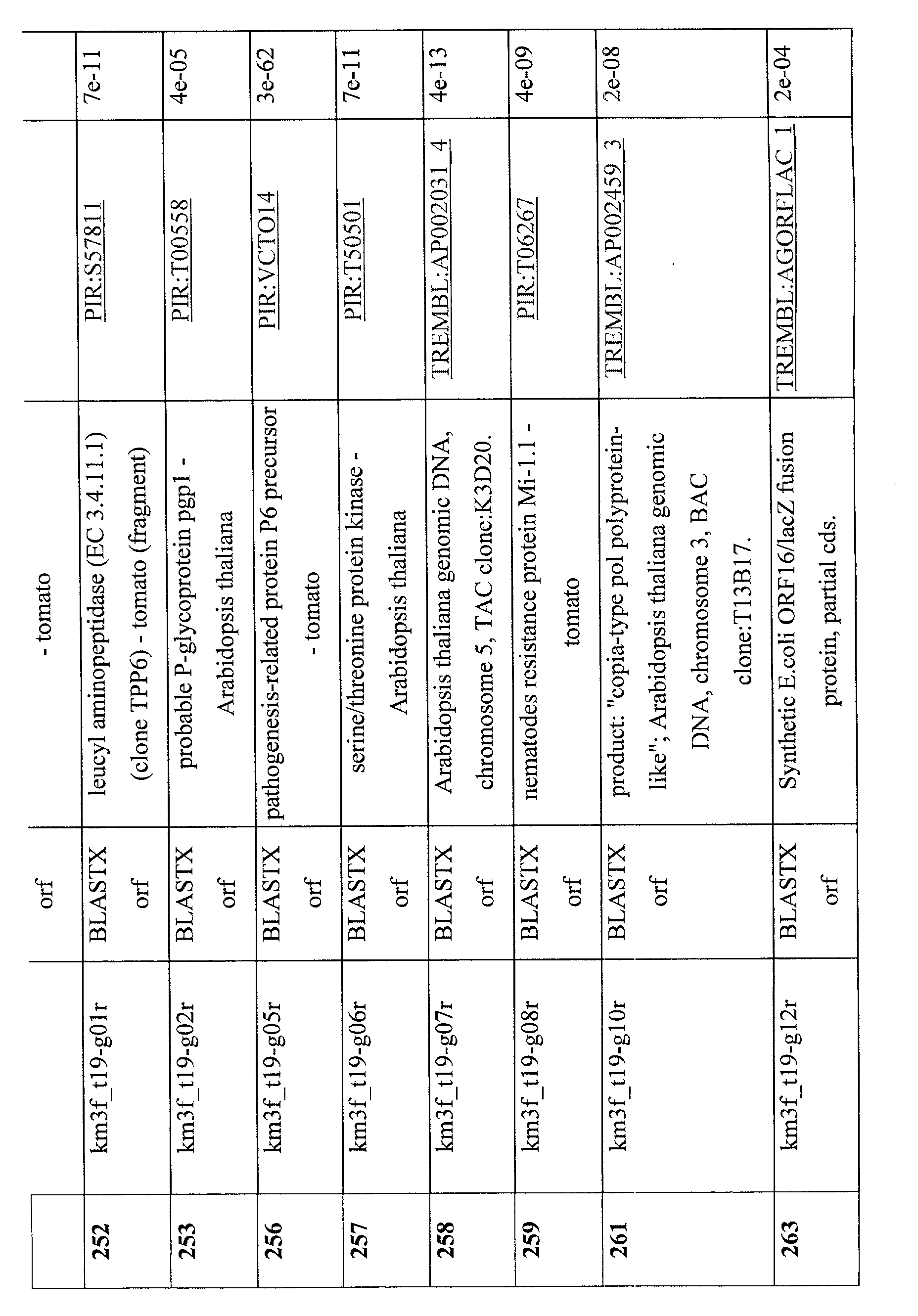

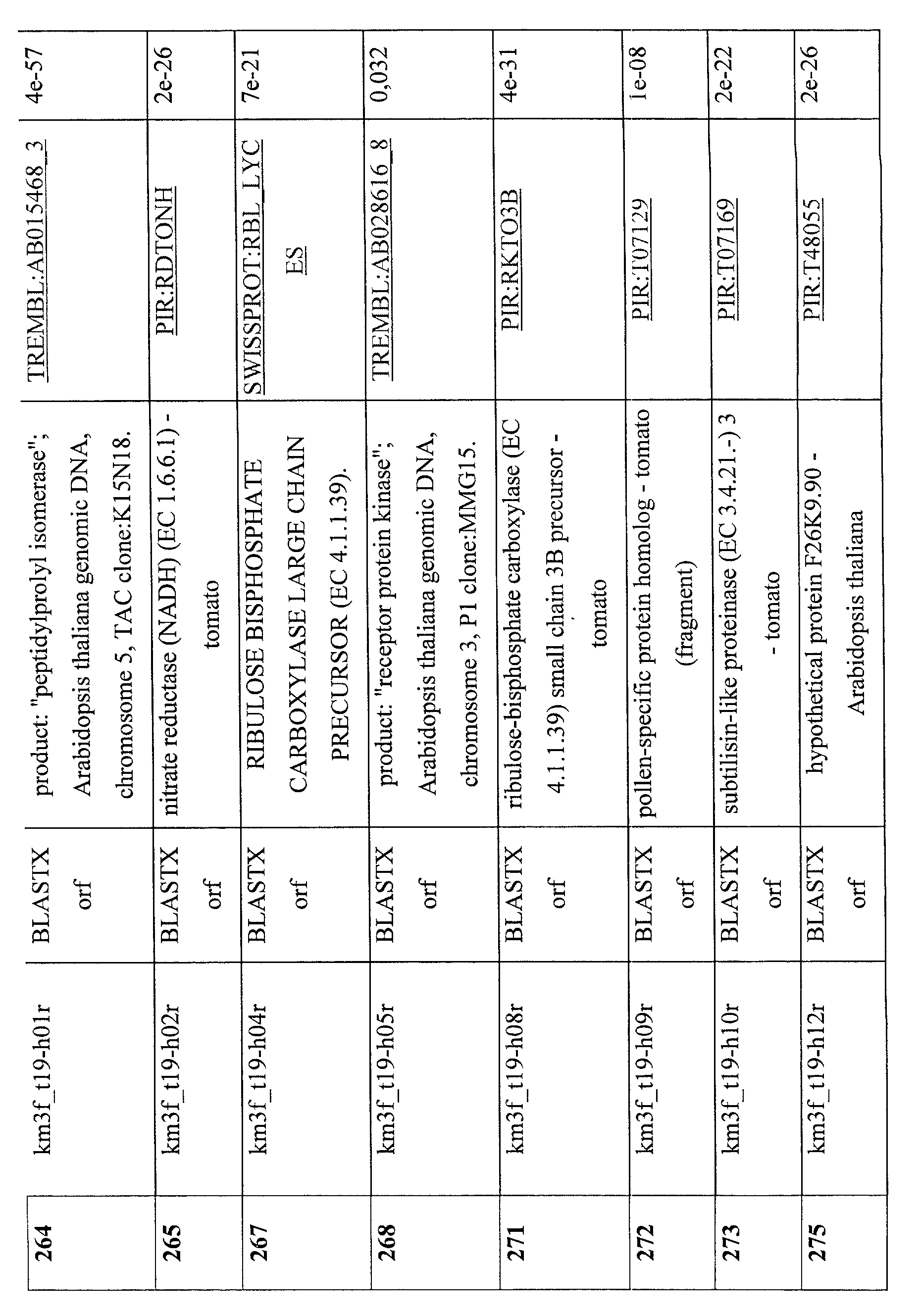

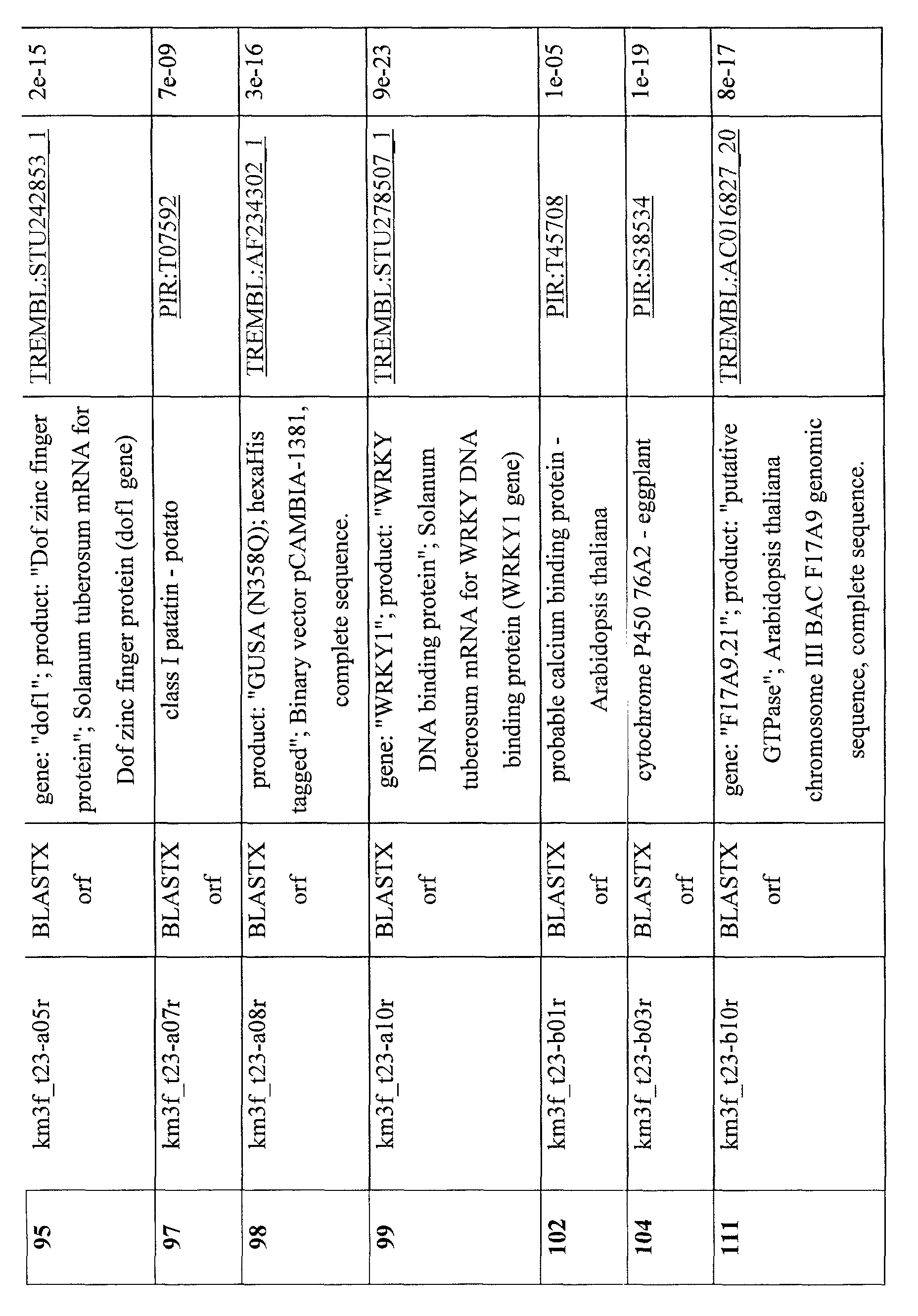

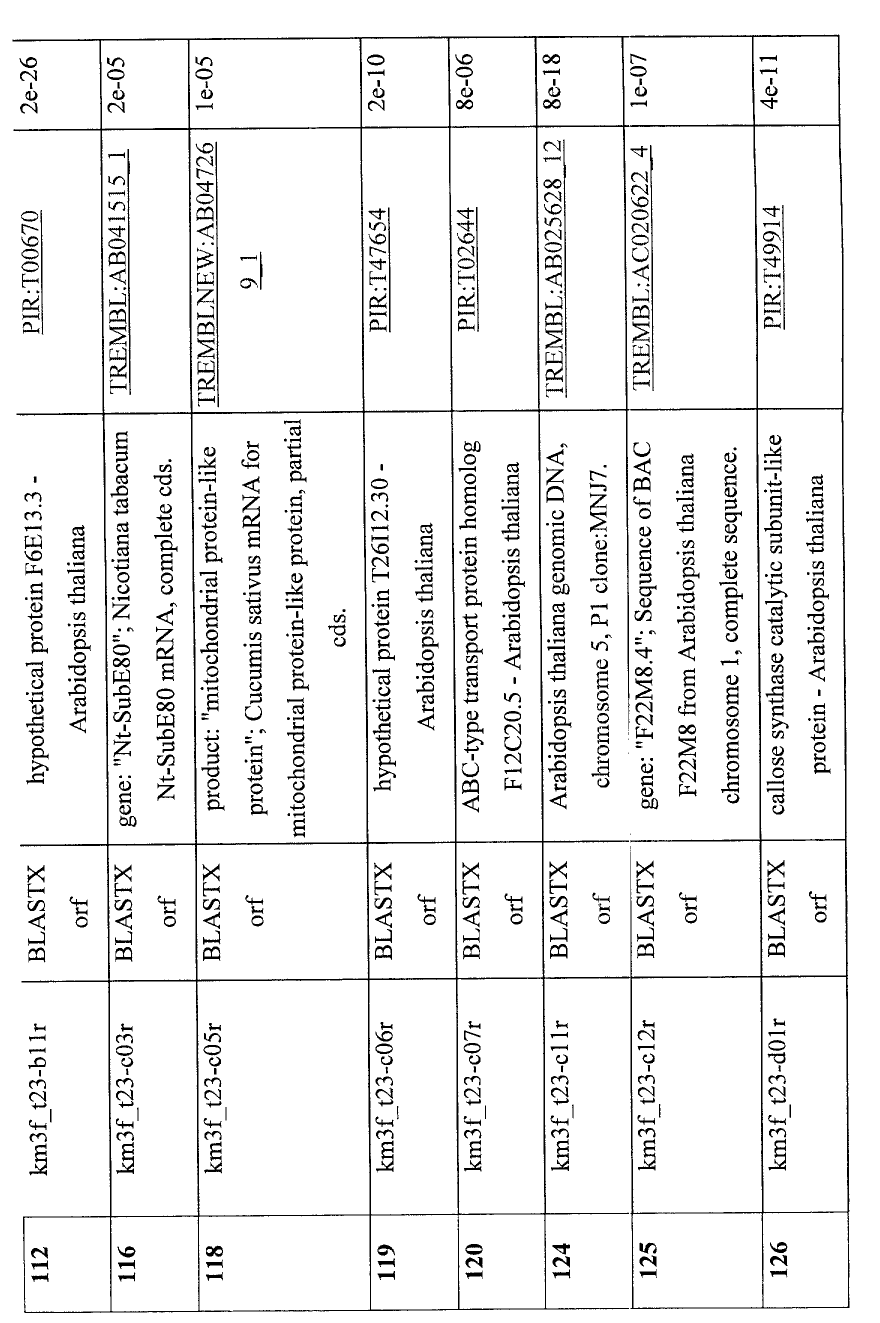

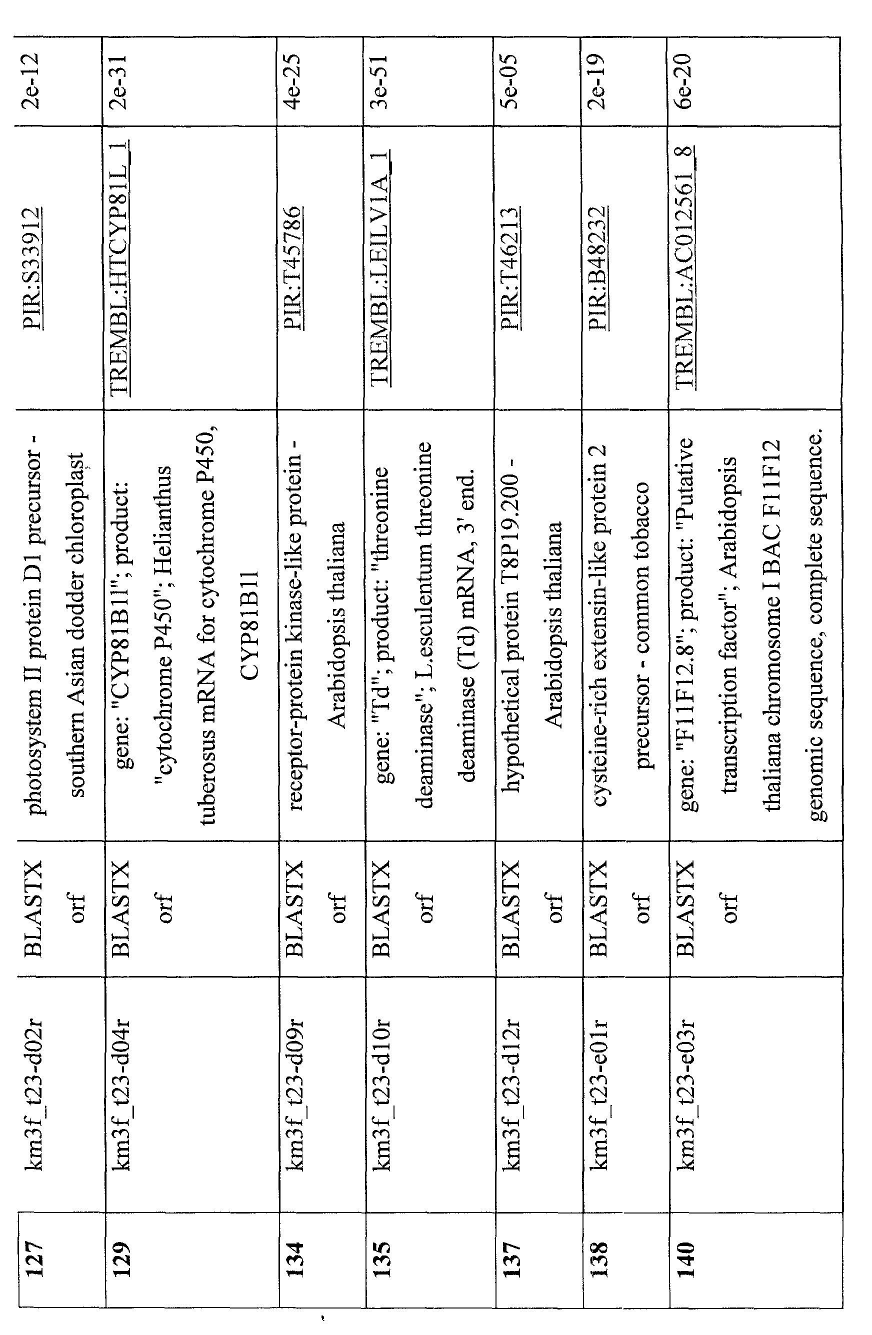

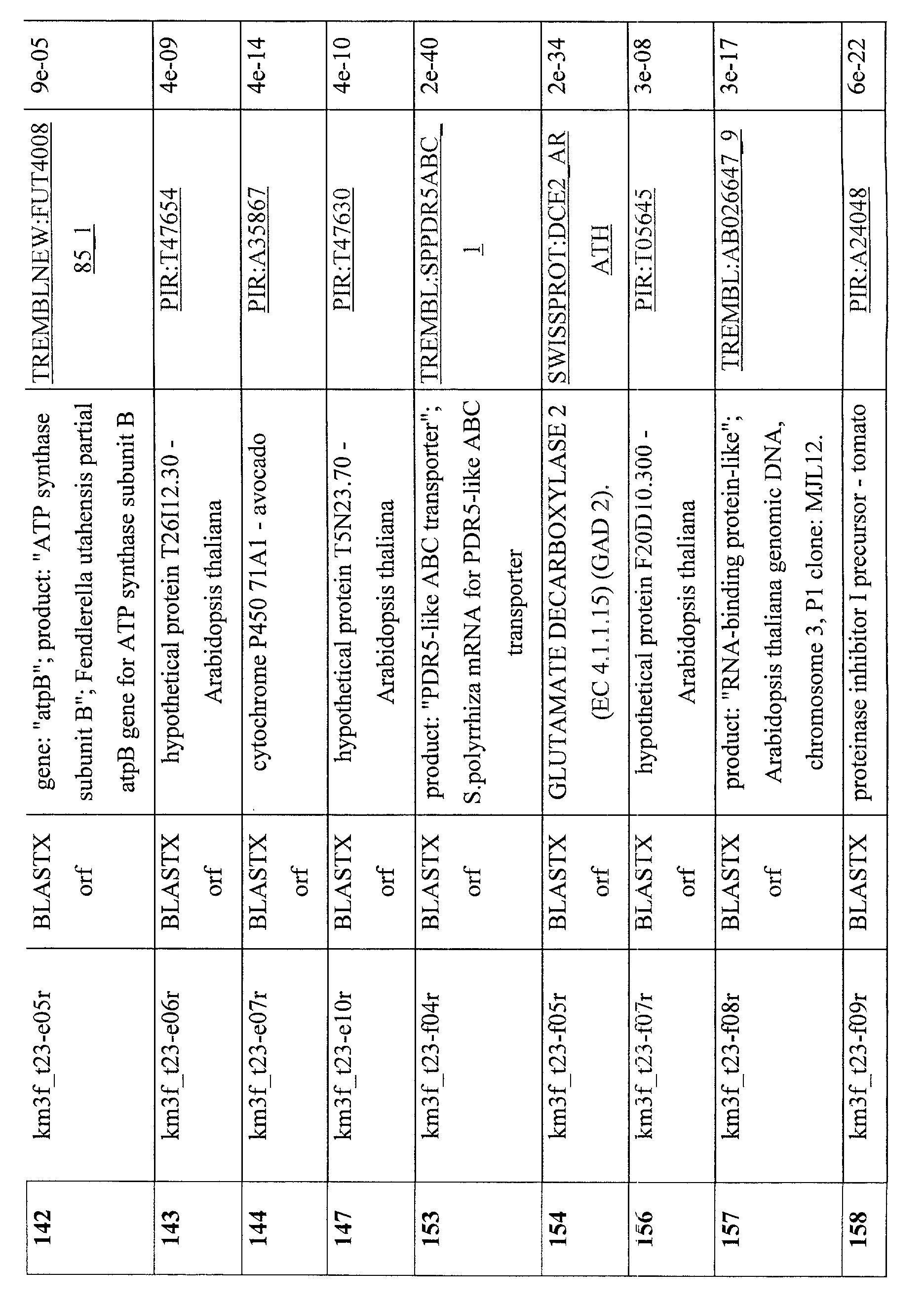

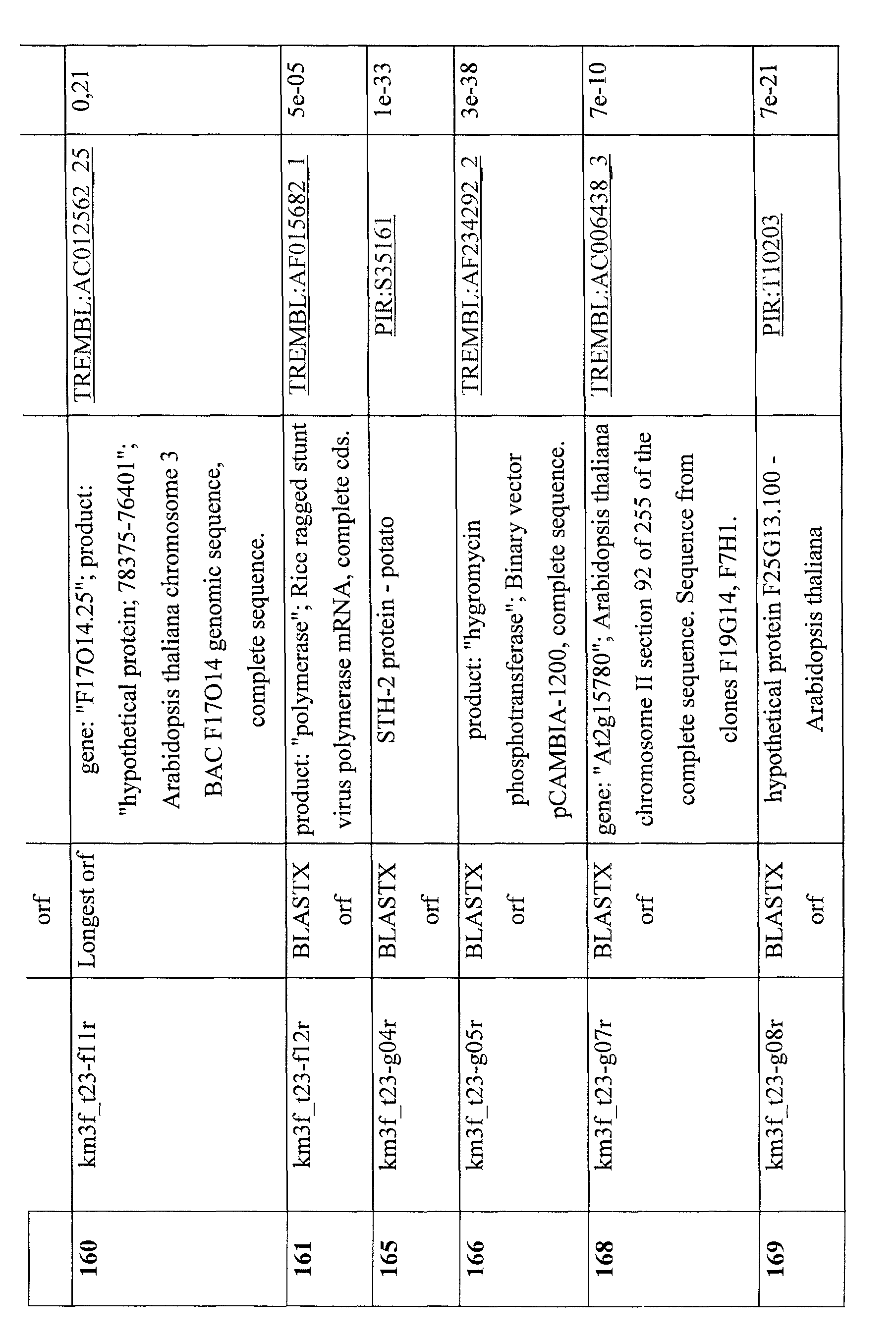

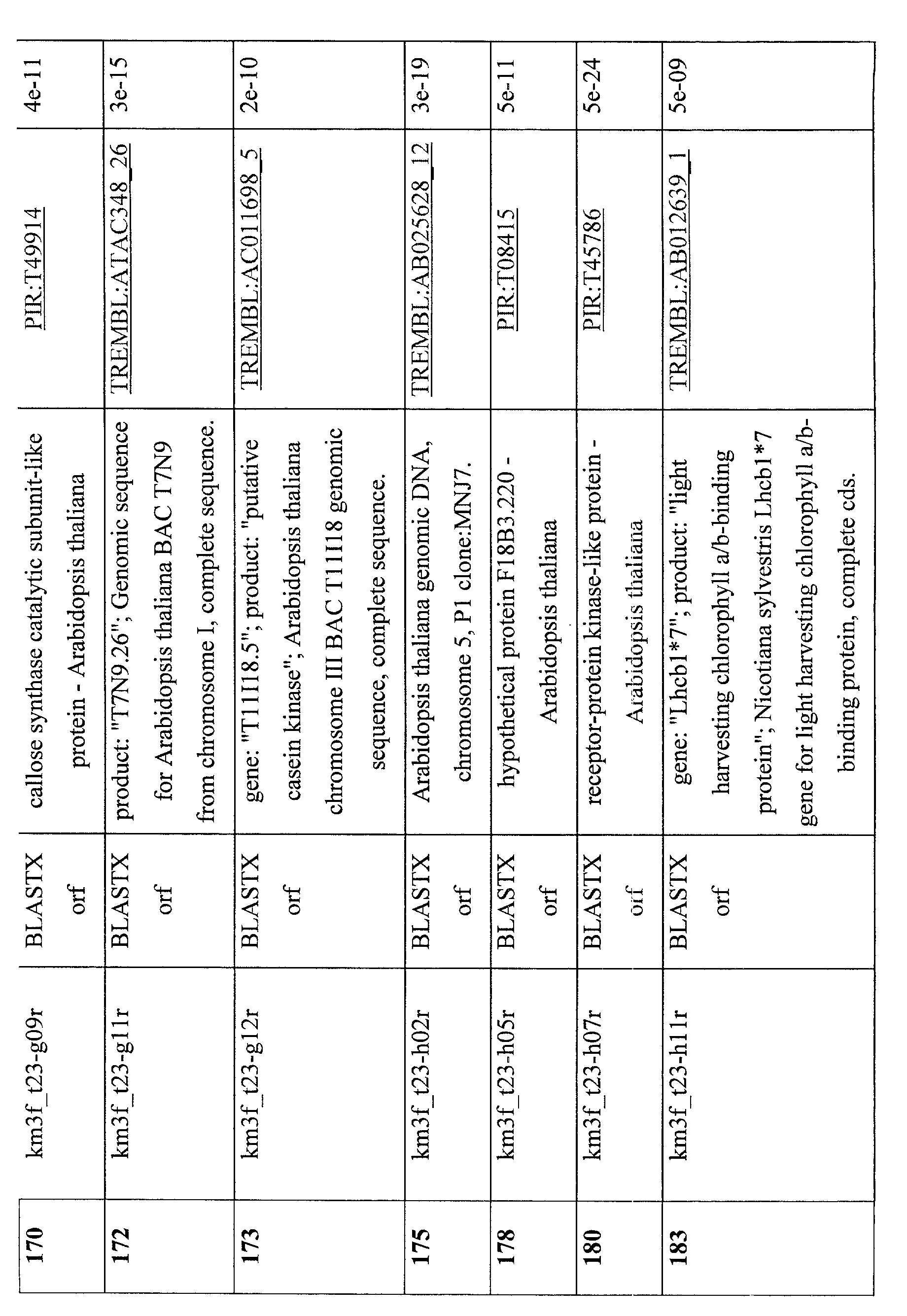

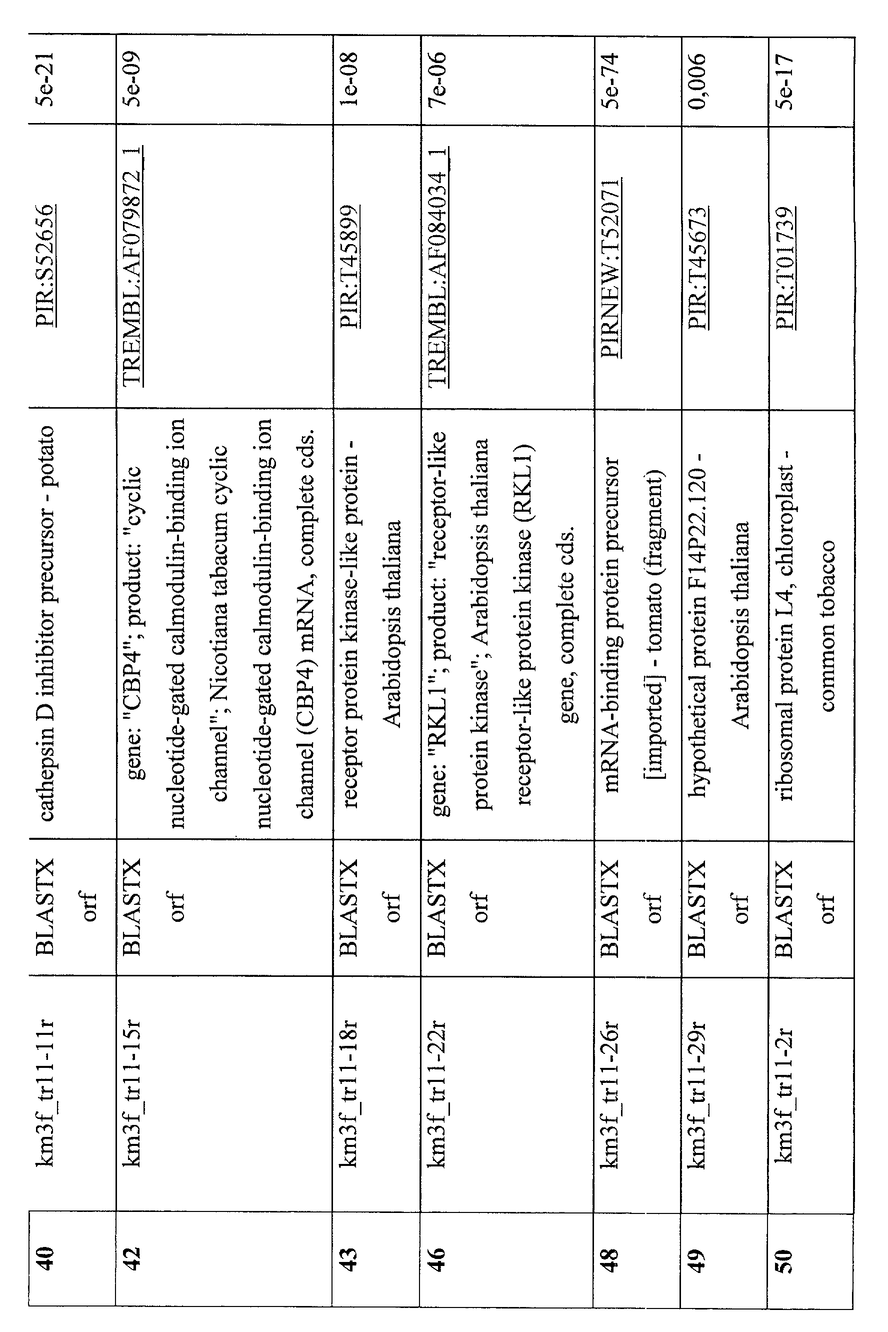

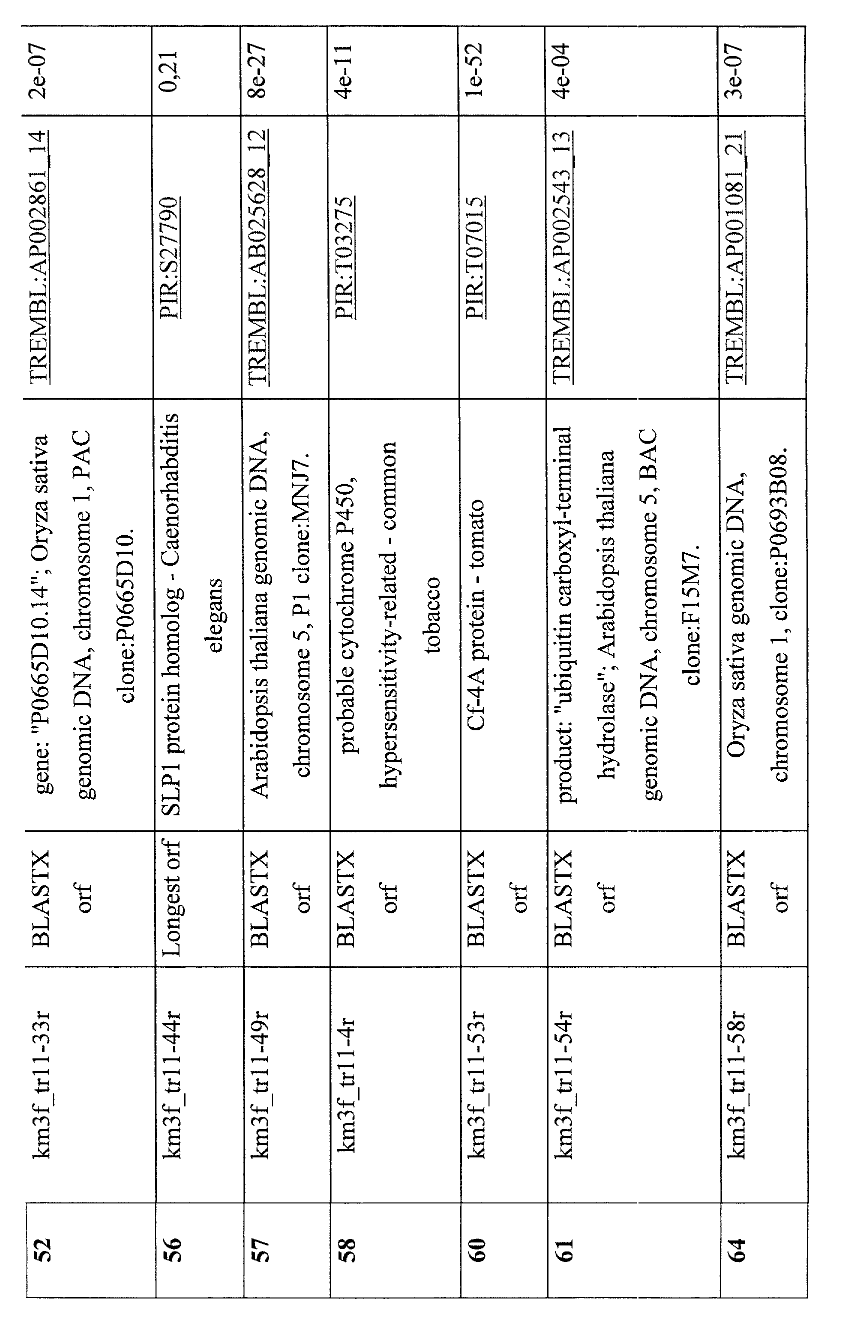

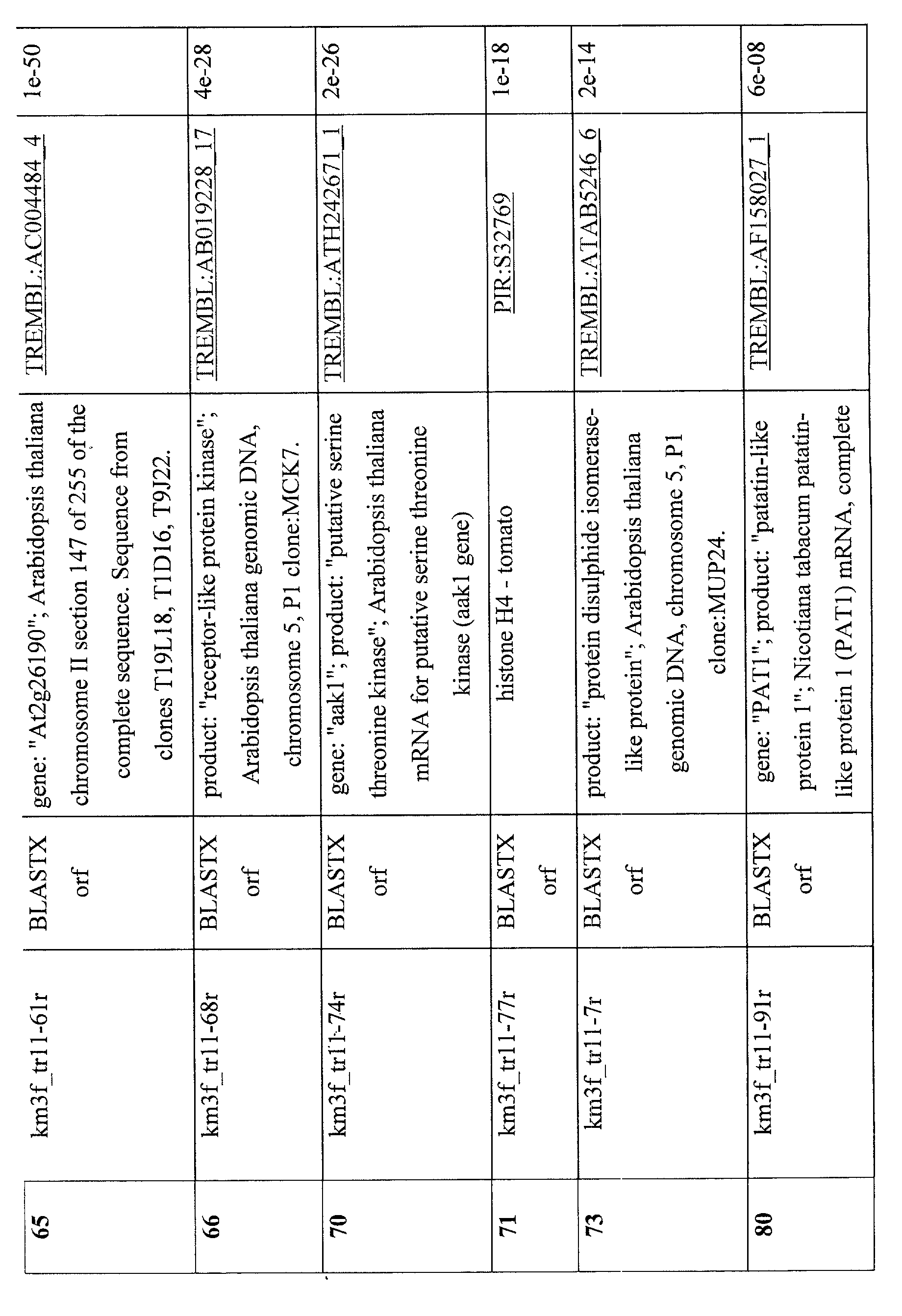

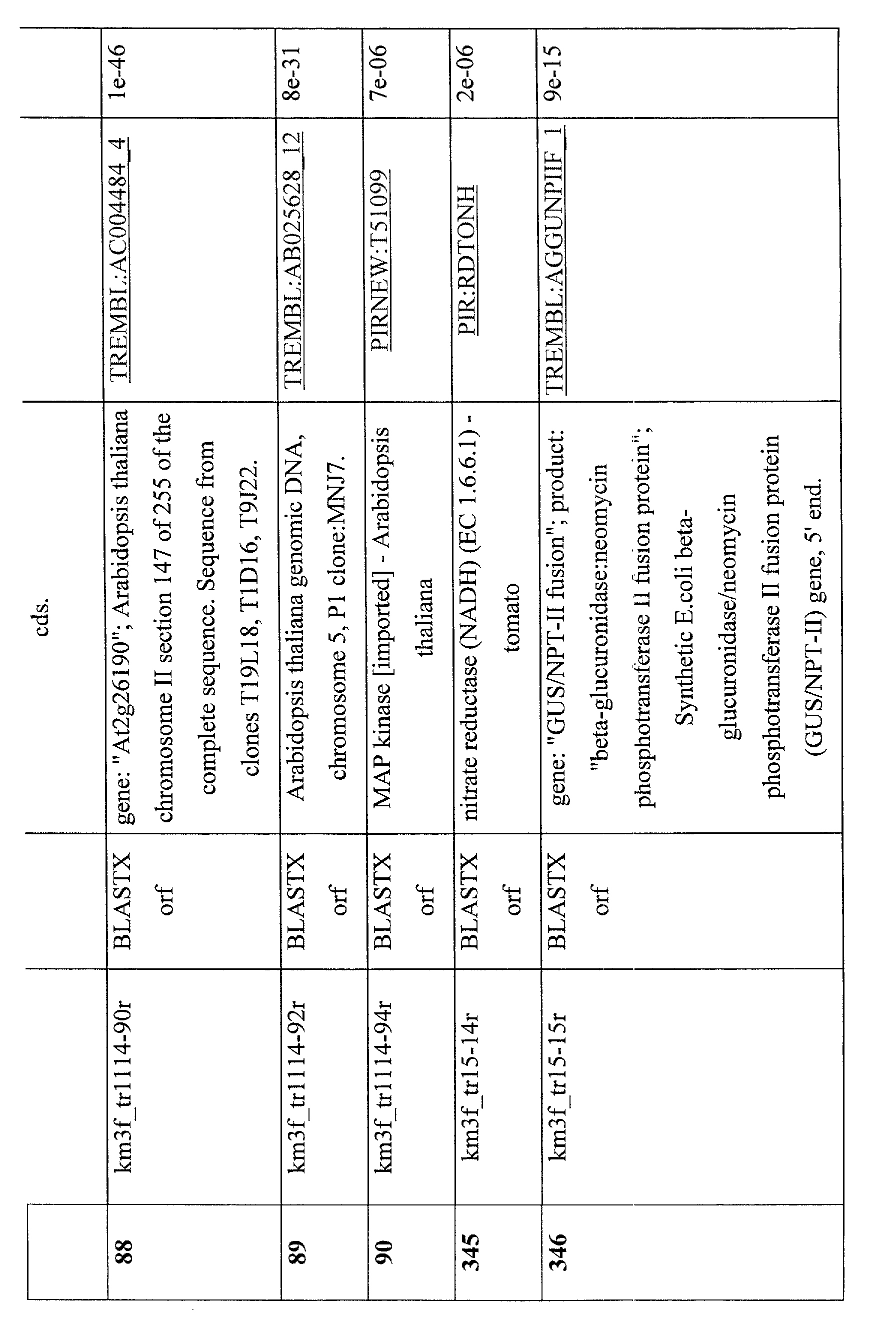

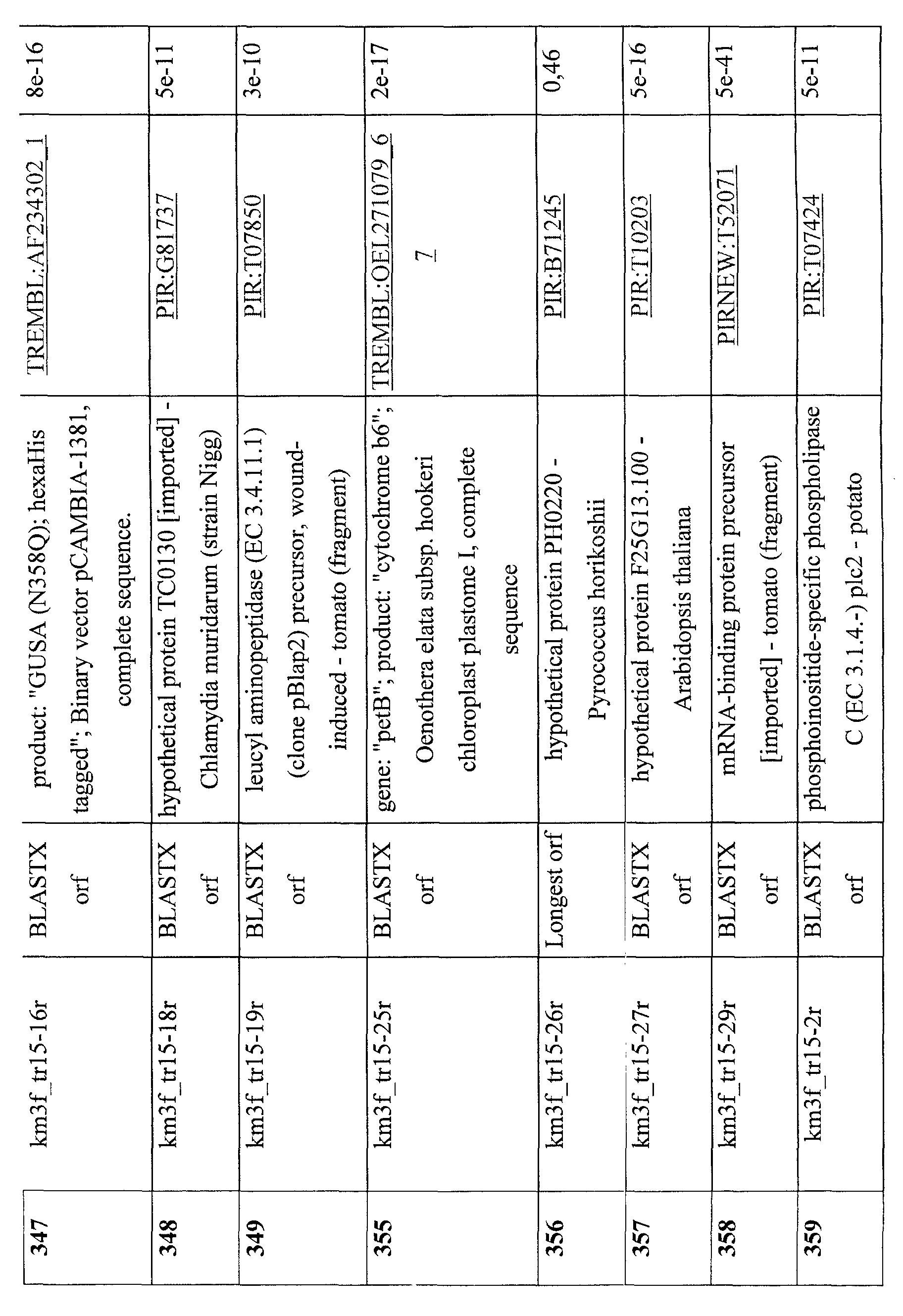

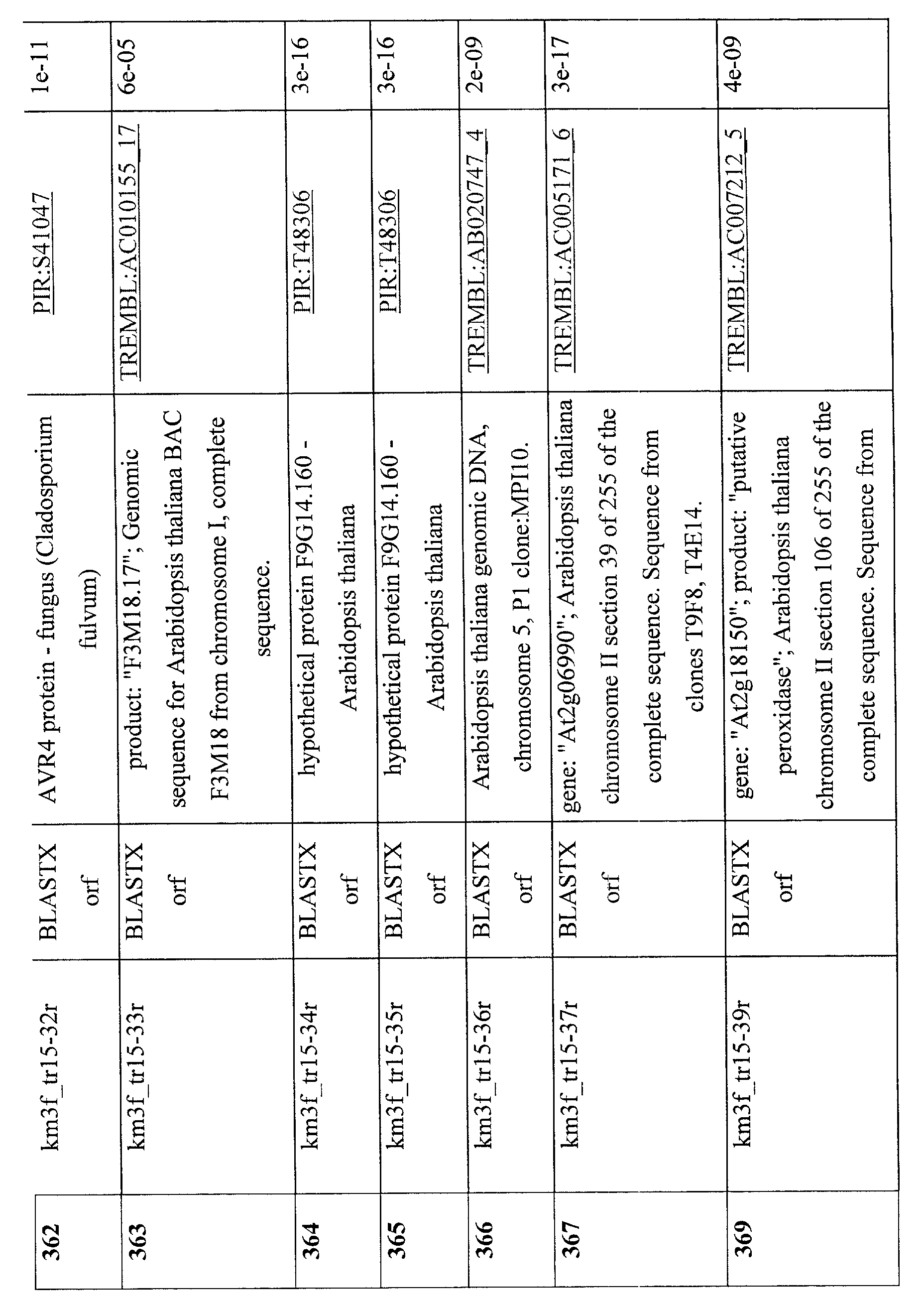

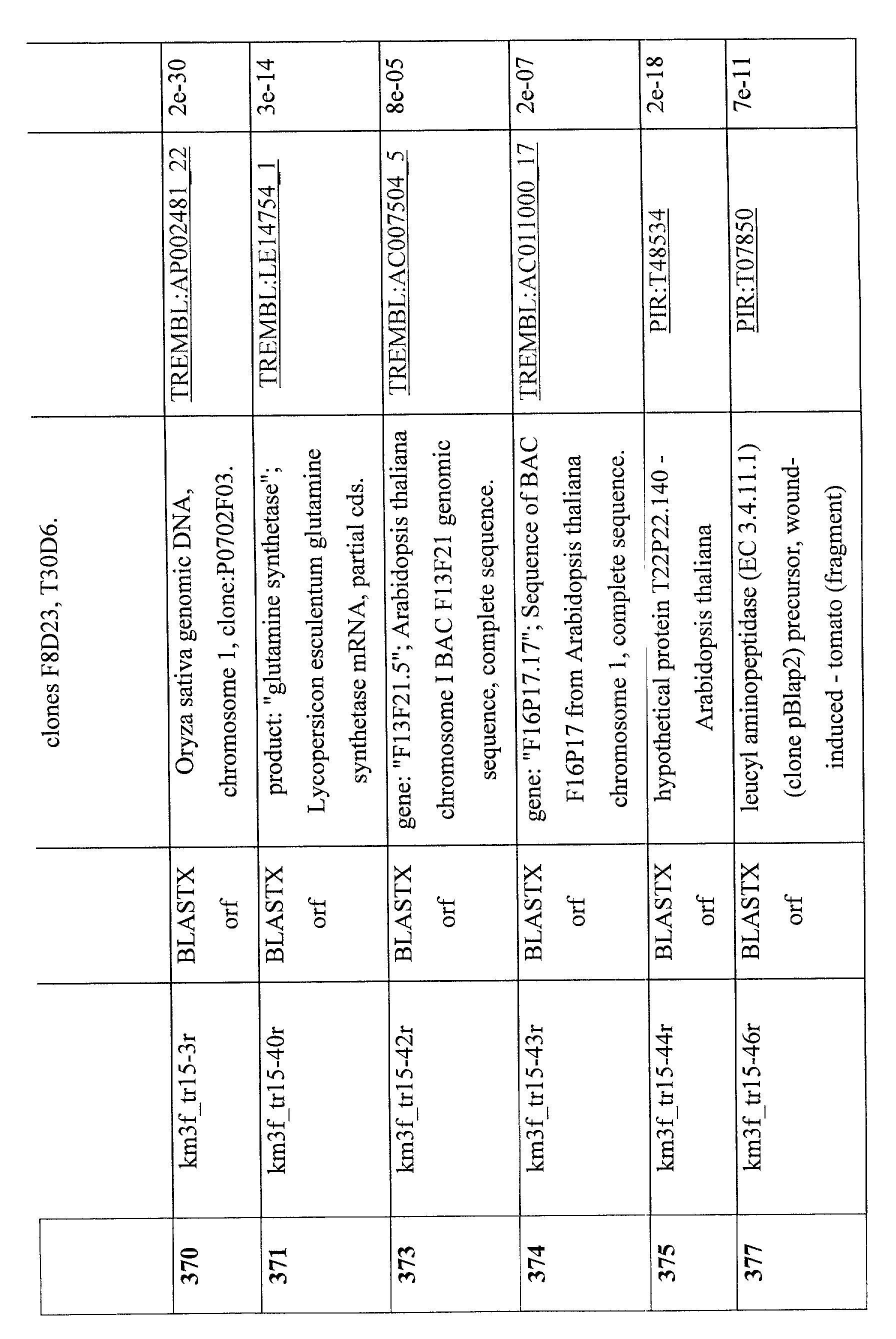

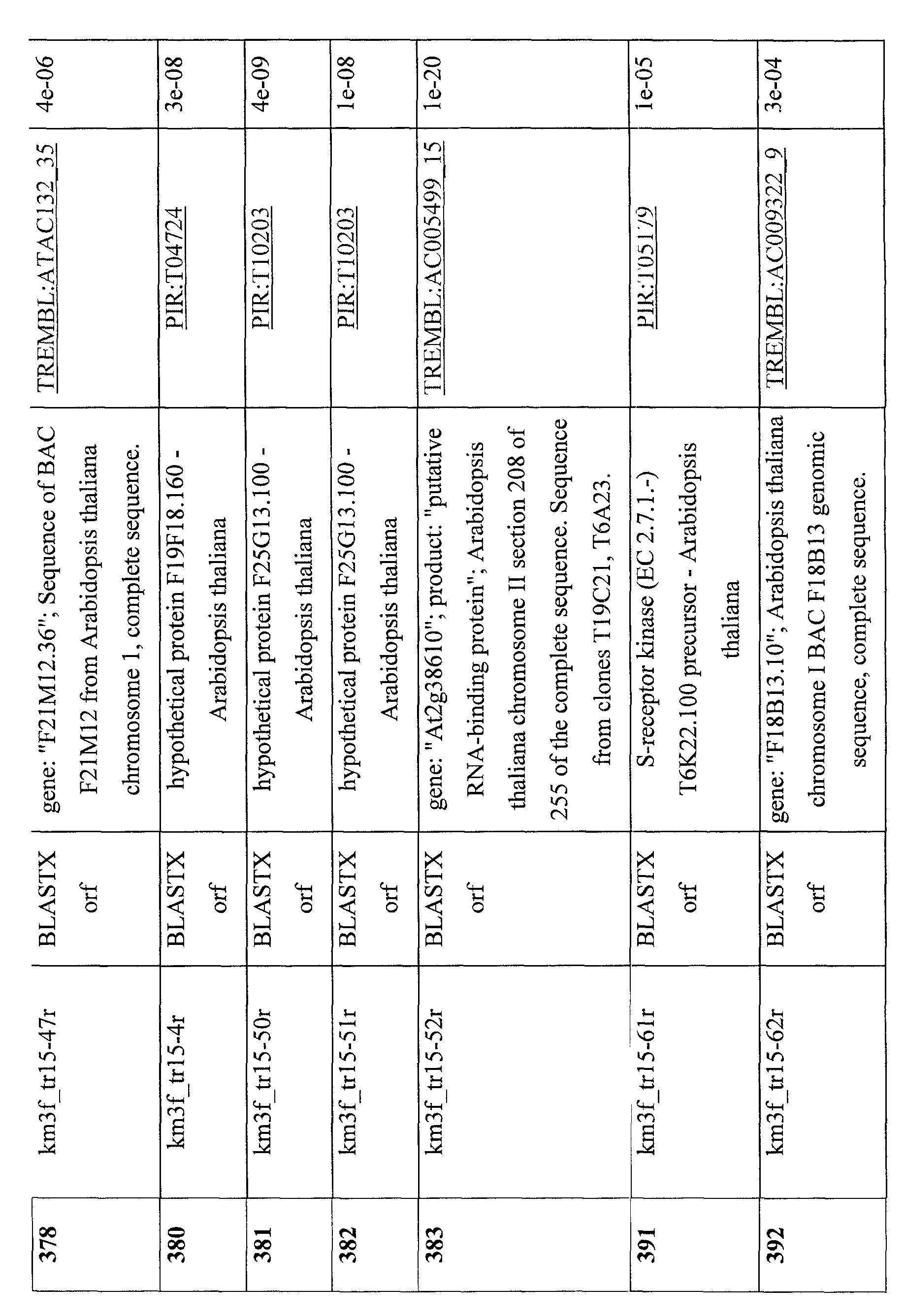

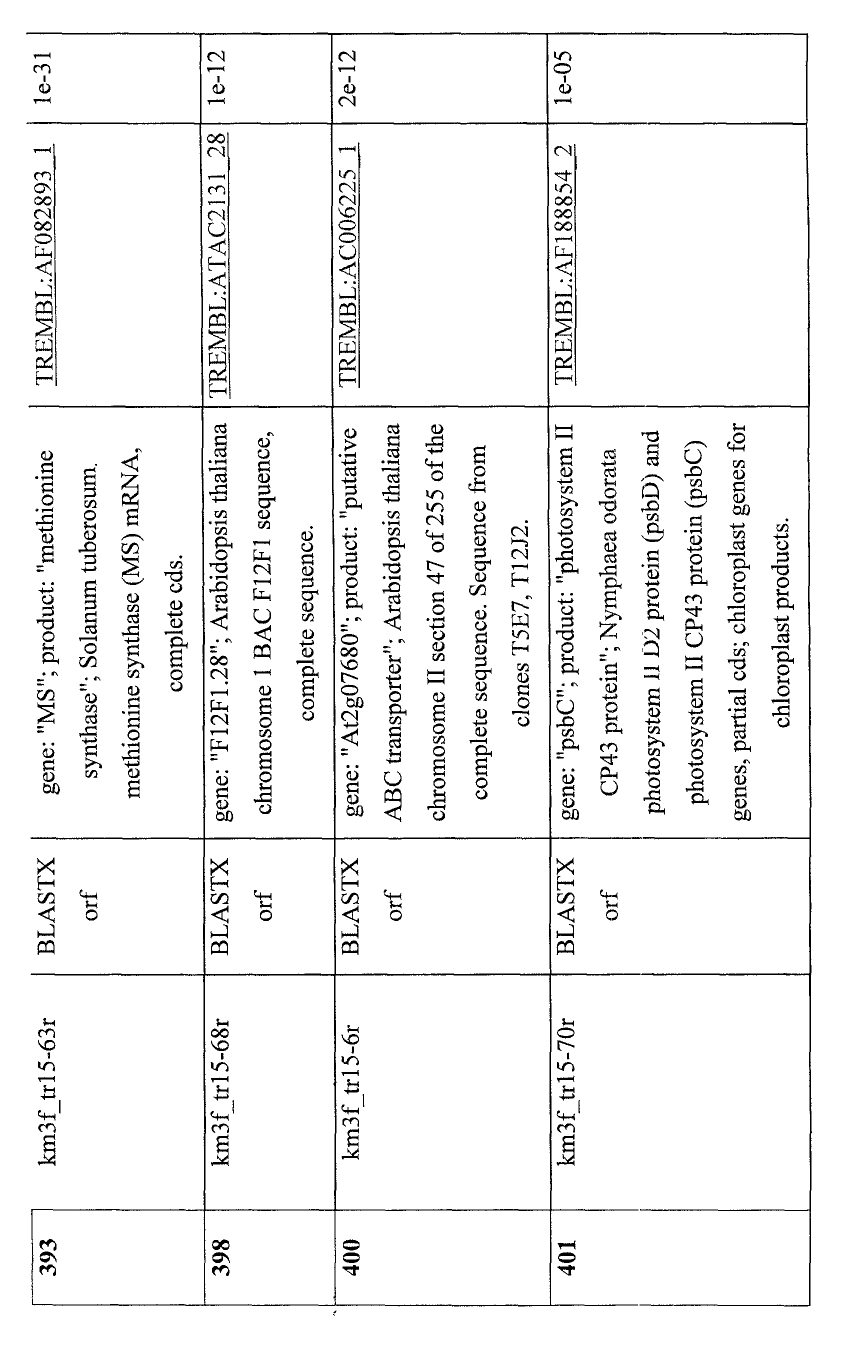

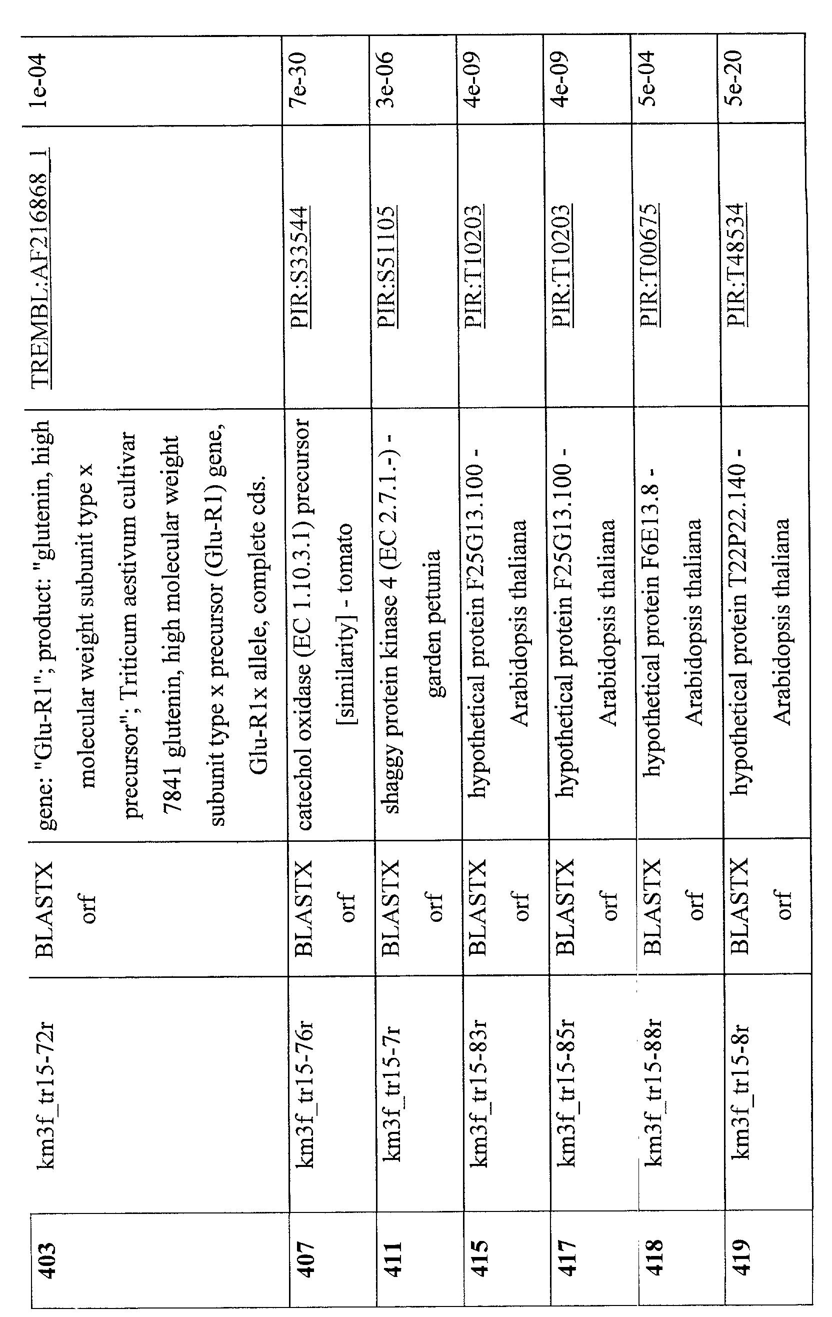

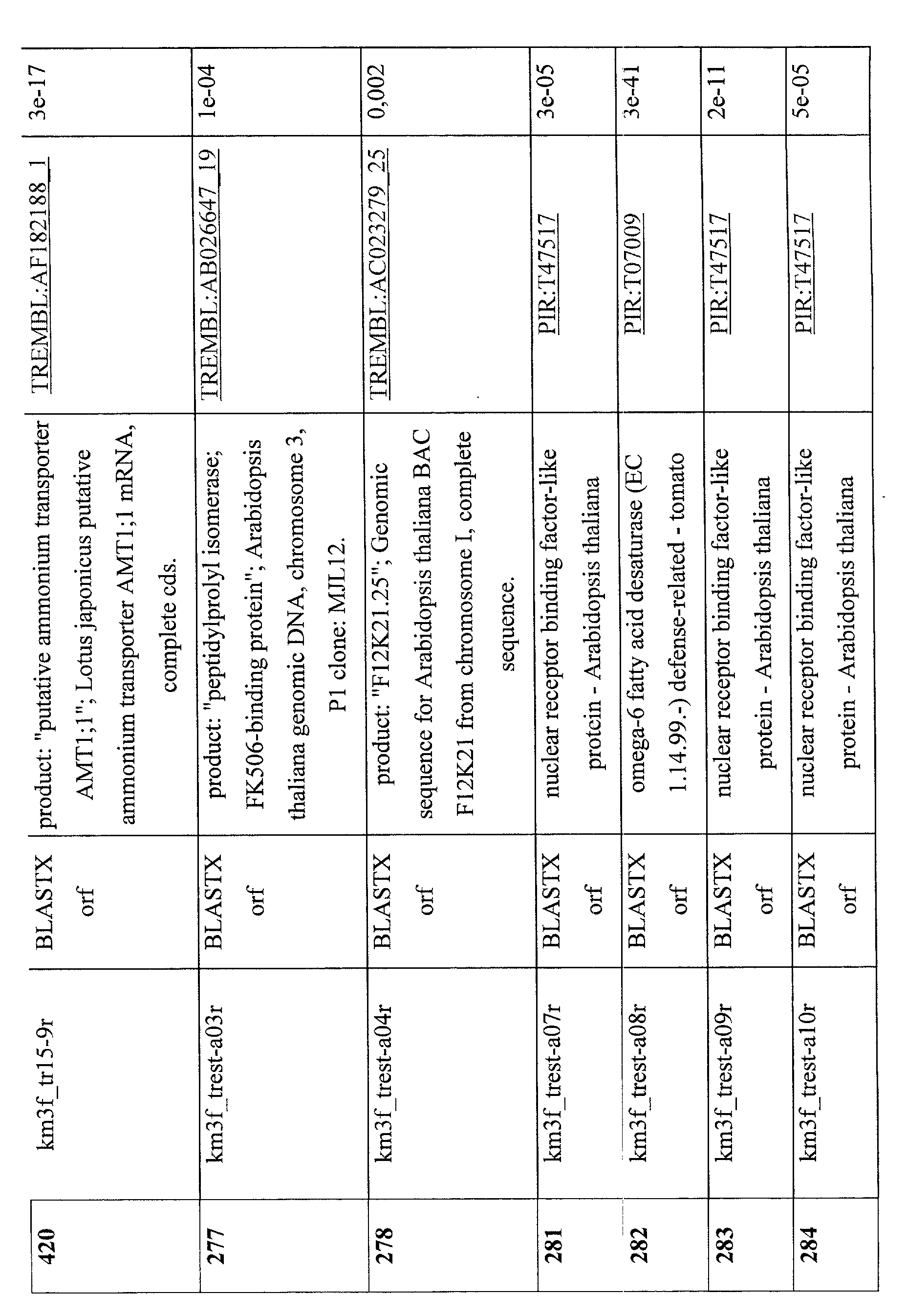

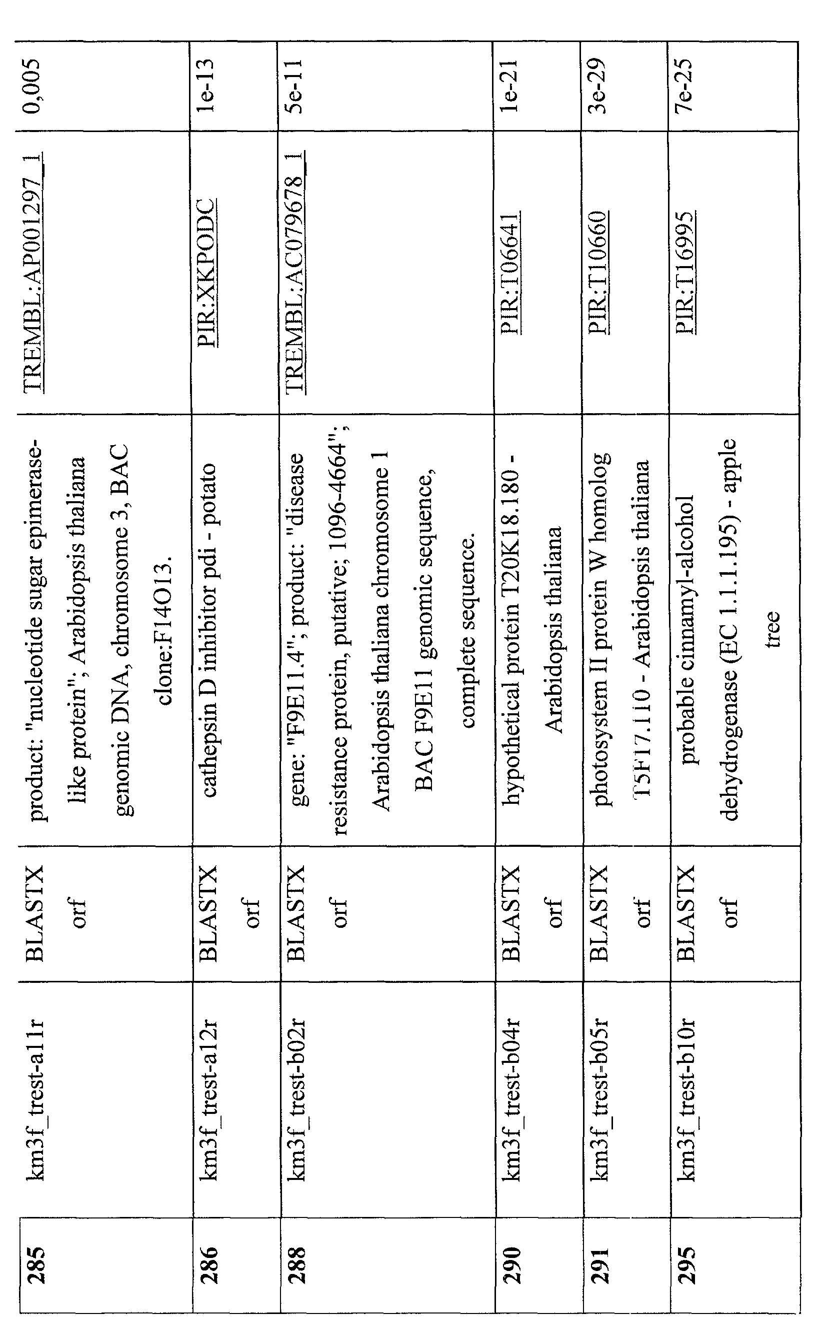

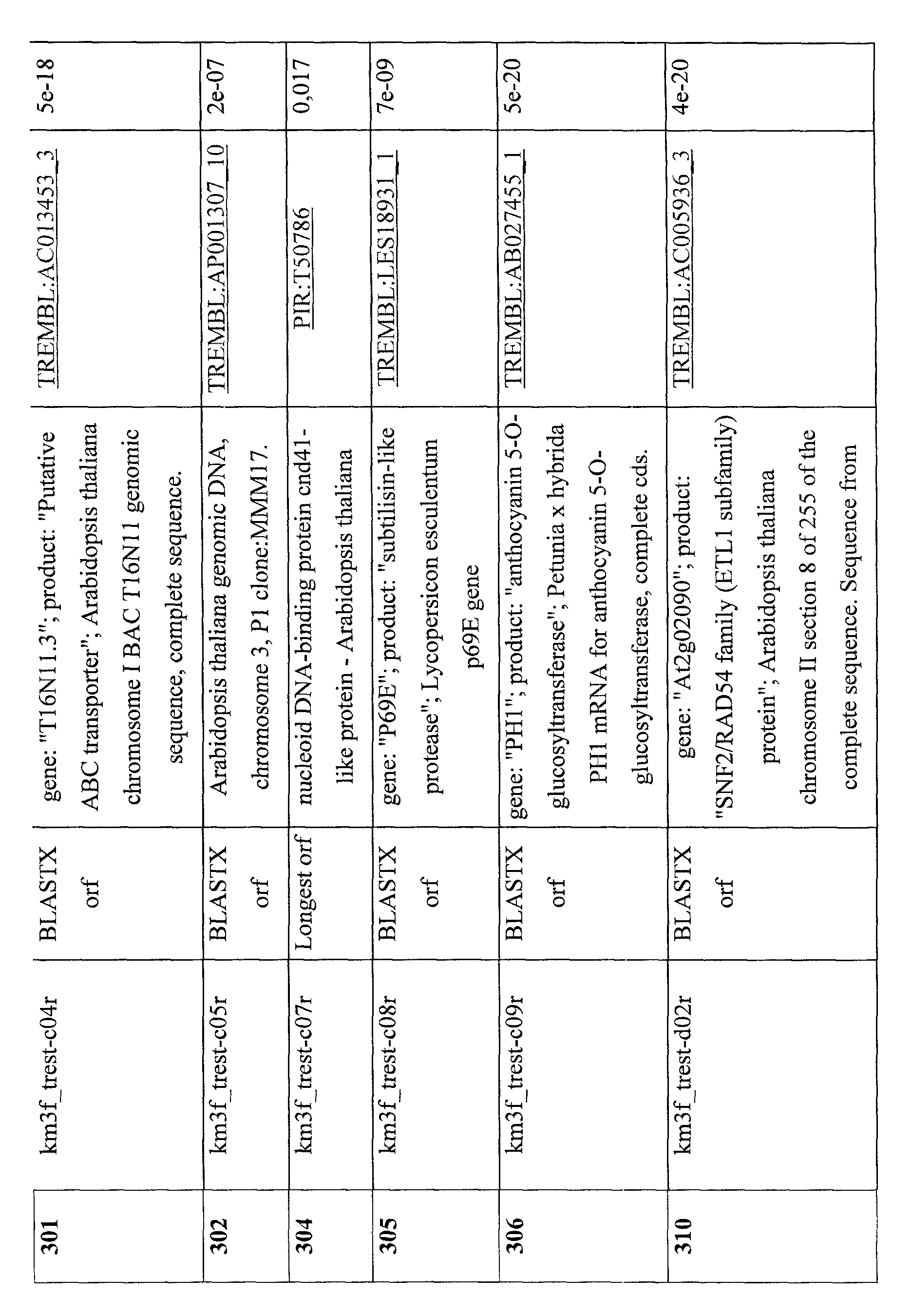

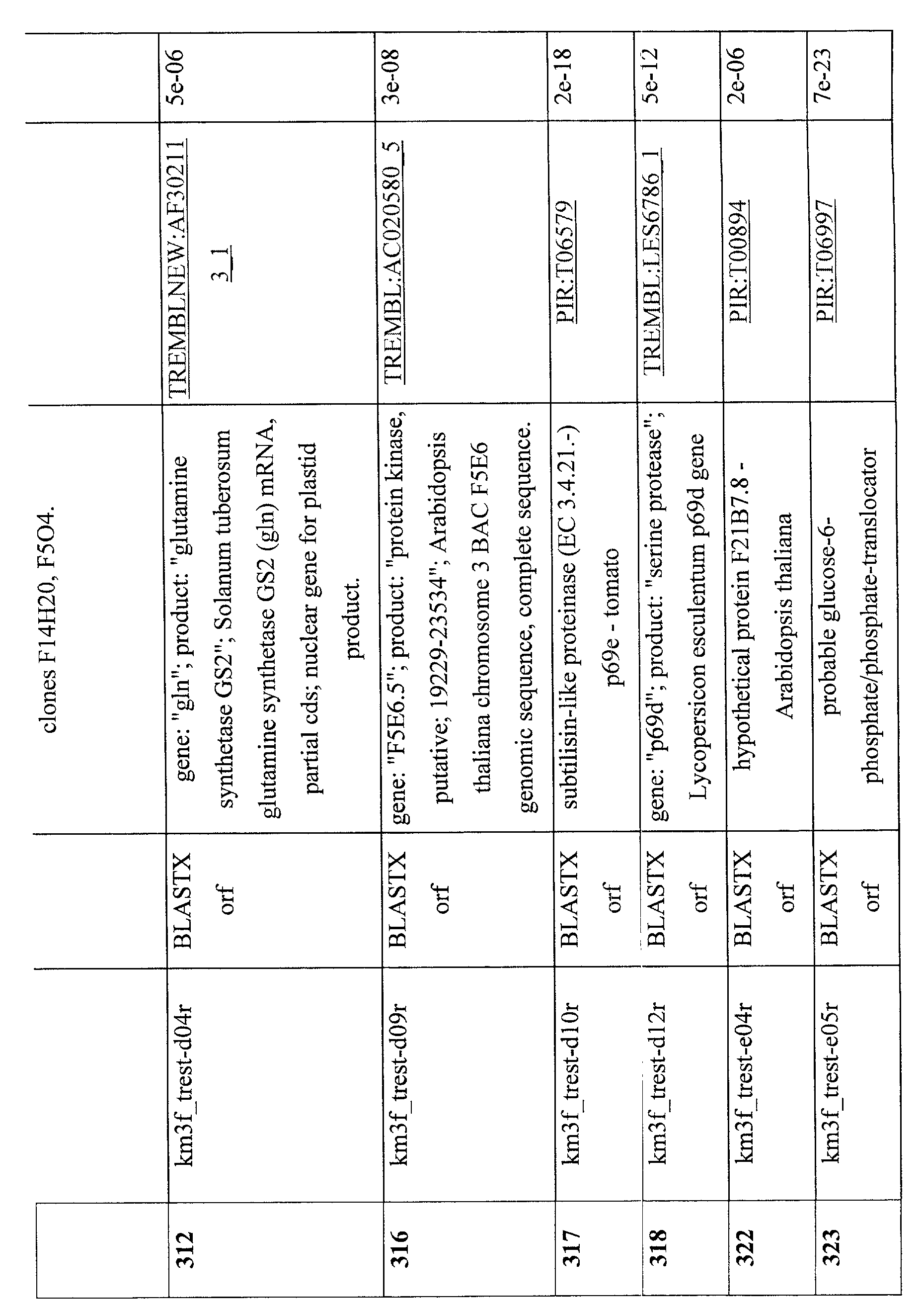

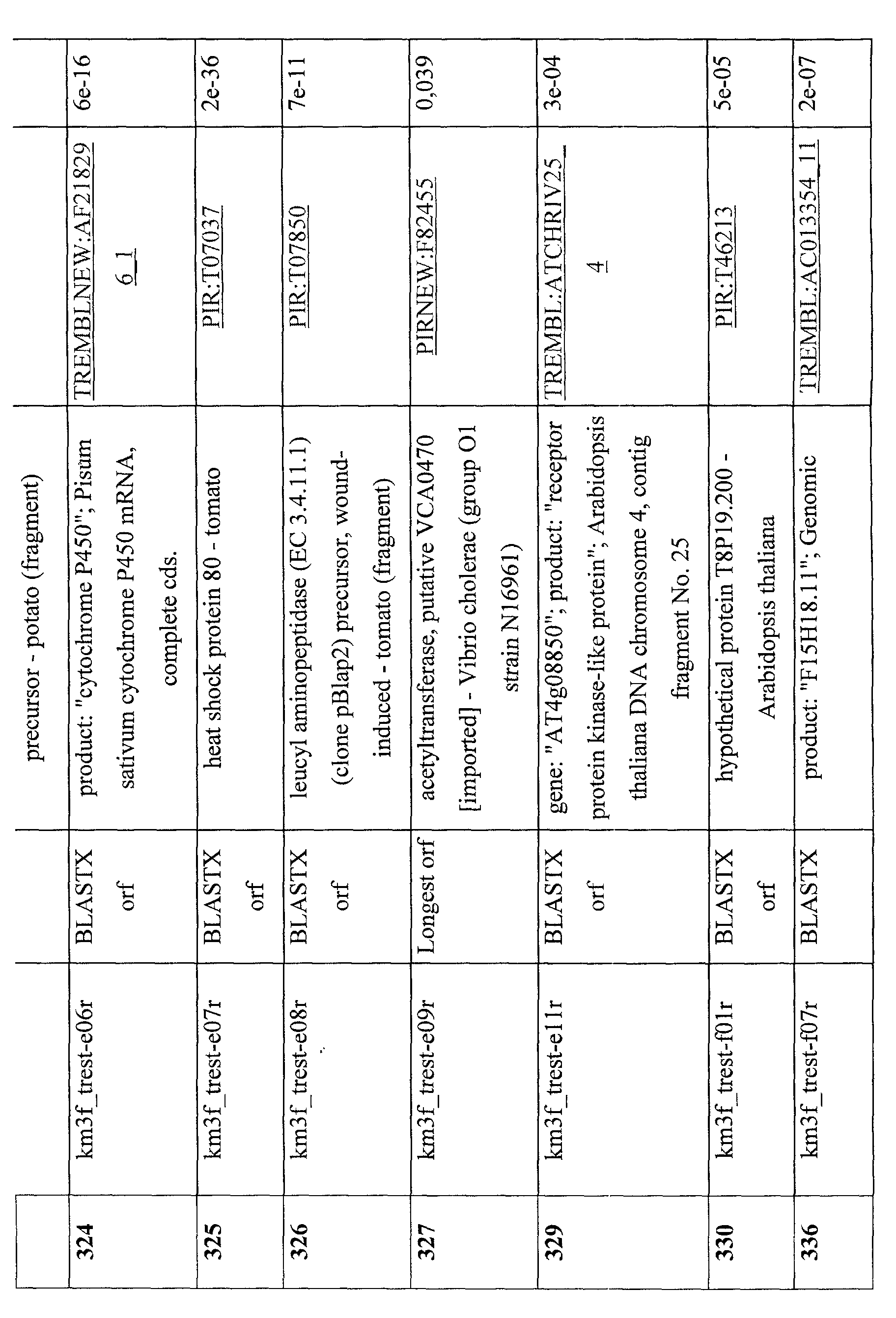

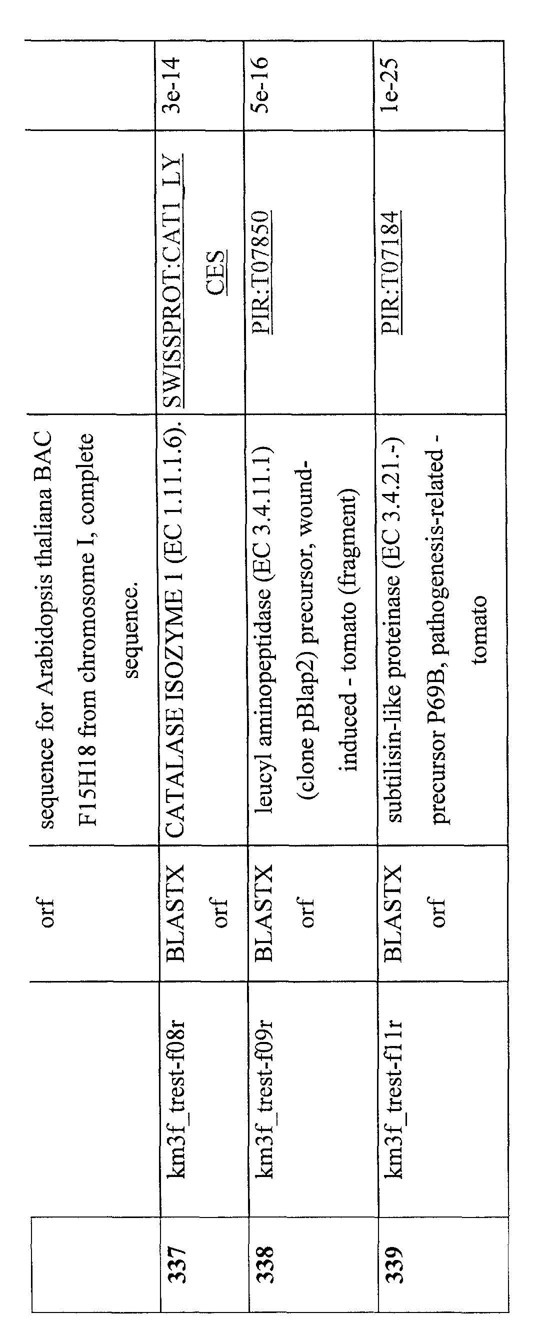

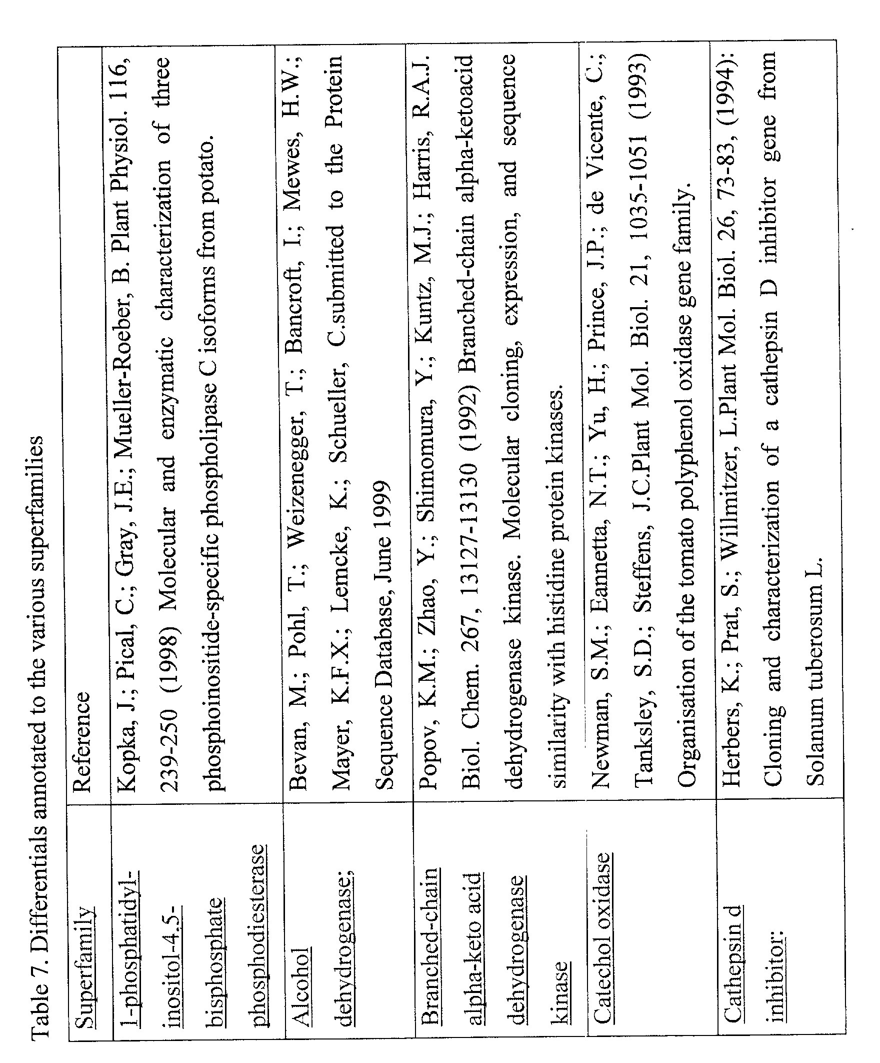

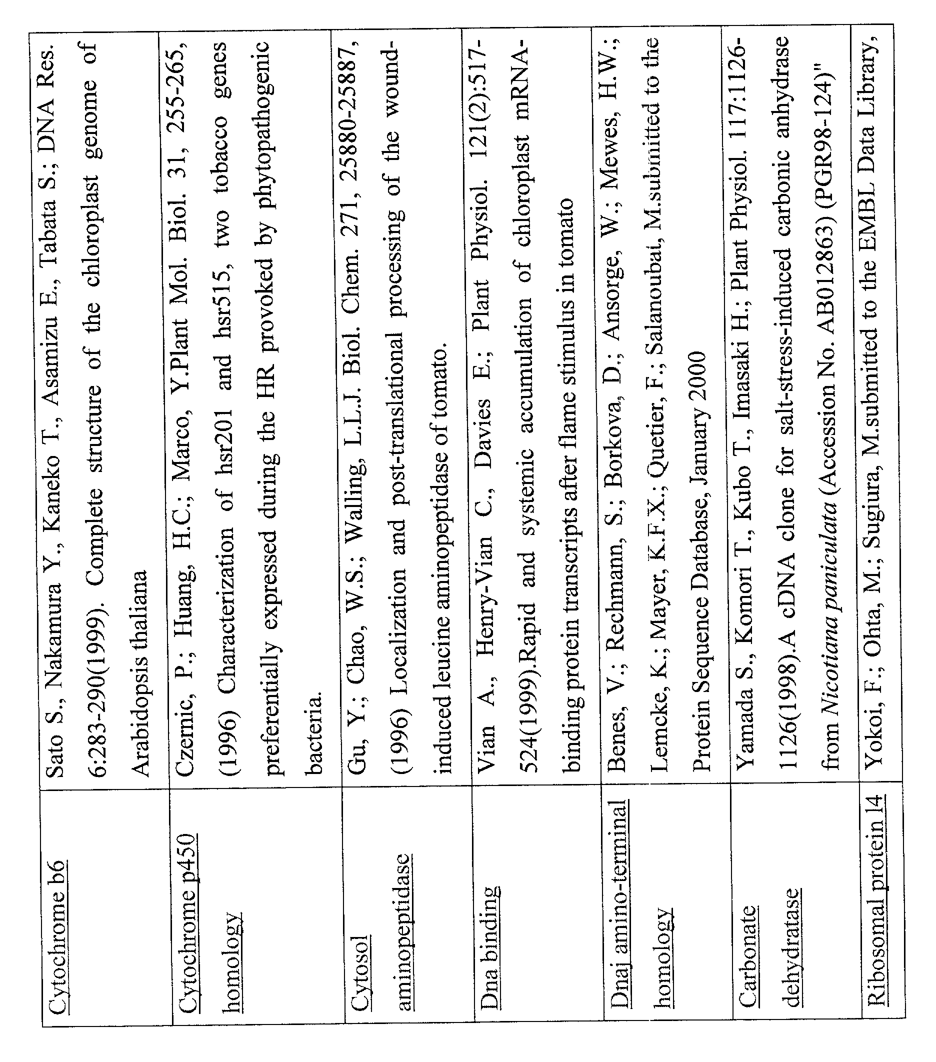

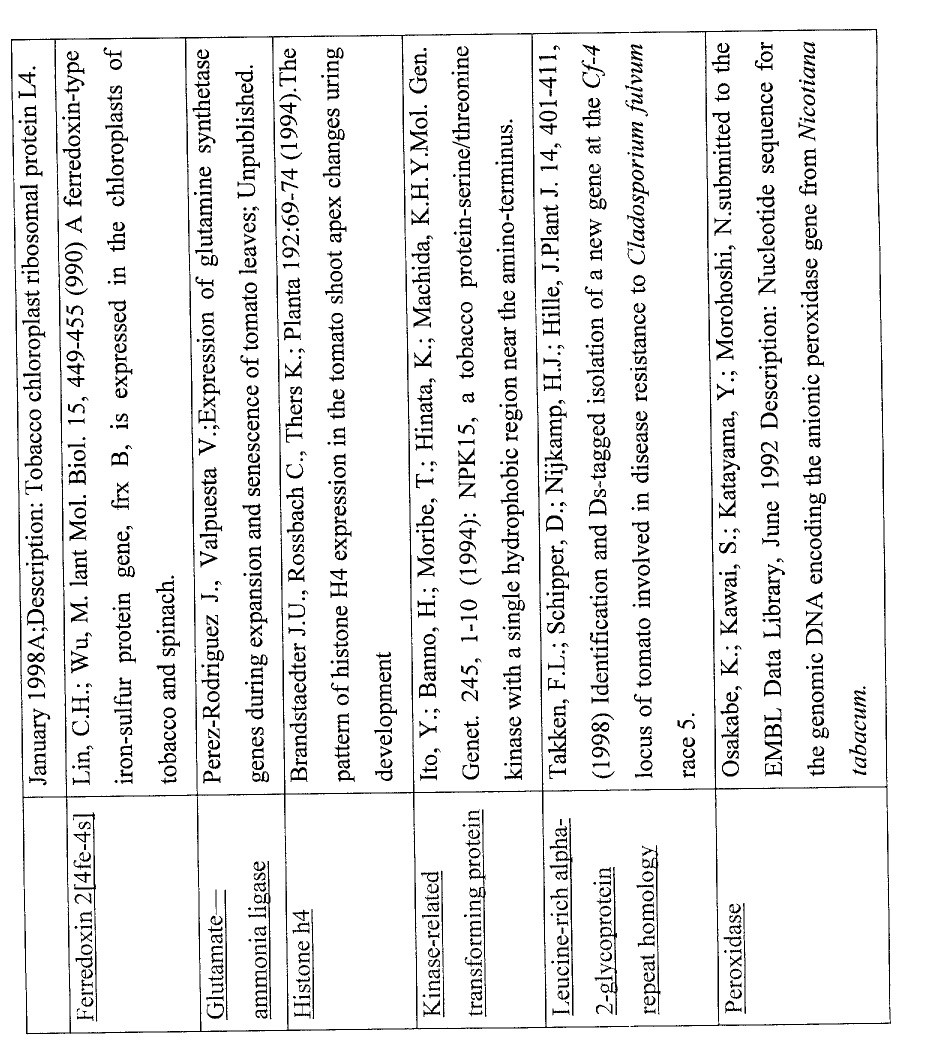

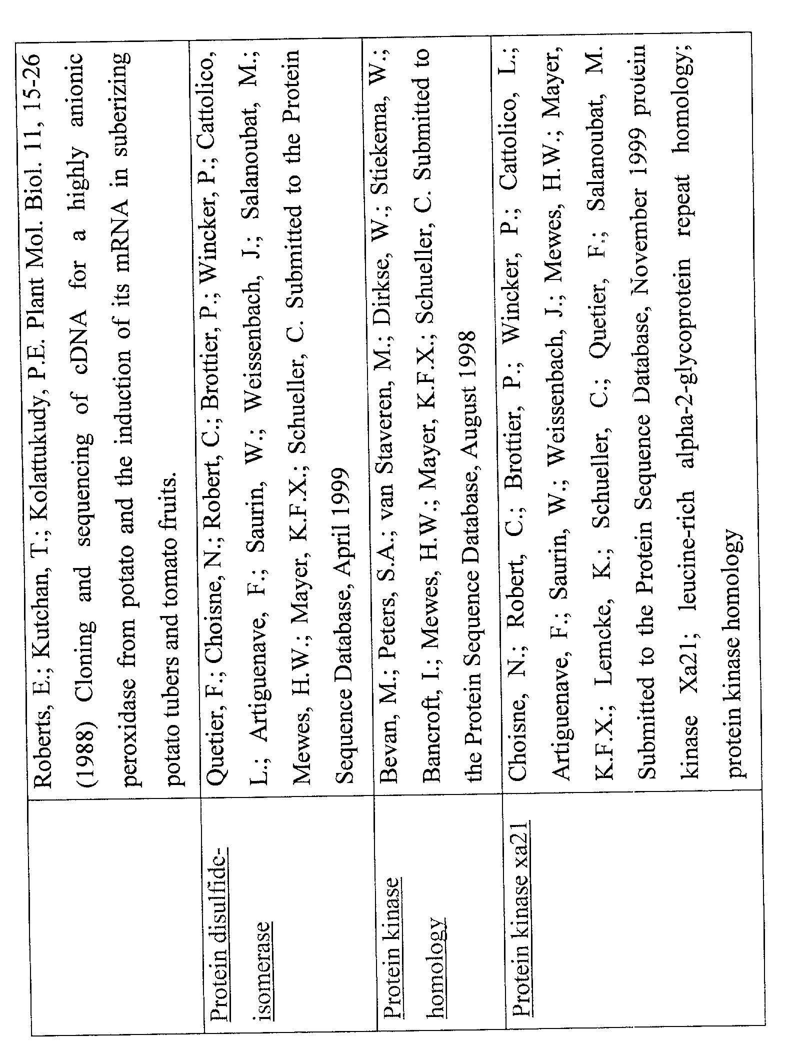

**Table 8:**

| Identification of sequences (see also the sequence listing for specific sequence information). | | | |
|---|---|---|---|
| **SEQ. ID NO** | **gelcode** | **Marker name** | **Sequence reference** |
| **1** | KM3F027R[3] | T14/m43_274.96 | Km3f_tr11-10r |
| **2** | KM3F027R[4] | T14/m44_470.66 | Km3f_tr11-13r |
| **3** | KM3F019L[4] | T12/m49_155.84 | Km3f_tr11-14r |
| **4** | KM3F022R[1] | T12/m51_263.82 | Km3f_tr11-19r |
| **5** | KM3F014L[4] | T12/m39_322.57 | Km3f_tr11-1r |
| **6** | KM3F027L[3] | T14/m48_157.75 | Km3f_tr11-25r |
| **7** | KM3F032R[2] | T13/m62_262.88 | Km3f_tr11-27r |
| **8** | KM3F034L[1] | T13/m55_152.62 | Km3f_tr11-28r |
| **9** | KM3F037L[1] | T13/m76_132.50 | Km3f_tr11-39r |
| **10** | KM3F015L[5] | T13/m40_152.17 | Km3f_tr11-3r |
| **11** | KM3F037L[2] | T13/m77_163.97 | Km3f_tr11-40r |
| **12** | KM3F029L[2] | T14/m92_360.48 | Km3f_tr11-43r |
| **13** | KM3F040R[1] | T12/m81_348.79 | Km3f_tr11-45r |
| **14** | KM3F040R[5] | T12/m85_115.69 | Km3f_tr11-46r |
| **15** | KM3F040R[5] | T12/m85_115.69 | Km3f_tr11-47r |
| **16** | KM3F018R[3] | T11/m43_132.26 | Km3f_tr11-52r |
| **17** | KM3F022L[5] | T12/m60_116.48 | Km3f_tr11-59r |
| **18** | KM3F019R[2] | T12/m42_202.77 | Km3f_tr11-5r |
| **19** | KM3F032L[1] | T13/m66_116.54 | Km3f_tr11-62r |
| **20** | KM3F032L[1] | T13/m66_116.54 | Km3f_tr11-63r |
| **21** | KM3F032L[2] | T13/m67_185.99 | Km3f_tr11-64r |
| **22** | KM3F032R[3] | T13/m63_257.70 | Km3f_tr11-65r |
| **23** | KM3F033R[2] | T14/m64_187.95 | Km3f_tr11-66r |
| **24** | KM3F035L[3] | T11/m78_161.83 | Km3f_tr11-67r |
| **25** | KM3F035R[1] | T11/m71_279.09 | Km3f_tr11-69r |
| **26** | KM3F019R[4] | T12/m44_191.57 | Km3f_tr11-6r |
| **27** | KM3F035R[1] | T11/m71_248.89 | Km3f_tr11-70r |
| **28** | KM3F041L[2] | T14/m69_173.70 | Km3f_tr11-76r |
| **29** | KM3F028L[3] | T12/m94_129.36 | Km3f_tr11-78r |
| **30** | KM3F028R[2] | T11/m92_289.36 | Km3f_tr11-81r |
| **31** | KM3F029L[1] | T14/m91_129.50 | Km3f_tr11-83r |
| **32** | KM3F029R[2] | T13/m92_244.73 | Km3f_tr11-84r |
| **33** | KM3F029R[3] | T13/m93_333.06 | Km3f_tr11-85r |
| **34** | KM3F038L[5] | T14/m80_190.22 | Km3f_tr11-88r |
| **35** | KM3F040R[4] | T12/m84_119.28 | Km3f_tr11-90r |
| **36** | KM3F040R[5] | T12/m85_212.74 | Km3f_tr11-92r |
| **37** | KM3F042L[2] | T14/m87_299.12 | Km3f_tr11-94r |
| **38** | KM3F042L[3] | T14/m88_228.31 | Km3f_tr11-95r |
| **39** | KM3F026L[1] | T13/m46_153.93 | Km3f_tr11-9r |
| **40** | KM3F027R[3] | T14/m43_274.96 | km3f_tr11-11R |
| **41** | KM3F027R[3] | T14/m43_274.96 | km3f_tr11-12R |
| **42** | KM3F023L[4] | T11/m59_162.51 | Km3f_tr11-15r |
| **43** | KM3F022R[5] | T12/m55_153.39 | Km3f_tr11-18r |
| **44** | KM3F022R[2] | T12/m52_132.78 | Km3f_tr11-20r |
| **45** | KM3F024R[2] | T13/m52_160.17 | Km3f_tr11-21r |
| **46** | KM3F025L[1] | T14/m57_146.95 | Km3f_tr11-22r |
| **47** | KM3F026L[2] | T13/m47_283.37 | Km3f_tr11-23r |
| **48** | KM3F031R[1] | T12/m61_423.01 | Km3f_tr11-26r |
| **49** | KM3F034R[4] | T13/m59_166.45 | Km3f_tr11-29r |
| **50** | KM3F014R[4] | T12/m34_195.62 | Km3f_tr11-2r |
| **51** | KM3F026L[2] | T13/m47_122.04 | Km3f_tr11-32r |
| **52** | KM3F030R[4] | T11/m64_116.67 | Km3f_tr11-33r |
| **53** | KM3F035R[3] | T11/m73_217.72 | Km3f_tr11-38r |
| **54** | KM3F028L[3] | T12/m94_169.15 | Km3f_tr11-41r |
| **55** | KM3F028R[1] | T11/m91_225.16 | Km3f_tr11-42r |
| **56** | KM3F038L[4] | T14/m79_179.14 | Km3f_tr11-44r |
| **57** | KM3F042L[2] | T14/m87_319.78 | Km3f_tr11-49r |
| **58** | KM3F015R[1] | T13/m31_284.99 | Km3f_tr11-4r |
| **59** | KM3F015R[1] | T13/m31_159.91 | Km3f_tr11-50r |
| **60** | KM3F018R[4] | T11/m44_322.91 | Km3f_tr11-53r |
| **61** | KM3F026R[3] | T13/m43_139.26 | Km3f_tr11-54r |
| **62** | KM3F027R[5] | T14/m45_143.31 | Km3f_tr11-55r |
| **63** | KM3F027R[5] | T14/m45_143.31 | Km3f_tr11-56r |
| **64** | KM3F019L[4] | T12/m49_149.24 | Km3f_tr11-58r |
| **65** | KM3F031L[5] | T12/m70_347.19 | Km3f_tr11-61r |
| **66** | KM3F035R[1] | T11/m71_322.22 | Km3f_tr11-68r |
| **67** | KM3F033R[2] | T14/m64_165.20 | Km3f_tr11-71r |
| **68** | KM3F036R[3] | T12/m73_130.27 | Km3f_tr11-72r |
| **69** | KM3F036R[3] | T12/m73_130.27 | Km3f_tr11-73r |
| **70** | KM3F038L[1] | T14/m76_238.28 | Km3f_tr11-74r |
| **71** | KM3F041L[2] | T14/m69_168.25 | Km3f_tr11-77r |
| **72** | KM3F028L[3] | T12/m94_129.36 | Km3f_tr11-79r |
| **73** | KM3F026L[1] | T13/m46_296.13 | Km3f_tr11-7r |
| **74** | KM3F028L[3] | T12/m94_129.36 | Km3f_tr11-80r |
| **75** | KM3F028R[4] | T11/m94_186.50 | Km3f_tr11-82r |
| **76** | KM3F035L[4] | T11/m79_198.36 | Km3f_tr11-86r |
| **77** | KM3F036L[5] | T12/m80_268.19 | Km3f_tr11-87r |
| **78** | KM3F038L[5] | T14/m80_133.82 | Km3f_tr11-89r |
| **79** | KM3F026L[1] | T13/m46_195.66 | Km3f_tr11-8r |
| **80** | KM3F040R[5] | T12/m85_212.74 | Km3f_tr11-91r |
| **81** | KM3F040R[5] | T12/m85_212.74 | Km3f_tr11-93r |
| **82** | KM3F042R[3] | T14/m83_248.13 | Km3f_tr11-96r |
| **83** | KM3F026L[5] | T13/m50_187.56 | Km3f_tr1114-86r |
| **84** | KM3F033R[2] | T14/m64_134.80 | Km3f_tr1114-89r |
| **85** | KM3F034L[5] | T12/m52_133.18 | Km3f_tr1114-93r |
| **86** | KM3F038R[3] | T14/m73_137.08 | Km3f_tr1114-95r |
| **87** | KM3F030L[4] | T11/m69_226.30 | km3f_tr1114-87R |
| **88** | KM3F035R[3] | T11/m73_237.91 | km3f_tr1114-90R |
| **89** | KM3F018R[3] | T11/m43_132.26 | km3f_tr1114-92R |
| **90** | KM3F035R[1] | T11/m71_279.09 | km3f_tr1114-94R |
| **91** | KM3F107R[2] | T26/m33_173.20 | km3f_t23-A01r |
| **92** | KM3F107R[2] | T26/m35_140.56 | km3f_t23-A02r |
| **93** | KM3F108L[2] | T23/m38_207.27 | km3f_t23-A03r |
| **94** | KM3F108L[2] | T23/m38_207.27 | km3f_t23-A04r |
| **95** | KM3F109R[1] | T24/m31_211.92 | km3f_t23-A05r |
| **96** | KM3F109R[1] | T24/m31_145.60 | km3f_t23-A06r |
| **97** | KM3F109R[2] | T24/m32_132.14 | km3f_t23-A07r |
| **98** | KM3F110L[2] | T25/m39_262.73 | km3f_t23-A08r |
| **99** | KM3F110R[1] | T25/m33_195.61 | km3f_t23-A10r |
| **100** | KM3F110R[1] | T25/m33_109.11 | km3f_t23-A11r |
| **101** | KM3F110R[2] | T25/m34_176.15 | km3f_t23-A12r |
| **102** | KM3F110R[2] | T25/m34_173.23 | km3f_t23-B01r |
| **103** | KM3F110R[2] | T25/m34_114.96 | km3f_t23-B02r |
| **104** | KM3F111R[1] | T23/m42_196.55 | km3f_t23-B03r |
| **105** | KM3F111R[1] | T23/m42_110.23 | km3f_t23-B04r |
| **106** | KM3F112L[2] | T24/m46_248.72 | km3f_t23-B05r |
| **107** | KM3F112L[2] | T24/m47_145.54 | km3f_t23-B06r |
| **108** | KM3F112L[2] | T24/m47_145.54 | km3f_t23-B07r |
| **109** | KM3F112L[2] | T24/m49_114.44 | km3f_t23-B08r |
| **110** | KM3F112R[2] | T24/m43_126.11 | km3f_t23-B09r |
| **111** | KM3F113R[1] | T25/m42_403.04 | km3f_t23-B10r |
| **112** | KM3F113R[1] | T25/m42_403.04 | km3f_t23-B11r |
| **113** | KM3F113R[1] | T25/m42_279.47 | km3f_t23-B12r |
| **114** | KM3F113R[1] | T25/m45_128.72 | km3f_t23-C01r |
| **115** | KM3F114L[1] | T26/m46_283.74 | km3f_t23-C02r |
| **116** | KM3F114L[1] | T26/m46_264.96 | km3f_t23-C03r |
| **117** | KM3F114L[1] | T26/m47_185.12 | km3f_t23-C04r |
| **118** | KM3F114R[1] | T26/m41_112.59 | km3f_t23-C05r |
| **119** | KM3F115R[2] | T26/m38_193.94 | km3f_t23-C06r |
| **120** | KM3F115R[2] | T26/m38_147.62 | km3f_t23-C07r |
| **121** | KM3F115R[4] | T26/m40_148.76 | km3f_t23-C08r |
| **122** | KM3F115R[5] | T26/m36_286.24 | km3f_t23-C09r |
| **123** | KM3F115R[5] | T26/m36_125.56 | km3f_t23-C10r |
| **124** | KM3F1161[3] | T23/m51_190.32 | km3f_t23-C11r |
| **125** | KM3F1161[3] | T23/m58_148.63 | km3f_t23-C12r |
| **126** | KM3F1161[3] | T23/m59_136.04 | km3f_t23-D01r |
| **127** | KM3F116L[3] | T23/m60_129.51 | km3f_t23-D02r |
| **128** | KM3F116R[1] | T23/m51_128.05 | km3f_t23-D03r |
| **129** | KM3F116R[1] | T23/m51_315.68 | km3f_t23-D04r |
| **130** | KM3F117L[3] | T24/m58_208.18 | km3f_t23-D05r |
| **131** | KM3F117L[3] | T24/m59_118.87 | km3f_t23-D06r |
| **132** | KM3F117L[3] | T24/m60_194.55 | km3f_t23-D07r |
| **133** | KM3F117R[1] | T24/m52_127.20 | km3f_t23-D08r |
| **134** | KM3F118L[2] | T25/m58_318.57 | km3f_t23-D09r |
| **135** | KM3F118L[2] | T25/m60_335.11 | km3f_t23-D10r |
| **136** | KM3F118R[2] | T25/m52_131.06 | km3f_t23-D11r |
| **137** | KM3F118R[2] | T25/m53_172.05 | km3f_t23-D12r |
| **138** | KM3F118R[2] | T25/m53_235.21 | km3f_t23-E01r |
| **139** | KM3F118R[2] | T25/m54_161.02 | km3f_t23-E02r |
| **140** | KM3F118R[2] | T25/m55_262.82 | km3f_t23-E03r |
| **141** | KM3F119L[2] | T26/m56_148.92 | km3f_t23-E04r |
| **142** | KM3F119L[2] | T26/m60_121.24 | km3f_t23-E05r |
| **143** | KM3F119L[2] | T23/m66_238.57 | km3f_t23-E06r |
| **144** | KM3F119L[2] | T23/m69_261.94 | km3f_t23-E07r |
| **145** | KM3F121L[1] | T24/m67_190.73 | km3f_t23-E08r |
| **146** | KM3F121L[2] | T24/m70_175.82 | km3f_t23-E09r |
| **147** | KM3F121L[2] | T24/m70_235.78 | km3f_t23-E10r |
| **148** | KM3F121L[2] | T24/m70_235.78 | km3f_t23-E11r |
| **149** | KM3F122L[2] | T25/m68_266.07 | km3f_t23-E12r |
| **150** | KM3F122R[1] | T25/m61_318.48 | km3f_t23-F01r |
| **151** | KM3F122R[1] | T25/m61_114.01 | km3f_t23-F02r |
| **152** | KM3F122R[2] | T25/m63_241.92 | km3f_t23-F03r |
| **153** | KM3F122R[2] | T25/m64_307.66 | km3f_t23-F04r |
| **154** | KM3F122R[2] | T25/m65_379.45 | km3f_t23-F05r |
| **155** | KM3F124R[1] | T23/m71_166.40 | km3f_t23-F06r |
| **156** | KM3F125L[2] | T24/m76_154.52 | km3f_t23-F07r |
| **157** | KM3F125L[2] | T24/m77_258.06 | km3f_t23-F08r |
| **158** | KM3F125L[2] | T24/m78_300.02 | km3f_t23-F09r |
| **159** | KM3F125L[5] | T24/m80_242.25 | km3f_t23-F10r |
| **160** | KM3F125L[5] | T24/m80_144.03 | km3f_t23-F11r |
| **161** | KM3F125L[5] | T24/m80_108.39 | km3f_t23-F12r |
| **162** | KM3F126L[5] | T25/m79_106.38 | km3f_t23-G01r |
| **163** | KM3F126L[5] | T25/m80_111.41 | km3f_t23-G02r |
| **164** | KM3F126R[5] | T25/m71_117.20 | km3f_t23-G03r |
| **165** | KM3F126R[3] | T25/m75_277.11 | km3f_t23-G04r |
| **166** | KM3F126R[3] | T25/m75_243.38 | km3f_t23-G05r |
| **167** | KM3F126R[3] | T25/m75_155.15 | km3f_t23-G06r |
| **168** | KM3F127L[1] | T26/m76_177.10 | km3f_t23-G07r |
| **169** | KM3F127L[3] | T26/m78_203.41 | km3f_t23-G08r |
| **170** | KM3F127L[3] | T26/m78_118.63 | km3f_t23-G09r |
| **171** | KM3F127L[3] | T26/m78_103.18 | km3f_t23-G10r |
| **172** | KM3F127R[3] | T26/m72_149.09 | km3f_t23-G11r |
| **173** | KM3F127R[2] | T26/m73_113.33 | km3f_t23-G12r |
| **174** | KM3F128L[2] | T23/m87_173.78 | km3f_t23-H01r |
| **175** | KM3F128L[2] | T23/m88_190.37 | km3f_t23-H02r |
| **176** | KM3F128R[1] | T23/m83_166.26 | km3f_t23-H03r |
| **177** | KM3F128R[1] | T23/m85_173.13 | km3f_t23-H04r |
| **178** | KM3F129R[1] | T24/m84_132.46 | km3f_t23-H05r |
| **179** | KM3F130L[1] | T25/m86_123.44 | km3f_t23-H06r |
| **180** | KM3F130L[4] | T25/m90_318.54 | km3f_t23-H07r |
| **181** | KM3F130L[4] | T25/m90_129.48 | km3f_t23-H08r |
| **182** | KM3F080L[1] | T21/m36_103.94 | km3f_t23-H10r |
| **183** | KM3F088R[2] | T19/M52_117.14 | km3f_t23-H11r |
| **184** | KM3F078L[1] | T20/m38_192.41 | Km3f_t19-A01r |
| **185** | KM3F078L[1] | T20/m38_131.93 | Km3f_t19-A02r |
| **186** | KM3F078R[1] | T20/m33_135.17 | Km3f_t19-A03r |
| **187** | KM3F078R[1] | T20/m33_99.10 | Km3f_t19-A04r |
| **188** | KM3F078R[1] | T20/m33_94.34 | Km3f_t19-A05r |
| **189** | KM3F078R[1] | T20/m35_109.43 | Km3f_t19-A06r |
| **190** | KM3F080L[1] | T21/m36_106.09 | Km3f_t19-A07r |
| **191** | KM3F080L[1] | T21/m38_236.82 | Km3f_t19-A09r |
| **192** | KM3F080r[1] | T21/m31_362.24 | Km3f_t19-A10r |
| **193** | KM3F080R[2] | T21/m32_252.41 | Km3f_t19-A11r |
| **194** | KM3F080R[2] | T21/m33_343.22 | Km3f_t19-A12r |
| **195** | KM3F080R[4] | T21/m34_117.55 | Km3f_t19-B01r |
| **196** | KM3F080R[4] | T21/m34_107.65 | Km3f_t19-B02r |
| **197** | KM3F080R[4] | T21/m35_139.69 | Km3f_t19-B03r |
| **198** | KM3F081L[4] | T22/m38_441.22 | Km3f_t19-B04r |
| **199** | KM3F081L[2] | T22/m39_153.77 | Km3f_t19-B06r |
| **200** | KM3F082L[1] | T19/m48_117.19 | Km3f_t19-B07r |
| **201** | KM3F082L[1] | T19/m48_113.60 | Km3f_t19-B08r |
| **202** | KM3F082R[1] | T19/m41_442.57 | Km3f_t19-B09r |
| **203** | KM3F082L[1] | T19/m42_280.88 | Km3f_t19-B10r |
| **204** | KM3F082L[1] | T19/m43_156.65 | Km3f_t19-B11r |
| **205** | KM3F082L[1] | T19/m45_153.53 | Km3f_t19-B12r |
| **206** | KM3F083L[1] | T20/m46_284.38 | Km3f_t19-C01r |
| **207** | KM3F083L[2] | T20/m47_321.79 | Km3f_t19-C02r |
| **208** | KM3F083L[2] | T20/m47_99.00 | Km3f_t19-C03r |
| **209** | KM3F083L[2] | T21/m46_249.52 | Km3f_t19-C04r |
| **210** | KM3F084R[1] | T21/m42_338.67 | Km3f_t19-C05r |
| **211** | KM3F084R[1] | T21/m42_220.55 | Km3f_t19-C06r |
| **212** | KM3F084R[1] | T21/m44_119.39 | Km3f_t19-C07r |
| **213** | KM3F085R[3] | T22/m45_186.69 | Km3f_t19-C08r |
| **214** | KM3F085R[3] | T22/m47_132.19 | Km3f_t19-C09r |
| **215** | KM3F087R[1] | T20/m44_254.43 | Km3f_t19-C10r |
| **216** | KM3F087R[1] | T20/m44_216.16 | Km3f_t19-C11r |
| **217** | KM3F087R[2] | T20/m45_183.03 | Km3f_t19-C12r |
| **218** | KM3F088L[2] | T19/m56_117.03 | Km3f_t19-D01r |
| **219** | KM3F088L[2] | T19/m59_197.55 | Km3f_t19-D02r |
| **220** | KM3F088L[2] | T19/m60_105.53 | Km3f_t19-D03r |
| **221** | KM3F088R[2] | T19/m51_111.14 | Km3f_t19-D04r |
| **222** | KM3F088R[2] | T19/m52_450.09 | Km3f_t19-D05r |
| **223** | KM3F088R[2] | T19/m52_195.49 | Km3f_t19-D06r |
| **224** | KM3F088R[3] | T19/m55_315.09 | Km3f_t19-D08r |
| **225** | KM3F089L[2] | T20/m60_139.30 | Km3f_t19-D10r |
| **226** | KM3F090R[1] | T21/m51_258.10 | Km3f_t19-D11r |
| **227** | KM3F090R[1] | T21/m53_164.99 | Km3f_t19-D12r |
| **228** | KM3F090R[1] | T21/m54_180.70 | Km3f_t19-E01r |
| **229** | KM3F091L[4] | T22/m58_246.81 | Km3f_t19-E02r |
| **230** | KM3F091R[4] | T22/m54_213.92 | Km3f_t19-E03r |
| **231** | KM3F092R[1] | T19/m62_142.37 | Km3f_t19-E04r |
| **232** | KM3F092R[1] | T19/m62_116.19 | Km3f_t19-E05r |
| **233** | KM3F092R[1] | T19/m62_105.69 | Km3f_t19-E06r |
| **234** | KM3F093R[1] | T20/m61_434.71 | Km3f_t19-E07r |
| **235** | KM3F093R[1] | T20/m61_178.68 | Km3f_t19-E08r |
| **236** | KM3F094L[1] | T21/m66_455.36 | Km3f_t19-E09r |
| **237** | KM3F094L[1] | T21/m66_237.97 | Km3f_t19-E10r |
| **238** | KM3F094L[1] | T21/m67_156.87 | Km3f_t19-E11r |
| **239** | KM3F094L[1] | T21/m68_273.60 | Km3f_t19-E12r |
| **240** | KM3F094R[1] | T21/m63_115.51 | Km3f_t19-F01r |
| **241** | KM3F094L[1] | T21/m64_149.70 | Km3f_t19-F02r |
| **242** | KM3F094L[1] | T21/m61_334.12 | Km3f_t19-F03r |
| **243** | KM3F095L[1] | T22/m66_142.80 | Km3f_t19-F04r |
| **244** | KM3F095L[1] | T22/m66_113.72 | Km3f_t19-F05r |
| **245** | KM3F095L[1] | T22/m70_269.73 | Km3f_t19-F06r |
| **246** | KM3F095R[1] | T22/m63_178.72 | Km3f_t19-F07r |
| **247** | KM3F095R[1] | T22/m64_307.25 | Km3f_t19-F08r |
| **248** | KM3F095L[1] | T22/m61_163.58 | Km3f_t19-F09r |
| **249** | KM3F095L[1] | T22/m65_216.30 | Km3f_t19-F10r |
| **250** | KM3F096L[1] | T19/m76_328.03 | Km3f_t19-F11r |
| **251** | KM3F096L[2] | T19/m80_253.80 | Km3f_t19-F12r |
| **252** | KM3F096L[2] | T19/m80_117.13 | Km3f_t19-G01r |
| **253** | KM3F096R[1] | T19/m73_128.53 | Km3f_t19-G02r |
| **254** | KM3F096R[1] | T19/m74_148.97 | Km3f_t19-G03r |
| **255** | KM3F096R[1] | T19/m75_150.10 | Km3f_t19-G04r |
| **256** | KM3F097L[3] | T20/m80_380.71 | Km3f_t19-G05r |
| **257** | KM3F097L[3] | T20/m80_131.76 | Km3f_t19-G06r |
| **258** | KM3F097R[3] | T20/m72_133.56 | Km3f_t19-G07r |
| **259** | KM3F098L[1] | T21/m76_182.35 | Km3f_t19-G08r |
| **260** | KM3F098L[1] | T21/m80_375.48 | Km3f_t19-G09r |
| **261** | KM3F098R[1] | T21/m72_151.64 | Km3f_t19-G10r |
| **262** | KM3F098R[1] | T21/m73_206.18 | Km3f_t19-G11r |
| **263** | KM3F099L[1] | T22/m76_263.77 | Km3f_t19-G12r |
| **264** | KM3F099L[3] | T22/m78_433.40 | Km3f_t19-H01r |
| **265** | KM3F099L[3] | T22/m78_243.16 | Km3f_t19-H02r |
| **266** | KM3F099L[3] | T22/m79_99.14 | Km3f_t19-H03r |
| **267** | KM3F099L[5] | T22/m80_170.23 | Km3f_t19-H04r |
| **268** | KM3F099R[5] | T22/m71_159.04 | Km3f_t19-H05r |
| **269** | KM3F099R[5] | T22/m72_197.82 | Km3f_t19-H06r |
| **270** | KM3F100L[2] | T19/M87_315.35 | Km3f_t19-H07r |
| **271** | KM3F100L[2] | T19/M87_205.62 | Km3f_t19-H08r |
| **272** | KM3F100L[1] | T19/M86_128.95 | Km3f_t19-H09r |
| **273** | KM3F100R[2] | T19/m83_265.73 | Km3f_t19-H10r |
| **274** | KM3F100R[3] | T19/m85_168.89 | Km3f_t19-H11r |
| **275** | KM3F100R[3] | T19/m85_265.33 | Km3f_t19-H12r |
| **276** | KM3F017L[2] | T11/m37_355.44 | Km3f_trest-A01r |
| **277** | KM3F017R[4] | T11/m34_115.00 | Km3f_trest-A02r |
| **278** | KM3F014L[1] | T12/m36_133.22 | Km3f_trest-A03r |
| **279** | KM3F014L[4] | T12/m39_131.36 | Km3f_trest-A04r |
| **280** | KM3F014L[5] | T12/m40_295.82 | Km3f_trest-A05r |
| **281** | KM3F014R[1] | T12/m33_141.33 | Km3f_trest-A06r |
| **282** | KM3F014R[3] | T12/m33_289.67 | Km3f_trest-A07r |
| **283** | KM3F014R[1] | T12/m33_141.33 | Km3f_trest-A08r |
| **284** | KM3F022L[1] | T12/m56_197.04 | Km3f_trest-A09r |
| **285** | KM3F022L[1] | T12/m56_414.48 | Km3f_trest-A10r |
| **286** | KM3F025L[3] | T14/m59_298.62 | Km3f_trest-A11r |
| **287** | KM3F032L[1] | T13/m66_159.78 | Km3f_trest-A12r |
| **288** | KM3F038R[1] | T14/m71_226.18 | Km3f_trest-B01r |
| **289** | KM3F038R[2] | T14/m72_127.10 | Km3f_trest-B02r |
| **290** | KM3F040R[1] | T12/m81_381.02 | Km3f_trest-B03r |
| **291** | KM3F050L[2] | T16/m46_246.46 | Km3f_trest-B04r |
| **292** | KM3F052L[1] | T18/m46_300.38 | Km3f_Trest-B05r |
| **293** | KM3F065R[1] | T15/m77_264.72 | Km3f_Trest-B06r |
| **294** | KM3F066R[1] | T16/m78_112.76 | Km3f_Trest-B07r |
| **295** | KM3F072L[2] | T18/m89_379.77 | Km3f_trest-B08r |
| **296** | KM3F101R[1] | T20/m82_255.31 | Km3f_trest-B10r |
| **297** | KM3F101R[1] | T20/m82_255.31 | Km3f_trest-B11r |
| **298** | KM3F101R[1] | T20/m82_253.18 | Km3f_trest-B12r |
| **299** | KM3F101R[1] | T21/m87_159.68 | Km3f_trest-C01r |
| **300** | KM3F101R[1] | T21/m89_355.66 | Km3f_trest-C02r |
| **301** | KM3F102L[3] | T21/m90_169.43 | Km3f_trest-C03r |
| **302** | KM3F102R[1] | T21/m81_130.62 | Km3f_trest-C04r |
| **303** | KM3F102R[1] | T21/m81_106.57 | Km3f_trest-C05r |
| **304** | KM3F102R[2] | T21/m82_294.56 | Km3f_trest-C06r |
| **305** | KM3F102R[2] | T21/m82_110.71 | Km3f_trest-C07r |
| **306** | KM3F103L[1] | T22/m87_209.70 | Km3f_trest-C08r |
| **307** | KM3F103L[1] | T22/m87_99.50 | Km3f_trest-C09r |
| **308** | KM3F103L[1] | T22/m89_116.52 | Km3f_trest-C10r |
| **309** | KM3F103L[1] | T22/m89_116.52 | Km3f_trest-C11r |
| **310** | KM3F103R[3] | T22/m84_176.12 | Km3f_trest-D01r |
| **311** | KM3F104L[3] | T22/m55_160.17 | Km3f_trest-D02r |
| **312** | KM3F105L[3] | T21/m92_131.87 | Km3f_trest-D03r |
| **313** | KM3F105L[3] | T21/m92_131.87 | Km3f_trest-D04r |
| **314** | KM3F106L[2] | T19/m35_286.21 | Km3f_trest-D06r |
| **315** | KM3F106L[2] | T19/m35_277.90 | Km3f_trest-D07r |
| **316** | KM3F106L[2] | T19/m35_256.65 | Km3f_trest-D08r |
| **317** | KM3F115L[1] | T21/m93_183.15 | Km3f_trest-D09r |
| **318** | KM3F115L[1] | T21/m93_180.82 | Km3f_trest-D10r |
| **319** | KM3F109R[3] | T24/m35_190.42 | Km3f_trest-D12r |
| **320** | KM3F111R[2] | T23/m43_242.15 | Km3f_trest-E01r |
| **321** | KM3F112R[2] | T24/m42_265.39 | Km3f_trest-E02r |
| **322** | KM3F123L[1] | T26/m69_396.15 | Km3f_trest-E03r |
| **323** | KM3F124L[1] | T23/m79_379.60 | Km3f_trest-E04r |
| **324** | KM3F124L[1] | T23/m80_206.00 | Km3f_trest-E05r |
| **325** | KM3F124R[1] | T23/m71_390.80 | Km3f_trest-E06r |
| **326** | KM3F130R[1] | T25/m83_230.64 | Km3f_trest-E07r |
| **327** | KM3F130R[1] | T25/m83_230.64 | Km3f_trest-E08r |
| **328** | KM3F130R[1] | T25/m83_178.42 | Km3f_trest-E09r |
| **329** | KM3F130R[1] | T25/m84_186.74 | Km3f_trest-E10r |
| **330** | KM3F130R[1] | T25/m85_171.99 | Km3f_trest-E11r |
| **331** | KM3F131L[1] | T26/m89_121.75 | Km3f_trest-F01r |
| **332** | KM3F131R[1] | T26/m82_152.12 | Km3f_trest-F02r |
| **333** | KM3F132L[1] | T24/m91_118.71 | Km3f_trest-F03r |
| **334** | KM3F132R[1] | T23/m94_140.81 | Km3f_trest-F04r |
| **335** | KM3F133L[1] | T26/m93_478.98 | Km3f_trest-F05r |
| **336** | KM3F134R[1] | T26/m62_208.72 | Km3f_trest-F06r |
| **337** | KM3F042R[5] | T14/m85_156.61 | Km3f_trest-F07r |
| **338** | KM3F043L[2] | T13/m87_152.46 | Km3f_trest-F08r |
| **339** | KM3F043L[3] | T13/m88_228.56 | Km3f_trest-F09r |
| **340** | KM3F043R[1] | T13/m81_122.23 | Km3f_trest-F11r |
| **341** | KM3F043R[3] | T13/m83_171.96 | Km3f_trest-F12r |
| **342** | KM3F050R[2] | T16/m43_123.27 | Km3f_tr15-10r |
| **343** | KM3F053L[2] | T15/m48_129.14 | Km3f_tr15-12r |
| **344** | KM3F062L[1] | T16/m66_127.57 | Km3f_tr15-13r |
| **345** | KM3F055L[1] | T17/m39_103.29 | Km3f_tr15-14r |
| **346** | KM3F055R[1] | T17/m32_138.72 | Km3f_tr15-15r |
| **347** | KM3F055R[1] | T17/m32_137.81 | Km3f_tr15-16r |
| **348** | KM3F056R[1] | T17/m42_284.69 | Km3f_tr15-18r |
| **349** | KM3F056R[1] | T17/m42_259.96 | Km3f_tr15-19r |
| **350** | KM3F046R[1] | T16/m36_104.54 | Km3f_tr15-1r |
| **351** | KM3F056R[1] | T17/m42_139.28 | Km3f_tr15-20r |
| **352** | KM3F057L[3] | T14/m59_171.85 | Km3f_tr15-21r |
| **353** | KM3F057L[3] | T14/m51_141.13 | Km3f_tr15-23r |
| **354** | KM3F058L[1] | T16/m56_241.58 | Km3f_tr15-24r |
| **355** | KM3F058L[1] | T16/m53_152.10 | Km3f_tr15-25r |
| **356** | KM3F058L[1] | T16/m54_194.58 | Km3f_tr15-26r |
| **357** | KM3F059R[1] | T17/m54_262.44 | Km3f_tr15-27r |
| **358** | KM3F060R[2] | T18/m53_268.40 | Km3f_tr15-29r |
| **359** | KM3F046L[2] | T16/m38_177.24 | Km3f_tr15-2r |
| **360** | KM3F060R[2] | T18/m53_199.18 | Km3f_tr15-30r |
| **361** | KM3F061L[1] | T15/m66_201.29 | Km3f_tr15-31r |
| **362** | KM3F061L[1] | T15/m67_253.60 | Km3f_tr15-32r |
| **363** | KM3F061L[1] | T15/m69_111.04 | Km3f_tr15-33r |
| **364** | KM3F061L[1] | T15/m61_167.19 | Km3f_tr15-34r |
| **365** | KM3F061L[1] | T15/m61_167.19 | Km3f_tr15-35r |
| **366** | KM3F061L[1] | T15/m62_225.54 | Km3f_tr15-36r |
| **367** | KM3F061L[1] | T15/m65_224.11 | Km3f_tr15-37r |
| **368** | KM3F062L[1] | T16/m66_130.52 | Km3f_tr15-38r |
| **369** | KM3F062L[1] | T16/m66_130.52 | Km3f_tr15-39r |
| **370** | KM3F046R[1] | T16/m31_213.31 | Km3f_tr15-3r |
| **371** | KM3F062L[1] | T16/m66_129.60 | Km3f_tr15-40r |
| **372** | KM3F062L[1] | T16/m66_126.64 | Km3f_tr15-41r |
| **373** | KM3F062L[1] | T16/m66_126.64 | Km3f_tr15-42r |
| **374** | KM3F062L[2] | T16/m61_105.18 | Km3f_tr15-43r |
| **375** | KM3F062R[2] | T16/m63_206.19 | Km3f_tr15-44r |
| **376** | KM3F062R[2] | T16/m63_119.24 | Km3f_tr15-45r |
| **377** | KM3F063R[1] | T17/m64_294.32 | Km3f_tr15-46r |
| **378** | KM3F063R[1] | T17/m64_211.16 | Km3f_tr15-47r |
| **379** | KM3F064R[1] | T18/m61_210.04 | Km3f_tr15-49r |
| **380** | KM3F048L[1] | T18/m38_152.26 | Km3f_tr15-4r |
| **381** | KM3F064R[1] | T18/m61_181.90 | Km3f_tr15-50r |
| **382** | KM3F064R[1] | T18/m62_180.75 | Km3f_tr15-51r |
| **383** | KM3F065R[1] | T15/m73_202.18 | Km3f_tr15-52r |
| **384** | KM3F066R[1] | T16/m80_119.58 | Km3f_tr15-54r |
| **385** | KM3F066R[1] | T16/m73_131.08 | Km3f_tr15-55r |
| **386** | KM3F066R[1] | T16/m74_315.97 | Km3f_tr15-56r |
| **387** | KM3F066R[3] | T16/m75_239.27 | Km3f_tr15-57r |
| **388** | KM3F068R[3] | T18/m76_169.66 | Km3f_tr15-58r |
| **389** | KM3F048L[2] | T18/m39_108.03 | Km3f_tr15-5r |
| **390** | KM3F068R[2] | T18/m78_111.26 | Km3f_tr15-60r |
| **391** | KM3F069R[2] | T15/m84_107.06 | Km3f_tr15-61r |
| **392** | KM3F070L[1] | T16/m87_118.01 | Km3f_tr15-62r |
| **393** | KM3F070L[1] | T16/m89_219.49 | Km3f_tr15-63r |
| **394** | KM3F070R[2] | T16/m82_249.46 | Km3f_tr15-64r |
| **395** | KM3F070R[2] | T16/m82_158.21 | Km3f_tr15-65r |
| **396** | KM3F070R[2] | T16/m82_158.21 | Km3f_tr15-66r |
| **397** | KM3F070R[3] | T16/m83_304.26 | Km3f_tr15-67r |
| **398** | KM3F070R[3] | T16/m84_170.22 | Km3f_tr15-68r |
| **399** | KM3F072L[1] | T18/m87_150.02 | Km3f_tr15-69r |
| **400** | KM3F048L[2] | T18/m35_173.35 | Km3f_tr15-6r |
| **401** | KM3F072L[1] | T18/m87_132.45 | Km3f_tr15-70r |
| **402** | KM3F072L[2] | T18/m89_106.44 | Km3f_tr15-71r |
| **403** | KM3F072L[4] | T18/m88_433.55 | Km3f_tr15-72r |
| **404** | KM3F072L[4] | T18/m81_372.94 | Km3f_tr15-73r |
| **405** | KM3F072R[2] | T18/m82_258.08 | Km3f_tr15-74r |
| **406** | KM3F072R[2] | T18/m82_140.67 | Km3f_tr15-75r |
| **407** | KM3F072R[2] | T18/m84_243.08 | Km3f_tr15-76r |
| **408** | KM3F073L[1] | T17/m87_196.39 | Km3f_tr15-77r |
| **409** | KM3F074L[2] | T17/m85_105.75 | Km3f_tr15-78r |
| **410** | KM3F074L[2] | T17/m85_96.86 | Km3f_tr15-79r |
| **411** | KM3F050L[2] | T16/m47_107.03 | Km3f_tr15-7r |
| **412** | KM3F074L[2] | T15/m91_288.03 | Km3f_tr15-80r |
| **413** | KM3F074R[2] | T15/m92_115.90 | Km3f_tr15-81r |
| **414** | KM3F074R[2] | T15/m92_115.90 | Km3f_tr15-82r |
| **415** | KM3F075L[2] | T18/m93_178.23 | Km3f_tr15-83r |
| **416** | KM3F075L[2] | T18/m93_149.84 | Km3f_tr15-84r |
| **417** | KM3F075L[2] | T18/m93_181.25 | Km3f_tr15-85r |
| **418** | KM3F055L[1] | T17/m39_103.29 | Km3f_tr15-88r |
| **419** | KM3F050R[2] | T16/m43_224.52 | Km3f_tr15-8r |
| **420** | KM3F050R[2] | T16/m43_153.87 | Km3f_tr15-9r |

## Claims

1. A method for identification, and optional isolation, of an expressed nucleic acid sequence that is associated with disease resistance in a plant against a pathogen comprising the steps of:
(a) providing a first plant material;
(b) providing a second plant material, which differs from the first plant material in a property relating to the hypersensitive response;
(c) optionally selecting an additional plant material, which differs from the first plant material in a property relating to the hypersensitive response;
(d) comparing a transcription profile generated for the first plant material with a transcription profile generated for the second plant material, and optionally with a transcription profile of the additional plant material;
(e) detecting/identifying in said transcription profiles a nucleic acid sequence that corresponds to a transcript that is differentially expressed between the first plant material and the second plant material, and optionally between the first plant material and any of the additional plant materials; and
(f) optionally, isolating, purifying and/or sequencing the expressed nucleic acid sequence identified in step (e).

2. A method according to claim 1, wherein the first plant material is obtained from a plant that does not produce the hypersensitive response; and wherein the second and optional additional plant material are obtained from one or more plants in which the hypersensitive response has been induced.

3. A method according to claims 1 or 2, wherein the induction of the hypersensitive response is suppressible.

4. A method according to claim 3, wherein the suppression is controlled by temperature.

5. A method according to any one of claims 1 - 4, wherein the second and additional plant materials have been obtained at different time intervals after induction of the hypersensitive response.

6. A method according to any one of claims 1 - 5, wherein the first plant material is obtained from a plant that does not comprise a plant pathogen derived avirulence gene.

7. A method according to any one of claims 1 - 6, wherein the second and optional additional plant material are obtained from transgenic plants comprising a matching pair of:
(a) a plant pathogen derived avirulence gene; and
(b) a plant resistance gene;
wherein matching pair is capable of inducing the hypersensitive response.

8. A method according to claim 7, wherein the plant pathogen is a fungus.

9. A method according to claim 8, wherein the fungus is *Cladosporium fulvum.*

10. A method according to claim 9, wherein the matching pair of the plant pathogen derived avirulence gene and the plant resistance gene is selected from the group consisting of the pairs: *Avr2* and *Cf-2*; *Avr4* and *Cf-4*; *Hcr9-4E* and *Cf-4E*; *Avr5* and *Cf-5*; and, *Avr9* and *Cf-9.*

11. A method according to any one of claims 1 - 10, wherein the transcript profiles of the plant materials are obtained using cDNA AFLP®.

12. An isolated nucleic acid molecule comprising a nucleotide sequence as defined in one of SEQ ID NO 1 to SEQ ID NO 420.

13. A nucleic acid molecule which is capable of hybridising under stringent conditions with at least part of a nucleotide sequence as defined in one of SEQ ID NO 1 to SEQ ID NO 420.

14. A nucleic acid molecule according to claim 12 or 13, wherein the nucleic acid molecule has a length of 10-50 nucleotides.

15. A collection of nucleic acid molecules comprising at least two different nucleic acids molecule as defined in any one of claims 12-14.

16. A collection according to claim 15, wherein the nucleic acid molecules are immobilised on a solid support whereby each different nucleic acid molecule is attached to a specific and distinct part of the solid support.

17. Use of a nucleic acid molecule according to any one of the claims 12-14, or a collection according to claim 15 or 16, in analysing a plant genome.

18. Use of a nucleic acid molecule according to any one of the claims 12-14, or a collection according to claim 15 or 16, in determining whether a plant is capable of expressing a desired property relating to disease resistance.

19. Use of a nucleic acid molecule according to any one of the claims 12-14, or a collection according to claim 15 or 16, in cloning a full-length copy of a plant gene or plant cDNA.

20. Use of a nucleic acid molecule according to claim 12 or 13 for providing a plant with resistance against at least one plant pathogen.

21. Use according to any one of the claims 17-20, wherein the plant is an economically and/or agronomically important crop or plant.

22. A nucleic acid molecule comprising a functional coding sequence for a plant polypeptide, **characterised in that**:
(a) the coding sequence is part of a plant transcript that comprises a nucleotide sequence as defined in one of SEQ ID NO 1 to SEQ ID NO 420 is; or
(b) the coding sequence encodes a polypeptide that has at least 40% amino acid identity with the polypeptide encoded by the coding sequence in (a).

23. A nucleic acid construct comprising a coding sequence as defined in claim 22, and further comprising an expression regulating sequence operably linked to the coding sequence whereby the a expression regulating sequence is capable of directing expression of the coding sequence in a suitable host cell.

24. A host cell containing a nucleic acid construct as defined in claim 23.

25. A host cell according to claim 24, wherein the host cell is a plant cell.

26. A host cell according to claim 24, wherein the host cell is a Agrobacterium cell.

27. A plant comprising a host cell as defined in claim 24 or 25.

28. Cultivation material for a plant of claim 27, wherein the cultivation material comprises a host cell defined in claim 24 or 25.

29. Plant material obtained from a plant as defined in claim 27.

30. A polypeptide encoded by a coding sequence as defined in claim 22.

31. A method for producing a polypeptide as defined in claim 30, wherein a host cell as defined in claims 24-26, or a plant as defined in claim 27 is grown under conditions conducive to the expression of the polypeptide, and optionally recovering the polypeptide.

32. A method for inducing a hypersensitive response in a transgenic plant, wherein the method comprises the steps of:
(a) growing a plant expressing a matching pair of a plant pathogen derived avirulence gene and a plant resistance gene, at a temperature that suppresses induction of the hypersensitive response; and
(b) reducing the temperature of the plant to a temperature that is permissive for the induction of the hypersensitive response.

33. A transgenic plant comprising a matching pair of a plant pathogen derived avirulence gene and a plant resistance gene.

34. A transgenic plant according to claim 33, wherein the plant is grown to a stage beyond the emergence of the hypocotyls.

35. A transgenic plant according to claim 34, wherein the plant is fullgrown.


SEQUENCE LISTING

<110> keygene N.V.

<120> nucleotide sequnces involved in disease resistance

<130> diff. seq. clado-tomaat

<140> BO 44607
<141> 2001-06-22

<160> 420

<170> PatentIn Ver. 2.1

<210> 1
<211> 271
<212> DNA
<213> Lycopersicon esculentum

<400> 1
ttaaatatac aatttggaaa gtgggagatt acaatgaaac tctaggcggg gtattattgg 60
agactggagg aagcataggg caaagagata gcagctattt caagattgtt ccatcaaaac 120
ttggttacaa cttagtcctt tgcgatccta ctcctatttt ctgtccattt tgccgtaagg 180
gtcagttatg tgtaaatgtg ggtgtagttt tccaagatgg aagaaggcgt ttggctctta 240
ctaaggatca gcctcttgat gtattattcg a 271

<210> 2
<211> 125
<212> DNA
<213> Lycopersicon esculentum

<400> 2
ttaaaggagc tgctcctctg gtgactacct gaatatggag tgtgtgtagc agtagcacca 60
ctgacaaaca attttgtgga taacataaga tctactagta gtcctcactg cccacatctc 120
ttcga 125

<210> 3
<211> 83
<212> DNA
<213> Lycopersicon esculentum

<400> 3
tcgatgcaat atgttacacc cacgattcac tacctaatta gtgtaaattc taggttgtgg 60
agtgatggtt gctacgcttt taa 83

<210> 4
<211> 149
<212> DNA
<213> Lycopersicon esculentum

<400> 4
tcgatggagt attttggtaa tataggttgg aagactaggt aaattacggc tgctgcgatg 60
cctatgagta cgattagtaa gaaaaatagg caacatgtcc aacataggca tcttacgcag 120
cagcctcttt tctttttggg tgattttaa 149

<210> 5

<211> 146
<212> DNA
<213> Lycopersicon esculentum

<400> 5
ttaaaattag agaataaaaa aactcagtct aacgctacag gatcccgaaa gctctgttcc 60
atgagcttca aaaattcaga aaattcaatt tttccatctc tgttttcatc aaaggccata 120
atcatcttct tgcaatgctc catcga 146

<210> 6
<211> 88
<212> DNA
<213> Lycopersicon esculentum

<400> 6
tcgatgaagc tctctttcaa atgatgttgt actattttct tgtgaatacc atgtagcttc 60
tgaaaagctc tcaagcctgc aattttaa 88

<210> 7
<211> 169
<212> DNA
<213> Lycopersicon esculentum

<400> 7
tcgatatcac tttcttcaaa cttgttgttt gatcctactg cttttgatat ctctgttttc 60
acttcagcca ttgcttgtgg atggcgcaaa agctctgtta gtgcccattc aacgctactg 120
cttgtcgtct ctgtaccagc tagaaacaat tccagtatga atactttaa 169

<210> 8
<211> 90
<212> DNA
<213> Lycopersicon esculentum

<400> 8
ttaaagtggg caccacagtt ggaaatattg gggcatttat ctacggggtg gatttatgaa 60
ctcactgtgt gatggaattc atgtattcga 90

<210> 9
<211> 174
<212> DNA
<213> Lycopersicon esculentum

<400> 9
tcgatcattc atcccgttac gtaaagcctt ttcgagctgg cgcctctgtc aaatactttt 60
aatccctgac atgttatggt ctcgggccga aactgcagat gcgatgcttt aagtaaatag 120
cgaaatctgc agcgtacgaa aacagttgct ccgaagaggg attcccgaat ttaa 174

<210> 10
<211> 118
<212> DNA
<213> Lycopersicon esculentum

<400> 10
tcgatccaaa tagagcatga agtctcacac agtaatactt tgccataaag atctttgctt 60
tgtccttatg gttcttctcc acatagctaa ctacgagttt tcccactggt attgttaa 118

<210> 11
<211> 117
<212> DNA
<213> Lycopersicon esculentum

<400> 11
ttaacaatgg tccagattgg atgggtttgt tctcagtaat tcctccatct ccattgtgat 60
gggattcaat tacagtgtca acaggttcca tattcatctt gggatcagtg tgatcga 117

<210> 12
<211> 88
<212> DNA
<213> Lycopersicon esculentum

<400> 12
tcgatcaaca agtccccggc atggaacaca catgtataaa tattggtgtt gcccaaccac 60
attccattcc ccatggtcct gctgttaa 88

<210> 13
<211> 137
<212> DNA
<213> Lycopersicon esculentum

<400> 13
ttaagtgatt tccggtcgtc caacaatgtt tcaaccggtg aaacacctat ccaacattat 60
tcaagccaat gaaatgcatg tccaaaacaa tgtctaacaa aactaaaatg atcataactt 120
tttactccga acttcga 137

<210> 14
<211> 100
<212> DNA
<213> Lycopersicon esculentum

<400> 14
tcgatccaca acatcaacac aacaataaag gggccaaagg atgattcaaa gatttactag 60
agttcttact tcttttttat gaatagggag taatatttaa 100

<210> 15
<211> 373
<212> DNA
<213> Lycopersicon esculentum

<400> 15
tcgatgaagt ttatggaagt gaatcctcca ttattggaag cctgaaatct gtcaacgatt 60
tcaacccagt tgaagttcca tcaaatcggc ccgttatctt tgataataac atgcttgagg 120
acaatttgca gcagccattg gctgaaagcg ataatgccac agaaaacctt gttagtgaga 180
acggagatga gccaatggac tcgggagaag gtgatggagc ccagacaagg ggcaaaagtg 240
ctagtagtag acaaaatgag aagttatacg gtgaggaagg catgttgaac actaaacaga 300
agaaagctga gaagaaaagg aggaagaaag acaagccctc aactgccatt gatatggacg 360
gtgatgactt taa 373

<210> 16
<211> 221
<212> DNA
<213> Lycopersicon esculentum

<400> 16
   tcgatggaat gatatggagt ggatgtgcaa atggattact tgtccaatgg gatagaaatg 60
   gcaatcgctt gcaagatttc cagtatcaca cattctccgt tcaatgttta tgcacatatg 120
   ggtcacggat ttgggcaggt tatgctagtg gttatattca ggtattggat cttagtggta 180
   atctacttgg aggatggata ggtcatagta gccccgtact a 221

<210> 17
<211> 255
<212> DNA
<213> Lycopersicon esculentum

<400> 17
ttaaatgtgc caccaccatc caccgataga gaaccacact aagacaacca tcaaacaatc 60
tcatccccac ttcgctccgg tgaaaccacc acaaacaaca accaaacaac tcatttcttt 120
ctcttctttc tccaacacac cagcaataag aagccacgaa ctccgaagaa tcggcgagct 180
caaagaagcg aatcagattt gaccacaaat caaagcaaat cagcacacaa caacgaagta 240
aactaacacc atcga 255

<210> 18
<211> 104
<212> DNA
<213> Lycopersicon esculentum

<400> 18
tcgatggctt agccttcatg ggaaatcgct tcactggana atatagcctc atatcctctt 60
tggagaacac tcacatcact tgagtacttg gatatttcat ttaa 104

<210> 19
<211> 254
<212> DNA
<213> Lycopersicon esculentum

<400> 19
tcgattgaag ttgtgttgaa catgcaagtc ttcacccttg ctttgtcacc gggcgaacac 60
gttgtagccg gggcgggagg gggatggcta tggctatgtg gtggaggtgg tggtgatcgt 120
ggtggtggag gtggtgggca tggttgtggt tgtggtcgtg gtcgtggtcg tggtcgtgga 180
gggcatggat atggtcgtgg acagggacat ggccatggta aataacatgg aatatcccat 240
ggccataaat ttaa 254

<210> 20
<211> 237
<212> DNA
<213> Lycopersicon esculentum

<400> 20
   tcgattcaca catagtattt tcgttctgtt tatcatgaat ctcaactgtt ttcaattgaa 60
   attttcagga gtatatttgc cagatttgtg cagcaaacag aggagcagcc cacaccgcgc 120
   aatgattgag ttattgagca gtggagctgc tgctgctact gatgcttctg ctccgatttc 180
   ttctgaagat attattgttg gtgctcctga tatttcccct gcttctttct aatttaa 237

<210> 21
<211> 162
<212> DNA
<213> Lycopersicon esculentum

<400> 21
ttaacaagtg tatcaagcat ggcaatccta ctagaatatg gcttttttga tgcatatgct 60
ggagaatcaa aggatcattc acccgactgg tgaaattcta cacatgggaa cattgtttcc 120
atgaaactct gcctctttct cccatcacga catgccaatc ga 162

<210> 22
<211> 88
<212> DNA
<213> Lycopersicon esculentum

<400> 22
tcgatttacc agttcccgga gtgtccataa agcaagtaac ctcttttcca tgctttacca 60
atcctgctat agtactcttt ccctttaa 88

<210> 23
<211> 166
<212> DNA
<213> Lycopersicon esculentum

<400> 23
ttaaactcct tgacctaaca ttttcatgaa ataaatcaga caaacaattc ccaatattat 60
ctggtgaaaa ttttactaac acctccaaat ctttttcccc actcaaaact accatatagt 120
actcataaaa atccctataa aaaggcacca atttccttgc aatcga 166

<210> 24
<211> 104
<212> DNA
<213> Lycopersicon esculentum

<400> 24
ttaattattc aataattcta tattgatgta tttgttattt gactttgatt acaattttgt 60
tggagcgtgc ctttgatttt ttggtttggt tttgcactat tcga 104

<210> 25
<211> 121
<212> DNA
<213> Lycopersicon esculentum

<400> 25
tcgattacaa acacgatttt gtgggatacg ttgcagtcat tcttgttgga atatcagttg 60
cttttctctt catttttgct tactcaatca aagcattcaa cttccaaaaa agatagttta 120
a 121

<210> 26
<211> 104
<212> DNA
<213> Lycopersicon esculentum

<400> 26
ttaaagccac ctagatctcc aagtccaaga ccgagtggtt tgaaatcctc agcaactgcg 60
gaagtaacat cagcagttac tggaggcaac gatgtggcaa tcga 104

<210> 27
<211> 239
<212> DNA

<213> Lycopersicon esculentum

<400> 27
ttaaaccaga gaagcgtcat ggagtatact tgagaaaaat gttcctcctt ctgcatctga 60
ttctcaaact tatagcgtct ttggtggtgc aaacttcaca ttcatactaa acagattcat 120
aaatatatca ttggttacaa agtcaggtaa acctaccaac caaattgtag agaaccgtgt 180
acactatgtt acaatgagct ggttgctaag ggagttcaac tgctatcctc ctcaatcga 239

<210> 28
<211> 76
<212> DNA
<213> Lycopersicon esculentum

<400> 28
ttaaaccaca ctaagcatat aaaaacatga tcccttacct agactataag tctatgtaaa 60
atatcacatg aatcga 76

<210> 29
<211> 164
<212> DNA
<213> Lycopersicon esculentum

<400> 29
ttaacgccaa tgggtgcagt atttgcaatg tacttgaagg ttttcaaagc agcaaatcca 60
gcttggtttt atatacatgt tgcttgtcaa acttctgcat atattgttgg tgttgctgga 120
tggggaactg gtctcaaact tggcagtgat tctaccggta tcga 164

<210> 30
<211> 123
<212> DNA
<213> Lycopersicon esculentum

<400> 30
ttaacgtcat catacaacat gtagaactga tccaagtact tctgttgcat ccgcattcta 60
gccttcagca gcttggattc aacaactgaa aaggattaca atggctacga attagtgtat 120
cga 123

<210> 31
<211> 110
<212> DNA
<213> Lycopersicon esculentum

<400> 31
tcgatagcct tatacccctc catcaaatca tcatcctggg ccatgtcaag gaaagattga 60
agctctagag ctctgcggta atacatcatc cctctcacag tcctagttaa 110

<210> 32
<211> 100
<212> DNA
<213> Lycopersicon esculentum

<400> 32
ttaacacttg ggctgatatc atcaaccgtg ctaaccttgg tatggaagtt atgcatgaac 60
gtaatgctca taacttccct ctagacctag ctgctatcga 100

<210> 33
<211> 101
<212> DNA
<213> Lycopersicon esculentum

<400> 33
ttaaccacta catgagtata ctatttgtgg aactatgaca ctgtc[?]gaac aaacaacaga 60
actagggcta ctactataaa agaaatctaa atctttatcg a 101

<210> 34
<211> 288
<212> DNA
<213> Lycopersicon esculentum

<400> 34
tcgatatggc cctgttcttc tcctccaatt cggttcccgg aaagttcttc tcgtttcatc 60
gcccgccgga gcggaagagt gtttcacgaa gaacgacgtc gttttcgcaa atcgccctca 120
tttgatggcc ggaaaacatc taggttataa ttttacatcg ttggcttgga gttcttatgg 180
agatcattgg agaaatctac ggagaattac ttccgttgag atgttttcaa ctcatcgtct 240
tcaaatgctt cacggaattc gtgtggatga cgtgaaatct atggttaa 288

<210> 35
<211> 320
<212> DNA
<213> Lycopersicon esculentum

<400> 35
ttaatagaat gggagaattg ttagagcttc cactttctac acaaaaccat ctgaaaagat 60
cagatgatgc ctgcgaactc tcacccattg taggtccaaa aatgggatcc tttgctttct 120
ccccagtccc agaaactgga taagctttca caaattttcc tttgttgagc cattcatcgg 180
ccggacttgg ctcgtctgaa tacttccctc cactaaaaga aaagccgtcc ccaactgaaa 240
caggagcatc cagcaaataa tcctccttat catcactact ggatcggcaa ttgaatcgca 300
aaatccttgc ctgagttcga 320

<210> 36
<211> 181
<212> DNA
<213> Lycopersicon esculentum

<400> 36
tcgatctagt acgaacacca agagaagaag actccataac atggccactt tttctcaagt 60
attttcccat gaatgttttg taatggacaa cagaggaggg aaaataaaaa gggatgagtt 120
tttaggtaaa gttagggttg aaacgggata cttatgactc aacactcaac agtaacgtta 180
a 181

<210> 37
<211> 92
<212> DNA
<213> Lycopersicon esculentum

<400> 37
ttaactagat agtctaccta aaggtgtaga cccgtcatac agacacaaac ctctccaaca 60
tgatgttcgc aagatagtag gaatgggatc ga 92

<210> 38
<211> 167

<212> DNA
<213> Lycopersicon esculentum

<400> 38
ttaactcttt gtacaaagta aagatctcta ttgaaatctt tcatgttttg aattatcctt 60
catgatgaaa acctcaaacc tacaatacca gaaaatccaa atatcctaaa accacttgag 120
cagccagcaa tatacaaaag ctatcgtcgt gtatgtgttt tgatcga 167

<210> 39
<211> 101
<212> DNA
<213> Lycopersicon esculentum

<400> 39
tcgatcccaa ctggtacata agggtggcag ctcaggggag aaagcctagc atgggccaat 60
cccaaattgt gtaattatga ggaccgcaac ccacgggtta a 101

<210> 40
<211> 101
<212> DNA
<213> Lycopersicon esculentum

<400> 40
tcgatcccaa ctggtacata agggtggcag ctcaggggag aaagcctagc atgggccaat 60
cccaaattgt gtaattatga ggaccgcaac ccacgggtta a 101

<210> 41
<211> 306
<212> DNA
<213> Lycopersicon esculentum

<400> 41
tcgatgatcc aggagtcatc aaaggccaag gtacaattgg aacagagata aaccgtcaac 60
tgaaggacat tcacgctgtg tttatacctg ttggtggtgg gggtttgata gctggtgttg 120
ctacctttct caaacaaatt gccccaaata caaaaattat tggagttgag ccatatggtg 180
cagcttcaat gacattgtca ttgcgtgaag gacatagagt gaaattatca aatgttgata 240
cttttgctga cggtgtagct gttgcactag ttggtgaata cacttttgca aaatgccaag 300
agttaa 306

<210> 42
<211> 134
<212> DNA
<213> Lycopersicon esculentum

<400> 42
tcgatgcaaa gcgaagaacc ttaccagtgg cttgacatgc cgcgaatcct cttgaaagag 60
aggggtgcct tcgggaacgc ggacacaggt ggtgcatggc tgtcgtcagc tcgtgccgta 120
aggtgtcggg ttaa 134

<210> 43
<211> 161
<212> DNA
<213> Lycopersicon esculentum

<400> 43
ttaaccgact gtttgtaggg ggtgacagtg ctggaggcaa tatagtttat aatatgatca 60

tgagagcggg gagagaaaaa ttgatcggag atgtgaaaat tttaggtgca atacttggat 120
ttccttattt gatgatacca tcggtacgca gtctacatcg a 161

<210> 44
<211> 207
<212> DNA
<213> Lycopersicon esculentum

<400> 44
ttaaccgaac agtgcacacc cctaatttca tccttactag gagtatttga aaagatttat 60
aaagatgata acttacccta caaggggtgc taacatcaca ataagcttca agagtgatca 120
agacttgatt gtccaaatcc tcagcatact tataacaagg gcaactagtt ccaagtatgc 180
tttggaatgt tggacaacac tcatcga 207

<210> 45
<211> 134
<212> DNA
<213> Lycopersicon esculentum

<400> 45
ttaacctcta tacacttcaa agcacaaaat aaatgcagcg aaaggagccg agttgataga 60
agtttgcgta tctttgttga tgaaattcac catctacttg gcagaatacc agtttttagc 120
acatttagca tcga 134

<210> 46
<211> 83
<212> DNA
<213> Lycopersicon esculentum

<400> 46
ttaatcgggc atgtctggtt taggattgga ttcggatgtg taggtctcag gaaaattatc 60
caatcctaag ggtatgggtt cga 83

<210> 47
<211> 235
<212> DNA
<213> Lycopersicon esculentum

<400> 47
ttaacgatga cttgcatgta agacaatagt tccaagccag ttggttcttt gcacttcaat 60
tttttatata tgatgcttcc tgacgatagt agtcgtgctc taatcccttc caacacgtat 120
gcacccaatc gttccatcgg ttccccagac acagacaccc gttgctctaa agcactcata 180
agagcttcag cagtagagat atcagcatct gacactgctt cagcacaggc atcga 235

<210> 48
<211> 129
<212> DNA
<213> Lycopersicon esculentum

<400> 48
tcgattggaa tttgtttgca tgattgtccg ctatctgtgt cgctgtcgtg gagaagagag 60
gagacgaaag cagcgctatt tcaccttgtt tgttgttatt ttgattatga tttactatga 120
ttgcgttaa 129

<210> 49

<211> 94
<212> DNA
<213> Lycopersicon esculentum

<400> 49
ttaactcttt gggcggtttc gtaatgttcc tcaccaacga tccgaggttg aagcatggtt 60
gaccttaaat cttagtgtac tgacccctaa tcga 94


<210> 50
<211> 207
<212> DNA
<213> Lycopersicon esculentum

<400> 50
ttaagatatc actgtctaga aagattgttt cgggttatcg gatctcttac gaaaagtaca 60
gggaatcact gagaagtggc ggagtgaagt cagttgtcag atttgcccct gacgatttgc 120
aaaattactt atcggattta ttctatggca ctgggttgtc ggagcaagca gcgacggcgt 180
acggctctgc ttccccgtcg gtatcga 207


<210> 51
<211> 235
<212> DNA
<213> Lycopersicon esculentum

<400> 51
tcgatacagc cgaaaaattc ctcaagactt atgatcatat ctttgctaca aggcctcata 60
acgaggccgc taagtatttg tcatatggtc ataagaattt ggtgtttgga acatatggac 120
catattggcg caacatgcgc aaattggtca ctctagacct tttgacacat caaaagatca 180
attcatttca atccgtcaga acagaacaag ttgatcttat gatccaatcg cttaa 235


<210> 52
<211> 165
<212> DNA
<213> Lycopersicon esculentum

<400> 52
tcgatcctaa gggtcggggg aaccccgaca gatagcgcgt ttcgcgcgta ctccgaaagg 60
gaatcgggtt aaaattcctg aaccgggacg tggcggttga cggcaacgtt aggaagtccg 120
gagacgtcgg cgggagcctc gggaagagtt atcttttctg cttaa 165


<210> 53
<211> 149
<212> DNA
<213> Lycopersicon esculentum

<400> 53
   tcgatccaaa gttcaagaag atgacatctg gactttagtc agcattctga gtaaatgcgc 60
   tcggatgtga attccgtcag cgtggttagc tcagaaatat caagtttggt ctagattgac 120
   cctttgttcg gggctcacaa ttagcttaa 149


<210> 54
<211> 209
<212> DNA
<213> Lycopersicon esculentum

<400> 54

tcgatcaaac tctctacttt cgcactaata ttagtgaaag ttgttgcaga tcccttcaaa 60
tgtagcagcc gaattgaact atccatgcta ttgaagaatt tttccaacat ttcatacttt 120
tccggcagct ttaccgagtt cacatgcttc ttccgttcag aaaccggcga tccagctacc 180
aatggagatt tcacttcacc gtagcttaa 209

<210> 55
<211> 210
<212> DNA
<213> Lycopersicon esculentum

<400> 55
tcgatcctga ttcagtgtat gaaacatggc atattttgta ttcgggagct actaatatgg 60
attcggccac ttatattcct gatactttgg agacagaaac aagacaagac ttgcagttca 120
ctgtagataa accttctaac ctgtctcagc atggggtcaa acagaatgat ggattggttg 180
aagtattgct tgatcaatct attagcttaa 210

<210> 56
<211> 240
<212> DNA
<213> Lycopersicon esculentum

<400> 56
ttaagcctaa aaacaagcgg aaaaagaaga aatcgtctaa tactgccgca gtttagagaa 60
tgcatgtgtg atctatcttc ttgaggtacg tagttgatta tgattgaaat tactctgcac 120
ggtaatagta aattctgtga ctcgttgcac agttggagag gcctcatgaa gttccagatg 180
caagcagttg tatcattgtt tctcaaattg aaggacatga attgaaacca ttgccatcga 240

<210> 57
<211> 83
<212> DNA
<213> Lycopersicon esculentum

<400> 57
ttaatcgcgt caagatctgg attctgcagg cgggaagtga tcaacaccac gtcatctccc 60
tcctccctaa tcaagtgggt cga 83

<210> 58
<211> 293
<212> DNA
<213> Lycopersicon esculentum

<400> 58
tcgatggatc agaagaaacg gttagcccta gaaagtaaag gtgatctgtt acctgacaaa 60
actgctggaa ggaaagaagc gggagttcct tcccaggaca atgctcagaa ggatctaaat 120
tctcttccag ccaaatcaat tgatgatgag tacagacaga gggccagcag tgatatagat 180
gtccgtaggg gtgattcaga tgagcttatg gacaaaagaa ctgtcaagaa agaggaagat 240
ggcacttttt tgaagcctaa atctgatgcc aaatcagctg atgcaacagt taa 293

<210> 59
<211> 87
<212> DNA
<213> Lycopersicon esculentum

<400> 59
ttaactgaat atggaacgtc tgatctggac gtaagagtca gacattgctt ttggcaatgt 60
ggatactact attattgcgc ccatcga 87

<210> 60
<211> 232
<212> DNA
<213> Lycopersicon esculentum

<400> 60
tcgatgaaaa gcttcgtgtt gaaatgtctc cacctttggc cttgttgatt tcagaaactc 60
tatatgcttg cttactcttg agcaacatgg tccacggaca gccctatgag aatattccag 120
agatcacgta gaaaaggaac agtgggtttc tgccgacgga cccggtcaag cttcacatca 180
gctatgaact gcaagtggca gctcgatgaa ctgggatttt gcgttgcttt aa 232

<210> 61
<211> 280
<212> DNA
<213> Lycopersicon esculentum

<400> 61
tcgatgtgat aaacctataa ggctcatcct tcttagccca atattgttgt tgatccttct 60
ttgatgttac ctcttgcaag aaatctgcca cgcctttcct ctcggggcat ttgaatccca 120
tggattcaaa gaagtcaaga acatcttccc gaggaccctg atagacaatt tgtgcatctg 180
acagcagaat tatgtcatca aacaagttgt aagtctcggg ggctggctgt aacagagata 240
ttacagcagt tcccttcaag agctgcaccg attgtcttaa 280

<210> 62
<211> 375
<212> DNA
<213> Lycopersicon esculentum

<400> 62
tcgatgtgtg aatatgatag cacgtttatt caatgcgcct ttagaagagg aagaagcagc 60
aattggtgtt ggcacagtgg ggtcatcaga ggccataatg ttagcgggcc tagccttcaa 120
gaggaactgg caaaacaaac gcaaagctga gggaaagcct tatgataagc ccaatattgt 180
tactggcgct aatgttcagg tgtgttggga gaaattttgc aacttacttt gaagtgtgca 240
attcgagcac tctagctcct cgaggacggg tgagtacgac gatgacgcca aaccaccagc 300
caccgccaga aaaaaggtca aacgcgacaa caaccaaagg cgtgcacaag caaaaacgag 360
ggtccaaacc cttaa 375

<210> 63
<211> 102
<212> DNA
<213> Lycopersicon esculentum

<400> 63
tcgatcccaa tttgtatata agggcagcag ctcaggggca aaagcctaag atgggcaaat 60
cccaatttga ggaattatga ggacagcatc ccaggggntt aa 102

<210> 64
<211> 117
<212> DNA
<213> Lycopersicon esculentum

<400> 64
tcgatcacat aggcatgttt ctggtcctgc ccgcagcttg attgatactc ttcaagctgc 60
tggtttaggc gctactggtg ttatgtctgt cctaataaag caatctggtg gacttaa 117

<210> 65
<211> 231
<212> DNA
<213> Lycopersicon esculentum

<400> 65
ttaagtgaca gtatcttatc attgatagct tgcattgtgg acgtaatgct catggagcca 60
tcatcttcag ggcgaccaag cttgcttata tcctgataaa acctaaatac cagccctgga 120
gaatggtgca gtatatgata atactgctgc acaaatgcat ttcccacaac ttgagcagat 180
acaggtagtt gagccgccgc cgccgccact gccgccgcca tcgccgatcg a 231

<210> 66
<211> 273
<212> DNA
<213> Lycopersicon esculentum

<400> 66
ttaagtttgc tcacatcatt gttttctttc ttcttgcgac ttcctttgaa actctcatgg 60
cacgaaaaga aattgatggg ccagaagtca tagaacttct aaaggaattt gactctaact 120
tgatgtgcga aggacaacaa atgtggccca gaacttattg gtgtaccaac aaagcttgct 180
aaggaaataa ttgagaagga aaatccatcc ataactaata ttccaatatt attgagtggt 240
tctccaatta cattagatta tctatgtgat cga 273

<210> 67
<211> 217
<212> DNA
<213> Lycopersicon esculentum

<400> 67
ttaataccac taaacatgtc agttgatttg tttcgtgtag tgtcatatgc acacatcagg 60
tcaagtaaaa ccttcatatg cctggtgata tatgttacct gcaccgagtt tggaaaattt 120
agatcttgaa acttattgta agtgcgagta ttgacatgtg caggttgatg ttccctcttg 180
ctttgttggt ctggtattgg aattggagaa tgatcga 217

<210> 68
<211> 104
<212> DNA
<213> Lycopersicon esculentum

<400> 68
ttaaataaat acatgtggtg gcgtgatttc tccaaacaaa atgtttctct atgagcaagg 60
ttttctttca atttcagcat tgaaccagaa tgacatggtt tcga 104

<210> 69
<211> 117
<212> DNA
<213> Lycopersicon esculentum

<400> 69
tcgatcccat tatggttctt tcagtcctat ttcaccattt ccttcaatga catgtgactt 60
gaggggacct aatgcaatgg cagaggatga aaaggcggaa accttggtgg gtattaa 117

<210> 70
<211> 82
<212> DNA

<213> Lycopersicon esculentum

<400> 70
ttaatacgcg atagaaaaca aaatatagcg cgcaaactag gataaattat cgcgcgcggt 60
gtcatctatg ttactagatc ga 82

<210> 71
<211> 80
<212> DNA
<213> Lycopersicon esculentum

<400> 71
tcgatggagc tttcaggacc tagatgatga catctgcttc tattcttgcc ttgaaaatct 60
gcctctgcct gtattattaa 80

<210> 72
<211> 85
<212> DNA
<213> Lycopersicon esculentum

<400> 72
tcgatgagtg gacgagagaa tgaaaagcac caccagtacc agcagctctg tttctgtata 60
atacattgtt atctatgggt attaa 85

<210> 73
<211> 89
<212> DNA
<213> Lycopersicon esculentum

<400> 73
tcgatgctag aggtgtcgaa aagttaccac agggataact ggcttgtggc agccaagcgt 60
tcatagcgac gttgcttttt gatccttaa 89

<210> 74
<211> 253
<212> DNA
<213> Lycopersicon esculentum

<400> 74
ttaagtactt gaagcacaag attcatgtga ttgatgacaa gaatttggta acaaaatatt 60
cacttattga aggtgatgtt cttggtgaca aattggaatc aattgcctat gatgtcaaat 120
ttgaagctgc tggagatgga ggttgtgttt gcaagacaac aactgagtat cacacaaagg 180
gtgatcatgt tgttagtgaa gaagaacaca atgtaggcaa agggaaagcc attgacctat 240
tcaaggccat cga 253

<210> 75
<211> 216
<212> DNA
<213> Lycopersicon esculentum

<400> 75
ttaagtagcg cgtctgctgc tccatacaag ccaaccacgg cctccagaag aagatgttgg 60
cgacctcgta ttgggaatcc ccgaacatcg cctcgctcca gtcaatgacc gctgttatgc 120
ggccattgtc cgtcaggaca ttgttggagc cgaaatccgc gtgcacgagg tgccggactt 180
cggggcagtc ctcggcccaa agcatcagct catcga 216

<210> 76
<211> 127
<212> DNA
<213> Lycopersicon esculentum

<400> 76
tcgatgcaga cacaaatata aacccagata tgtggaaata ggaacacaat aacaatggag 60
gaactaagtg tattgataaa cgacatattc tagagcctcc ccttctttcg cactctctca 120
tacttaa 127

<210> 77
<211> 150
<212> DNA
<213> Lycopersicon esculentum

<400> 77
tcgattatat ggattgggct cgcaagaatg gccctttctt cgtaaatgat acattagttt 60
tcaagtatga tgcaccaaac gcaaatggag gatttccaca cagtgtatac ttattaccaa 120
actattggag ctttatcaag tgcgacttaa 150

<210> 78
<211> 182
<212> DNA
<213> Lycopersicon esculentum

<400> 78
ttaagttcaa gtggaaaggt tatagatata actcctggag ttagcgccat gtggaccttt 60
gcggtgaaga aattgacctt ctcaaagaaa cagctggtgc cactgcttct gattccgaag 120
aaggaaaatc tggaattgat tgttgggctt gtgaaggaag ggaaactgaa aacagtaatc 180
ga 182

<210> 79
<211> 90
<212> DNA
<213> Lycopersicon esculentum

<400> 79
ttaacgccta cattttatga ttgcacttgc ccaaatgtca tacacattgt acgtgatgtt 60
atggagcaaa ttcaacgtag agatgttcga 90

<210> 80
<211> 92
<212> DNA
<213> Lycopersicon esculentum

<400> 80
tcgattccat tatggccacc ctgatatttt tgacagactt ttccacctta caaggggtgg 60
gattagcaaa gcttctaaga ttatcaactt aa 92

<210> 81
<211> 77
<212> DNA
<213> Lycopersicon esculentum

<400> 81

tcgattggcg gtgcgaacaa tggaaatgaa gccatttgtg atgaatgcga ttgaattagc 60
aatttgctga aacttaa 77

<210> 82
<211> 121
<212> DNA
<213> Lycopersicon esculentum

<400> 82
tcgattggca gaacaatcaa aatgaaggat caagggatag agcacctggt ttagggacag 60
aggccattga gccaccaaat tggccattac aattctgtga agtttgggat accggcctta 120
a 121

<210> 83
<211> 90
<212> DNA
<213> Lycopersicon esculentum

<400> 83
ttaagggccg tcagggagac ttttacatct tggtcatgga catgctggga cccagtttgt 60
gggatgtctg gaattcttta ggccaatcga 90

<210> 84
<211> 147
<212> DNA
<213> Lycopersicon esculentum

<400> 84
ttaatgagat atgcgagacg cctatgatcg catgatattt gctttcaatt ctgttgtgca 60
cgttgtaaaa aacctgagca tgtgtagctc agatccttac cgccggtttc ggttcattct 120
aatgaatata tcacccgtta ctatcga 147

<210> 85
<211> 164
<212> DNA
<213> Lycopersicon esculentum

<400> 85
ttaatgccaa tgggtgcagt atttgcaagg tacttgaagg ttttcaaagc agcaaatcca 60
gcttggtttt atatacatgt tgcttgtcaa acttctgcat atattgttgg tgttgctgga 120
tggggaactg gtctcaaact tggcagtgat tctaccggta tcga 164

<210> 86
<211> 150
<212> DNA
<213> Lycopersicon esculentum

<400> 86
tcgataacag tggatatcat tatcaatttg tctttgtgga ttccagacaa caattacttt 60
tgagaccacg ccgcactgat ttcctcagta tattacatgc tgctcctttc ttgagttcca 120
tcaccaccaa gttatataga acctgattaa 150

<210> 87
<211> 145
<212> DNA

<213> Lycopersicon esculentum

<400> 87
ttaatcgccc aattcagctg caccagtcca ttctcatagt attttcttct cagaagggac 60
cacactcata ttgtatacaa ttgggggact ctttataata ttcagcaaga gaaatgaagg 120
cgctttctca cccttcttgt atcga 145


<210> 88
<211> 106
<212> DNA
<213> Lycopersicon esculentum

<400> 88
ttaatcccta agtaattgtt agcaagaagt ttgaatccac aaggtgtgca atatgtcatg 60
tgaatgtgca tgtccattct accaggtcgt aacaatgcag gatcga 106


<210> 89
<211> 97
<212> DNA
<213> Lycopersicon esculentum

<400> 89
ttaatctgtt gatctttgtg agagtctttt gttttgagct atgtgatcaa tgacggaact 60
agagcggcga actggtattc acctgagccc ccatcga 97


<210> 90
<211> 164
<212> DNA
<213> Lycopersicon esculentum

<400> 90
ttaaatcatc agcttttgag agaagacatg gcaaacaaat cttacgagga ggccattgtt 60
tccctccaga accttatcag tgagaaggga gagctgggac catttgtagc agaaagaatt 120
gatgaaatga cagctgagtt acaaacaagc agtaaaccgt tcga 164


<210> 91
<211> 323
<212> DNA
<213> Lycopersicon esculentum

<400> 91
tcgatgtaac tgcgtaccga tgtaactgcg taccgatggg agatgtgctg tggaatcttg 60
agtttgctct gcaacttcag gagagtgcag aggagtgtgg aaagggtttt gggaaaatgg 120
acattgaaga agggtttgat gatgttacat gcaaaggaaa gaaggatttg aatgaatccc 180
caggttacga tgcaagcatg actgattcaa gaagcagtgg aatatctatg agcattggcg 240
gccgcagcct tgccagcgaa gactcagatg ggctaacacc tagtgctgtt ttctctcaaa 300
tcatgaatcc aaaaggacat taa 323


<210> 92
<211> 104
<212> DNA
<213> Lycopersicon esculentum

<400> 92
ttaatgtttg gatacaggta gcccagcgac gtgggtgcgc tgcaagtcgt gcaccaaagg 60
ctgcgaatca aacaatcgtt tgtacgattt ttcaatatca tcga 104

<210> 93
<211> 78
<212> DNA
<213> Lycopersicon esculentum

<400> 93
tcgaggtgaa ccagtttttg gttgtgactt ctcggtattt tcggtgacag ctaagttcta 60
taacaagaga tggtttaa 78


<210> 94
<211> 90
<212> DNA
<213> Lycopersicon esculentum

<400> 94
tcgagaacat agctaatcgt ccattcttga tttccttgac cttcaactca gcaaatgcct 60
ccggatcatc agcaaggccc agtgggttaa 90


<210> 95
<211> 165
<212> DNA
<213> Lycopersicon esculentum

<400> 95
tcgagctcgt cgcatagtgt gatttgaacg gttctattta gctgtttgac attattctga 60
gttacatatc cttataagga tggaaagcta aagacaaagg actaacggcc ctaattagca 120
cagccattga ttttcaccta tagaagtgag atacatttct tttaa 165


<210> 96
<211> 107
<212> DNA
<213> Lycopersicon esculentum

<400> 96
ttaaaagtac gagctccaac attttctccg atcaaatcac tccattgatc tccaatatta 60
ccaataattc tatatccagc cttcaccaac gctgttctct tgctcga 107


<210> 97
<211> 103
<212> DNA
<213> Lycopersicon esculentum

<400> 97
tcgagccgag tcactgtacc atccataaga ttagccatgt caaaattgtt gaagcctgat 60
ccggaattgg ctgaaacaat ttatagcaaa tgctcaagtt taa 103


<210> 98
<211> 72
<212> DNA
<213> Lycopersicon esculentum

<400> 98
ttaaacttcg tcaattgaca ttcctatcgc cctatatgcc tatacattgt ggttgcttca 60
ccatgagctc ga 72

<210> 99
<211> 68
<212> DNA
<213> Lycopersicon esculentum

<400> 99
tcgagcacag atagattttg caatgatgct ggtatatgac cttccaagac attatgagat 60
aagtttaa 68


<210> 100
<211> 82
<212> DNA
<213> Lycopersicon esculentum

<400> 100
ttaaagctac taacaaaaga atgctcatgg atatgcgact tagctctgaa atagagtccc 60
agcagtacaa ggatgagctc ga 82


<210> 101
<211> 90
<212> DNA
<213> Lycopersicon esculentum

<400> 101
ttaagatgca cgccctccgc gtcttttctc ttctagaatt gcagcctcat tcaacatgtc 60
agctgcatct ttgtgcatgt ctagcttcga 90


<210> 102
<211> 79
<212> DNA
<213> Lycopersicon esculentum

<400> 102
ttaaacccag tcaaaatatc ttagttgaca gacccatata tccacccaat ctctttaccc 60
cattcaatct tgtcctcga 79


<210> 103
<211> 208
<212> DNA
<213> Lycopersicon esculentum

<400> 103
tcgaggactt tttcaccttt gctgagcagc aacaacaacg cctttttatg gacaagtaca 60
actttgatgt ggtaaatgat gtaccgcttt ctggccgtta tgaatggatt agggtgagtc 120
actaagctgg tggggctgat gaaattgctg gatatttgat gttctctccc ataactaaac 180
tattgtataa ataggtggtg tagtttaa 208


<210> 104
<211> 368
<212> DNA
<213> Lycopersicon esculentum

<400> 104
tcgagggaag aattcatgcc atttataaac accgatgcga actctcatga tatcttttgt 60

tggacctaca agaggttgta tctctacgta aaaactgaat gccaattttg cagccaggag 120
gagaacccaa agtagtgtgt atttgaaaag agaaaatgca tcttcatgca tgcctcttcc 180
aacataaagc cgaggctggg accaccacat taccaaactc acaatcttgt aatctgacct 240
ctcaagaaaa cggcggatga aagggaacac aaacagcaat gcagatagca tgtttggtga 300
caggtaaaaa agaaccgcta tgatgaatag agaaggcgaa cttgaaccat tcccaaacca 360
gtttttaa 368

<210> 105
<211> 224
<212> DNA
<213> Lycopersicon esculentum

<400> 105
ttaaaacaca tattcataat cacttgctca catctggatc acttagcatg cataaactat 60
tacaaccaag gctcatctgt caacaaacat aagacacatt gctcatggag aggagccact 120
tgctacatct tcattattct tagaaaattc tattgcgtct tcatcctcat ccatatgatt 180
ctcatgattt gttgcagcag caataacaga gtctagcacc tcga 224

<210> 106
<211> 316
<212> DNA
<213> Lycopersicon esculentum

<400> 106
   tcgaggtgaa aagaagtacc actccttttc tccatacaat gccaaatcta aaacacaaaa 60
   cacaaacaaa aaaatttaga tccatctgaa tttcagaaat tttcaactgt tcagtactaa 120
   caaaaactaa tttaccagga agatcccatg gatcaaactt gtagagatca atttctgcta 180
   caattggtac agcaattggc tgtgaagtgc attttctgca taaataatgc gtcacaagtt 240
   cttcatcagt tggatgaaat ctgaatcctg gaggtaactg caattcagct gctgtcattt 300
   tttctttttc ttttaa 316

<210> 107
<211> 89
<212> DNA
<213> Lycopersicon esculentum

<400> 107
tcgaggaggt cgactaagct gttttcgctc ctcaatcctc atgtttttgg tttcgtcagt 60
tagttttatc cccaaaaact ccattttaa 89

<210> 108
<211> 82
<212> DNA
<213> Lycopersicon esculentum

<400> 108
tcgagtgcca aagatgggtc atcaatgcgc cgaggggcac taatatggca cgtatatggg 60
gtcgtactac ttgcaatgtt aa 82

<210> 109
<211> 112
<212> DNA
<213> Lycopersicon esculentum

<400> 109
ttaaacacaa aaagaagaag caagacccct tttgaagctt caggtctacc tagtcataaa 60

atgcctcttg gaggcaacct atgattcaga cactactttt gaatggcctc ga 112

<210> 110
<211> 413
<212> DNA
<213> Lycopersicon esculentum

<400> 110
tcgagtactg ataaattttg aaatgatgcc ggtatatgac cttccaagac attatgagac 60
aaattcaacg tacgaagtcc aacaagatct ccaatagtgc ttggaatacg accttcaaat 120
ctgttctttg agagattgat aatcatgtta gaagtaaaaa ttctaacaga atcataatct 180
tgtccctttg tagtaattgt cgtcaaataa ttgtaataaa tatcagaaat atactctggt 240
gttcttgtac tctcatcaat ttttttcatg gcttgcaaat tccccaaaat actttcgggt 300
aaattcccac taaatccatt agatgataga tcaagaattt gaagacgcgt aaacaagttt 360
gtattccctg aagatttgat gggaccatgc aacttatttg atctcaagtt taa 413

<210> 111
<211> 127
<212> DNA
<213> Lycopersicon esculentum

<400> 111
tcgagtaaaa ccaaggagtg ccctatctat gagcagctgt gcacaatatt tgctgactcg 60
ggtgctgatg ggaagtatgc ccaatcaagt cactatgaag gaggagccaa gttgccactg 120
tctttaa 127

<210> 112
<211> 438
<212> DNA
<213> Lycopersicon esculentum

<400> 112
ttaaaggttg ggtggaaatt tcttttaccg atttctctag gtaatctatt attgacaacc 60
tcgttccaac ttctttcact gtaaaagacc acaatatcct agattcacga cttgtctcaa 120
acaagagaaa gaaacaagca taaaatcttt tttatagata ttcaacatat gctccctatg 180
ataactgaat tcataaatta tggtcaacaa acaatacgag ccgccagata tatcggccaa 240
ggtttcatga ttaccctgtc ccacgcaaat cgtttacctg taactattca atacccctac 300
gaaaaattga tcacatcgga acgtttccga ggccgaatac actttgaatt tgataaatgc 360
attgcttgtg aagtatgtgt gcgtgtatgt cctatagatt tacccgttgt tgattggaag 420
ttggaaactg atattcga 438

<210> 113
<211> 220
<212> DNA
<213> Lycopersicon esculentum

<400> 113
ttaagagacc ccctccttgc tttgtgaata ctccacactt taaacagtac tcctcattga 60
gactgattat atagccgtat ctaccttatg tcccccacac tgacgcccaa ttccacatcc 120
ctgtggaggg ccccattagc gctataagat atggccccac aatgtcgtgt gccccggttt 180
acaactgtct cacgtgaaaa accctccccc cttaaatcga 220

<210> 114
<211> 91
<212> DNA
<213> Lycopersicon esculentum

<400> 114
ttaacacggg gcctggtaat ggcggtgcta taactggtgc tctcttcctc aaacaatttg 60
ttgacgagaa ggttcagtgg ttgcatctcg a 91

<210> 115
<211> 91
<212> DNA
<213> Lycopersicon esculentum

<400> 115
ttaacacggg gcctggtaat ggcggtgcta taactggtgc tctcttcctc aaacaatttg 60
ttgacgagaa ggttcagtgg ttgcatctcg a 91

<210> 116
<211> 415
<212> DNA
<213> Lycopersicon esculentum

<400> 116
ttaaaggaaa tctgatctcc cttgtgtgga gcatattttt agttgctaat aatttatgtg 60
ctgttgatgc cctttgcata atgtagtcac tgccatagac cttgtttcct tggcatctat 120
tgctgtcttc tgagttctct gcattgcatg gagtgtacca tgaagtcttt caacatcatc 180
aatggtgcct agttcacatg ctacacttcc tagtttgtgt acattagggt agattttagt 240
ttgttgcttg aaaatgttat tatgtgttat cttgattggc ttggaaagat gagtctctta 300
catagctttt ctcttctctt tttttttctt cgtatgaatc cgctccgagt ccataatgga 360
tttcttctgt tacattctct cgttgagccg taaaggctca aacctctact ctcga 415

<210> 117
<211> 252
<212> DNA
<213> Lycopersicon esculentum

<400> 117
ttaaagtcat tccgaaacat gttggggaac tttggaacgt acaaaccctc attgtcaaca 60
cacaacagat caaccttgat attcaagcag acatattgaa catgccccgg ctgaggcatc 120
tgctcaccaa cacgtctgct aaattgcctg cgcttgctaa ccccaaaaca agtaagacta 180
ccttggtaaa tcaaagcctg caaaccctct ccacaattgc accagaaagc tgcactgagt 240
atgttctctc ga 252

<210> 118
<211> 130
<212> DNA
<213> Lycopersicon esculentum

<400> 118
tcgagaacat atagtctacc ttttgaccta ataagaaaat tatcccacta ttcctttgat 60
atctaccaga ttattagcta aatgtccttg tacatagagt acggtcagac ttctcctcaa 120
ggttatttaa 130

<210> 119
<211> 127
<212> DNA
<213> Lycopersicon esculentum

<400> 119

tcgagagtct ggacctatcc cacaacagct tgaatgggac aattcctgtt gacctacttg 60
agctacactt tttggcaata ttcagcgtag catacaataa cttatctggt gcagtaccac 120
catttaa 127

<210> 120
<211> 256
<212> DNA
<213> Lycopersicon esculentum

<400> 120
tcgagctatc gcattatttc cacattttgg ggtgcgttag gtggtgatgt atacctaggt 60
aaatctccaa gatcatctgc cccttgtcta gatggtgtgt ttcgttacaa ttctgatgtt 120
ggaactgtcg gtacacccgt tagattcatt cctttatctg gaggtatatt tgaagatcaa 180
ctaatgaacc tacaattcaa cattgcaaca gtgaaattgt gtgttagtta tacaacttgg 240
aaagccggta atttaa 256

<210> 121
<211> 289
<212> DNA
<213> Lycopersicon esculentum

<400> 121
tcgagcagac aaaagcggca ctccccactc ttggtcgcct tatccactca aatgatgaag 60
aagtcttgac agatgcatgc tgggctcttt catatctttc tgatggaact aatgacaaaa 120
tccaggctgt tattgaagct ggagtgtgtt cccgccttgt tgagctgcta cttcattctt 180
caccatctgt tctcattcct gccttgcgca ctgttggtaa catagtcaca ggagacgata 240
tccaaactca ggttatgatt gaccatcatg ctcttccctg ccttgttaa 289

<210> 122
<211> 74
<212> DNA
<213> Lycopersicon esculentum

<400> 122
ttaacaaaaa gaactgtgtt tttgacaatt tcgtgatgga gaccgaagtg cttgtaacct 60
tatactgggc tcga 74

<210> 123
<211> 117
<212> DNA
<213> Lycopersicon esculentum

<400> 123
tcgagctgca gacgaacggg atgaagcaga tgcaacaatc tttacaactt catcatttgt 60
aagtacatcc catatcccat cagttgccag aacaatgaac tcatcttttc ctgttaa 117

<210> 124
<211> 158
<212> DNA
<213> Lycopersicon esculentum

<400> 124
ttaagccaaa acaagaaacc gaagcatgat gttccaagct ctagccgtgc aaatggttct 60
gaagactcaa ctattactag taaagatccg gagaatatga tgacgacaaa agattaccct 120
ctagctctct taggattccc gttgttagag tccttcga 158

<210> 125
<211> 310
<212> DNA
<213> Lycopersicon esculentum

<400> 125
   ttaaagtttg gtgctctgag ttccatgttc ccgcaaatca gacatgtgat ggttgagcaa 60
   tcaaaagcga cacctagtgt cttctcaaga tatggagttc acagctttcc atcaatattg 120
   attgtgactc agacatcaag agtgcgatat cacggccaga aagatcttcc ctcccttgtg 180
   aacttctaca agaggactac agggcttcat cctatggttg aagtgaccga aagtcaaatt 240
   acctacaagg cagatggtcg caaagctttt cagaaatgga aagggtcctc tttgaaggaa 300
   ctatcctcga 310

<210> 126
<211> 192
<212> DNA
<213> Lycopersicon esculentum

<400> 126
ttaaagtctc aatgataatc tcatctcata ccatctaaac gaatatcaca atttgggcat 60
aacccttaca tcaattcaac aagaccatgt ataggtcgta aaaaagaaca atattcaaat 120
aacaagaaaa ggaatgatag agtcaataag ctcataacaa aatccaaagc tacattatgg 180
cactgtcctc ga 192

<210> 127
<211> 92
<212> DNA
<213> Lycopersicon esculentum

<400> 127
ttaaatcatt agaaaccatg tttgaaacag agaccaaact tgaaccaaga aagaagcttc 60
aactagccag agaactcgga ttgcaacctc ga 92

<210> 128
<211> 159
<212> DNA
<213> Lycopersicon esculentum

<400> 128
tcgagtgcat gtgttttatt catattggat gagataagaa aaagatctgc tagagatggg 60
ctgaagacta ctggagatgg gctggacttg ggagtccttt tatcatttgg gcctggcctt 120
acaattgaga ccgttgtgct tcgtagtatg cccatttaa 159

<210> 129
<211> 105
<212> DNA
<213> Lycopersicon esculentum

<400> 129
ttaacaaata acaactctag aatgcaacac tgacaaaggg caagaagatt acctggttct 60
gcgcagcgac gagtcttcca agacgactcc acctcttcaa ctcga 105

<210> 130
<211> 227
<212> DNA

<213> Lycopersicon esculentum

<400> 130
tcgagccgac tcataatcat gtccctttgt tgtaattgtg gtcaaataat cataatgacg 60
agcgaattga tcagaaacat aatgtggggt tcttgcactc ttatcaattt ttttcatggc 120
ttgcaaattc ccaaaaagac ttattggtaa attaccacta aatccattgg atgatagatc 180
cagaagttga agctgcgtaa acaagtttgt attccctgaa gatttaa 227


<210> 131
<211> 189
<212> DNA
<213> Lycopersicon esculentum

<400> 131
tcgagcacac tttcaaacaa ccaactagtg atgtccacca acatattggg tatttgcatc 60
agcactgccc tgaacctgat gtacagcata aggcgcaggg tacagattct ctccatatcc 120
tgtttcagga tttccataat tggcagaata tgtaaaattc ggagttgctg cagaaatctg 180
gcgatttaa 189


<210> 132
<211> 159
<212> DNA
<213> Lycopersicon esculentum

<400> 132
tcgatccatg tgaagggtca gcgcgcatca tccctaaaca ccttcagatt catgctgagc 60
ttgggatgaa gttagtgact tgatgttctt ctgtctaagc aaagcactta tatattgaag 120
ttgactaccc gtgatatcga cttatcccct tccatttaa 159


<210> 133
<211> 91
<212> DNA
<213> Lycopersicon esculentum

<400> 133
tcgagatgca accactgaac cttctcgtca acaaattgtt tgagcaagag agcaccagtt 60
atagcaccgc cattaccagg ccccgcgtta a 91


<210> 134
<211> 170
<212> DNA
<213> Lycopersicon esculentum

<400> 134
ttaactacat aaatcaatgc gagtagcata ataccgacta tggttcagcg tacccaatta 60
ttcccaaaac tactagtcaa gtatgttgta aatccctcta tgggcgatca gtttagtgat 120
cacgccagca tgcctttata cctctggtag ggtatattgg gtcctctcga 170


<210> 135
<211> 228
<212> DNA
<213> Lycopersicon esculentum

<400> 135
ttaagaagga ctattacgcg attcttggtc tatagaaggg ttgttcagtt gaggagatta 60
ggaagtcgta caggaaactg tcattgaaag ttcatcccga caaaaataag gctccaggct 120

ctgaagaggc attttaattt ctgtttgcat caactggggt agagtttttt ctcctgtcaa 180
acgcacagtt ttggaaggtg ttgactgggg atgtgtaatg tccttcga 228

<210> 136
<211> 78
<212> DNA
<213> Lycopersicon esculentum

<400> 136
tcgagatcca agaggaatac gtgaaggacg aactgaagaa tctccgccgt gaacatttac 60
gggcacagga agagttaa 78

<210> 137
<211> 83
<212> DNA
<213> Lycopersicon esculentum

<400> 137
ttaaccataa tcatcatgga ttttgatgcc ggaatttcct tgtctgtgac tgaaggacct 60
tcctctttgt caccgtctct cga 83

<210> 138
<211> 423
<212> DNA
<213> Lycopersicon esculentum

<400> 138
ttaacccatc caggacatct agtgaaatgc tacccttggt tgaactctga gcagaagcgc 60
tctcctcatc accaaagtct tccacatgag aagcaggtgt tccttccagc agataccgaa 120
gaatatcccg cttggataaa cagacaccag caccagcctc ataatacaac tcaacagcat 180
cacgagtgta agcatttctt ttgtcccaag gaagaggtgg acaaccttct gaaaacatca 240
tatcaagatg agctgaaaac atgtcggtct cacagaactc ctctatgaag tcactggaca 300
tgacctctgg atacagaaga agaactgccc aatgaagaat attatttttg tctaatgttg 360
gttttcttag tccagtgagt tcttgataca tgggcttcgc aatcttcaat cctctagtct 420
cga 423

<210> 139
<211> 170
<212> DNA
<213> Lycopersicon esculentum

<400> 139
tcgagaaccc attttcaatg tgaaaattga atcgtatttg gtttgagatc tctaatgtat 60
tggaaaaact gcttaccgga gccggcgact tgaaagaggt ttccgacgac cggccaacct 120
ggaggtccgg gaggtagatt tagggttttg gatttgggtt ttagggttaa 170

<210> 140
<211> 90
<212> DNA
<213> Lycopersicon esculentum

<400> 140
tcgagaacat agctaatcgt gcattcttga ttaccttgac cttcgattca ccaaatgcct 60
ccggatcatc agcaaggccc attgggttaa 90

<210> 141
<211> 133
<212> DNA
<213> Lycopersicon esculentum

<400> 141
tcgagagggt gggattacca tcgtggtcca tgcctatggg atcacggaag ccagtggacc 60
agccctggtt gtgtgagatt tcagtaccat gtggaacaat tttagcgtgg agaagatcac 120
tcccaggttt taa 133

<210> 142
<211> 230
<212> DNA
<213> Lycopersicon esculentum

<400> 142
tcgaggtcac aatcttgtat tggaaataca gtggacattg ccttcaaatc caaaacatcc 60
aacaaagaca agaatgtgga gaggagtgat ggcatcttac tcttttgttg caatgtgtgt 120
atttcccttg gcaattggag gatattgggc ttatgggaat atgatgcctg gaaatggaat 180
tatgggtgca attgcaaagt accaccaaga gagtacacca aaatggttaa 230

<210> 143
<211> 138
<212> DNA
<213> Lycopersicon esculentum

<400> 143
tcgaggaaat caagtaacaa atgttgaaat ttgcagctag actatactag ctacctacct 60
actatgttgt aaaataaaca cctgctaagg gatatttagc atggttttct aaataaacta 120
tctttccttc tcggttaa 138

<210> 144
<211> 154
<212> DNA
<213> Lycopersicon esculentum

<400> 144
ttaacctcct ctttgtttat cccaggaaca tccactttga agacctgagc ttgtggggtc 60
tctttccaat caattttcgc atttgcaaaa gcagagattt cacgagcaga ggatggatcg 120
ttggcaatgg tggttgaaat tgggaagccc tcga 154

<210> 145
<211> 220
<212> DNA
<213> Lycopersicon esculentum

<400> 145
tcgagtcggc ctgcgcggaa gatgtaacgg ggctcaaacc atacaccgaa gctacgggta 60
tcatcttcgg atgatgcggt agaggagcgt tctgtaagcc tgtgaaggtg agttgagaag 120
cttgctggag gtatcagaag tgcgaatgct gacatgagta acgacaatgg gtgtgaaaaa 180
cacccacgcc gaaagaccaa tgtttcctgc gcaacgttaa 220

<210> 146
<211> 632
<212> DNA
<213> Lycopersicon esculentum

<400> 146
ttaagacaga tccgaggttc catagcagtg ttattgatgg tttacaagag gatgcggagg 60
gctactttgc ggggcgcttt gaaaagacca ttctttgggg ccgccctgcg acgaggggga 120
ctattatgct ccgggatatt atggttgctg agaggagtgt gtaccctcta aacgggggcg 180
cgttcttaat tactggtggg gggggcctat atgggggctt taatggggct gggtaaccac 240
agtgtggagt gaggggcggg ttttattaca cagctggggg ggggggggag aagaattcac 300
ctctcgcggg tgaaatatnt ntcctacgag tattttgagg gtcacacacc tcggaccatt 360
ttatcttgca ctgtatcggt ggcgtgtgga aagaagattt tctgtgcgtt gtcangtgat 420
gtgctgctca tgcgacttcc tcacttcgtt ctattctgct cgcgcttgct ttgtggtata 480
atcggctacg gactatgcct ggagcgcgtc ggtgagatcg atatgacgct gccagtttgc 540
tacactgata aatctttgta gttgtgggat ctgtggttac ttggcgatca ctgggttttt 600
gtcgtatact cgatattgcg gtctattgtc ga 632

<210> 147
<211> 187
<212> DNA
<213> Lycopersicon esculentum

<400> 147
tcgagtaccc tattgtttca attgaagatc catttgacca agatgactgg gagacctatg 60
ctaagctcac tgctgagatt ggggagcaag tacagattgt cggtgatgac cttcttgtca 120
ccaaccctaa gagggtcgcc aaggcaattg cagagaagac ttgcaatgct cttcttctca 180
aggttaa 187

<210> 148
<211> 116
<212> DNA
<213> Lycopersicon esculentum

<400> 148
ttaactttca cgacaacgcc tttaggtgaa gctatctgca ccgtctaaga tgatttggca 60
tcaccaacat tggtcacagt tctggtgtat gtctgaggag ttgatccaag tctcga 116

<210> 149
<211> 91
<212> DNA
<213> Lycopersicon esculentum

<400> 149
tcgagatgca accactgaac cttctcgtca acaaattgtt tgaggaagag agcaccagtt 60
atagcaccgc cattaccagg tcccaagtta a 91

<210> 150
<211> 79
<212> DNA
<213> Lycopersicon esculentum

<400> 150
tcgagaatgt caatgctgat ggtgctaagg atcttatggc tcatttggtg aagctgcaat 60
cttccattga tgaagttaa 79

<210> 151
<211> 412
<212> DNA
<213> Lycopersicon esculentum

<400> 151
ttaactgttc tttccgggcc ttacgaacat cagtggaatt agtatctttg gttactttag 60
gttcatacgc cctgaatctt ctttttggag ctccacatac tgggcagaag tatttgtcag 120
gtaatttctc aaaaggagtt ttgtcattgt aaatataccc gcaatcccgg caaatatagg 180
cttgcttgga ggtaacgcgc atggagattt tcggggcagc agcaggagct gctctagaga 240
atgatggagg agggagcaag agatgaattg atggagaaaa aaatgaagat tttagggcta 300
aaccatttgg tgcctgtctg aggccaccat tgggggctaa attagctgga attctagtgg 360
gtacttgcag ggcggccatt gcttatacaa attgcccttt cttctagctc ga 412

<210> 152
<211> 154
<212> DNA
<213> Lycopersicon esculentum

<400> 152
tcgagcatgg aaacttattc aaaactagat tgaataacat acatgcttca ctaacatcgc 60
cttacaaggg ttatacatca tgtttatcgc aacacaatgc cgcggtccca agtttcccta 120
gaaggttcat tacaacaggt gctacttcag ttaa 154

<210> 153
<211> 431
<212> DNA
<213> Lycopersicon esculentum

<400> 153
tcgaggaaaa gaactctagt agtatccccc tcggtgtgca tggtcatttt gcaaatggag 60
aggcaagttt tggacctgca tctggtttga tttcttactc aggtcacata gcacattcgg 120
gtaacatctc ccttcgttca gatagcagca caaccagtgc cagatccttc gccttcccag 180
tattacaatc tgaatggaac agtagtccag taagaatggc gaaggcagaa cggagacatt 240
ataagggttg gaggcaaagc cttctctgtt gtaaattcta agtccttttt gtttcttttc 300
gtagagtttt cagttcaaaa atatacatca tttcatcatc ctttgtcagt tacaagttga 360
tattcttagg aatgaatcac atgtcattgg taataggttc aaagggctct ttgagtgtgt 420
aatcatctta a 431

<210> 154
<211> 211
<212> DNA
<213> Lycopersicon esculentum

<400> 154
ttaagattgc tatgggtgct gcacgtggac tggatttcct tcatacatct caaaacaggg 60
tgatacatcg tgatatgaaa acctcaaaca ttttgttgga tgaaaactgg gaaagtaaga 120
tttcagattt tgggttgtcc aaaatggggc ctggaaatga atcagctact catgttagta 180
cacaagtcaa aggcacattt ggttacctcg a 211

<210> 155
<211> 130
<212> DNA
<213> Lycopersicon esculentum

<400> 155
tcgaggatga agataaagcc atagtgctcc taaactcgtt gccatcttcc tacgatactt 60
tatcaacaac cattttacat ggtaaggatt ctattgagtt gaagcatgtc acatcagccc 120
ttttgcttaa 130

<210> 156
<211> 246
<212> DNA
<213> Lycopersicon esculentum

<400> 156
ttaagccatt catcctccaa aatttcaggc acggtaatac gtgtcgtagg attcggatcc 60
aagatgcgag taatcaactt gatagcacca aaggagatcc aaggtgggca cgtaaattca 120
gcagcagaga ttttgttata gaggttcata agattggagt catcaaaagg caagtaacct 180
gcaagcagta caaagagtat gactccgcac gaccagaggt ctgctgttgc cccatcgtat 240
cctcga 246

<210> 157
<211> 35
<212> DNA
<213> Lycopersicon esculentum

<400> 157
ttaagtagac agatatcact gattgcttct ttcga 35

<210> 158
<211> 89
<212> DNA
<213> Lycopersicon esculentum

<400> 158
ttaagaagaa ggatagtatc atgtgaatgt gtatagatta ctgccaattg aatagaatca 60
ctattcagaa caagcatccc ttgcctcga 89

<210> 159
<211> 122
<212> DNA
<213> Lycopersicon esculentum

<400> 159
tcgagggata gggaatttat tgtcttctac cgagggaagg acttcttgcc ctctgcagtc 60
tcttcagcaa tagaagaacg gagaaagcaa gtttttgaag aggaaaagcg gaatggtctt 120
aa 122

<210> 160
<211> 305
<212> DNA
<213> Lycopersicon esculentum

<400> 160
ttaactgcaa tcaactcatc taacaaagtt cataccacct tctacaatca tttcagcagt 60
ggcacccata gcgccaatac cactactact caaaagagcc aacgctaact cctcctcgcc 120
ccactgtttc aaacccatac tgatactctt caaaacctct acatccacct ctagtaacca 180
gtttggtgtg catctaacta tcaagctgat gagcccggag acataagatt tccaagttga 240
tttgttgcaa ccgagggata ttttcccatg tagaacacct gcaacgaact ccaagtgtgc 300
ctcga 305

<210> 161
<211> 117
<212> DNA
<213> Lycopersicon esculentum

<400> 161
tcgagtcaac gcgtttcgga gaatgatgaa gaaccaacct attgc[?]cgga ttgataaaga 60
aaaacaataa gataaatctt gaaatgtgct taggatgctg agaat[?]gtac atcttaa 117

<210> 162
<211> 87
<212> DNA
<213> Lycopersicon esculentum

<400> 162
ttaagatttc gctcaagaac atcctagact atgcaatata ttctcagaac caatgatgca 60
agcaagaact gaatcccatt tactcga 87

<210> 163
<211> 242
<212> DNA
<213> Lycopersicon esculentum

<400> 163
tcgagtagga cggagcacga gaaactttgt ctgaatatgg ggggaccatc ctccaaggct 60
aaatactact gactgaccga tagtgaacta gtaccgtgag ggaaaggcga aaagaacccc 120
ggagagggga gtgaaataga tcctgaaacc gtatgcgtac aagcagtggg agcagacttt 180
gttctgtgac tgcgtacctt ttgtataatg ggtcagcgac ttattttcag tggcgagctt 240
aa 242

<210> 164
<211> 151
<212> DNA
<213> Lycopersicon esculentum

<400> 164
tcgagttgaa gaggtggagt cgtcttggga gactcgtcgc tgcgcagaac caggtaatct 60
tcttgccctt tgtcagtgtt gcattctaga gttgttattt gtttagattc aatatgatta 120
gtggtgtgaa tgacagtgga agtcttctta a 151

<210> 165
<211> 279
<212> DNA
<213> Lycopersicon esculentum

<400> 165
ttaagacgag ctctgaaaca gtatacccct cgtatagaag tggatttgat gaattttttg 60
caaaataaaa aagaaagaca acgttccaag tacgatccat atgacattac tggcacatct 120
tctgaagagg gatatgtagg agcttcaaag aaaaataatt tatttggaag gtattctgct 180
ggttcagttg atagtgatgg tgccagaaag tggaattctg ttccagaccc gacctatatg 240
gctagttctg taggtcactc attgtcggat gatactcga 279

<210> 166
<211> 137
<212> DNA
<213> Lycopersicon esculentum

<400> 166
ttaactgcct gcaacggtat ccttccccat tccaattatc tttgctttta ttccagtgaa 60
aaacttgaac aagaatcacc aacgaatctg ctttctctga agtattggta aaattccttc 120

cagacatctc cactcga                                                    137

<210> 167
<211> 185
<212> DNA
<213> Lycopersicon esculentum

<400> 167
tcgagtagtt gtgatgaaaa actgagatcc atttgtgtta ggtccagaat tggccataga 60
taacatcccc ttcctttcat gcttcaattc aaaattttca tcctcaaatt ttagcccata 120
gatggattct cccccagtac catctccagc agaaatatca ccaccttgca ccatgaagct 180
cttaa                                                                185

<210> 168
<211> 251
<212> DNA
<213> Lycopersicon esculentum

<400> 168
ttaaggaaag taaagacctc aaaacacaaa atgctactcc acttcaaaac aactttacat 60
aatatgcttg tactattatg tagtctatct tgctgtgact tgccttggag atagtccctc 120
ctctgaaaca tcagaaaata gatcaatact tttagacaac gcatacacaa tttcaatcat 180
gcctggcctt cttgaaggat ctttattcaa acatgaaata gccacagaca tcacattcac 240
aacactttcg a                                                          251

<210> 169
<211> 301
<212> DNA
<213> Lycopersicon esculentum

<400> 169
ttaagtcgtc tcgtcgtaaa atccgtgttg ttgacattgg ttctttggag aaaatcagat 60
ctgggaaagt tgaggttgtt cctggaatat tcaacaagtt ttcatgtgct actagtactg 120
gcttacgctc tgacccgagt agcatggggc acgtggaatc ccgtgtgaat cagcaaggac 180
cacgcaatgt tccgtattgg ccaaagttta ggttcagatt gttgaagatt tttgggaggc 240
gtcattgttg aaggcttgga ttcttgcttt catccaagga gggtaggtcc tcaactctcg 300
a                                                                    301

<210> 170
<211> 226
<212> DNA
<213> Lycopersicon esculentum

<400> 170
ttaatactag caacatgtca taagtataca tcattttagt aaggacgttg tccgatccag 60
ttgcctacag gatcatagtt gcaagaaatg aaccaccatc cgttgttgca acgtgcccga 120
ccacaaccta gtcggactga gttgcgccag actacttgag tataatgtct acactttttt 180
ccaccaacac attggttggt agcgtagtta tagcttggcc tctcga                   226

<210> 171
<211> 91
<212> DNA
<213> Lycopersicon esculentum

<400> 171
ttaatacggg gcctggtaat ggcggtgcta taactggtgc tctcttcctc aaacaatttg 60

ttgacgagaa ggttcagtgg ttgcatctcg a                                    91


<210> 172
<211> 103
<212> DNA
<213> Lycopersicon esculentum

<400> 172
tcgagaacca ccatccttnc tggagacttg ttgagcttag ccttgcagag tggctttatg 60
ctctactagg gagcacgggc gctgcaatat ttggttccct taa                      103


<210> 173
<211> 123
<212> DNA
<213> Lycopersicon esculentum

<400> 173
tcgagaatga ggttgagttc actaacataa atggcctcaa tttggtgttg atcttccatt 60
catgttatcc cgaggattct caggaggttg agctcctgaa aacatctcca taacagacct 120
taa                                                                  123


<210> 174
<211> 131
<212> DNA
<213> Lycopersicon esculentum

<400> 174
tcgagatgga cggccaaaaa taaagttact ctcccagtgt gttggtcgtg ccttgagcgt 60
aactctgtgc taggtggtga tttgttgaat ctaatattgg ataacctata gctttgtcac 120
tctgtactta a                                                         131


<210> 175
<211> 351
<212> DNA
<213> Lycopersicon esculentum

<400> 175
ttaatactag caacatgtca taagtataca tcattttagt aaggacgttg tccgatccag 60
ttgcctacag gatcatagtt gcaagaaatg aaccaccatc cgttgttgca acgtgcccga 120
ccacaaccta gtcggactga gttgcgccag actacttgag tataatgtct acactttttt 180
ccaccaacac attggttggt agcgtagtta tagcttggcc tctcggacac ccacaattgc 240
acggctgccc tccccgtgaa gtcaccacca cccttggcaa gattctcccc agcaccagaa 300
tgaatcaagt tacaatcacc agctcttgag ttggcatagt tttgtgctcg a             351


<210> 176
<211> 105
<212> DNA
<213> Lycopersicon esculentum

<400> 176
tcgagcatat gcatagcttt gatcctcctt atgcacataa tgatgtcaaa cctggtaatg 60
tcctgctaac tcatagaaga gaacaaccac ctcttgcagt attaa                    105


<210> 177
<211> 107

<212> DNA
<213> Lycopersicon esculentum

<400> 177
ttaaggcacg gtgaatgact atcaatgttt ttagagcaac cgcccaatta tgtgtctttg 60
acaaccttct cgcaagagca tgaatgcagt atgcaacatc agctcga 107


<210> 178
<211> 155
<212> DNA
<213> Lycopersicon esculentum

<400> 178
ttaagtcatt tgcaaccttg agccacaagg acgacttcct atccattttt gcaagcattc 60
cagcaatcag aacaaccaca agcggtaatc ccttacagtg tagggcaact tgcaaccctg 120
cttccagtag atcgggggga caactctctc ctcga 155


<210> 179
<211> 265
<212> DNA
<213> Lycopersicon esculentum

<400> 179
   ttaaattgaa caattgatgc attgatttan aaccgcgacc aatatgagat gaattggcat 60
   atggcatagc agggtttgta tattaacaaa aaggaaagat ttttcagtgt gaggatcggg 120
   taatacgaat gcaaagctct ggctttcagg tgcagcaaaa gggtggtctc acaagtcaca 180
   acatccggcc cctcaacatg ggttgtgctt tccttacctg cttcaggggg gcaacgatga 240
   aaaccttgtg tggcggtccc ttcga 265


<210> 180
<211> 347
<212> DNA
<213> Lycopersicon esculentum

<400> 180
   tcgagggctg gagatctctc ttatccttgc tggaaacaat ccaaacaccg gtttataatc 60
   tctctgacaa gttctaccaa ggccaggatc tgaaagagag gattcacttt ccgaaattcc 120
   aagtgacatg attcctcctt cagaagcttc atcttcctct gatcttgtac agagtaagag 180
   tagttcatat tgttctatca catttaggag cctttagtag agtactttat gttattagta 240
   attattgtaa aatctggtaa tgtacagttt tcagatatgt aaaatcataa ttttgactct 300
   acatgatccc tcattattat tgcactgaac attatggttt gtattaa 347


<210> 181
<211> 125
<212> DNA
<213> Lycopersicon esculentum

<400> 181
ttaaggcaag atttgctgat tgtcattttg atgaatcagt atacccaaca ttagggggag 60
aacataagtc attgggaaaa gagatagatt ggaattcatc atctctatct catttggatc 120
ctcga 125


<210> 182
<211> 179
<212> DNA
<213> Lycopersicon esculentum

<400> 182
ttaagggcga cagactatac tgatatagtg cagggaacga catttgtgct gcaaagggaa 60
actgaaccaa ggcaaaagtt gctttcgtcc atttcaacgg gtacaagggt agaaaaggcg 120
attgacgagt ttatttcctt gaaccaaaat gttgtgaatc ctgaactgga actcctcga 179

<210> 183
<211> 236
<212> DNA
<213> Lycopersicon esculentum

<400> 183
ttaagtcccg taacgagcgc aacccttgtc cttagttacc agcacgtaat ggtgggcact 60
ctaaggagac tgccggtgac aaaccggagg aaggtgggga tgacgtcaag tcatcatggc 120
ccttacggcc tgggctacac acgtgctaca atggtcggta caaagggttg ccaagccgcg 180
aggtggagct aatcccataa aaccgatcgt agtccggatc gcag[?]ctgca actcga 236

<210> 184
<211> 405
<212> DNA
<213> Lycopersicon esculentum

<400> 184
tcgagtttac gacagatgag gagcaagtta ttgatgggtt ggatagagct gctatgtcaa 60
tgaagaaggg tgaagtggca ctgctaataa ttgcacctga ttatgccttc ggtctatctg 120
aatcaaagca agacttaggt gttgttcctc ccaactcaac tgtatattat gaggttgagc 180
tggtttcctt tgtcaaggaa aaggaatcat gggatatgaa tacccaggag aaaattgagg 240
ccactggtaa aaagaaagag gaaggaaatg ctatcttcaa ggctggtaag tatgcaagag 300
catcaaagag atatgagaag gctgctaaat ttattgaata tgatactgac tttagtgaag 360
aggaaaagaa acaagctaaa gctttgaaga tcagctgcaa cttaa 405

<210> 185
<211> 215
<212> DNA
<213> Lycopersicon esculentum

<400> 185
ttaagttgat cgtgttactg atggaggacg gttactagta agtaactggt aatgttgata 60
tactcttctt tagtgttgga ggagccactg ctgatatctc caagtgtctc tcctttgcca 120
tccatccacc cgattgattt ccaggttgag tcggatgctc aaacactata ccaatctctc 180
ccttgtgagg tttgcacaca ttcatcttca ctcga 215

<210> 186
<211> 72
<212> DNA
<213> Lycopersicon esculentum

<400> 186
ttaataatag tggtttcatg tctgaggagg agctaaggtc tgatcctgct tacttatctt 60
actattactc ga 72

<210> 187
<211> 143
<212> DNA
<213> Lycopersicon esculentum

<400> 187
tcgagtagct ctagaagcat gtgtaaaagc tcgtaatgaa ggacgtgatc ttgctcggga 60
aggtaatgag attattcgcg aggcttgcaa atggagcccg gaactagctg ctgcttgtga 120
ggtatggaaa gagatcgtat taa 143

<210> 188
<211> 133
<212> DNA
<213> Lycopersicon esculentum

<400> 188
ttaaggatat tagatttgga tgcctcaact tcagcttcta gtatcagatt atgcaccaga 60
aggaagttta caagccatac tgcacgaaag gccttcgtct tcaacttccc ttcccctgtc 120
ttggtccact cga 133

<210> 189
<211> 171
<212> DNA
<213> Lycopersicon esculentum

<400> 189
ttaaggccct caagcctaaa gcttgagtgc aagttcccac tcacaacttt gcgtaccaat 60
attcttgtgc ctattattct tattgcggac caaataaaga agcttcattg cttggagctc 120
ttagaatcca tgaaagtatg tagacacttg ctttggtctc cctccactcg a 171

<210> 190
<211> 221
<212> DNA
<213> Lycopersicon esculentum

<400> 190
ttaaggatac aacttcaaac atgaacagaa tctggtttgt tggaaaaata agtttactaa 60
cacaccacaa cctcaaatca aacaaataaa gaatcacaat cttgaatttt tacattgaca 120
tggattgtac ttgaggagct tgattcactt gttggttccc atagtgtcgc tgagcaacta 180
taccattcca ccctagagcg ggatcaagtc ccctgtttcg a 221

<210> 191
<211> 297
<212> DNA
<213> Lycopersicon esculentum

<400> 191
ttaagtactg gttagatgtg ttcaacaccc tttccatcgt attattgctg catggtgtgc 60
gtgatgaact caccaactca cttctgtagc catctccaaa acgtaacgta gtgttgtttt 120
gggaatgact atgataccga ctaacaactc cggtacccta gtcttcccac cgtctctgct 180
cacctactgt ctcagaagtg gtaacgaatg tcgtgtgtga tacactcacg ccaacctgtc 240
gctgatgatc gctgagcaag ggtgtcttca acatcatcat ctccactcac ttctcga 297

<210> 192
<211> 178
<212> DNA
<213> Lycopersicon esculentum

<400> 192
ttaatgatac ggacccatgc ctgtgggtaa gccttctttg cctcctgcac ctcagccaag 60
acctgggttg catcagtgca cccgaacatg ggcaacttcc acatggtcca gtatctgcca 120

tcgtagtatc ctggtgactt atggttctca cggtacacaa atccgtgctc agtctcga   178

<210> 193
<211> 102
<212> DNA
<213> Lycopersicon esculentum

<400> 193
ttaatctgct tgcagctgac cattgtccac catccatccc tacgacaaag aagatatggc 60
catcaatgtg ccatgactgg acagtatctt ctggattctc ga                    102

<210> 194
<211> 228
<212> DNA
<213> Lycopersicon esculentum

<400> 194
   ttaatcagca ataatgacca ctgccaaccc tatggataac actcagaagc gaatcaccgt 60
   agctactacc aacgaaccaa caacaccata cggcattgga tcagggctca ttgatcccaa 120
   cagagcactt gatcccgggc taatatacga tgcaactcct caagactatg tcaatcttct 180
   atgctccaag aatcaccaca actgctcaaa tccatcagat gatctcga              228

<210> 195
<211> 142
<212> DNA
<213> Lycopersicon esculentum

<400> 195
   ttaatcggga aaacaaacat caacaacgta ctcagtatac tcctataagt ggagtctaga 60
   gaaggtaggg tgtacaatgg gcttaccact acctttgtat tgtagaaggg ttgttgccga 120
   tagacccccg actccgtctc ga                                           142

<210> 196
<211> 238
<212> DNA
<213> Lycopersicon esculentum

<400> 196
   tcgagattgg tttgggatac cagaaaatgg aagaatacac tctcaggaca tcttgaatgg 60
   tgtaaaattg aatgtacagc ggctattgaa ccacgcagat actcaacatc aagagtaatc 120
   gcaacatgaa caagcgaagg atcgcaaacc ccagttttcc ccaattttct tctttcatta 180
   gaagtgcatt caccggcatt tcggaataca ggggctttac gaaatgagaa acgattaa   238

<210> 197
<211> 65
<212> DNA
<213> Lycopersicon esculentum

<400> 197
   tcgaaacttt tcagtgataa aaagcttgag agaaagtgaa aatctacagg taaaagatga 60
   cactg                                                             65

<210> 198
<211> 88
<212> DNA

<213> Lycopersicon esculentum

<400> 198
ttaaaatgaa attgatcggc aagctacaag atggcacagt cttcataaaa aagggccaca 60
atggagaaaa tgaagacgag cttttcga 88


<210> 199
<211> 107
<212> DNA
<213> Lycopersicon esculentum

<400> 199
ttaaaccttg ggtggaaata aattgcgagg cacaattcca gcaaatattg gtaatttacg 60
aaagttgcag caactacaat tggagaacaa ttacatggtg ggttcga 107


<210> 200
<211> 105
<212> DNA
<213> Lycopersicon esculentum

<400> 200
ttaaagaaga aaaaagaagg gggaatagag ttgaagaaag gtaaattgat tatgaacatg 60
gaaataagta gagtgataga tggatttgtt gtcgttgagg ttcga 105


<210> 201
<211> 147
<212> DNA
<213> Lycopersicon esculentum

<400> 201
ttaagcggga agggactggc tgctattggg cgaagtgccg gggcaggatc tcctgtcatc 60
tcaccttgct cctgccgaga aagtatccat catggctgat gcaa[?]gcggc ggctgcatac 120
gcttgatccg gctacctgcc cattcga 147


<210> 202
<211> 268
<212> DNA
<213> Lycopersicon esculentum

<400> 202
tcgaaccctt acggccaaca atacaagcat atcctttttg aaagttggaa ctatcacttg 60
agtttgtaca gggtttcctc tcagtttcta atgaagatgg ggcaactttt tccacttggg 120
tggtaatttt atcttttacc tcactctgca aattagattt actgccagca aattcgtcct 180
gtgacgtatc tataaaatca ctctgatgaa ttttggggac caaaattgca tatctcatta 240
gtgaaacccc agctgtcatc agctttaa 268


<210> 203
<211> 153
<212> DNA
<213> Lycopersicon esculentum

<400> 203
ttaaagcaag gtggcactat gtgttacata tggacgggat gtcaaagaag ccaattactg 60
tatctactac acattttata ttcaaggata tttcattgaa aggatttctg ttacgagaaa 120
agaatttaga tgaagcacaa tataagtgtt cga 153

<210> 204
<211> 262
<212> DNA
<213> Lycopersicon esculentum

<400> 204
tcgaacgatc tctcgtaaaa tcattttcgt ttcttattca ggatctcata ctcgtctaca 60
tctttaatta catcgccgac acttactttc acctcattcc aaccccatat agttatgtag 120
catggaccac ttactggatt gctcaaggtt gtgtagggga aggaatatgg atcctagctc 180
atgaatgtgg tcatcatggc tttagtgatt accaatgggt agatgacact gttggtctta 240
tccttcactc ttcacttatt aa 262

<210> 205
<211> 151
<212> DNA
<213> Lycopersicon esculentum

<400> 205
tcgaacactt atattgtgct tcatctaaat tcttttctcg taacagaaat cctttcaatg 60
aaatatcctt gaatataaaa tgtgtagtag atacagtaat tggcttcttt gacatccctc 120
catatgtaac catagtgcca ccttgcttta a 151

<210> 206
<211> 155
<212> DNA
<213> Lycopersicon esculentum

<400> 206
ttaacgcaat ggtggcacta tgtgttacat atgtgaggga tgtcaaagaa gccaattact 60
gtatctacta cacattttat attcaaggat atttcattga caggatttct gttacgagca 120
aaagaattta catgaagcac aatatacgtg ttcga 155

<210> 207
<211> 55
<212> DNA
<213> Lycopersicon esculentum

<400> 207
ttaacccgta aacagaactt gaactcgccc aaacaatggc gggttgtggg ttcga 55

<210> 208
<211> 272
<212> DNA
<213> Lycopersicon esculentum

<400> 208
tcgaataata catcaagagg ctgatcctta gtaagagcca aacgccttct tccatcttga 60
gaaaactaca cccacattta cacataactg acccttacgg caaaatggac agaaaatagg 120
agtaggatcg caaaggacta agttgtaacc aagttttgat ggaacaatct tgaaatagct 180
gctatctctt tgccctatgc ttcctccagt ctccaataat accccgccta gagtttcatt 240
gtaatctccc actttccaaa ttgtatagtt aa 272

<210> 209
<211> 130
<212> DNA

<213> Lycopersicon esculentum

<400> 209
tcgaaggata ggatgaagat catgtaaata gttcactaga atctgatgta tttctgaaga 60
tcaatgggca ttctatctgg ggaagaacat aacgtttgag tgactataag ttacattcag 120
tacatcttaa 130


<210> 210
<211> 199
<212> DNA
<213> Lycopersicon esculentum

<400> 210
ttaaggagga gatattacca agccaggacg gtatgttacc tgacaaattg ttgtaactca 60
agtccaatgt acgtaaagga gcagtggata acgtctgtgg aattggtccg gagaagttgt 120
tcccatccaa gtataagtaa ttgaaattgt tgatgttggc tgaagcagga aatatttccc 180
ctttcaacat gttattcga 199


<210> 211
<211> 101
<212> DNA
<213> Lycopersicon esculentum

<400> 211
ttaaggcgat gacacagtgc atggaatcgc tctcgtcttt actcatttga accaataaaa 60
tctccaagaa acttgcaaga ggaataatca tatggattcg a 101


<210> 212
<211> 82
<212> DNA
<213> Lycopersicon esculentum

<400> 212
ttaattttag gcaaggaaag ctttgagagc actaaaggga ctagtgaggc ttcaagctat 60
tgttcgtggt agggctgttc ga 82


<210> 213
<211> 352
<212> DNA
<213> Lycopersicon esculentum

<400> 213
ttaatagaat gggagaattg ttagagcttc cactttctac acaaaaccat ctattgcaca 60
aaatcacatt caatgggtaa ccctgaaaag atcagatgat gcctgcgaac tctcacccat 120
tgtaagtcca aaaatgggat cctttgcttt ctccccagtc ccagaaactg gataagcttt 180
cacaaatttt cctttgttga gccattcatc ggccggactt ggctcgtctg aatacttccc 240
tccactaaaa gaaaagccgt ccccaactga aacaggagca tccagcaaat aatcctcctt 300
atcatcacta ctggatcggc aattgaatcg caaaatcctt gcctgagttc ga 352


<210> 214
<211> 219
<212> DNA
<213> Lycopersicon esculentum

<400> 214
ttaacaaact tagcattgac atcaactgac gttagaaccc cttcttcgtc aatcatgtag 60

aatccagtga tatcccctac ttcaccagat gaatcaaata cggagggttg atcaaacctg 120
aatatagcca taccatttgt tccatccctt gactttgtta gtttcacatc tggtattgtt 180
tgctcatcag ttccttgtat gaactgaatt tttggtcga 219

<210> 215
<211> 275
<212> DNA
<213> Lycopersicon esculentum

<400> 215
ttaaattata gagtaaacca actaacaata caaacatatc aaggattata ttgcttccct 60
tgcatggtgg ttcctctgat gaacaggtgg acgtgcatcc attgatgata acttatgaga 120
gatgaagtgc attgttggcc ttgagtttgg agtttggagc gaacaagagc atgctagatt 180
gatgataaaa accaaaacat cttttactcc tatcttcagg atatggaagg cgttcagtcc 240
agcaaaatgc taagctgctc aggatctaga ctcga 275

<210> 216
<211> 236
<212> DNA
<213> Lycopersicon esculentum

<400> 216
ttaagtgcag tgtacatttt tcaaccatat gataaaaaca cgacaaaagt tctcaagatc 60
cccaaaaaca cacacacaaa aaccaggaaa ggaagtatca agacccccca aaacacacaa 120
acaaaagcac ttcacacatg ataataaatc aaggaatcaa gttgcaggaa actactctga 180
tgatgcacag ttctcaaatt tctcatttgg gattcatgac agcccaatgg tgtcga 236

<210> 217
<211> 86
<212> DNA
<213> Lycopersicon esculentum

<400> 217
tcgacccatg tgaccatact gtacctctac tatcgctctt ccatttcaat ttgtttttct 60
tatttatttc ttctacttca acttaa 86

<210> 218
<211> 351
<212> DNA
<213> Lycopersicon esculentum

<400> 218
ttaatggttg agagggttgc acactattct gatatattga agatattgcg tgacctttat 60
cctactatgc aacttccaga aaagtgtgct gatgacaacc cattgatgca aaattatcaa 120
gtatcaaagg agaaggcaaa aagcttgggt attgagttta ctacccttga agaaagcatc 180
aaagaaactg ttgaaagttt gaaggaaaag aagttttttg gaggttcatc ttctatgtaa 240
aaggcttctc aaagctttta tggttttgtt gaacaatact acccacccca ccctacccta 300
cacacttttt ttttttactt ctttaagcta attatagaat caagaagtcg a 351

<210> 219
<211> 258
<212> DNA
<213> Lycopersicon esculentum

<400> 219
ttaatatgat ttgttggatt atatgtgttg ttggtattgt tgtggataat ttgggttgtt 60

gttggattgg gcatgcacag tcacagcaca cacatcaggg gccagtgctg cctggcattt 120
tcgttcagca ttcattcacc tagcttcaca tacgtcagtc cagcgcgcac catttatctc 180
aattgctggc acgcattgtt gcattgttat agactttata cctataagac agtaacatat 240
tggacagtac gagctcga 258

<210> 220
<211> 229
<212> DNA
<213> Lycopersicon esculentum

<400> 220
tcgagcaatt gtgcttagta gtagcttgtg aagaatgtaa aacttcacgg tccagttcca 60
gataagtcta cttttagcaa atcctagtag aagacagtag cattatgaga atctgatgat 120
tctcttttac atcaagtact ttagtatgtg tactatatgt tctttagggt ctttacatat 180
gcactaatgg tagttcatga agcttgaaac tgttcttgta ctatattaa 229

<210> 221
<211> 227
<212> DNA
<213> Lycopersicon esculentum

<400> 221
tcgagccgta cgtccaatat ttactgctca aggtataaat ctataacaag caacaatcgt 60
gccgcaatta gataaatgtc gcgcttacta cttatgtaag ctagctaata atctaacgaa 120
aatccggcag cacttcccct atttctttta cttcatccca atccaacaac aacccaaatt 180
atccacaaca ataccaacaa cacttataat ccaacaaatc atattaa 227

<210> 222
<211> 134
<212> DNA
<213> Lycopersicon esculentum

<400> 222
ttaatgaaag cagttatgaa gtgaaatcct tttctttgtg gtcctccact tcccatcagt 60
tgcacggagc ctaaagagat accgaaatca cctcttgaac cagaatgttg tgaagacgat 120
actggtttcc tcga 134

<210> 223
<211> 129
<212> DNA
<213> Lycopersicon esculentum

<400> 223
ttaacagcat agtgtagtga attccaaccc catacatcat attcttcgca taaatctccc 60
ttccaatgca atagtgccat cgcgcattcc gcgtgttctt gaatcacagc tgcatgtaga 120
ggtgttcga 129

<210> 224
<211> 95
<212> DNA
<213> Lycopersicon esculentum

<400> 224
ttaattcaca gtttgcaaga actcaaatat ctcccaacct agggggagga cctgctccta 60
cacttgtcaa agctgagcta ccgtggtcag ttcga 95

<210> 225
<211> 331
<212> DNA
<213> Lycopersicon esculentum

<400> 225
tcgagtgcat gagagaacat cacaaaataa aactgcagcg agtatgaaaa gagatgcaca 60
acaaaagagc cctatggtaa aaacaaattt taccgttgtg tggcagaaga caattgccag 120
gagaagcttc tcccatcccc caacacagaa gttatgcatt ttatgttcac agttcaaagg 180
attatgtatt atgacgaaca agcttcagtt atcttcacta acagatctcc tttgaccaga 240
ccccatttac atgtcatccg aactaggaaa tttcagtctt attaggtata catgtgtctt 300
aacatagtcc ctaaaaaagg ttggccatta a 331

<210> 226
<211> 142
<212> DNA
<213> Lycopersicon esculentum

<400> 226
ttaatgtagc caccggtttt cctaccagct aagacatcca taagtgtggt cttaccagct 60
ccactaacac ccattagagc tgtcaaaact cctggtctaa aagcaccact tacccctttc 120
aagagttcaa gtcggtcctc ga 142

<210> 227
<211> 105
<212> DNA
<213> Lycopersicon esculentum

<400> 227
tcgaggccta gtctgaggat cctgccttat gcacaaagat gcagcatgca ccatgcaata 60
cacctcgtgc tccttgtagc tgcttcctag acgagcatct attaa 105

<210> 228
<211> 79
<212> DNA
<213> Lycopersicon esculentum

<400> 228
tcgaggcaaa ggtgggaaat cctcacagaa ctcaaaggtc cataagcaat tgagtggttg 60
gtgaagtata cactattaa 79

<210> 229
<211> 266
<212> DNA
<213> Lycopersicon esculentum

<400> 229
   ttaatatcag ctcgttgcta ccaaaaccat actgccaaga atctacctca ggcccagcaa 60
   tcggatcgca gaaattgaaa atcgtaccaa gacatggacc ctgctatcca aatgctaaaa 120
   ctccatcgtc cgattccgag caacttatga attgggataa agttagagtt cgttcaatca 180
   ataaaaaacc taaaactact ccacttgttc ttagtaatta tgattacgga agctatactg 240
   taaaaatagg tcttggcaca cctcga 266

<210> 230
<211> 84

<212> DNA
<213> Lycopersicon esculentum

<400> 230
ttaatatcct cgctgcttgg cctacttctg tggatgacaa caaggacacc aaatccacat 60
tcaatattat atcaggcacc tcga 84


<210> 231
<211> 182
<212> DNA
<213> Lycopersicon esculentum

<400> 231
tcgagttgtg aacaccatgg tacaattttt cctattttct ccaactcttc aatacaacca 60
attacatttt tctcattttc tttgtttttt tcatcttgtt ttataatcca taaaaatggt 120
ctccctatac ctatcaaccc ttttgatatc tcctccattt ggcttattga tggattcatt 180
aa 182


<210> 232
<211> 73
<212> DNA
<213> Lycopersicon esculentum

<400> 232
tcgagtagtg aagccaatgt cgtctgatac agttccgtct ttcatctggt cagcttcaag 60
ttctactcat taa 73


<210> 233
<211> 90
<212> DNA
<213> Lycopersicon esculentum

<400> 233
ttaatggagt tcttgaaagt ggctagttat gtaaacttga tggctattga tgcaaacaca 60
acaatggttc attcatgtcc acttactcga 90


<210> 234
<211> 91
<212> DNA
<213> Lycopersicon esculentum

<400> 234
tcgagtatgc catcaaaggc aagtgaggtc ttctcttcta acttcaatgc tccaacatta 60
gcttcaagat gatcagttcc cgtgccatta a 91


<210> 235
<211> 102
<212> DNA
<213> Lycopersicon esculentum

<400> 235
ttaaggcctt ccccttcaat acattcttga tatggtactg gatgactgta ttccttgccc 60
tgagacagag cattatcttc tgtgacttcc ccgttcattc ga 102


<210> 236

<211> 157
<212> DNA
<213> Lycopersicon esculentum

<400> 236
tcgagtatag ctgcatccaa tgtaagtttt cgctttgcaa tctcatatac attctcatca 60
acagtattcc tagtaaccag cctgtatact gtaactggtt tgttttggcc tatccggtgg 120
cagcgatctt cagcttgccg atctatctgt ggattaa 157

<210> 237
<211> 144
<212> DNA
<213> Lycopersicon esculentum

<400> 237
ttaacgagtg cctggaatat tagaaggaac agtaacaggt tcatacttgt gcagcaattt 60
ccagacgctc ccatgtcaac cttcgtctct ccgctgctac atttgacttt gcattttctt 120
cttcaagctt gtgttggcac tcga 144

<210> 238
<211> 104
<212> DNA
<213> Lycopersicon esculentum

<400> 238
tcgaggaggt atctagcaca ccagatatga tctccacctt caattccaac actatgacct 60
acttgaaatg ctcactacaa tgcttgcctg tatgctggaa ttaa 104

<210> 239
<211> 104
<212> DNA
<213> Lycopersicon esculentum

<400> 239
   tcgaggagga gctgaagacc aactctactg atccaactgc acctttggtt tctctcacca 60
   aaaacctatg attcagacac tacttttgaa tggccttgaa ttaa 104

<210> 240
<211> 258
<212> DNA
<213> Lycopersicon esculentum

<400> 240
tcgagacggg tgtccttgtg acacgaagca atcggaagtg gttccaagaa aagcctctaa 60
gcttcagtca tacgagaccg taccgcaaac cgacacaggt gggcgagatg agtattctaa 120
ggcgcttgag agaactcggg agaaggaact cggcaaattg gtaccgtaac ttcgggataa 180
ggtacgcccc ggtagcttga ctgctttact gcagaagagt gaaagggttg caataaactg 240
gtagctgcga ctgtttaa 258

<210> 241
<211> 252
<212> DNA
<213> Lycopersicon esculentum

<400> 241
tcgagacttt agctagtact ggaatacatg tagcacctat aaatacattt ccgtgaacca 60

ttattcccat acatatgttc taaggctgtt ctcttggttg ggttgaatcc tccgtctggt 120
catgcacatc ttagaagata gttgaagcaa taagctcttg aagaaacaca gctctttgcc 180
ttttctcttc atactgttct tccaaggctg gaccacatac tggcattttg ctgctttgta 240
tcatatgttt aa 252

<210> 242
<211> 229
<212> DNA
<213> Lycopersicon esculentum

<400> 242
tcgagagctg ttggcatagt gagccacagt tacgcccaac attcctagag ttggtagata 60
aactcaagga tatgctgagg cagtatgcca ttcatgctca ggcagcacgt aatgttgcag 120
gaggtaacag ccagaaggaa ctataaataa agatcgtgtt atttgcgtga agcttttgtg 180
gaatagccag aagatacact gataacccag agccttcagt gctg[?]ttaa 229

<210> 243
<211> 157
<212> DNA
<213> Lycopersicon esculentum

<400> 243
tcgagggcaa atgatccatg actagtttat gataccccat ttgaggatta tgtaccttat 60
ttatgtggtt tgaactacac aaatccacag gtaggtaaat tgttacgacg catggtgaat 120
tgctcggagg ttgaaagtat ccctgaagca caattaa 157

<210> 244
<211> 158
<212> DNA
<213> Lycopersicon esculentum

<400> 244
tcgagggcaa atgatccagg actagtttat gataccccat ttgaggatta tgtaccttat 60
ttatgtggtt tgaactacac aaatccacag gtagtgtaaa ttgttacgac gcatgctgaa 120
ttgctcggag gttgaaagta tccctgaagc acaattaa 158

<210> 245
<211> 57
<212> DNA
<213> Lycopersicon esculentum

<400> 245
ttaaacagaa aagataactc ttcccgagtg ctacccgccg acgtctccgg acttcct 57

<210> 246
<211> 320
<212> DNA
<213> Lycopersicon esculentum

<400> 246
ttaaggggcc tctttctatc ttaagacaac caatctaagc acatcaataa gaaatgatat 60
gggagatgca tgtgatgaac acatgactat cttacaggat atatcatgcg agtagggcac 120
tcttttctct aaaaaaatat tatcatgaat gcgcaagaat aatgggttaa gggacaacaa 180
aaagattctt cagaggaatc gggtaggggg ttgtcttctg ggatccgctt acaaggcggg 240
taggtggagc cttcctggcc ggggggtaca aatgagggcc cgctaacggg gggaccacaa 300
atcctaaaac attcagtatt 320

<210> 247
<211> 214
<212> DNA
<213> Lycopersicon esculentum

<400> 247
ttaaataaat gttgattgac ataagaacaa ttcatattgc aaaacaaaga aaaaattcac 60
ttattatgaa taattcctat aataggtata aaatttccct gtaaagtata cagaacttat 120
gcctcattac atcacagatt agtcaataat gcaatggtat caaccaaaac taaaacaaca 180
ccgcgatttt ttcaatctga tatcagtata tcga 214

<210> 248
<211> 238
<212> DNA
<213> Lycopersicon esculentum

<400> 248
tcgatcacaa gcaatgttct ttgataagaa gcaaacatcc aata[?]tatag agctgcagct 60
ttccagctct acatttacaa tacaattatt ccgggctgct gggagcattg acttgcacta 120
tctaaggttc ttacgcactt cccaaaccag taaaggggct gctaggtgta gcttcttata 180
tattctcctg ttctgagttg acctccaccc ttgcaccctc catg[?]tatta tactttaa 238

<210> 249
<211> 372
<212> DNA
<213> Lycopersicon esculentum

<400> 249
   tcgattggat ggacctgcaa catgtgttgg ttctttgcaa actttcaaga tttcaatcaa 60
   agcatcatga aaacctgact ccgctgccag ataaagtggt gtctcctctg ccttatttgc 120
   tgggaagtcg tgttcaggat ctagtaataa attcatcaag atcctcacaa tgtcaatgtg 180
   tccacttcta acggccttgt gcaaggccgt atctccaatg tcatccgtca tcctcacaag 240
   tactgtctca ctgtccttgt atgctaagca gttcatttac cacttcactt tgtcctacat 300
   tagctgcaat gtgaagcgca gaaacattaa acattatctt ataaagtaac aaagcccggg 360
   ggtacgcctt aa 372

<210> 250
<211> 352
<212> DNA
<213> Lycopersicon esculentum

<400> 250
tcgataccaa tcaacattga gtttggccaa gaagctcagg ctgctgctac tcagaagctc 60
aagattggac tctattttgc aacatggtgg gctttgaatg ttgttttcaa tatttacaac 120
aagaaggttt tgaatgcatt tccatttcca tagcttactt ctactctttc tctggctgct 180
ggctctctta tgatgtaggt ttctatgggc tactaagatt gctgaaactc caaagactga 240
ctttgattct tggaaagcct tgtttcctgt tgctgtggct cacacaattg gccatgtggc 300
tgcaacactg agcatgtcac aagttgctgt catcattcac tcatattatt aa 352

<210> 251
<211> 179
<212> DNA
<213> Lycopersicon esculentum

<400> 251

tcgatataag gtaacttaga gaagtcctct tcttctaccc atcgttctct tccaatggct 60
ctgtcaagtt cttgttgtgc cttctccgtg atgtttggac tccgcaaaag ttcttgaaat 120
gcccattcta ttgttgctgc tgaagtatcg gttgcacctg atatcaaatc atgtattaa 179

<210> 252
<211> 373
<212> DNA
<213> Lycopersicon esculentum

<400> 252
tcgatactca gaccaagctt cagcatcctg tgaattctgt tgccaaatgt gtttggctca 60
tcaaggctaa aacctgaagt gagaagagca gtctcaaaca acaatagaac caaatccttg 120
acggacttgt cattcttatc agcatcagct ctcttcctga gctcatccat gatggcattc 180
tcaggattga tctccatggt cttcttgcta gacatgtatc cagccatgct aaagtccctg 240
agagcttgtg ccttcatgat tctctccatg taggcagtct aaccatactc tccagtaacc 300
aagcagcaag gagaatccac tacacggtca gagaccacca ctttctcaac ctagacacct 360
aaaacatcct taa 373

<210> 253
<211> 203
<212> DNA
<213> Lycopersicon esculentum

<400> 253
tcgatgtagc tggccccgta tggagcgatg aaaagaaaaa cgccacgggt tatggtgttt 60
caactctggt ggaatgggtg ctgaggaact agtcaagatg ttgatggcta aacatattag 120
ggactaatga tgtttggaaa aataaatgca tcaagttgta tgaataaagg caatagtata 180
ggctttcttg ttatcttgat taa 203

<210> 254
<211> 205
<212> DNA
<213> Lycopersicon esculentum

<400> 254
ttaatcagtg catcagacta ccggatgaaa tcacaggaat gaaaccaaag ggaataggag 60
ctgattttgc taggggatac ttgagtaatg tatgcgttgc tggagaattg caaagaaatg 120
gcctcggcta cgctcttatt tgtaaagcaa agacggttgc taaagacatg ggaataagtg 180
atctatacgt ccatgttgcc atcga 205

<210> 255
<211> 168
<212> DNA
<213> Lycopersicon esculentum

<400> 255
tcgactgccc ttgctggatt caatagtatt tttgctacct gcagaaactt gagaggacaa 60
aatacttgac gattacttgt atcggaaacg tggttgtgcc ttctgaacgg acacccttac 120
cgctactctt gaaaaccgcg actgacttat tactacaaac ttgattaa 168

<210> 256
<211> 160
<212> DNA
<213> Lycopersicon esculentum

<400> 256

ttaatcccag tcaatgccct tacttcattt atgaagcttt tgggatgtgt attctgaaat 60
gaagaatgaa gtctcttcac agctatatct cctaataatg gaaggtttac cttgtaaaca 120
cttccctgac ctccttgccc aatgcaaaat tttgcatcga 160

<210> 257
<211> 305
<212> DNA
<213> Lycopersicon esculentum

<400> 257
ttaaggggaa tcttgacatg aaacagtgga cactatatat agacattaca acagcctatg 60
tgagaaaatt attagctcca aaacgaaaag ggaaagggac ttcacctgtt atgaaacatg 120
gagattttgg atatcaattc gcaatgacat cagtttcttc tcaataattg tcaattcttc 180
tgcagtctct aaaagttcat ccagtctgtt ttgagctcta acttgatacg gcttactgat 240
gagttttaca aagtcctcca cattactagt cgtgtccaag agctccttct gcatagcctc 300
ttcga 305

<210> 258
<211> 144
<212> DNA
<213> Lycopersicon esculentum

<400> 258
ttaatcgact gtttgtaggg ggtgacagtg ctggaggcaa tatagtttat aatatgatca 60
tgagagcggg gagagaaaaa ttgatcggag atgtgaaaat tttaggtgca atacttggat 120
ttccttattt gatgatacca tcga 144

<210> 259
<211> 94
<212> DNA
<213> Lycopersicon esculentum

<400> 259
ttaatggata tatactggaa gtatgagacc cgacccactt gtgaatcatc cgaatcttgt 60
tccccatcgg agtacctccg tcaccagaaa tcga 94

<210> 260
<211> 124
<212> DNA
<213> Lycopersicon esculentum

<400> 260
ttaatatggg gtggagtgac tcaaacttgg taagtttttc atataaattt tttcttagtt 60
agaagaaagt aaacagagtc ggagtcaaca tttgagttgc tgattctaaa ttcgggtgaa 120
tcga 124

<210> 261
<211> 92
<212> DNA
<213> Lycopersicon esculentum

<400> 261
tcgatcccat tcctactatc ttgcgaacat catgttggag aggt[?]tgtgt ctgtatgacg 60
ggtctacacc tttaagtaga ctatctaatt aa 92

<210> 262
<211> 116
<212> DNA
<213> Lycopersicon esculentum

<400> 262
tcgatattgt aaacatttag tgtgtttgtt acttcattag cattggtgca agataacgtc 60
tagaccctct tgtttgtact tctctcttca gagaattgtt attacgaata aattaa 116

<210> 263
<211> 245
<212> DNA
<213> Lycopersicon esculentum

<400> 263
ttaattgcaa ggcccaaatg accaaatgca cctgccgtag aagccactga gcccactgcc 60
gtggaaatgg gaacattgaa tttagctgct ttatctatta tatcacccac attacatgtt 120
acaccaacca catcaccaaa cctattcatt ttttctagcg caccacggag acgatgctcc 180
gacgatgggt ctgaatcaac aataccgcga ggatccttga ggtacctgta caagacgtag 240
caaag 245

<210> 264
<211> 181
<212> DNA
<213> Lycopersicon esculentum

<400> 264
ttaacttgat tcagcaataa cttagctctc ttctcattat caaattcact agtctgaatc 60
tcttttagag tatccaaaac ttcaaccata gtaggcctca tgtccctatc tgtctgtaag 120
catagaaaag ccaactctgc tactgaacta gtcatttccc aaatattggt atctgaatcg 180
a 181

<210> 265
<211> 132
<212> DNA
<213> Lycopersicon esculentum

<400> 265
tcgaatgtat gcaaactctc agtgaagtga acgagaagaa atcaaggatc ctccagttct 60
cctgaatgtg tgactttgga tttggtttca atgcacgaat cgtgtcaggg aacgacttag 120
catcacgatt aa 132

<210> 266
<211> 125
<212> DNA
<213> Lycopersicon esculentum

<400> 266
ttaatgagct ctctaccgat tataacacct gcacatacat gttctgcata cttgattttc 60
ctctctattt caggtccagt tcccagtcca agaatatcca aagattccaa agttgatttc 120
ttcga 125

<210> 267
<211> 201
<212> DNA
<213> Lycopersicon esculentum

<400> 267
tcgaagttga accctccgag ctaaatttct ccgagttgaa ccagaagttg acataccaag 60
tgacattttc caagacaact aatagctcaa accctgaggt tattgaggga ttcttgaagt 120
ggacttctaa taggcactca gtgagaagtc caattgcagt tgtgtctgcc tagtcaaaaa 180
ttggctatat aagtgcatta a 201

<210> 268
<211> 163
<212> DNA
<213> Lycopersicon esculentum

<400> 268
ttaatacaca ggttgctgat ttcgggctgt caagggaaga aatggtcagt aaacatgcat 60
ccaacatccg tggaacattc ggatatcttg atcctgaata tatatcaact aggtccttca 120
ctaagaaaag tgatgtttac agctttgggg ttttactatt cga 163

<210> 269
<211> 111
<212> DNA
<213> Lycopersicon esculentum

<400> 269
tcgatctgtt gcttgtgata ctgccatatc tattccataa tctcctttgg ttgagattgt 60
gaagtgcgaa atgttcgatc ctcttcgcta tcagcgaaca gagattatta a 111

<210> 270
<211> 145
<212> DNA
<213> Lycopersicon esculentum

<400> 270
ttaatcaaca aaaggtgtaa gaaataagag cttcgggaat gacatgcttg atttttccca 60
ttgataatgg aaaacgaact tagagtagga tgtaagagtt ctgcagaacc cagtcacttt 120
gatctaaaac tgttgttgcc ttcga 145

<210> 271
<211> 97
<212> DNA
<213> Lycopersicon esculentum

<400> 271
tcgacctgat tgattacttc caatttttct acaaacacta tgtctcagtc ccaaacaagt 60
tgggcttggc tatatgaata ctcacttctt tatttaa 97

<210> 272
<211> 103
<212> DNA
<213> Lycopersicon esculentum

<400> 272
ttaacactta ccccggtgag tctcgcctgg tttcactgca tcgccggagc tgctggctgc 60
actgccttgc tagttctctc cgattattgg tgcttactgt cga 103

<210> 273

<211> 101
<212> DNA
<213> Lycopersicon esculentum

<400> 273
ttaagatccg tacaacacct gcaagtatgt gaggacttga ggaaagtaag gaagtagagg 60
ccttgcatga actagatcat gatgatgatg atgatggtcg a 101

<210> 274
<211> 76
<212> DNA
<213> Lycopersicon esculentum

<400> 274
ttaaagagct tgttcaaaca ccaacaagga gtgtagctct catcccaagg gaaaaaatcc 60
cttgcaaact cgtcga 76

<210> 275
<211> 112
<212> DNA
<213> Lycopersicon esculentum

<400> 275
tcgacggcct gtgggcattc agtctggatc gcgaagactg tggaattgat cagcgttggt 60
gggaaagcgc gttacgagaa agccgggcaa ttgctgtgcc aggcagtttt aa 112

<210> 276
<211> 112
<212> DNA
<213> Lycopersicon esculentum

<400> 276
tcgacggcct gtgggcattc agtctggatc gcgaaaactg tggaattgat cagcgttggt 60
gggaaagcgc gttacaagaa agccgggcaa ttgctgtgcc aggcagtttt aa 112

<210> 277
<211> 259
<212> DNA
<213> Lycopersicon esculentum

<400> 277
   ttaaagtggt acgcgagctg ggtttagaac gtcgtgagac agttcggtcc ctatctgccg 60
   tggacgtttg agatttgaga ggggctgctc ctagtacgag aggaccggag tggacgaacc 120
   tctggtgttc cggttgtcac gccagtggca ttgccgggta gctatgttcg ggaaagataa 180
   ccgctggaag catctaagcg ggaaacttgc ctcaagatga aatttcactg gaaccttgag 240
   ttccctgaag gggcgtcga 259

<210> 278
<211> 233
<212> DNA
<213> Lycopersicon esculentum

<400> 278
ttaaagtggc aattttattc aggcattnta ttcatctaga aaacaagaaa gcctattcta 60
ttgcctttat tcatacaact ggatgcattt attttctcaa acatcatgag cccctaatat 120
gcttagcctt caccatcttg actagtccct cagcacccat tccaccagag ttgaaacacc 180

ataacccgtg gcgtttttct tttcatcgct ccatacgggg ccagctacgt cga 233

<210> 279
<211> 91
<212> DNA
<213> Lycopersicon esculentum

<400> 279
ttaatgctgc cgcacaactc caatgcaact gctccgccac gctaactgat tccgatttgc 60
aacaattgga tactactcct actatgttcg a 91

<210> 280
<211> 78
<212> DNA
<213> Lycopersicon esculentum

<400> 280
tcgaccccga actctttgct caacttgatt atgttttgat gaatgtctcc ataatctcat 60
ggaatgtgag ggtttaa 78

<210> 281
<211> 60
<212> DNA
<213> Lycopersicon esculentum

<400> 281
tcgacgagaa agagacagga agccattttt ttgtgtgtgg gggggggaga taaaaagttt 60

<210> 282
<211> 145
<212> DNA
<213> Lycopersicon esculentum

<400> 282
ttaactaagt ctctgaaagc aacaagagtg attacaatct tgtcttgtaa taattatcta 60
tttggtttta gcgaaaaata tcttctgcta aatgaaactg gagttgaaat tgttcatttc 120
atgtagggta tgcccattat gtcga 145

<210> 283
<211> 114
<212> DNA
<213> Lycopersicon esculentum

<400> 283
ttaaccattt gctctcttga ctgcacagtt gcaagaccca tgcttcggta ccttgcagct 60
aatgcagcac ttatagccac atcattggtc cgtgtaggcc tcagtttctg tcga 114

<210> 284
<211> 214
<212> DNA
<213> Lycopersicon esculentum

<400> 284
tcgaccatca tgatttcatg actgaggtgc acatttggag gcagacatga caaaaccatc 60
tcaagggacc tcctcttgtt tatcctttat gggctacttg cacttttcat gaatatatgt 120

aatcattttg acttcaattg tttctagttt ccatctgttt tagcatattc agctaaacta 180
acatttgcaa caccaatctg ggtttttgcg ttaa 214

<210> 285
<211> 122
<212> DNA
<213> Lycopersicon esculentum

<400> 285
tcgaccaaag gtgatcctat tacaggaata gcgtcaggga cacctgttac tattttcact 60
gcccaatagc caatttggtc ccaaggtaag gaataaccag ttacgccaaa agatgcggtt 120
aa 122

<210> 286
<211> 168
<212> DNA
<213> Lycopersicon esculentum

<400> 286
tcgaccgata gtgaaccagt accgtgaggg aaaggcgaaa aaaaccccgg gaggggagtg 60
aaatagatcc tgaaaccgtg tgcatacaaa cagtcggagc ctcttcgtgg ggtgacggcg 120
taccttttgt ataatgggtc agcgacttac attcagtggc gaggttaa 168

<210> 287
<211> 236
<212> DNA
<213> Lycopersicon esculentum

<400> 287
ttaacctcag aacgattgac aagggcctga agagctgtaa gacctgcaac aataagacct 60
gcaccttttg cagctgatac tttagcgagt cttggaacag tcaaactctc attagccacc 120
gcatattcag ccgatccacc tccattcatg gcgttgagca tagccacaac cttgttacca 180
cccttgaatc tttttacatt agatcctacc tctacaaacc tctcctccca cgtcga 236

<210> 288
<211> 242
<212> DNA
<213> Lycopersicon esculentum

<400> 288
tcgactaata catgaccagc atcagctttc acagcatccc cttcaacatg aggaggttca 60
tctgtgggct tgtagattcc agcgctgctg ataaacaaaa attgcttgac accagaactc 120
ttggcccagt cagctacagg acttacagaa tccaagtctt taccattgtt gtccaaaaca 180
gcatcaaata cttcaccctc taaaatcttt ccaacatctg caggattacc ccatacggtt 240
aa 242

<210> 289
<211> 150
<212> DNA
<213> Lycopersicon esculentum

<400> 289
ttaaactaat caaatgtttg aaagccataa aggaggatgc cttctcattt tctgaatatc 60
ctgttatact cacttttgaa gaccaccttc atccttatcc acaccttcaa gaaaaagtag 120
ctcagatggt gaagagcaca tttgggtcga 150

<210> 290
<211> 95
<212> DNA
<213> Lycopersicon esculentum

<400> 290
ttaatagttc tgctgctgaa gaagtggaga atttgcctgt tgtatcaacc agtgtagctt 60
atggtgttta tatggcagtt tccagcaacc ttcga 95


<210> 291
<211> 172
<212> DNA
<213> Lycopersicon esculentum

<400> 291
tcgacttctg cactggagag ctgtgtgacg gcaagcttgt gcgactgaga gctgcgacac 60
cctttggtct ctgttgctgc cgaactctgc gacctgctgc tgaggctgcg ccggtagtga 120
atgatcatgc aagagaactt attgaaacaa aaagctcaaa tagatcggtt aa 172


<210> 292
<211> 177
<212> DNA
<213> Lycopersicon esculentum

<400> 292
ttaagatcag gaaatattct aagggtcaag caggcaaatt atggtcaaag ctagctttta 60
ttagtaggta gtatgagaag atttaggagc tcccttcagg cacttcacta ttcagttggc 120
aaagaaatgg agttgcgaaa ctagatagta tgcggaagta cttctatctg gtgtcga 177


<210> 293
<211> 85
<212> DNA
<213> Lycopersicon esculentum

<400> 293
tcgacaccaa gtgtatgggt cccaaggatt gtctctaccc gaaccccgac agttgtacaa 60
cctacataca gtgtgtaccg cttaa 85


<210> 294
<211> 140
<212> DNA
<213> Lycopersicon esculentum

<400> 294
tcgacaacaa tcaatttgtt ctgattttca tccaccgcat aacccaacaa gttcaccaac 60
tgaggatggt gcactctaga aagaatctca atctcatttt ccgccggaga attactagaa 120
ctgttacggg gttcagttaa 140


<210> 295
<211> 201
<212> DNA
<213> Lycopersicon esculentum

<400> 295
tcgacaggca taccaggttt ccatccagaa ggcactacga tatttggagg gacaaggccc 60

tgaattgctg aaagatttgc aagtgagttg gtgtctgcaa gtggttcaat gagtggctca 120
acaatcttct tgccttcatc tgttttaggt aaaccaacat caagatcagc aaagatatat 180
aattgagatg cacgcagtta a 201

<210> 296
<211> 201
<212> DNA
<213> Lycopersicon esculentum

<400> 296
tcgacaggca taccaggttt ccatccagaa ggcactacga tatttggagg gacaaggccc 60
tgaattgctg aaagatttgc aagtgagttg gtgtctgcaa gtggttcaat gagtggctca 120
acaatcttct tgccttcatc tgttttaggt aaaccaacat caagatcagc aaagatatat 180
aattgagatg cacgcagtta a 201

<210> 297
<211> 199
<212> DNA
<213> Lycopersicon esculentum

<400> 297
ttaacttggt tcctctggag gataagtttg ataacaccac agctatgctg atctatgaaa 60
gattctggaa aaagaagatg cctctcaata gaacgatgtt tggggattat gatgtgatgc 120
atattatgta tccagggctg cctttttctc caccgtctgg cattggttct ggtaacgggc 180
caactggaag tgctgtcga 199

<210> 298
<211> 153
<212> DNA
<213> Lycopersicon esculentum

<400> 298
ttaagagctg agctaactaa accattacaa cagctgcaag atactgcaag aagaatagca 60
gagatacaac gtgaatgcaa gttggagata aacattgaag agtatgtaga ggcatcagta 120
cggccattct tgatggatgt catctactgt cga 153

<210> 299
<211> 103
<212> DNA
<213> Lycopersicon esculentum

<400> 299
tcgaccccca tcagaagcag aaaggcttgg aggatttaca gtagatgaat ccgcatataa 60
atgatctttg tcatcaagat acaaggcagg aggatgatct taa 103

<210> 300
<211> 104
<212> DNA
<213> Lycopersicon esculentum

<400> 300
tcgaccactt cgccccgtct atggtgaaga tggggaacat atctcctttg acaggttcca 60
gtggggaaat caggaagact tgcaggaaga tcaactcatc ttaa 104

<210> 301

<211> 272
<212> DNA
<213> Lycopersicon esculentum

<400> 301
ttaatgagat gttcaaatcc ggtgtggctt tggacgctgt aacattcaac actatgatct 60
ttatttgtgg aagtcatggc tacttggaag aggcggaagc attgctgaat aagatggagg 120
aaagaggaat atctcctgat actaaaacat acaacatctt cctatctctt tatgctaatg 180
cagctaagat tgatagggct cttcagtggt atagaaagat aaggaggacg ggactttttc 240
ctgatgctgt gacttgtaag gctattattc ga 272

<210> 302
<211> 186
<212> DNA
<213> Lycopersicon esculentum

<400> 302
ttaaaaaccg tcaacatcca tgtagcattc tcaacttctt gaggattaga tttgacataa 60
cgatgattga aggtacagcc atcatgcata tcaactttgt gatcatcttt taggtgtgca 120
acgaggtact gaatatcacc ggtaacagcg cattcagatc ctgcatatgg gcaattgtat 180
ggtcga 186

<210> 303
<211> 102
<212> DNA
<213> Lycopersicon esculentum

<400> 303
tcgaccccaa gcctattccg ggcgactgga atggtgcagg tgctcacaca aattacagca 60
ccaagtctat gagggaagac ggaggctatg aaataatctt aa 102

<210> 304
<211> 101
<212> DNA
<213> Lycopersicon esculentum

<400> 304
tcgaccatag accacaacta catgtaacat acacaccatg ctatccagaa cccccccccc 60
acctaaaacg aagtctattg taaacaatgt ctctatctta a 101

<210> 305
<211> 140
<212> DNA
<213> Lycopersicon esculentum

<400> 305
tcgacctcaa tcagtgagtt gtcttactgt gcacatgaga ggaagcttta tatcaccctg 60
cttccttttc ttgctggtgt tgctattgct ggcatcttac tcaggactca tcgtcttatt 120
cttgcctaca ttgatcttaa 140

<210> 306
<211> 78
<212> DNA
<213> Lycopersicon esculentum

<400> 306

tcgacccgac ccatattcgt attctattcc ttgagcttca cagaccttac cgagtaaacc 60
accaatccaa ccagttaa 78

<210> 307
<211> 179
<212> DNA
<213> Lycopersicon esculentum

<400> 307
ttaagaagac ttgtggctgg agttggggtt gaaaacctat ttcatctgta ctctcccaac 60
ctaaaggaga taaaagtgtt ggacaagaaa agagtgagga gggccaagct atactatctc 120
agggacaaaa tgaatgccct gaaaaaacac taacaagcta gctcatgtgg aagggtcga 179

<210> 308
<211> 93
<212> DNA
<213> Lycopersicon esculentum

<400> 308
ttaagaacca acaggatgaa gttctctaca acccttgtgt ttgtgatttt agccttgttg 60
ctcactacaa cttatgcaga acaatgtggt cga 93

<210> 309
<211> 266
<212> DNA
<213> Lycopersicon esculentum

<400> 309
   ttaagacggg aataataaat ttcatttgaa aaggtaaagt ggcaatttta tttaggcatt 60
   ttattcatct agaaaacaag aaagcctatt ctattgcctt tattcataca acttgatgca 120
   tttattttct caaacatcat gagtccctaa tatgcttagc catcaacatc ttgactagtt 180
   cctcagcacc cattccacca gagttgaaac accataaccc gtggcgtttt tcttttcatc 240
   gctccatacg gggccagcta cgtcga 266

<210> 310
<211> 183
<212> DNA
<213> Lycopersicon esculentum

<400> 310
ttaagaccaa caatgagaaa tgtagtcaaa atgctggaaa atgcagaacc ttgcagatta 60
gttgggataa ttgtaagcaa agatgatggt agtaacaaga cagaacaatt gaaggatcac 120
accaagatgt agcctttgtt cgtttcactc ttcaacctga tctc[?]gtcct catggaacgt 180
cga 183

<210> 311
<211> 182
<212> DNA
<213> Lycopersicon esculentum

<400> 311
tcgactccca cgagtacgga atagaaggca tcgtgtgtta gacgagtttg tatttgtgtt 60
tttcccataa cattattatg gtcagagcca agagttctcg tccatcggct ttcgtgaaat 120
tatctactgc tgtatgattg atacttttat agagtaaata gaagtaatga gtttccagtt 180
aa 182

<210> 312
<211> 204
<212> DNA
<213> Lycopersicon esculentum

<400> 312
ttaatgcant acaagaccat ttatcctgcc ctgttgagga acctgttgca gagccccaaa 60
agcatactta tgcttctatt ctacaagtta ctaaagggaa ttctgcacaa ggactgggac 120
agtcttctct caacaaatct acacctcctc cttcagagtg gcaacatgtg ccacagcctc 180
ctgctttgcc atcagttcat tcga 204

<210> 313
<211> 123
<212> DNA
<213> Lycopersicon esculentum

<400> 313
ttaaactgca agtatgctat atgacttttg atagcacata cgaagttgca acagcttgtg 60
ccttagctct aggagcagaa aaactaattt gtatcataaa cggcccaatt cttgatgagt 120
cga 123

<210> 314
<211> 155
<212> DNA
<213> Lycopersicon esculentum

<400> 314
tcgactcaaa atttcctctg agcaaggctg aagatgcttg gagcaagagt attgatggac 60
atgccaccgg aaagattatt gtggagccat agagcagtgt tctcccaaat tatgtaataa 120
aatgtttgcg aggcttgtgc agtatgacca gttaa 155

<210> 315
<211> 153
<212> DNA
<213> Lycopersicon esculentum

<400> 315
tcgactccag atttcctctg agcaaggcag aagatgcttg gagcaagagt attgatggac 60
atgctaccgg aaagattgtt gtggagccat agagtactgg tctaaaaagg cttgtgtgca 120
gtttgtcatc tggctaggaa accaaaaagt taa 153

<210> 316
<211> 175
<212> DNA
<213> Lycopersicon esculentum

<400> 316
ttaagggttt ccggaatgct gccgaaccaa ggatttggtg aactagacag attgcgacat 60
agaagtccca gtcctatggg ttcagcaaac cttatgtcaa atgttactgg agcaggattg 120
agtggttgga atggacttcc ccaggagagg ttgagtggac ctcctggaat gtcga 175

<210> 317
<211> 93
<212> DNA
<213> Lycopersicon esculentum

<400> 317
tcgacccccg ggtatagaaa agatcatgtt gggagcagaa tgggccctgc aatgcacaaa 60
ttgggattag tcgggctaca atgtatgtat taa 93

<210> 318
<211> 104
<212> DNA
<213> Lycopersicon esculentum

<400> 318
ttaagggggt gttctctttg taataaaaag agaagaaata aagttgttgt aacaatatat 60
aatgcctatc tatttggttg aggttttatg ggtttttggg tcga 104

<210> 319
<211> 288
<212> DNA
<213> Lycopersicon esculentum

<400> 319
tcgaccggca tgagcttacg gagcaagtcc ttcaccctca ccggcgcacc ttctcccgaa 60
gttacggtgc cattttgcct agttccttca cccgagttct ctcaagcgcc ttggtattct 120
ctacccaacc acctgtgtcg gtttggggta cggttcctgg ttacctgaag cttataagct 180
tttcttggaa gcatggcatc aaccacttcg tgttctaaaa gaacactcgt catcagctct 240
cggccttaga atcccggatt tacctaagat tccagcctac caccttaa 288

<210> 320
<211> 212
<212> DNA
<213> Lycopersicon esculentum

<400> 320
tcgacccctc caccattctg gaaagaaatg ggaagtagta tctgccacat gatagagtta 60
gcgtatcaat tatttcggaa tattgtgaga gaagagcaat aatttggtgt tcaatttgca 120
tattgtcttc atatctcata acaatagaga ataaaagaag agaaaacaga ctgttttgta 180
ctttggattg tatctggaaa attgttactt aa 212

<210> 321
<211> 142
<212> DNA
<213> Lycopersicon esculentum

<400> 321
ttaagtccca atatcaagtg gagaaagata ccatgggctt gccgatgaaa agcaaaacat 60
atgggcacga agtgtgttga acatgagaca tgaataccct ccaattacaa gatgtcaatt 120
ggcgccaaga tcttggagtc ga 142

<210> 322
<211> 80
<212> DNA
<213> Lycopersicon esculentum

<400> 322
ttaagatgtg cttttggagc actatacctt tcatagaccg atgcacgcct cctacttatt 60
acgcgttcaa tatgagtcga 80

<210> 323
<211> 271
<212> DNA
<213> Lycopersicon esculentum

<400> 323
ttaaatatac aatttggaaa gtgggagatt acaatgaaac tctaggcggg gtattattgg 60
agactggagg aagcataggg caaagagata gcagctattt caagattgtt ccatcaaaac 120
ttggttacaa cttagtcctt tgcgatccta ctcctatttt ctgtccattt tgccgtaagg 180
gtcagttatg tgtaaatgtg ggtgtagttt tccaagatgg aagaaggcgt ttggctctta 240
ctaaggatca gcctcttgat gtattattcg a 271

<210> 324
<211> 84
<212> DNA
<213> Lycopersicon esculentum

<400> 324
tcgactagtg atttgttttt ttgacttgat ttggtgttga aaaaaaattc ctactatatt 60
tggtttgggt tggtttcaac ttaa 84

<210> 325
<211> 105
<212> DNA
<213> Lycopersicon esculentum

<400> 325
ttaagggcaa tccaagtcgg tctattatgc gtgcaacaat gtcctgaaaa taggccaaat 60
atgtcttctg tcattatgat gttgggtaac agccatggga gtcga 105

<210> 326
<211> 91
<212> DNA
<213> Lycopersicon esculentum

<400> 326
ttaatgatga acggtatcta aagaatgtgt tttggattga ttccagatca agagcggcat 60
atggttattt tggtgatgtg gttgtggtcg a 91

<210> 327
<211> 192
<212> DNA
<213> Lycopersicon esculentum

<400> 327
tcgaccaatc atctatggtg atgtgagccg ccctaagcca atgactgtct tctggtcctc 60
aaaagctcaa gagatgacca agaggccaat gaagggaatg cttactggcc ctgtcaccat 120
tctcaactgg tcttttgtca gaaatgacca acctaggttt gagacctgct accagattgc 180
tttggccatt aa 192

<210> 328
<211> 222
<212> DNA
<213> Lycopersicon esculentum

<400> 328
tcgacccgaa gctccgcaag tactacagaa attggaggag ataaaaccag agatgatgtt 60
agcagctact agttctggag atgatggcac aacagttcaa gaaaagagtg atgaataaac 120
tctgctgcag ttttggaatt tttgagcttt tcacttgata tctatttgtt tattctttca 180
tttattttta cagttagtag tagaaatgtt gtttcatatt aa 222

<210> 329
<211> 133
<212> DNA
<213> Lycopersicon esculentum

<400> 329
ttaatatctt tctcggcggt ctggagacgg gtaaacattt tgacagtgaa ggactctgta 60
taaaattgtt ttatatgctt tttacaggct gacataaact tttcaggacc aatttcattt 120
tcatatgggt cga 133

<210> 330
<211> 132
<212> DNA
<213> Lycopersicon esculentum

<400> 330
ttaatattcc tgcaccgtcg tatgatgcga tggggggacg gatcgcggaa ggttgtccga 60
ctgttggaat agacggtttt tgactcatag aaggtgctta tgcaaatccg ggcgcgtaat 120
tcaaggggtc ga 132

<210> 331
<211> 276
<212> DNA
<213> Lycopersicon esculentum

<400> 331
tcgacctgct atgaagagtc actttctatg gttagagggg aattcaattg aagaaatcac 60
catcaaatga cattgaaatg gatagttttg aagttattgt tcatgaggca agtggggtca 120
aaagttcaaa aagtattcta ttattgctca agggattagg aacaaagttg catcttgatg 180
cccagattcc cctttcacaa caaattgatg gagtaataca aaacattgta agtggcaaat 240
gagcgagttg aaatggattg agctgtgatt gattaa 276

<210> 332
<211> 144
<212> DNA
<213> Lycopersicon esculentum

<400> 332
tcgaccttgc atatccagtg agagccagcc aaagtgttgc aatgttattt gaacccctca 60
tgagataata caatcctcca atggaacaca aagcatacat actctcagta tatattgttg 120
agtagaatat ggaggcggga ttaa 144

<210> 333
<211> 124
<212> DNA
<213> Lycopersicon esculentum

<400> 333
tcgactatga ataactccgg agccctgaca atcaaaaaca aactgaacgg gtaacaccgg 60
agacgcggtc gaagtaccca atcaaaatca actatctaga ccatggctcg gaataagaga 120

ttaa 124

<210> 334
<211> 236
<212> DNA
<213> Lycopersicon esculentum

<400> 334
ttaaccaact caaaaaccat caaatgcaga tgtgttggca catagggaaa tgtaaagttt 60
gggtccccat aaatgttgac cttaggtgca cttccatatt cgcgcaagca aatagaacgg 120
gcatcctctg tagcgttgcg tgcaacctcc aagggggaca tttttgtatg tatatagccc 180
acacaatcag gaggtggatt aggatcgtgt agagcgacat gctgccctgt gttcga 236

<210> 335
<211> 271
<212> DNA
<213> Lycopersicon esculentum

<400> 335
tcgaataaga catcaagagg ctgatcctta gtaagagcca aacgccttct tccatcttgg 60
aaaactacac ccacatttac acataactga cccttacggc aaaatggaca gaaaatagga 120
gtaggatcgc aaaggactaa gttgtaacca agttttgatg gaacaatctt gaaatagctg 180
ctatctcttt gccctatgct tcctccagtc tccaataata ccccgcctag agtttcattg 240
taatctccca ctttccaaat tgtatattta a 271

<210> 336
<211> 146
<212> DNA
<213> Lycopersicon esculentum

<400> 336
tcgactagga aatacttgga ttcatggagc gctttcttat gggcaacaac acctacactt 60
ttttactcgt tcacattgag actatatact ttgaagggcc atcaacttga tgcatcgacg 120
gttgttactt gtggcgcact gtttaa 146

<210> 337
<211> 106
<212> DNA
<213> Lycopersicon esculentum

<400> 337
ttaatgaaac tttagcctta gctggtcatg accacgatac tacatgtttc gttaggtggg 60
ccaggaatgc ccgacaaatc aatttatctg gtaaactact agtcga 106

<210> 338
<211> 79
<212> DNA
<213> Lycopersicon esculentum

<400> 338
ttaatggtga taactatcag atttgggcgg taaggataga aacatatcta gatgccatgg 60
atatgtgaga ggcagtcga 79

<210> 339
<211> 403

<212> DNA
<213> Lycopersicon esculentum

<400> 339
tcgactggac atgattttgc atcccaggcc ttcctatata tgcttgagat tgttgaggtt 60
gtgagggagg tggtaaggtg gcattttgct gcggttgaat atacccatgc aaactttgag 120
gcaccacatt aggaagacct tgtgggcgaa attgagactg caacggctgc tggtgagtaa 180
aatgaccttg ctggtgagat tggcttggaa atggaccagg aaatgtatga tgactgtact 240
gttgagacat tggctgttga ttagactgac caggatgttg ctgaattgag tgtcctgctt 300
gctgagcaat aggataaagt tgagattgtg caggaacttg accttgtgaa ggtaccagcc 360
ccggttgctg attgccgatt agacccagag gtgggcgcat taa 403


<210> 340
<211> 343
<212> DNA
<213> Lycopersicon esculentum

<400> 340
ttaatagcat acgcagccga cgaggctgcc actacacaat acacaatgat gttcacaacc 60
gacaaggctg cacttgtaca aatggacatc ccaaacaaac catgtccaga ccattatcca 120
aaacataatt atacaaccca cacgatgtct acagacctct aagagtatag tagtgaaact 180
tgacgggaca gggccccgcc atacccatag acaagcaaaa gactacataa gaagttatgt 240
accaaaacga ctgggctccg gaacagtgga gctctcccaa acagcagagt agatatccta 300
ggcgggagga tcaccgaact gagcgtctac accgaatagt cga 343


<210> 341
<211> 230
<212> DNA
<213> Lycopersicon esculentum

<400> 341
tcgactcagc agtagatcaa gccagtcttc actccatcgg aaattcaggg tgagctaatg 60
cgtctagctt ggactgggtc ttcttcttca agtcttgaag ccttgaactt ccggcatgga 120
ctagattttt cgttattttt agcttcttag atattcttag atagtttagt ttagtaattt 180
gattttagat gttcttgtga tgatgacttc cagattttgg ggaatattaa 230


<210> 342
<211> 115
<212> DNA
<213> Lycopersicon esculentum

<400> 342
ttaatatgac gaccggtgag ggacttaacg aaagactgcc tcttttgcaa acagccttgt 60
aaagcaatca actgtatatg ttatttttga ctttccatat cagtttccaa gtcga 115


<210> 343
<211> 169
<212> DNA
<213> Lycopersicon esculentum

<400> 343
ttaatgatcc aacttttgct ttaccttact ggaattggga tcatccaaaa ggtatgcgta 60
tacctcccat gtttgatcgt gagggttcat ctctttacga tgagaaacgt aaccaaaatc 120
atcgcaatgg aactattatt gatcttggtc attttggtaa ggacgtcga 169


<210> 344

<211> 79
<212> DNA
<213> Lycopersicon esculentum

<400> 344
tcgacgaccc atctgaaaat cttgctgaat tttgagttat ggctgttgaa attgttgatt 60
cggatgaaaa cccgattaa 79


<210> 345
<211> 71
<212> DNA
<213> Lycopersicon esculentum

<400> 345
ttaatcggca actaatcgtc ggtggccgga gcgggcgacg acggtccctt taggtgcaat 60
ttttccgtcg a 71


<210> 346
<211> 97
<212> DNA
<213> Lycopersicon esculentum

<400> 346
ttgggtggaa atttctttta ccgatttctc taggtaatct attattgaca acctcgttcc 60
aacttctttc actgtaaaag accacaatat cctttaa 97


<210> 347
<211> 261
<212> DNA
<213> Lycopersicon esculentum

<400> 347
ttaattatgt acagagaaaa ggtacaatgt tcatggtgaa atggtattca cagatagagt 60
tctatttcct cttccacaag tatcatactg ttccattgga caaatctaaa ctaggtattt 120
cattacaagg acgaatgcaa tacaactaca gtatcaatct ccatttgata tccctatctc 180
agagtccttg gcaggctggg aatttatgtt tgtcagcgga ctagctacgc tcccacaaac 240
tgangctgca gttgctgtcg a 261


<210> 348
<211> 76
<212> DNA
<213> Lycopersicon esculentum

<400> 348
tcgaccacag aaaaggatga gctttatcta aatttggttc tagaatttgt acctgaaact 60
ctctaccgtg tgttaa 76


<210> 349
<211> 261
<212> DNA
<213> Lycopersicon esculentum

<400> 349
ttaattatgt agagagaaaa ggtacaatgt tcatggtgaa atggtattca cagatagagt 60
tctatttcct cttccacaag tatcatactg ttccattgga caaatctaaa ctaggtattt 120
cattacaagg acgaatgcaa tacaactaca gtatcaatct ccatttgata tccctatctc 180

agagtccttg gcaggctggg aatttatgtt tgtcagcgga ctagctacgc tcccagaagc 240
tgaggctgca gttgctgtcg a 261

<210> 350
<211> 96
<212> DNA
<213> Lycopersicon esculentum

<400> 350
ttaattcccc tttgcatgaa cgactatatc gtcattctac tctcaagatt tcaactcgga 60
tctctcacct tcccaaacat catattgttg tgtcga 96

<210> 351
<211> 95
<212> DNA
<213> Lycopersicon esculentum

<400> 351
ttaattcata acattatgca gcaacctgat gttagaaaaa catcaagaat ttacaatctg 60
agcacgaaat cattcacacg ctggtttcgt gtcga 95

<210> 352
<211> 155
<212> DNA
<213> Lycopersicon esculentum

<400> 352
ttaattggtc atactgcaca agcctcgcaa acattttatt acataatttg ggagaacact 60
gctctatggc tccacaataa tctttccggt ggcatgtcca tcaatactct tgctccaagc 120
atcttcagcc ttgctcagag gaaattttga gtcga 155

<210> 353
<211> 124
<212> DNA
<213> Lycopersicon esculentum

<400> 353
ttaattggta tgaatcttgg gagttatcta ggggtgaggg tgctcctcca ccggtatggg 60
atgaatttgt ggaggctttc cagggccact tcctgcctcc agagatgagg cgagctatag 120
tcga 124

<210> 354
<211> 155
<212> DNA
<213> Lycopersicon esculentum

<400> 354
ttaattggtc atactgcaca agcctcgcaa acattttatt acataatttg ggagaacact 60
gctctatggc tccacaataa tctttccggt ggcatgtcca tcaatactct tgctccaagc 120
atcttcagcc ttgctcagag gaaattttga gtcga 155

<210> 355
<211> 77
<212> DNA
<213> Lycopersicon esculentum

<400> 355
tcgacgcttg aaggcaaatg acattgcatt tgaaggcaag gaaatgctag gaataaatgt 60
catttgctca tctttaa 77

<210> 356
<211> 197
<212> DNA
<213> Lycopersicon esculentum

<400> 356
ttaaatacta cattcagact aagaagactt gtggctggag ttggggttga aaacctattt 60
catctgtact cttccaacct aaaggagata aaagtgttgg acaagaaaag agtgaggagg 120
gccaatctat actatctcag ggacaaaatg aatgccctga aaaaacacta acaaactagc 180
tcatgtggaa gggtcga 197

<210> 357
<211> 135
<212> DNA
<213> Lycopersicon esculentum

<400> 357
tcgaaatcgg aacactactg gaggaaatat gagggtctaa agcccatgtc aaaagctcat 60
caccacagaa gtcacccgcc ttgagatgaa cagagttgaa aaaccactcc ttccaccata 120
tgtgggcgta gttaa 135

<210> 358
<211> 128
<212> DNA
<213> Lycopersicon esculentum

<400> 358
tcgacccggt ttacccgggt atacttgatc cacaaactcc cctttagcga ataacccggt 60
aaaaattatt ccccatgcac cacaaccccc atgtaattgt gctgcttcaa gtggatcgtc 120
gtatttaa 128

<210> 359
<211> 127
<212> DNA
<213> Lycopersicon esculentum

<400> 359
tcgaacatgc ttactgacaa tatgacgaca attgatctgt catacaacaa cctcaacgga 60
actattcctt caaacttttc aagccttcca catttgcaga aactgtcgct agaaaacaat 120
tcgttaa 127

<210> 360
<211> 37
<212> DNA
<213> Lycopersicon esculentum

<400> 360
tcgaaactga cacgtgtccc ttcctgttga acgttaa 37

<210> 361

<211> 133
<212> DNA
<213> Lycopersicon esculentum

<400> 361
tcgaagcaac gcgaagaacc ttaccaggcc ttgacatcca atgaactttc cagagatgga 60
ttggtgcctt cgggaacatt gagacaggtg ctgcatggct gtcgtcagct cgtgtcgtga 120
gatgttgggt taa 133


<210> 362
<211> 120
<212> DNA
<213> Lycopersicon esculentum

<400> 362
tcgaatcaac tcaggatcaa acacttgtgt tgtccccctc tcttgaacta tacatttcac 60
ccatttaggc aggtcaattc cctcgtcgtt gatgacacta ctaggatttt tacccgttaa 120


<210> 363
<211> 405
<212> DNA
<213> Lycopersicon esculentum

<400> 363
tcgaaacggg gctaacaaac ttacctagcc ttgccaccca ctgaacttta taaagatcga 60
tatgagccta aggtaacatt gggacacgtg ctttatggct gttttcacca cacagtttag 120
agaggtctat tttgcgcatc taatcttgaa ccaccaacgc cagtttaaaa cacctctttg 180
ttaagagcgc cactttagcg gccatataaa acaccccgcg cacataaaaa tattgtgaaa 240
tttcgcctcc gtgtgttaaa aaacactagt ggtctcttat ggtgtgggga aaaaatccct 300
cagcgggcgc gtataactct gttagaacga ttacctgtta gccaactact tcctctttcc 360
cgtctctctc tctcttgtct attacgtcac cgatattaat attaa 405


<210> 364
<211> 395
<212> DNA
<213> Lycopersicon esculentum

<400> 364
tcgaaccgta tggggtaatc ctgcagatgt tgggaagatt ttagagggtg aagtatttga 60
tgctgttttg gacaacaatg gtaaagactt ggattctgta agtcctgtag ctgactgggc 120
caagagttct ggtgtcaagc aatttttgtt tatcagcagc gctggaatct acaagcccac 180
agatgaacct cctcatgttg aaggggatgc tgtgaaagct gatgctggtc atgtattagt 240
agagaaatac atatcagaga tatttggtag ttgggcaagt ttccgtcctc agtacatgat 300
tggctctggc aataacaagg attgtgagga atggttcttt gatcgtattg ttcgtggacg 360
accagttcta attcctggct ctggaatgca gttaa 395


<210> 365
<211> 141
<212> DNA
<213> Lycopersicon esculentum

<400> 365
tcgaagggct gacaagtaga cgagtgatga ggtctctttg ttgaagcctc gttctcacta 60
ttttcatcac ttttggcact gtttctttgt tccaaataac ttgcacaaca ccataggctt 120
gctcttattt tcaatagtta a 141

<210> 366
<211> 294
<212> DNA
<213> Lycopersicon esculentum

<400> 366
ttaaagatgg tactctttta gcaccaaagg tattcaatgt gcaattggag ggtgcattca 60
agagttttga tacagaatgt gagatgttgc gcaacctccg ccaccgaaat ctgaccaaag 120
tcatcaccag ttgctccaac cttgatttca aagccttagt gctggaatac atgtccaatg 180
ggacacttga taaatggcta tactctcaca acttgttctt gaatttattt caaagattag 240
atataatgat agatgttgca tctgcaatgg tctatctcca caatgggtgt tcga 294

<210> 367
<211> 168
<212> DNA
<213> Lycopersicon esculentum

<400> 367
tcgaaccgaa ctcctttacg accaggtggt ggggttgtgt ttgataccac caccccgtac 60
ttgggctcgg gtaagggtgg aagcagtacc gcgacgttgg ttgcgcaggt cagtggtaag 120
cccacgatga acgacggcgc gtgcggtatc aagaggggcg gattttaa 168

<210> 368
<211> 79
<212> DNA
<213> Lycopersicon esculentum

<400> 368
tcgaaccctt aaggaagagg attaaaatgt ggaatgacat atagttgttg atgggttttg 60
agccgtgtta tatggttaa 79

<210> 369
<211> 79
<212> DNA
<213> Lycopersicon esculentum

<400> 369
tcgaaccctt caagaagagg atgagaatct ggaagttcat atacttgatg ttcagttctg 60
agccggctta tatgcttaa 79

<210> 370
<211> 191
<212> DNA
<213> Lycopersicon esculentum

<400> 370
tcgaaaggaa agaaaatcaa gttcttcaag tgcagatgat ctaatcactc caggcatatt 60
caatggtgca caactatagt ccatgtgcaa cctggaaaac actcttttcc aaatgcggta 120
aaaccatcaa cagactttga agcaaaagcg gaggtcggaa catgaatgag cattgcatgt 180
gattccctta a 191

<210> 371
<211> 142
<212> DNA
<213> Lycopersicon esculentum

<400> 371
tcgaactagg agcagccata gcacctttgg cgtttctcaa ctttc[?][?]cacc ttgggtaaag 60
gaccatcatc ataatcttca tcactagaat cagaagaccc atttccatat gtttccttca 120
tacaccaatc cgagcaaatt aa 142

<210> 372
<211> 198
<212> DNA
<213> Lycopersicon esculentum

<400> 372
tcgaaaacag gttttactat ccgacccatt tcttttgctt gatcaggacc acgagtcatg 60
ttcacttgtt cgtttatagt atcctgacct ctgactaaag atggagaagt agatgccgcc 120
cactccctat aaaccggatt tattcgggca ttcatgtttg acttatttgt gcgacaaggc 180
gtcatttcgc ctaattaa 198

<210> 373
<211> 152
<212> DNA
<213> Lycopersicon esculentum

<400> 373
tcgaatggag tcagcaatag gctaccgcct catggaactg tgatacttgt acttggtggt 60
gtcaccgcgg gtgaatcagc ggcgctacaa cgaaaccccg atatctcgct aattgcagct 120
agcgacctta ccgatggcca tcgccttatt aa 152

<210> 374
<211> 293
<212> DNA
<213> Lycopersicon esculentum

<400> 374
tcgaattcac tactcacagg aacattggca tcactctctt ttgcctcgga acacttcagg 60
tatttgcttt gcttctgagg ccaaagccag accacaagta cagattatac tggaacatct 120
accaccatgc tgttggatac gcggtaataa gtctgagcat cgcgaatgtt tacaaaggat 180
ttgatgcatt gaatggacag aagaattgga agagggctta cactggtgtg actcatagcc 240
ataggtgcca ttgctgtttt attagaagct tttacttggt tcattgtcat taa 293

<210> 375
<211> 175
<212> DNA
<213> Lycopersicon esculentum

<400> 375
tcgaacaaca atggctaact tgttgcatgg attcaattgg aaattaggag gagatatgag 60
gccagaagat ataagcatgg acgagattta tggattgact acgcacccaa ataagcctgt 120
atctgtgatt atggaaccta gacttcccct ccatctttat tagaactgac tttaa 175

<210> 376
<211> 103
<212> DNA
<213> Lycopersicon esculentum

<400> 376
tcgaagatat cattcctggg ttgcagtagg caatgtttca tgcaaatcta tcaaaataga 60
gtctgaaaaa gtttttacca gaagtattta cagaagtgtt taa 103

<210> 377
<211> 131
<212> DNA
<213> Lycopersicon esculentum

<400> 377
ttaacagcaa agtaacctaa catggtaact tgatacaaaa tcaaggtagc tatcatgcgc 60
gtgtaaatat ggggccacat tcttccgtag ctccggaatc aggcactcaa agtgtatgtt 120
ccctcattcg a 131


<210> 378
<211> 295
<212> DNA
<213> Lycopersicon esculentum

<400> 378
tcgaaaacga tgctggatat tgagttgacc acattatgta tattacggac aaaccaataa 60
caagtccaca accgtaaccc atgagaaccc cctgccaact gatcattggt gaatcttctt 120
cttcttgttg ttgatctagc tccgctggag ttgtcacttg atcatcaccg ccacaatgtg 180
ttgagagtgg aaatccgcgt aacccatcat ttccttggtg cgaagtgttc cccaacgaat 240
caaattgttt tcctttgggg atgcatccaa ccacatgatt gtgagagaga tttaa 295


<210> 379
<211> 116
<212> DNA
<213> Lycopersicon esculentum

<400> 379
tcgaagtagt tccacagatt atctaattgt agatccgaac aggcttctca aaattgtgac 60
cggtctggtg ggcacaatat gatcttcttg gaggacaatc cgagtgctct atttaa 116


<210> 380
<211> 82
<212> DNA
<213> Lycopersicon esculentum

<400> 380
tcgaatcaat ggttccaact caacaaattc ttcttgtgtt agcccttgcg gaactaataa 60
ttgtctatca ccatcccatt aa 82


<210> 381
<211> 82
<212> DNA
<213> Lycopersicon esculentum

<400> 381
tcgaatcaat ggttccaact caacaaattc ttcttgtgtt agcccttgcg gaactaataa 60
ttgtctatca ccatccattt aa 82


<210> 382
<211> 123
<212> DNA
<213> Lycopersicon esculentum

<400> 382
tcgaacaaac caatgtgagc aagaagttca aagaataatt tatttgcaga acattgcaaa 60
tcaactacca gatgcattta ctaatcttcc aaggattact aaatcgcata ttccagctgt 120
taa 123

<210> 383
<211> 319
<212> DNA
<213> Lycopersicon esculentum

<400> 383
tcgaaccata tatcgtagta gaagtgtaag ttgtgaccat accggtgtcg tggatcaata 60
gcttctagcc aatgctgcag agcaagtttc tgagcttttt catcttttga caatccttta 120
ccaactttgg cagctcgtgt acgtgctcta gcccaccgcg atacagcagt ttccggtttc 180
tccacgttga agaacgaaac agaactccgt ttgagagctg cgaaatccaa agccttccac 240
catagttctt caaccacaac agcacaatct gcaagatttc ttcttgtcct ataactctta 300
tacactttct gaagcttaa 319

<210> 384
<211> 294
<212> DNA
<213> Lycopersicon esculentum

<400> 384
tcgaaacttc aagtggaaca ctaactcttc catttgcaca tggcatggcg taagttgcag 60
ctcagatggc actcgtgttg ttgcacttcg ccttcctgga cttgcacttt atggtcctat 120
tccagacaat accataggaa gactagatgc actaacaaca ctcagccttc attccaatgc 180
cctcactgga aatcttcctt cagacatcac ctctcttccc tccctccgtt tcatatttat 240
acaacaaaac aaattttctg gtgaaatacc ttcttctcta tctctactcc ttaa 294

<210> 385
<211> 139
<212> DNA
<213> Lycopersicon esculentum

<400> 385
tcgaatatgc caacacatta ctaaagcaag aaagagagag agaaatacat actttgagtt 60
ggaactcaaa atgggttttg gcctacataa agatgaagaa ggaacaggga agtctcacag 120
gcagcctttg gtgtcttaa 139

<210> 386
<211> 102
<212> DNA
<213> Lycopersicon esculentum

<400> 386
tcgaacaggc agacacaact cattgcatca gaacaaagag gatagacatc tatagcaacc 60
ataaagcttc aagcacacag tgataccaac taacaccctt aa 102

<210> 387
<211> 102
<212> DNA
<213> Lycopersicon esculentum

<400> 387
tcgaacaaac agacacaact cattgcatca gaacaaagag gatagacatc tatagcaacc 60

ataaagcttc aagcacacag tgataccaac taacaccctt aa 102

<210> 388
<211> 238
<212> DNA
<213> Lycopersicon esculentum

<400> 388
ttaacttggc tacactacac gaacacgcca tatttgtcca ccaccaagga tcttctggat 60
cttcactgct ttccaatccc aaaaccactc ccacatttga gaaacctccc acttttcttt 120
atggatccac ctacccaaat actagaaaac atcccaaacc ctcctaaaat tacaccaact 180
tctctcttaa ccccccaccc caccctctta tattcaccct taaaaaaaac tccttcga 238

<210> 389
<211> 211
<212> DNA
<213> Lycopersicon esculentum

<400> 389
tcgaatagca ctaccccaaa agaatacaca tcagattttt ctgtcaactg ttgtcgctta 60
tagtattcag gatctagata tccaaagctt cctttcacaa cagtgctaac atgagttttt 120
cctgatccac taagtggacc gattttggat aacccaaaat ctgaaacctt agcaacccat 180
ttatcatcca ataaaatgtt ggttgactta a 211

<210> 390
<211> 141
<212> DNA
<213> Lycopersicon esculentum

<400> 390
   tcgaatagct gggttgattt acgaagagac tcgtggtgtt ctgaagatct tttggaaaaa 60
   tgtaattcgt gattccgtga catacacaga gcacgctagg agaaagacgg ttactgctat 120
   ggatgttgtt tatgcgctta a 141

<210> 391
<211> 80
<212> DNA
<213> Lycopersicon esculentum

<400> 391
tcgaacgtag tttgtatttg acagcttcac ttgagtaaaa aagaacttgt cttcagtaaa 60
taaggaggca gataaattaa 80

<210> 392
<211> 168
<212> DNA
<213> Lycopersicon esculentum

<400> 392
tcgaagatgt tggaaaacca gtttcagagt actttggtgt tagcggtgat gccccaagag 60
ttctcgcata tacaggaaat gaggatggga ggaagtttat actggaaggt gaaataacct 120
tggatggtgt caagtcattt ggggagaagt ttttggaaga caatttaa 168

<210> 393
<211> 82

<212> DNA
<213> Lycopersicon esculentum

<400> 393
tcgaacgagc aattgaaaag attctggatc atcttcggga taaggatgat gcacacatct 60
tctgtttaga agatcaaatt aa 82


<210> 394
<211> 160
<212> DNA
<213> Lycopersicon esculentum

<400> 394
tcgaaagcag caaaacaaat tagtcgttac aagttcccgg cgcgtgtacg aaatcgctcc 60
ttgtgagatc gtctttcgtt caaatcacac aaattagctg ttacaagttc ctagctggca 120
gccatttgaa ttccattcat ccaaattccc cccaaattaa 160


<210> 395
<211> 167
<212> DNA
<213> Lycopersicon esculentum

<400> 395
tcgaaagaca agttctttgt tcatttttcc catctcattt actgatagag aatgtgatgg 60
tggaaaagcc ctgacatcat acagtaaata gaaggattag taatgtaaga cgccaagacc 120
aaactccctt tacttgtacc ttctttttca gttttttccg ttattaa 167


<210> 396
<211> 40
<212> DNA
<213> Lycopersicon esculentum

<400> 396
tcgaccacat tatactttgc cctgatcttt tacattttaa 40


<210> 397
<211> 107
<212> DNA
<213> Lycopersicon esculentum

<400> 397
tcgaattcag cgggttgggg aaatgcctgc tgcttgtgtt actgttattt gttgttacat 60
cgtcccaaca tagtagctag tcttagttgc tcgttttctt gtattaa 107


<210> 398
<211> 127
<212> DNA
<213> Lycopersicon esculentum

<400> 398
tcgaagaggt gaattattgg acgaacaaaa ttggattcat caatttcatt tgccgaggat 60
ctccttcatc catccctatc aaatgagggg cagttgttga cacccatttt gaccctccac 120
aatttaa 127


<210> 399

<211> 125
<212> DNA
<213> Lycopersicon esculentum

<400> 399
ttaacgagct ctctaccgag tataacacct gcacatacat gttctgcata cttgattttc 60
ctctctattt caggtccagt tcccagtcca agaatatcca aagattccaa agttgatttc 120
ttcga 125


<210> 400
<211> 96
<212> DNA
<213> Lycopersicon esculentum

<400> 400
tcgaaggtaa ctatcttcac tacgaagggc ttgaaacaca acagaattat gataatcaac 60
catatcagca cttgattgtg taaatacttc tattaa 96


<210> 401
<211> 95
<212> DNA
<213> Lycopersicon esculentum

<400> 401
tcgaaggttg ctggaaactg ccatataaac accataagct acactggttg atacaacagg 60
caaattctcc acttcttcag cagcagaact attaa 95


<210> 402
<211> 60
<212> DNA
<213> Lycopersicon esculentum

<400> 402
tcgaatcaca atggtaatca tggtaacctg agttgagagt ggtagttgtt ttctctacga 60


<210> 403
<211> 155
<212> DNA
<213> Lycopersicon esculentum

<400> 403
ttaattggtc gtactgcaca agcctcgcaa acattttatt acataatttg ggagaacact 60
gctctatggc tccacaataa tctttccggt ggcatgtcca tcaatactct tgctccaagc 120
atcttcagcc ttgctcagag gaaattttga gtcga 155


<210> 404
<211> 90
<212> DNA
<213> Lycopersicon esculentum

<400> 404
ttaagatgca ccccctccgc gtcttttctc ttctagaatt gcagcctcat tcaacatgtc 60
agctgcatct ttgtgcatgt ctagcttcga 90


<210> 405

<211> 83
<212> DNA
<213> Lycopersicon esculentum

<400> 405
ttaatcgggc atgtctggtt taggattgga ttcggatgtg taggtctcag gaaaattatc 60
caatcctaag ggtatgggtt cga 83


<210> 406
<211> 125
<212> DNA
<213> Lycopersicon esculentum

<400> 406
ttaacgagct ctctaccgag tataacacct gcacatacat gttctgcata cttgattttc 60
ctctctattt caggtccagt tcccagtcca agaatatcca aagattccaa agttgatttc 120
ttcga 125


<210> 407
<211> 102
<212> DNA
<213> Lycopersicon esculentum

<400> 407
tcgaacagac agacacaact cattgcatca gaacaaagag gatagacatc tatagcaacc 60
ataaagcttc aagcacacag tgataccaac taacaccctt aa 102


<210> 408
<211> 319
<212> DNA
<213> Lycopersicon esculentum

<400> 408
tcgaaccata tatcctanta gaagtgtaag ttgtgaccat accggtgtcg tggatcaata 60
gcttctagcc aatgctgcag agcaagtttc tgagcttttt catcttttga caatccttta 120
ccaactttgg cagctcgtgt acgtgctcta gcccaccgcg atacagcagt ttccggtttc 180
tccacgttga agaacgaaac agaactccgt ttgagagctg cgaaatccaa agccttccac 240
catagttctt caaccacaac agcacaatct gcaagatttc ttcttgtcct ataactctta 300
tacactttct gaagcttaa 319


<210> 409
<211> 292
<212> DNA
<213> Lycopersicon esculentum

<400> 409
tcgaattcac tactcacagg aacattggca tcactctctt ttgcctcgga acacttcagg 60
tatttgcttt gcttctgagg ccaaagccag accacaagta cagattatac tggaacatct 120
accaccatgc tgttggatac gcggtaataa gtctgagcat cgcgaatgtt tacaaaggat 180
ttgatgcatt gaatggacag aagaattgga agagggctta cactggtgtg atcatagcca 240
taggtgccat tgctgtttta ttagaagctt ttacttggtt cattgtcatt aa 292


<210> 410
<211> 455
<212> DNA
<213> Lycopersicon esculentum

<400> 410
ttaagggcac cccccctgga catacttctg gaattccgct tattcacccc gcggtattca 60
ctgggggcaa agaggtgcaa gataccacca agcgcaaact tgcttataaa gcccgcaaca 120
cgcgccgcac accctcacac tgagggggcc ctcttggcgc taaagagcgt ggcaccacat 180
tccactggac cggggtacaa caacgacccg agggggaaaa ccctggcggg cgatactatg 240
tgggtccaga gaaaaacacc caacctcngn nnnaaatcaa gcagtccata ggtcgtctcc 300
cggtgcgcaa aatgtgttac ttcccgccca ggtcgcaatc caacctgatc agtcaggagc 360
gcaccatcat actcagccta tgacgcatcc tgtcctgcga ctacagtcag ataccctcta 420
cgtcaagccg gacgaatctc acggatagtc ctcga 455

<210> 411
<211> 211
<212> DNA
<213> Lycopersicon esculentum

<400> 411
tcgaaatagt ttgtactttg agaaagatgg tataatcttc aggattcatt gttccggtga 60
ttgtcatgtt tgtaccatct agtactgaat catcaggctg aaaacgtata atttcattct 120
gtttttcgtt tggcatatac ttttgacatg acaaatttat ggctcgaggt aaatcttcat 180
gttcatcaac agcaaggaat ggatccctta a 211

<210> 412
<211> 146
<212> DNA
<213> Lycopersicon esculentum

<400> 412
ttaaaagatg gaacagagat ccgaaaaggg cattgaaatt tcagatcata cagaaaatta 60
taagactgaa ttggagaaac cgattcggaa ttcgtatatc caaacgctgt aaatacaaaa 120
cctaatacca atatgtatct aatcga 146

<210> 413
<211> 127
<212> DNA
<213> Lycopersicon esculentum

<400> 413
tcgattgaag actagtgcta gaaaaagtta ttgacgttgc tgtgatggaa tcagaaagat 60
acaaacctta tttgttctgt ttgaagtgaa atgtgcacnt atnnncctct ccctaaaatg 120
tttttaa 127

<210> 414
<211> 180
<212> DNA
<213> Lycopersicon esculentum

<400> 414
ttaaactgaa cataaacatt gacaatatca aacaaaatta ttctcttagc aactgtgcct 60
atgaatgctt ccacactgat attgacaata tgaagtttgg atctcctttc aaactccacc 120
tactttgtat cctaacccaa aagctggtac tgtcactaca aatatacctc aggctatcga 180

<210> 415
<211> 179
<212> DNA
<213> Lycopersicon esculentum

<400> 415
ttaaacttga gaagaaaagg actaactgat gagggagttc aaaagattgt gaaagatcca 60
gcaccatatg gacctggtcc atggggtgga gaaggtggaa aaccatggga tgatggagtt 120
ttcactggga tcaagcagat aatcctaaca caaacatcag aggccatttc ttgtatcga 179

<210> 416
<211> 170
<212> DNA
<213> Lycopersicon esculentum

<400> 416
ttaaaaacct aagtgatgaa attcccttct tctttttttt tcagttacaa caggctgacg 60
gccatcaaca aggaaatact gggctggagc ttccaactgt gcagccccca ccacctatgc 120
aggtgggggc tagtccgggc tcaatcagac caggttctat ggtggatcga 170

<210> 417
<211> 142
<212> DNA
<213> Lycopersicon esculentum

<400> 417
tcgatggcag aagacagaat tctctttatc aacaatcaaa catttcttct aatataacag 60
gattacatgt tgcttccaga tataaaagaa atattgaagc cctattgata ttcatgtact 120
gatcttcagt agtgtacatt aa 142

<210> 418
<211> 105
<212> DNA
<213> Lycopersicon esculentum

<400> 418
tcgatcaatg agtgattgtg caaaccttgt tagagcttgc tcataggttt caggattgtc 60
tttggaaact ggtttcttca ataaattttt accaacttgt tttaa 105

<210> 419
<211> 234
<212> DNA
<213> Lycopersicon esculentum

<400> 419
ttaatgaaat cgtgggagta agactgatat tcttgccgaa ttcatctgtg ttcaccattc 60
ccggcgggat agtctgccag tacagttcgt tgttctttca attggtgata cgtacacttt 120
tcccggcagg aacatacggc gtgacatatt cttcaaatgg tgtatacccg ccctgatgct 180
ccatcacttc ctgattattg agccatactt tggcgtaatg agtgaccgcg tcga 234

<210> 420
<211> 170
<212> DNA
<213> Lycopersicon esculentum

<400> 420
tcgatgcaca catcaatgga tgtaacgtga agaaacaagt acaaagatta tcaaaggatg 60
aagaagtagt agttactact tatgaaggta tgcattcaca tccaattgac aaatctacgg 120
ataactttga gcacattttg agtcaaatgc aaatctataa ttccttttaa 170